(19) **Europäisches Patentamt**
**European Patent Office**
**Office européen des brevets**

(11) **EP 3 810 774 B1**

(12) **EUROPEAN PATENT SPECIFICATION**

(45) Date of publication and mention of the grant of the patent:
**13.09.2023 Bulletin 2023/37**

(51) International Patent Classification (IPC):
**C12N 15/10** (2006.01)   **C12Q 1/6806** (2018.01)
**C12Q 1/6816** (2018.01)   **C12Q 1/6844** (2018.01)
**C12Q 1/6813** (2018.01)   **C12Q 1/6869** (2018.01)

(21) Application number: **19814056.8**

(22) Date of filing: **04.06.2019**

(52) Cooperative Patent Classification (CPC):
**C12N 15/1093**

(86) International application number:
**PCT/US2019/035422**

(87) International publication number:
**WO 2019/236599 (12.12.2019 Gazette 2019/50)**

(54) **METHODS OF MAKING HIGH-THROUGHPUT SINGLE-CELL TRANSCRIPTOME LIBRARIES**

VERFAHREN ZUR HERSTELLUNG VON EINZELZELLTRANSKRIPTOMBIBLIOTHEKEN MIT HOHEM DURCHSATZ

PROCÉDÉS DE PRODUCTION DES BIBLIOTHÈQUES DE TRANSCRIPTOMES À UNE SEULE CELLULE À HAUT DÉBIT ET LEURS

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **04.06.2018 US 201862680259 P**
**21.03.2019 US 201962821678 P**

(43) Date of publication of application:
**28.04.2021 Bulletin 2021/17**

(60) Divisional application:
**23187488.4**

(73) Proprietors:
 • **Illumina, Inc.**
   **San Diego, CA 92122 (US)**
 • **University of Washington**
   **Seattle, WA 98105 (US)**

(72) Inventors:
 • **STEEMERS, Frank J.**
   **San Diego, CA 92122 (US)**
 • **SHENDURE, Jay**
   **Seattle, WA 98105 (US)**
 • **CAO, Junyue**
   **Seattle, WA 98105 (US)**
 • **TOME, Jacob**
   **Seattle, WA 98105 (US)**
 • **GASPERINI, Molly**
   **Seattle, WA 98105 (US)**

(74) Representative: **Murgitroyd & Company**
   **Murgitroyd House**
   **165-169 Scotland Street**
   **Glasgow G5 8PL (GB)**

(56) References cited:
   **WO-A1-2015/200609     WO-A1-2018/125982**
   **WO-A2-2016/130704     US-A1- 2003 082 584**
   **US-A1- 2018 023 119**

 • **RAPOLAS ZILIONIS ET AL: "Single-cell barcoding and sequencing using droplet microfluidics", NATURE PROTOCOLS, vol. 12, no. 1, 8 December 2016 (2016-12-08), pages 44-73, XP055532179, GB ISSN: 1754-2189, DOI: 10.1038/nprot.2016.154**
 • **ALLON M. KLEIN ET AL: "Droplet Barcoding for Single-Cell Transcriptomics Applied to Embryonic Stem Cells", CELL, vol. 161, no. 5, 1 May 2015 (2015-05-01), pages 1187-1201, XP055731640, Amsterdam NL ISSN: 0092-8674, DOI: 10.1016/j.cell.2015.04.044**

• Patrone G. ET AL: "Nuclear Run-On Assay Using Biotin Labeling, Magnetic Bead Capture and Analysis by Fluorescence-Based RT-PCR", BioTechniques, 1 November 2000 (2000-11-01), pages 1012-1017, XP055913071, DOI: 10.2144/00295st02 Retrieved from the Internet: URL:https://www.future-science.com/doi/epd f/10.2144/00295st02 [retrieved on 2022-04-14]

**Description**

FIELD

Embodiments of the present disclosure relate to sequencing nucleic acids. In particular, embodiments of the methods and compositions provided herein relate to producing indexed single-cell transcriptome libraries and obtaining sequence data therefrom.

BACKGROUND

Cells transit across functionally and molecularly distinct states during various processes, such as during development of a multicellular organism and in response to different conditions such as exposure to a therapeutic agent. Characterizing the cell state transition path, or cell fate, is useful in understanding pathways including development and the molecular response of cells to changing environments. For instance, regulators of developmental defects can be identified and a better understanding of how therapeutic agents affect cells can achieved.

Single cell combinatorial indexing ('sci-') is a methodological framework that employs split-pool barcoding to uniquely label the nucleic acid contents of large numbers of single cells or nuclei. Current single cell genomic techniques, however, lack the throughput and resolution to obtain a global view of the molecular states and trajectories of a rapidly diversifying and expanding number of cell types typically present during development of a multicellular organism. Current single cell genomic techniques only capture a snapshot of a cell's state, thus cannot provide information on cell transition dynamics regulated by intrinsic (e.g., a cell's intrinsic cell cycle program) and extrinsic (e.g., a cell's response to an external stimulus such as a therapeutic agent) factors.

US 2003/082584 A1 and Rapolas Zillionis et al.: "Single-cell barcoding and sequencing using droplet microfluidics", NATURE PROTOCOLS, 2016, 12(1):44-73 disclose methods of preparing sequencing libraries in droplets comprising single cells involving a droplet specific indexing of the RNA.

SUMMARY

The invention is defined by the appendant claims.

Provided herein are methods to identify cell state transition dynamics by labeling newly synthesized RNA. Both whole and newly synthesized RNA transcriptomes are captured, allowing characterization of transcriptome dynamics between time points at the single cell level. Also provided herein are methods that focus single-cell sequencing on mRNAs of interest, thereby addressing the limited power of current to detect changes in the abundance of any given transcript. Further provided are methods that overcome the rate of cell loss and limited reaction efficiencies to result in the profiling of greater numbers of single cells then previously possible.

In one embodiment, a method includes providing a plurality of nuclei or cells in a first plurality of compartments, where each compartment comprises a subset of nuclei or cells, and labeling newly synthesized RNA in the subsets of cells or nuclei obtained from the cells. RNA molecules in each subset of nuclei or cells are processed to generate indexed nuclei or cells, where the processing includes adding to RNA nucleic acids present in each subset of nuclei or cells a first compartment specific index sequence to result in indexed DNA nucleic acids present in indexed nuclei or cells, and then combining the indexed nuclei or cells to generate pooled indexed nuclei or cells.

In another embodiment, a method includes providing a plurality of nuclei or cells in a first plurality of compartments, where each compartment comprises a subset of nuclei or cells. Each subset is contacted with reverse transcriptase and a primer that anneals to a predetermined RNA nucleic acid, resulting in double stranded DNA nucleic acids with the primer and the corresponding DNA nucleotide sequence of the template RNA nucleic acids. The DNA molecules in each subset of nuclei or cells are processed to generate indexed nuclei or cells, where the processing includes adding to DNA nucleic acids present in each subset of nuclei or cells a first compartment specific index sequence to result in indexed nucleic acids present in indexed nuclei or cells, and then combining the indexed nuclei or cells to generate pooled indexed nuclei or cells.

In another embodiment, a method includes providing a plurality of nuclei or cells in a first plurality of compartments, where each compartment comprises a subset of nuclei or cells. Each subset is contacted with reverse transcriptase and a primer that anneals to a predetermined RNA nucleic acid, resulting in double stranded DNA nucleic acids with the primer and the corresponding DNA nucleotide sequence of the template RNA nucleic acids. The DNA molecules in each subset of nuclei or cells are processed to generate indexed nuclei or cells, where the processing includes adding to DNA nucleic acids present in each subset of nuclei or cells a first compartment specific index sequence to result in indexed nucleic acids present in indexed nuclei or cells, and then combining the indexed nuclei or cells to generate pooled indexed nuclei or cells. The pooled indexed nuclei or cells are split and then further processed to add a second compartment specific index to the DNA molecules, combined, split, and further processed to add a third compartment specific index to the DNA molecules.

Definitions

Terms used herein will be understood to take on their ordinary meaning in the relevant art unless specified otherwise. Several terms used herein and their meanings are set forth below.

As used herein, the terms "organism," "subject," are used interchangeably and refer to microbes (e.g., prokaryotic or eukaryotic) animals and plants. An example of an animal is a mammal, such as a human.

As used herein, the term "cell type" is intended to identify cells based on morphology, phenotype, developmental origin or other known or recognizable distinguishing cellular characteristic. A variety of different cell types can be obtained from a single organism (or from the same species of organism). Exemplary cell types include, but are not limited to, gametes (including female gametes, e.g., ova or egg cells, and male gametes, e.g., sperm), ovary epithelial, ovary fibroblast, testicular, urinary bladder, immune cells, B cells, T cells, natural killer cells, dendritic cells, cancer cells, eukaryotic cells, stem cells, blood cells, muscle cells, fat cells, skin cells, nerve cells, bone cells, pancreatic cells, endothelial cells, pancreatic epithelial, pancreatic alpha, pancreatic beta, pancreatic endothelial, bone marrow lymphoblast, bone marrow B lymphoblast, bone marrow macrophage, bone marrow erythroblast, bone marrow dendritic, bone marrow adipocyte, bone marrow osteocyte, bone marrow chondrocyte, promyeloblast, bone marrow megakaryoblast, bladder, brain B lymphocyte, brain glial, neuron, brain astrocyte, neuroectoderm, brain macrophage, brain microglia, brain epithelial, cortical neuron, brain fibroblast, breast epithelial, colon epithelial, colon B lymphocyte, mammary epithelial, mammary myoepithelial, mammary fibroblast, colon enterocyte, cervix epithelial, breast duct epithelial, tongue epithelial, tonsil dendritic, tonsil B lymphocyte, peripheral blood lymphoblast, peripheral blood T lymphoblast, peripheral blood cutaneous T lymphocyte, peripheral blood natural killer, peripheral blood B lymphoblast, peripheral blood monocyte, peripheral blood myeloblast, peripheral blood monoblast, peripheral blood promyeloblast, peripheral blood macrophage, peripheral blood basophil, liver endothelial, liver mast, liver epithelial, liver B lymphocyte, spleen endothelial, spleen epithelial, spleen B lymphocyte, liver hepatocyte, liver, fibroblast, lung epithelial, bronchus epithelial, lung fibroblast, lung B lymphocyte, lung Schwann, lung squamous, lung macrophage, lung osteoblast, neuroendocrine, lung alveolar, stomach epithelial, and stomach fibroblast.

As used herein, the term "tissue" is intended to mean a collection or aggregation of cells that act together to perform one or more specific functions in an organism. The cells can optionally be morphologically similar. Exemplary tissues include, but are not limited to, embryonic, epididymidis, eye, muscle, skin, tendon, vein, artery, blood, heart, spleen, lymph node, bone, bone marrow, lung, bronchi, trachea, gut, small intestine, large intestine, colon, rectum, salivary gland, tongue, gall bladder, appendix, liver, pancreas, brain, stomach, skin, kidney, ureter, bladder, urethra, gonad, testicle, ovary, uterus, fallopian tube, thymus, pituitary, thyroid, adrenal, or parathyroid. Tissue can be derived from any of a variety of organs of a human or other organism. A tissue can be a healthy tissue or an unhealthy tissue. Examples of unhealthy tissues include, but are not limited to, malignancies in reproductive tissue, lung, breast, colorectum, prostate, nasopharynx, stomach, testes, skin, nervous system, bone, ovary, liver, hematologic tissues, pancreas, uterus, kidney, lymphoid tissues, etc. The malignancies may be of a variety of histological subtypes, for example, carcinoma, adenocarcinoma, sarcoma, fibroadenocarcinoma, neuroendocrine, or undifferentiated.

As used herein, the term "compartment" is intended to mean an area or volume that separates or isolates something from other things. Exemplary compartments include, but are not limited to, vials, tubes, wells, droplets, boluses, beads, vessels, surface features, or areas or volumes separated by physical forces such as fluid flow, magnetism, electrical current or the like. In one embodiment, a compartment is a well of a multi-well plate, such as a 96- or 384-well plate. As used herein, a droplet may include a hydrogel bead, which is a bead for encapsulating one or more nuclei or cell, and includes a hydrogel composition. In some embodiments, the droplet is a homogeneous droplet of hydrogel material or is a hollow droplet having a polymer hydrogel shell. Whether homogenous or hollow, a droplet may be capable of encapsulating one or more nuclei or cells. In some embodiments, the droplet is a surfactant stabilized droplet.

As used herein, a "transposome complex" refers to an integration enzyme and a nucleic acid including an integration recognition site. A "transposome complex" is a functional complex formed by a transposase and a transposase recognition site that is capable of catalyzing a transposition reaction (see, for instance, Gunderson et al., WO 2016/130704). Examples of integration enzymes include, but are not limited to, an integrase or a transposase. Examples of integration recognition sites include, but are not limited to, a transposase recognition site.

As used herein, the term "nucleic acid" is intended to be consistent with its use in the art and includes naturally occurring nucleic acids or functional analogs thereof. Particularly useful functional analogs are capable of hybridizing to a nucleic acid in a sequence specific fashion or capable of being used as a template for replication of a particular nucleotide sequence. Naturally occurring nucleic acids generally have a backbone containing phosphodiester bonds. An analog structure can have an alternate backbone linkage including any of a variety of those known in the art. Naturally occurring nucleic acids generally have a deoxyribose sugar (e.g. found in deoxyribonucleic acid (DNA)) or a ribose sugar (e.g. found in ribonucleic acid (RNA)). A nucleic acid can contain any of a variety of analogs of these sugar moieties that are known in the art. A nucleic acid can include native or non-native bases. In this regard, a native deoxyribonucleic acid can have one or more bases selected from the group consisting of adenine, thymine, cytosine or guanine and a ribonucleic acid can have one or more bases selected from the group consisting of adenine, uracil, cytosine or guanine. Useful non-native bases that can be included in a nucleic acid are known in the art. Examples of non-native bases include a locked nucleic acid (LNA), a bridged nucleic acid (BNA), and pseudocomplementary bases (Trilink Biotechnologies, San Diego, CA). LNA and BNA bases can be incorporated into a DNA oligonucleotide and increase oligonucleotide hybridization strength and specificity. LNA and BNA bases and the uses of such bases are known to the person skilled in the art and are routine. Unless indicated otherwise, the term "nucleic acid" includes natural and non-natural mRNA, non-coding RNA, e.g., RNA without poly-A at 3' end, nucleic acids derived from a RNA, e.g., cDNA, and DNA.

As used herein, the term "target," when used in reference to a nucleic acid, is intended as a semantic identifier for the nucleic acid in the context of a method or composition set forth herein and does not necessarily limit the structure or function of the nucleic acid beyond what is otherwise explicitly indicated. A target nucleic acid may be essentially any nucleic acid of known or unknown sequence. It may be, for example, a fragment of genomic DNA (e.g., chromosomal DNA), extra-chromosomal DNA such as a plasmid, cell-free DNA, RNA (e.g., RNA or non-coding RNA), proteins (e.g. cellular or cell surface proteins), or cDNA. Sequencing may result in determination of the sequence of the whole, or a part of the target molecule. The targets can be derived from a primary nucleic acid sample, such as a nucleus. In one embodiment, the targets can be processed into templates suitable for amplification by the placement of universal sequences at one or both ends of each target fragment. The targets can also be obtained from a primary RNA sample by reverse transcription into cDNA. In one embodiment, target is used in reference to a subset of DNA, RNA, or proteins present in the cell. Targeted sequencing uses selection and isolation of genes or regions or proteins of interest, typically by either PCR amplification (e.g., region-specific primers) or hybridization-based capture method or antibodies. Targeted enrichment can occur at various stages of the method. For instance, a targeted RNA representation can be obtained using target specific primers in the reverse transcription step or hybridization-based enrichment of a subset out of a more complex library. An example is exome sequencing or the L1000 assay (Subramanian et al., 2017, Cell, 171;1437-1452). Targeted sequencing can include any of the enrichment processes known to one of ordinary skill in the art.

As used herein, the term "universal," when used to describe a nucleotide sequence, refers to a region of sequence that is common to two or more nucleic acid molecules where the molecules also have regions of sequence that differ from each other. A universal sequence that is present in different members of a collection of molecules can allow capture of multiple different nucleic acids using a population of universal capture nucleic acids, e.g., capture oligonucleotides that are complementary to a portion of the universal sequence, e.g., a universal capture sequence. Non-limiting examples of universal capture sequences include sequences that are identical to or complementary to P5 and P7 primers. Similarly, a universal sequence present in different members of a collection of molecules can allow the replication (e.g., sequencing) or amplification of multiple different nucleic acids using a population of universal primers that are complementary to a portion of the universal sequence, e.g., a universal anchor sequence. In one embodiment universal anchor sequences are used as a site to which a universal primer (e.g., a sequencing primer for read 1 or read 2) anneals for sequencing. A capture oligonucleotide or a universal primer therefore includes a sequence that can hybridize specifically to a universal sequence.

The terms "P5" and "P7" may be used when referring to a universal capture sequence or a capture oligonucleotide. The terms "P5' " (P5 prime) and "P7' " (P7 prime) refer to the complement of P5 and P7, respectively. It will be understood that any suitable universal capture sequence or a capture oligonucleotide can be used in the methods presented herein, and that the use of P5 and P7 are exemplary embodiments only. Uses of capture oligonucleotides such as P5 and P7 or their complements on flowcells are known in the art, as exemplified by the disclosures of WO 2007/010251, WO 2006/064199, WO 2005/065814, WO 2015/106941, WO 1998/044151, and WO 2000/018957. For example, any suitable forward amplification primer, whether immobilized or in solution, can be useful in the methods presented herein for hybridization to a complementary sequence and amplification of a sequence. Similarly, any suitable reverse amplification primer, whether immobilized or in solution, can be useful in the methods presented herein for hybridization to a complementary sequence and amplification of a sequence. One of skill in the art will understand how to design and use primer sequences that are suitable for capture and/or amplification of nucleic acids as presented herein.

As used herein, the term "primer" and its derivatives refer generally to any nucleic acid that can hybridize to a target sequence of interest. Typically, the primer functions as a substrate onto which nucleotides can be polymerized by a polymerase or to which a nucleotide sequence such as an index can be ligated; in some embodiments, however, the primer can become incorporated into the synthesized nucleic acid strand and provide a site to which another primer can hybridize to prime synthesis of a new strand that is complementary to the synthesized nucleic acid molecule. The primer can include any combination of nucleotides or analogs thereof. In some embodiments, the primer is a single-stranded oligonucleotide or polynucleotide. The terms "polynucleotide" and "oligonucleotide" are used interchangeably herein to refer to a polymeric form of nucleotides of any length, and may include ribonucleotides, deoxyribonucleotides, analogs thereof, or mixtures thereof. The terms should be understood to include, as equivalents, analogs of either DNA, RNA, cDNA or antibody-oligo conjugates made from nucleotide analogs and to be applicable to single stranded (such as sense or antisense) and double stranded polynucleotides. The term as used herein also encompasses cDNA, that is complementary or copy DNA produced from a RNA template, for example by the action of reverse transcriptase. This term refers only to the primary structure of the molecule. Thus, the term includes triple-, double- and single-stranded deoxyribonucleic acid ("DNA"), as well as triple-, double- and single-stranded ribonucleic acid ("RNA").

As used herein, the term "adapter" and its derivatives, e.g., universal adapter, refers generally to any linear oligonucleotide which can be attached to a nucleic acid molecule of the disclosure. In some embodiments, the adapter is substantially non-complementary to the 3' end or the 5' end of any target sequence present in the sample. In some embodiments, suitable adapter lengths are in the range of about 10-100 nucleotides, about 12-60 nucleotides, or about 15-50 nucleotides in length. Generally, the adapter can include any combination of nucleotides and/or nucleic acids. In some aspects, the adapter can include one or more cleavable groups at one or more locations. In another aspect, the adapter can include a sequence that is substantially identical, or substantially complementary, to at least a portion of a primer, for example a universal primer. In some embodiments, the adapter can include a barcode (also referred to herein as a tag or index) to assist with downstream error correction, identification, or sequencing. The terms "adaptor" and "adapter" are used interchangeably.

As used herein, the term "each," when used in reference to a collection of items, is intended to identify an individual item in the collection but does not necessarily refer to every item in the collection unless the context clearly dictates otherwise.

As used herein, the term "transport" refers to movement of a molecule through a fluid. The term can include passive transport such as movement of molecules along their concentration gradient (e.g. passive diffusion). The term can also include active transport whereby molecules can move along their concentration gradient or against their concentration gradient. Thus, transport can include applying energy to move one or more molecule in a desired direction or to a desired location such as an amplification site.

As used herein, "amplify", "amplifying" or "amplification reaction" and their derivatives, refer generally to any action or process whereby at least a portion of a nucleic acid molecule is replicated or copied into at least one additional nucleic acid molecule. The additional nucleic acid molecule optionally includes sequence that is substantially identical or substantially complementary to at least some portion of the template nucleic acid molecule. The template nucleic acid molecule can be single-stranded or double-stranded and the additional nucleic acid molecule can independently be single-stranded or double-stranded. Amplification optionally includes linear or exponential replication of a nucleic acid molecule. In some embodiments, such amplification can be performed using isothermal conditions; in other embodiments, such amplification can include thermocycling. In some embodiments, the amplification is a multiplex amplification that includes the simultaneous amplification of a plurality of target sequences in a single amplification reaction. In some embodiments, "amplification" includes amplification of at least some portion of DNA and RNA based nucleic acids alone, or in combination. The amplification reaction can include any of the amplification processes known to one of ordinary skill in the art. In some embodiments, the amplification reaction includes polymerase chain reaction (PCR).

As used herein, "amplification conditions" and its derivatives, generally refers to conditions suitable for amplifying one or more nucleic acid sequences. Such amplification can be linear or exponential. In some embodiments, the amplification conditions can include isothermal conditions or alternatively can include thermocycling conditions, or a combination of isothermal and thermocycling conditions. In some embodiments, the conditions suitable for amplifying one or more nucleic acid sequences include polymerase chain reaction (PCR) conditions. Typically, the amplification conditions refer to a reaction mixture that is sufficient to amplify nucleic acids such as one or more target sequences flanked by a universal sequence, or to amplify an amplified target sequence ligated to one or more adapters. Generally, the amplification conditions include a catalyst for amplification or for nucleic acid synthesis, for example a polymerase; a primer that possesses some degree of complementarity to the nucleic acid to be amplified; and nucleotides, such as deoxyribonucleotide triphosphates (dNTPs) to promote extension of the primer once hybridized to the nucleic acid. The amplification conditions can require hybridization or annealing of a primer to a nucleic acid, extension of the primer and a denaturing step in which the extended primer is separated from the nucleic acid sequence undergoing amplification. Typically, but not necessarily, amplification conditions can include thermocycling; in some embodiments, amplification conditions include a plurality of cycles where the steps of annealing, extending and separating are repeated. Typically, the amplification conditions include cations such as $Mg^{2+}$ or $Mn^{2+}$ and can also include various modifiers of ionic strength.

As used herein, "re-amplification" and their derivatives refer generally to any process whereby at least a portion of an amplified nucleic acid molecule is further amplified via any suitable amplification process (referred to in some embodiments as a "secondary" amplification), thereby producing a reamplified nucleic acid molecule. The secondary amplification need not be identical to the original amplification process whereby the amplified nucleic acid molecule was produced; nor need the reamplified nucleic acid molecule be completely identical or completely complementary to the amplified nucleic acid molecule; all that is required is that the reamplified nucleic acid molecule include at least a portion of the amplified nucleic acid molecule or its complement. For example, the re-amplification can involve the use of different amplification conditions and/or different primers, including different target-specific primers than the primary amplification.

As used herein, the term "polymerase chain reaction" ("PCR") refers to the method of Mullis U.S. Pat. Nos. 4,683,195 and 4,683,202, which describe a method for increasing the concentration of a segment of a polynucleotide of interest in a mixture of genomic DNA without cloning or purification. This process for amplifying the polynucleotide of interest consists of introducing a large excess of two oligonucleotide primers to the DNA mixture containing the desired polynucleotide of interest, followed by a series of thermal cycling in the presence of a DNA polymerase. The two primers are complementary to their respective strands of the double stranded polynucleotide of interest. The mixture is denatured at a higher temperature first and the primers are then annealed to complementary sequences within the polynucleotide of interest molecule. Following annealing, the primers are extended with a polymerase to form a new pair of complementary strands. The steps of denaturation, primer annealing and polymerase extension can be repeated many times (referred to as thermocycling) to obtain a high concentration of an amplified segment of the desired polynucleotide of interest. The length of the amplified segment of the desired polynucleotide of interest (amplicon) is determined by the relative positions of the primers with respect to each other, and therefore, this length is a controllable parameter. By virtue of repeating the process, the method is referred to as PCR. Because the desired amplified segments of the polynucleotide of interest become the predominant nucleic acid sequences (in terms of concentration) in the mixture, they are said to be "PCR amplified". In a modification to the method discussed above, the target nucleic acid molecules can be PCR amplified using a plurality of different primer pairs, in some cases, one or more primer pairs per target nucleic acid molecule of interest, thereby forming a multiplex PCR reaction.

As defined herein "multiplex amplification" refers to selective and non-random amplification of two or more target sequences within a sample using at least one target-specific primer. In some embodiments, multiplex amplification is performed such that some or all of the target sequences are amplified within a single reaction vessel. The "plexy" or "plex" of a given multiplex amplification refers generally to the number of different target-specific sequences that are amplified during that single multiplex amplification. In some embodiments, the plexy can be about 12-plex, 24-plex, 48-plex, 96-plex, 192-plex, 384-plex, 768-plex, 1536-plex, 3072-plex, 6144-plex or higher. It is also possible to detect the amplified target sequences by several different methodologies (e.g., gel electrophoresis followed by densitometry, quantitation with a bioanalyzer or quantitative PCR, hybridization with a labeled probe; incorporation of biotinylated primers followed by avidin-enzyme conjugate detection; incorporation of $^{32}$P-labeled deoxynucleotide triphosphates into the amplified target sequence).

As used herein, "amplified target sequences" and its derivatives, refers generally to a nucleic acid sequence produced by the amplifying the target sequences using target-specific primers and the methods provided herein. The amplified target sequences may be either of the same sense (i.e. the positive strand) or antisense (i.e., the negative strand) with respect to the target sequences.

As used herein, the terms "ligating", "ligation" and their derivatives refer generally to the process for covalently linking two or more molecules together, for example covalently linking two or more nucleic acid molecules to each other. In some embodiments, ligation includes joining nicks between adjacent nucleotides of nucleic acids. In some embodiments, ligation includes forming a covalent bond between an end of a first and an end of a second nucleic acid molecule. In some embodiments, the ligation can include forming a covalent bond between a 5' phosphate group of one nucleic acid and a 3' hydroxyl group of a second nucleic acid thereby forming a ligated nucleic acid molecule. Generally, for the purposes of this disclosure, an amplified target sequence can be ligated to an adapter to generate an adapter-ligated amplified target sequence.

As used herein, "ligase" and its derivatives, refers generally to any agent capable of catalyzing the ligation of two substrate molecules. In some embodiments, the ligase includes an enzyme capable of catalyzing the joining of nicks between adjacent nucleotides of a nucleic acid. In some embodiments, the ligase includes an enzyme capable of catalyzing the formation of a covalent bond between a 5' phosphate of one nucleic acid molecule to a 3' hydroxyl of another nucleic acid molecule thereby forming a ligated nucleic acid molecule. Suitable ligases may include, but are not limited to, T4 DNA ligase, T4 RNA ligase, and E. coli DNA ligase.

As used herein, "ligation conditions" and its derivatives, generally refers to conditions suitable for ligating two molecules to each other. In some embodiments, the ligation conditions are suitable for sealing nicks or gaps between nucleic acids. As used herein, the term nick or gap is consistent with the use of the term in the art. Typically, a nick or gap can be ligated in the presence of an enzyme, such as ligase at an appropriate temperature and pH. In some embodiments, T4 DNA ligase can join a nick between nucleic acids at a temperature of about 70-72° C.

The term "flowcell" as used herein refers to a chamber comprising a solid surface across which one or more fluid reagents can be flowed. Examples of flowcells and related fluidic systems and detection platforms that can be readily used in the methods of the present disclosure are described, for example, in Bentley et al., Nature 456:53-59 (2008), WO 04/018497; US 7,057,026; WO 91/06678; WO 07/123744; US 7,329,492; US 7,211,414; US 7,315,019; US 7,405,281, and US 2008/0108082.

As used herein, the term "amplicon," when used in reference to a nucleic acid, means the product of copying the nucleic acid, wherein the product has a nucleotide sequence that is the same as or complementary to at least a portion of the nucleotide sequence of the nucleic acid. An amplicon can be produced by any of a variety of amplification methods that use the nucleic acid, or an amplicon thereof, as a template including, for example, polymerase extension, polymerase chain reaction (PCR), rolling circle amplification (RCA), ligation extension, or ligation chain reaction. An amplicon can be a nucleic acid molecule having a single copy of a particular nucleotide sequence (e.g. a PCR product) or multiple copies of the nucleotide sequence (e.g. a concatameric product of RCA). A first amplicon of a target nucleic acid is typically a complementary copy. Subsequent amplicons are copies that are created, after generation of the first amplicon, from the target nucleic acid or from the first amplicon. A subsequent amplicon can have a sequence that is substantially complementary to the target nucleic acid or substantially identical to the target nucleic acid.

As used herein, the term "amplification site" refers to a site in or on an array where one or more amplicons can be generated. An amplification site can be further configured to contain, hold or attach at least one amplicon that is generated at the site.

As used herein, the term "array" refers to a population of sites that can be differentiated from each other according to relative location. Different molecules that are at different sites of an array can be differentiated from each other according to the locations of the sites in the array. An individual site of an array can include one or more molecules of a particular type. For example, a site can include a single target nucleic acid molecule having a particular sequence or a site can include several nucleic acid molecules having the same sequence (and/or complementary sequence, thereof). The sites of an array can be different features located on the same substrate. Exemplary features include without limitation, wells in a substrate, beads (or other particles) in or on a substrate, projections from a substrate, ridges on a substrate or channels in a substrate. The sites of an array can be separate substrates each bearing a different molecule. Different molecules attached to separate substrates can be identified according to the locations of the substrates on a surface to which the substrates are associated or according to the locations of the substrates in a liquid or gel. Exemplary arrays in which separate substrates are located on a surface include, without limitation, those having beads in wells.

As used herein, the term "capacity," when used in reference to a site and nucleic acid material, means the maximum amount of nucleic acid material that can occupy the site. For example, the term can refer to the total number of nucleic acid molecules that can occupy the site in a particular condition. Other measures can be used as well including, for example, the total mass of nucleic acid material or the total number of copies of a particular nucleotide sequence that can occupy the site in a particular condition. Typically, the capacity of a site for a target nucleic acid will be substantially equivalent to the capacity of the site for amplicons of the target nucleic acid.

As used herein, the term "capture agent" refers to a material, chemical, molecule or moiety thereof that is capable of attaching, retaining or binding to a target molecule (e.g. a target nucleic acid). Exemplary capture agents include, without limitation, a capture nucleic acid (also referred to herein as a capture oligonucleotide) that is complementary to at least a portion of a target nucleic acid, a member of a receptor-ligand binding pair (e.g. avidin, streptavidin, biotin, lectin, carbohydrate, nucleic acid binding protein, epitope, antibody, etc.) capable of binding to a target nucleic acid (or linking moiety attached thereto), or a chemical reagent capable of forming a covalent bond with a target nucleic acid (or linking moiety attached thereto).

As used herein, the term "reporter moiety" can refer to any identifiable tag, label, indices, barcodes, or group that enables to determine the composition, identity, and/or the source of an analyte that is investigated. In some embodiments, a reporter moiety may include an antibody that specifically binds to a protein. In some embodiments, the antibody may include a detectable label. In some embodiments, the reporter can include an antibody or affinity reagent labeled with a nucleic acid tag. The nucleic acid tag can be detectable, for example, via a proximity ligation assay (PLA) or proximity extension assay (PEA) or sequencing based readout (Shahi et al. Scientific Reports volume 7, Article number: 44447, 2017) or CITE-seq (Stoeckius et al. Nature Methods 14:865-868, 2017).

As used herein, the term "clonal population" refers to a population of nucleic acids that is homogeneous with respect to a particular nucleotide sequence. The homogenous sequence is typically at least 10 nucleotides long, but can be even longer including for example, at least 50, 100, 250, 500 or 1000 nucleotides long. A clonal population can be derived from a single target nucleic acid or template nucleic acid. Typically, all of the nucleic acids in a clonal population will have the same nucleotide sequence. It will be understood that a small number of mutations (e.g. due to amplification artifacts) can occur in a clonal population without departing from clonality.

As used herein, the term "unique molecular identifier" or "UMI" refers to a molecular tag, either random, non-random, or semi-random, that may be attached to a nucleic acid. When incorporated into a nucleic acid, a UMI can be used to correct for subsequent amplification bias by directly counting unique molecular identifiers (UMIs) that are sequenced after amplification.

As used herein, an "exogenous" compound, e.g., an exogenous enzyme, refers to a compound that is not normally or naturally found in particular composition. For instance, when the particular composition includes a cell lysate, an exogenous enzyme is an enzyme that is not normally or naturally found in the cell lysate.

As used herein, "providing" in the context of a composition, an article, a nucleic acid, or a nucleus means making the composition, article, nucleic acid, or nucleus, purchasing the composition, article, nucleic acid, or nucleus, or otherwise obtaining the compound, composition, article, or nucleus.

The term "and/or" means one or all of the listed elements or a combination of any two or more of the listed elements.

The words "preferred" and "preferably" refer to embodiments of the disclosure that may afford certain benefits, under certain circumstances. However, other embodiments may also be preferred, under the same or other circumstances. Furthermore, the recitation of one or more preferred embodiments does not imply that other embodiments are not useful, and is not intended to exclude other embodiments from the scope of the disclosure.

The terms "comprises" and variations thereof do not have a limiting meaning where these terms appear in the description and claims.

It is understood that wherever embodiments are described herein with the language "include," "includes," or "including," and the like, otherwise analogous embodiments described in terms of "consisting of" and/or "consisting essentially of" are also provided.

Unless otherwise specified, "a," "an," "the," and "at least one" are used interchangeably and mean one or more than one.

Also herein, the recitations of numerical ranges by endpoints include all numbers subsumed within that range (e.g., 1 to 5 includes 1, 1.5, 2, 2.75, 3, 3.80, 4, 5, etc.).

For any method disclosed herein that includes discrete steps, the steps may be conducted in any feasible order. And, as appropriate, any combination of two or more steps may be conducted simultaneously.

Reference throughout this specification to "one embodiment," "an embodiment," "certain embodiments," or "some embodiments," etc., means that a particular feature, configuration, composition, or characteristic described in connection with the embodiment is included in at least one embodiment of the disclosure. Thus, the appearances of such phrases in various places throughout this specification are not necessarily referring to the same embodiment of the disclosure. Furthermore, the particular features, configurations, compositions, or characteristics may be combined in any suitable manner in one or more embodiments.

## BRIEF DESCRIPTION OF THE FIGURES

The following detailed description of illustrative embodiments of the present disclosure may be best understood when read in conjunction with the following drawings.

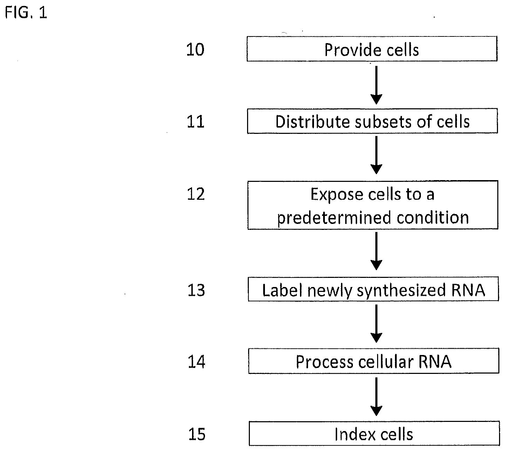

FIG. 1 shows a general block diagram of a general illustrative method for single-cell combinatorial indexing according to the present disclosure.

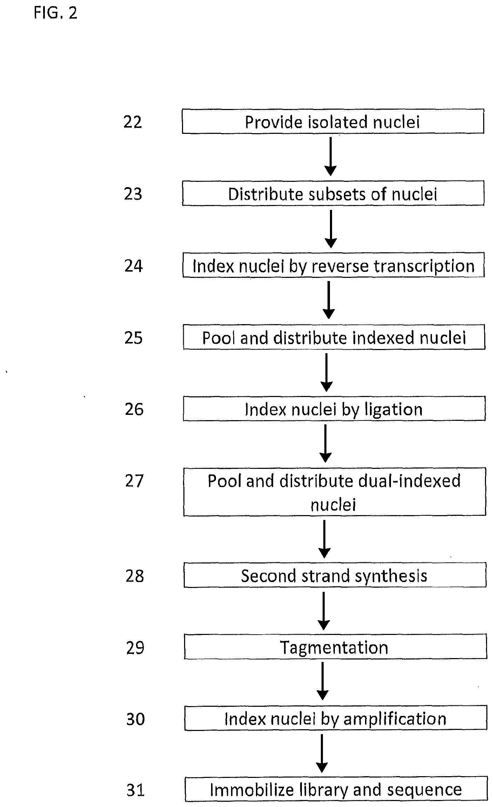

FIG. 2 shows a general block diagram of a general illustrative method for single-cell combinatorial indexing according to the present disclosure.

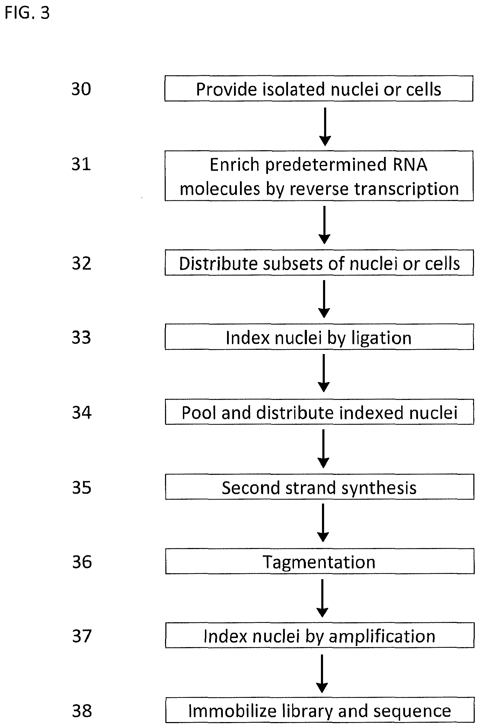

FIG. 3 shows a general block diagram of a general illustrative method for single-cell combinatorial indexing according to the present disclosure.

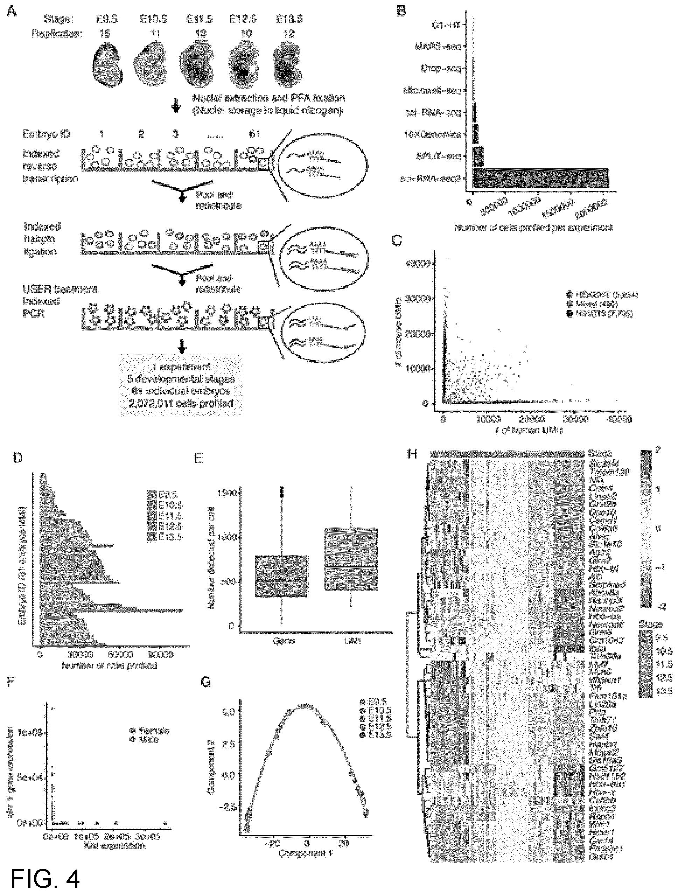

Fig. 4 shows sci-RNA-seq3 enables profiling of ~2 million cells from 61 mouse embryos across 5 developmental stages in one experiment. (A) sci-RNA-seq3 workflow and experimental scheme. (B) Comparison of per-experiment throughput here vs. recent reports. (C) Scatter plot of mouse vs. human UMI count from HEK293T and NIH/3T3 cells. (D) Bar plot showing number of cells profiled from each of 61 mouse embryos. (E) Box plot showing the number of genes and UMIs detected per cell. (F) Scatter plot of unique reads aligning to Xist (chr X) vs. chrY genes per mouse embryo. (G) Pseudotime trajectory of pseudobulk RNA-seq profiles of mouse embryos. (H) Heatmap of changes in marker gene expression E9.5 to E13.5 from pseudobulk RNA-seq profiles of mouse embryos.

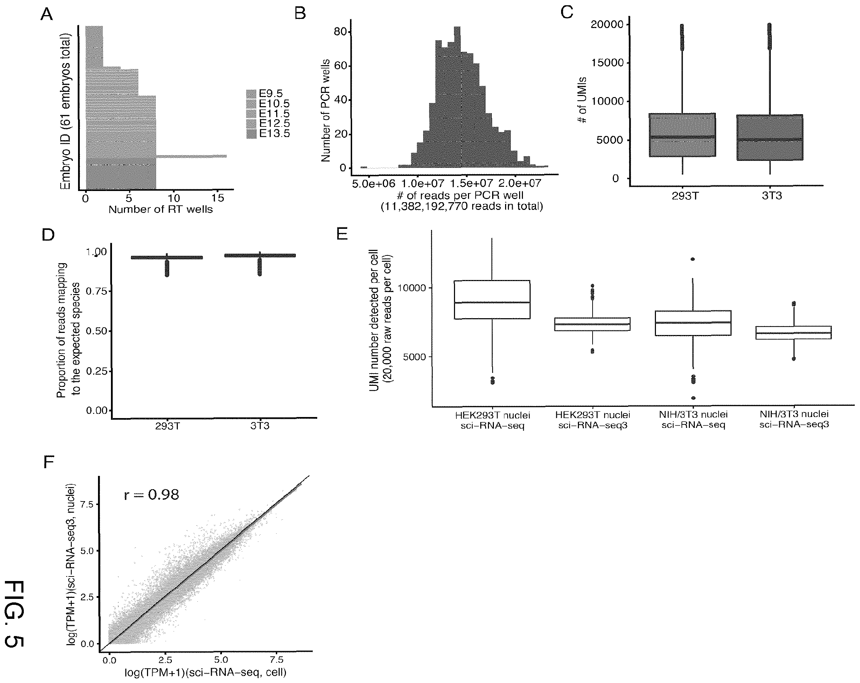

Fig. 5 shows performance and QC-related analyses for sci-RNA-seq3. (A) Bar plot showing the number of RT wells used for each of 61 mouse embryos. (B) Histogram showing the distribution of raw sequencing reads from each PCR well in sci-RNA-seq3. (C) Box plot showing the number of UMIs per cell from HEK293T and NIH/3T3 cells. (D) Box plot showing the proportion of reads mapping to the expected species for HEK293T (human) and NIH/3T3 (mouse) cells. (E) Box plot comparing the number of UMIs per cell (downsampled to 20,000 raw reads per cell) for sci-RNA-seq3 vs. sci-RNA-seq. (F) Correlation between gene expression measurements in aggregated profiles of HEK293T cells from sci-RNA-seq3 vs. sci-RNA-seq.

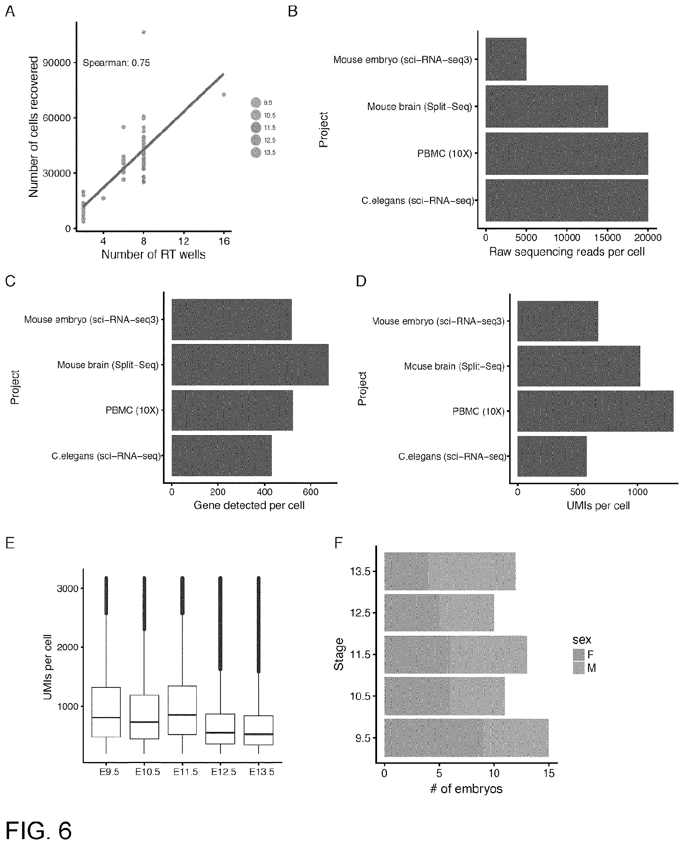

Fig. 6 shows additional performance and QC-related analyses for sci-RNA-seq3. (A) Scatter plot showing correlation between number of RT wells used and number of cells recovered per embryo. (B-D) Bar plot comparing the number of raw sequencing reads (B), detected genes (C) and UMIs (D) per cell between sci-RNA-seq3 and other methods. (E) Box plot showing the number of UMIs detected per cell from embryos across five developmental stages. (F) Bar plot showing the number of male and female embryos profiled at each developmental stage.

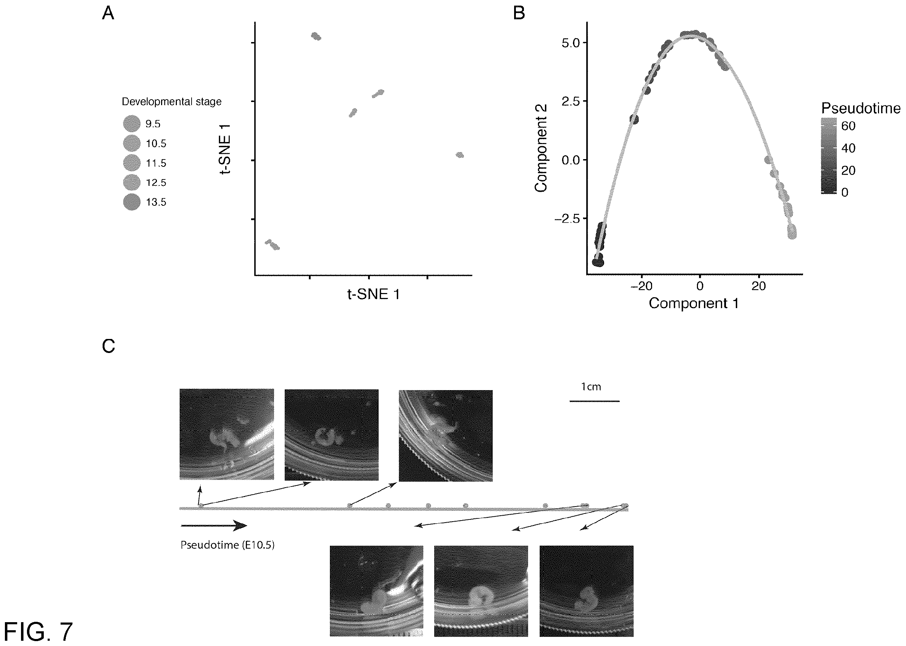

Fig. 7 shows pseudobulk RNA-seq profiles of mouse embryos are readily separated by developmental stage. (A) t-SNE of the aggregated transcriptomes of single cells derived from each of 61 mouse embryos results in five tightly clustered groups perfectly matching their developmental stages. (B) Pseudotime trajectory of pseudobulk RNA-seq profiles of mouse embryos, identical to Fig. 4G, but colored by pseudotime. (C) The 61 profiled embryos were ordered by pseudotime. The three earliest and three latest (in pseudotime) E10.5 embryos are shown in photos, and appear to be morphologically distinct.

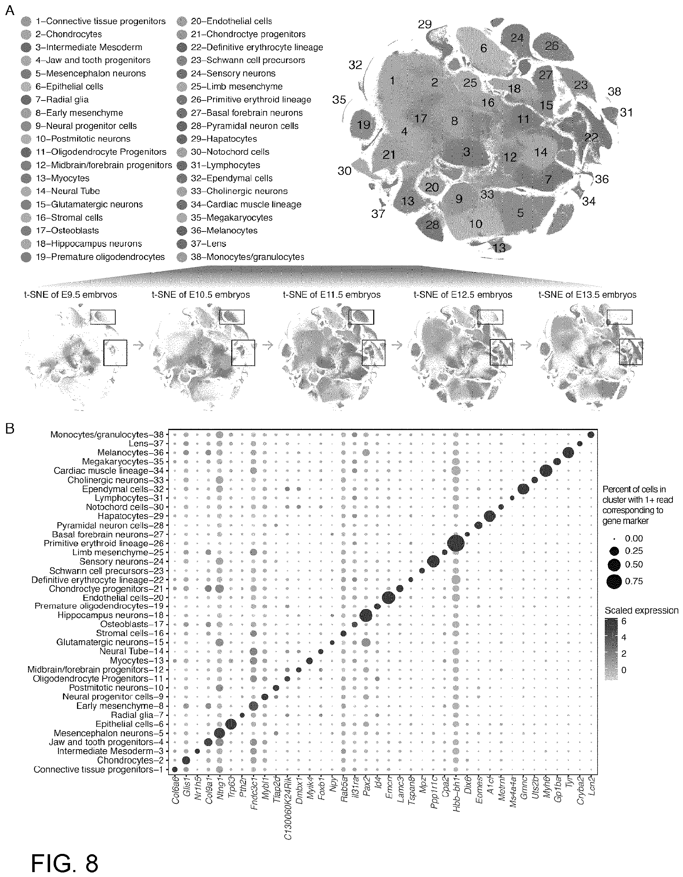

Fig. 8 shows identifying the major cell types of mouse organogenesis. (A) t-SNE visualization of 2,026,641 mouse embryo cells, colored by cluster id from Louvain clustering, and annotated based on marker genes. The same t-SNE is plotted below, showing only cells from each developmental stage. Primitive erythroid (transient) and definitive erythroid (expanding) clusters are boxed to illustrate their proliferative dynamics (B) Dot plot showing expression of one selected marker gene per cell type. The size of the dot encodes the percentage of cells within a cell type, and its color encodes the average expression level.

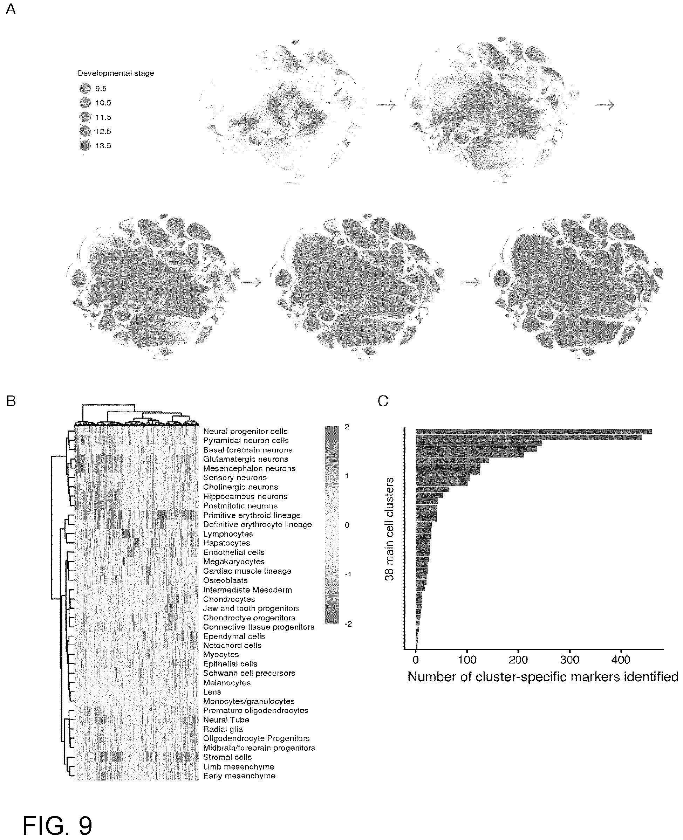

Fig. 9 shows identifying the major cell types of mouse organogenesis and the corresponding sets of cell type-specific markers genes. (A) t-SNE visualization of cell state transition from E9.5 to E13.5, colored by development stages. This is the same t-SNE as shown in Fig. 8A, but in each plot only cells derived from a single timepoint are shown. (B) Heatmap showing the relative expression of genes across the major cell types identified. (C) Bar plot showing the number of marker genes in each cell type with more than two-fold higher expression when compared with the second highest expressing cell type (FDR of 5%).

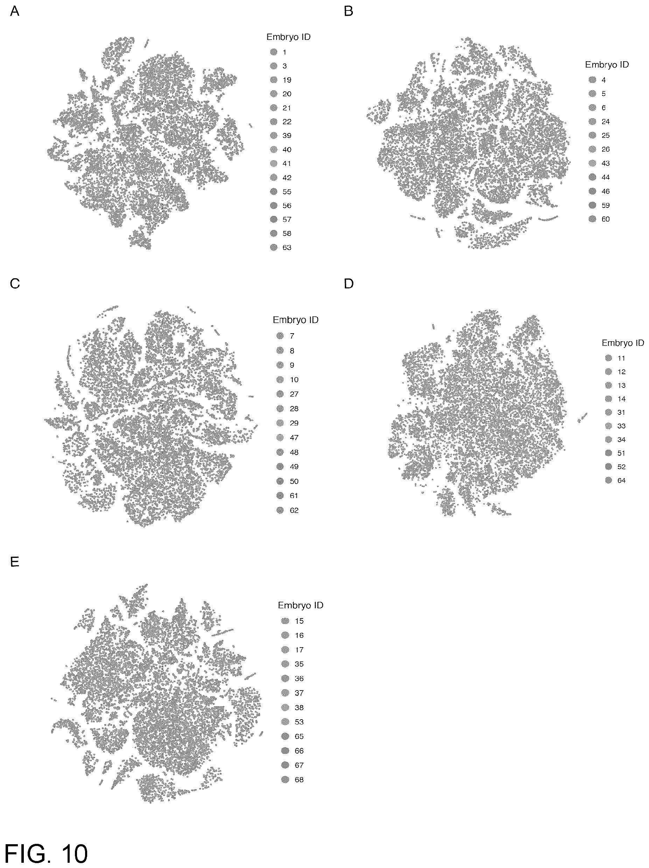

Fig. 10 shows cells derived from replicate embryos of the same timepoint do not exhibit obvious batch effects. (A-E) t-SNE visualization of mouse embryo cells from different developmental stages: E9.5 (A), E10.5 (B), E11.5 (C), E12.5 (D), E13.5 (E), colored by embryo ID in each stage.

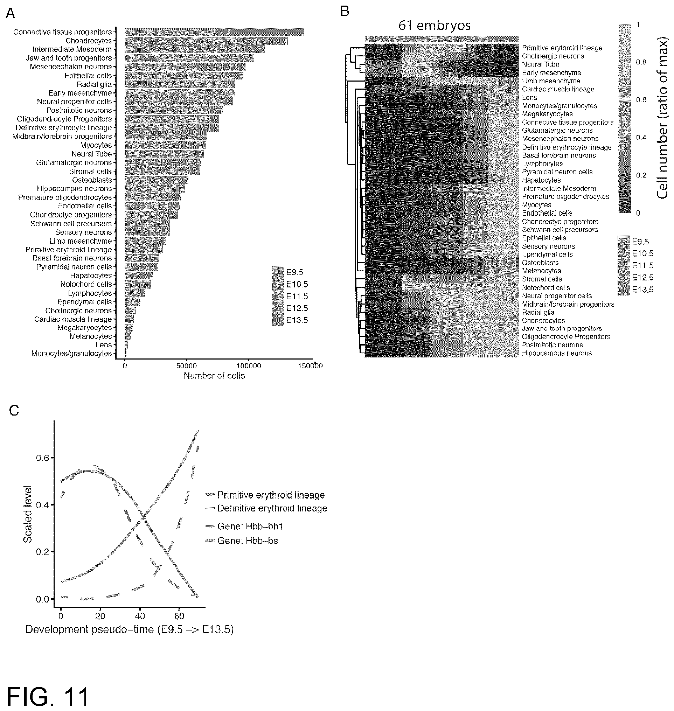

Fig. 11 shows the dynamics of cell type numbers during mouse organogenesis. (A) Bar plot showing the number of cells profiled for each cell type, split out by development stage. (B) Heatmap showing the relative cell number of each cell type (rows) in 61 mouse embryos (columns). An estimate of the absolute cell number per cell type per embryo was calculated by multiplying the proportion that cell type contributed to a given embryo by the estimated total number of cells at that development stage. For presentation, these estimates are normalized in each row by the maximum estimated cell number for a given cell type across all 61 embryos. Embryos are sorted left-to-right by developmental pseudotime. (C) Line plot showing the relative cell number change for primitive erythroid and definitive erythroid lineages, calculated as in panel B. Dashed lines show relative expression of marker genes for primitive erythroid (Hbb-bh1) and definitive erythroid (Hbb-bs). Data points for individual embryos were ordered by development pseudotime and smoothed by loess method.

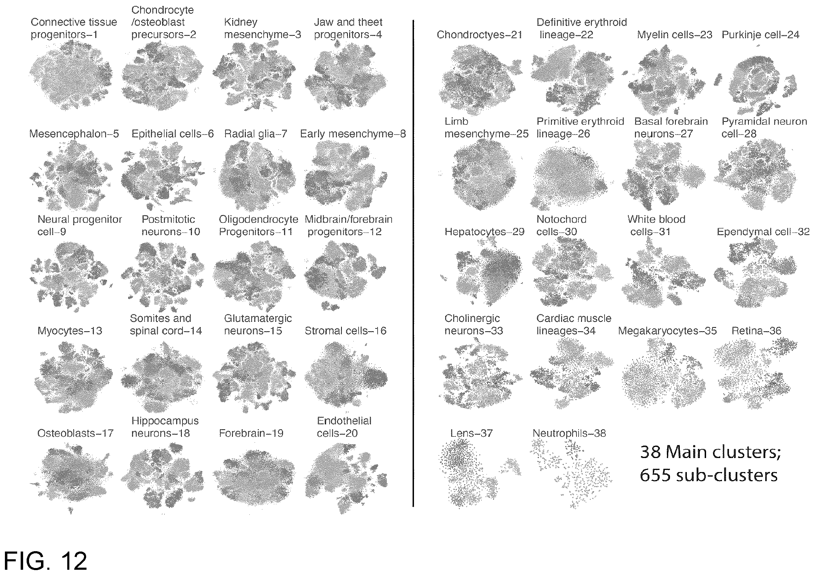

Fig. 12 shows louvain clustering and t-SNE visualization of subclusters of the each of 38 main cell types. As cell type heterogeneity was readily apparent within many of the 38 clusters shown in Fig. 8A, we adopted an iterative strategy, repeating Louvain clustering on each main cell type to identify subclusters. After subclusters dominated by one or two embryos were removed and highly similar subclusters merged, a total of 655 subclusters (also termed 'subtypes' to distinguish them from the 38 main cell types identified by the initial clustering).

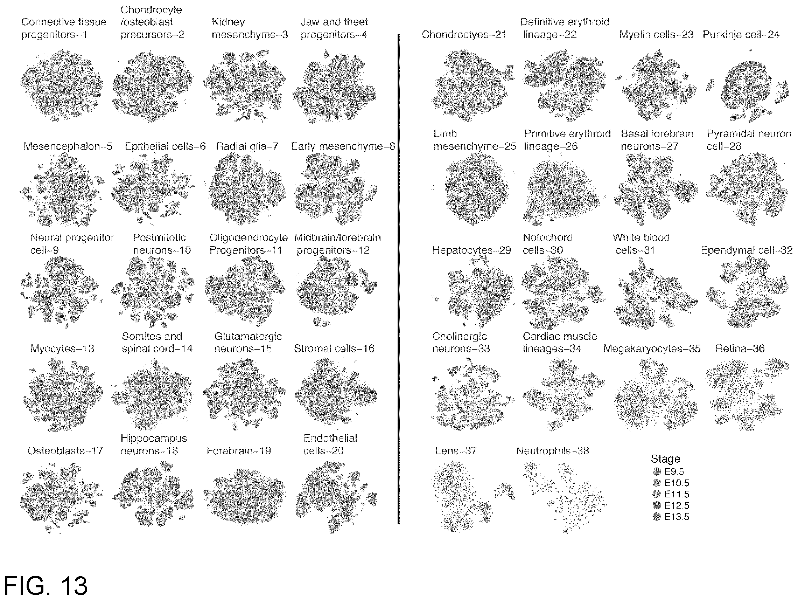

Fig. 13 shows louvain clustering and t-SNE visualization of subclusters of the each of 38 main cell types across developmental stages. Identical to Fig. 12, but with cells colored by developmental stage rather than subcluster ID.

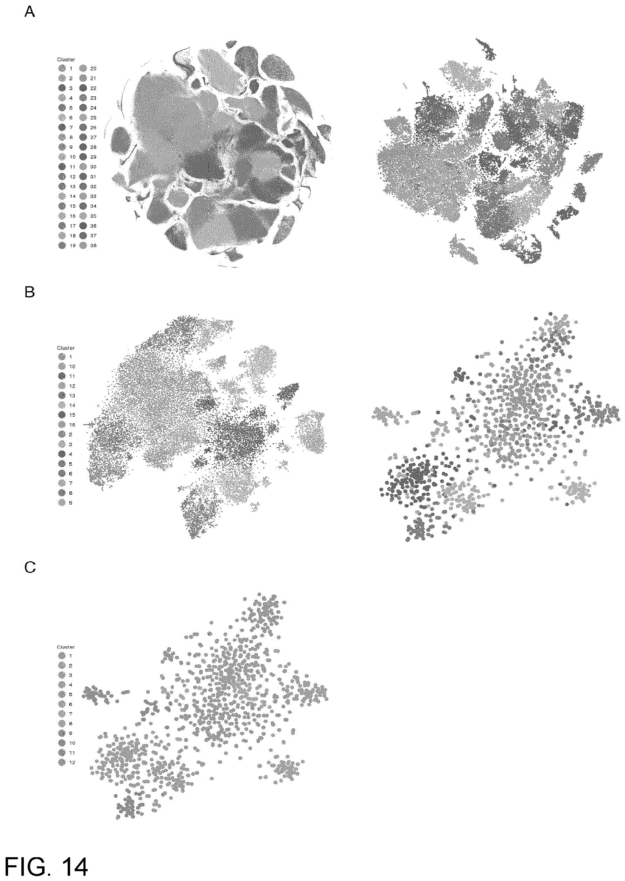

Fig. 14 shows sensitivity to detect cell types is a function of cellular coverage. (A) t-SNE visualization of all cells (left plot, n = 2,026,641) and downsampled subset (right plot, n = 50,000), colored by Louvain cluster IDs from Fig. 8A. (B) t-SNE visualization of all endothelial cells (left plot, n = 35,878) and those from the downsampled subset (right plot, n = 1,173), colored by Louvain cluster ID computed based on the 35,878 endothelial cells. (C) t-SNE visualization of the 1,173 endothelial cells, colored by Louvain cluster ID computed based on the 1,173 endothelial cells.

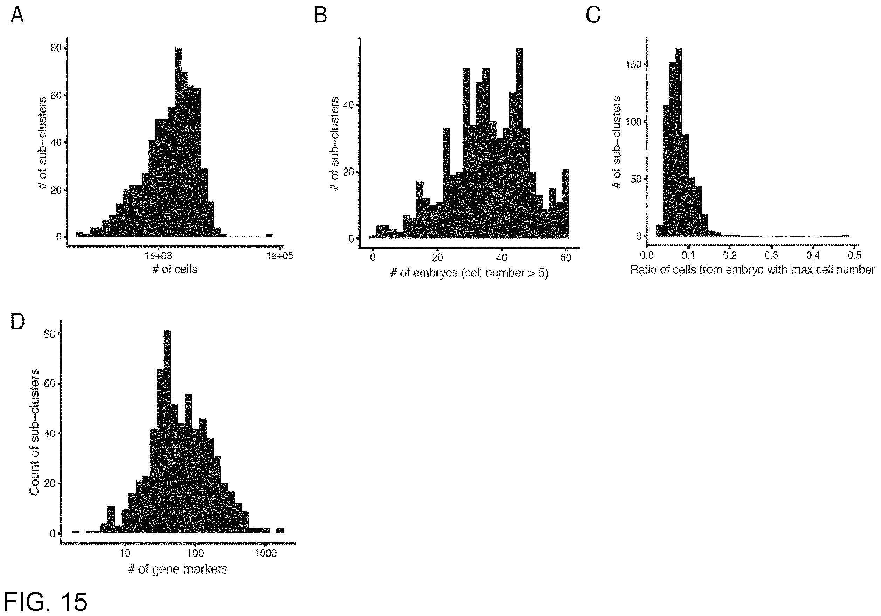

Fig. 15 shows the 655 cell subtypes derive from multiple embryos and are defined by sets of markers. (A) Histogram showing the distribution of subclusters with respect to cell number (median 1,869; range 51-65,894). (B) Histogram showing the distribution of subclusters with respect to the number of contributing embryos (>5 cells to qualify as a contributor). (C) Histogram showing the distribution of subclusters with respect to the ratio of cells derived from the most highly contributing embryo. (D) Histogram showing the distribution of subclusters with respect to the number of marker genes (at least 1.5-fold higher expression when compared with the second highest expressing cell subtype within the same main cluster; 5% FDR).

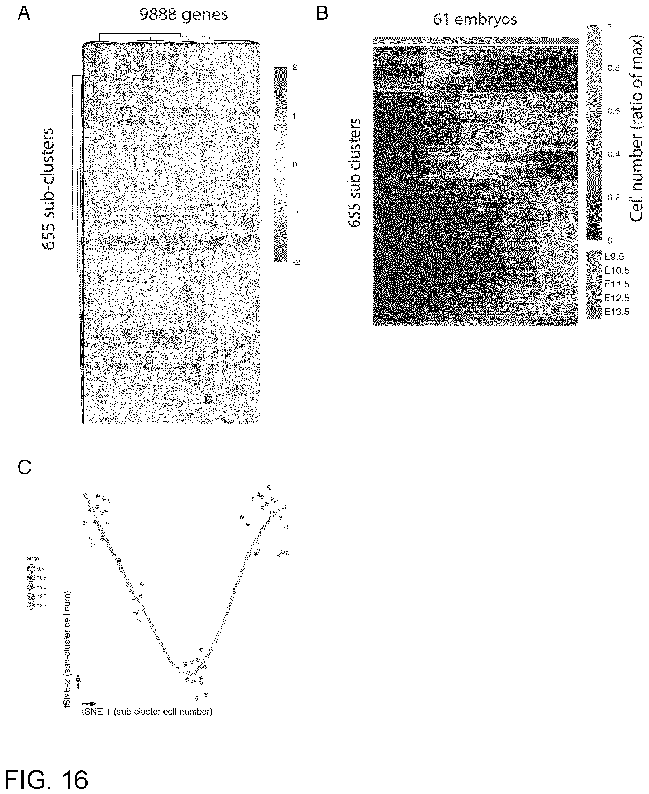

Fig. 16 shows the dynamics of cell subtype numbers during mouse organogenesis. (A) Heatmap showing the relative expression of genes across the 655 identified subclusters. (B) Heatmap showing the relative cell number of each cell subtype (rows) in 61 mouse embryos (columns). An estimate of the absolute cell number per cell subtype per embryo was calculated as in Fig. 11B. (C) t-SNE visualization of all 61 mouse embryos based solely on the proportions of the 655 cell subtypes in each embryo.

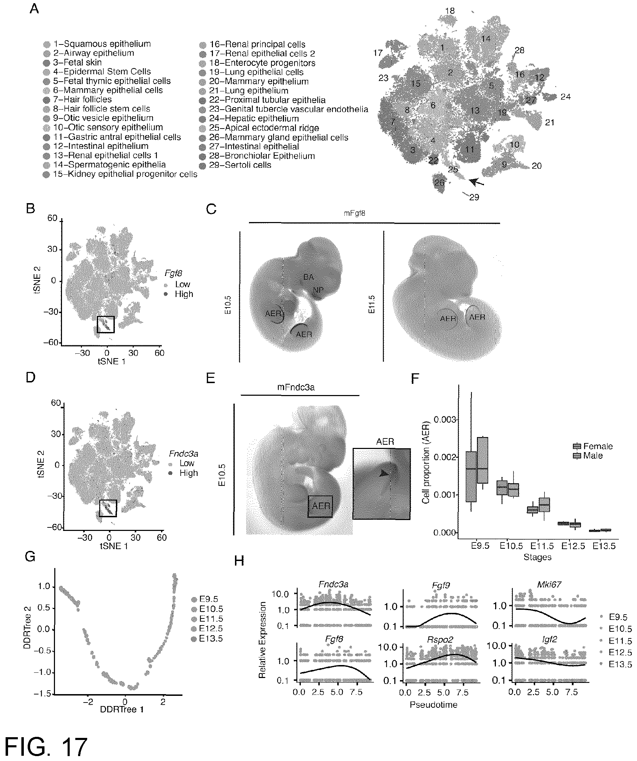

Fig. 17 shows identification and characterization of epithelial cell subtypes and the limb apical ectodermal ridge (AER). (A) t-SNE visualization and marker-based annotation of of epithelial cell subtypes. (B) t-SNE visualization of all epithelial cells colored by expression level of Fgf8. (C) In situ hybridization images of Fgf8 in E10.5 (left) and E11.5 (right) embryos. (D) t-SNE visualization of all epithelial cells colored by expression level of Fndc3a. (E) In situ hybridization images of Fndc3a in E10.5 embryo. Arrow: site of gene expression. (F) Box plot showing the proportion of AER cells per embryo at different developmental stages. (G) Pseudotime trajectory of AER single cell transcriptomes, colored by development stage. (H) Line plot showing relative expression of AER marker genes across developmental pseudotime.

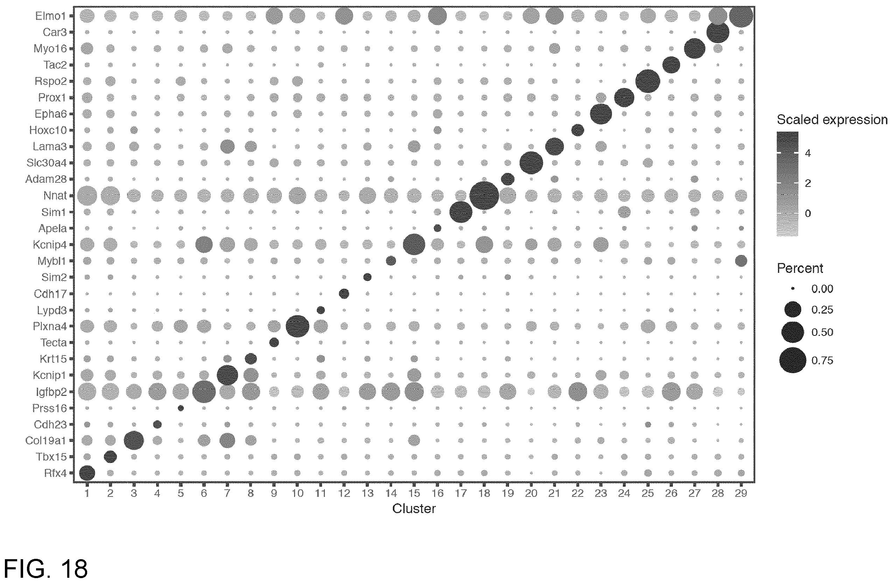

Fig. 18 shows identifying the subtypes of mouse epithelium. Dot plot showing expression of one selected marker gene per epithelial subtype. The size of the dot encodes the percentage of cells within a cell type, and its color encodes the average expression level.

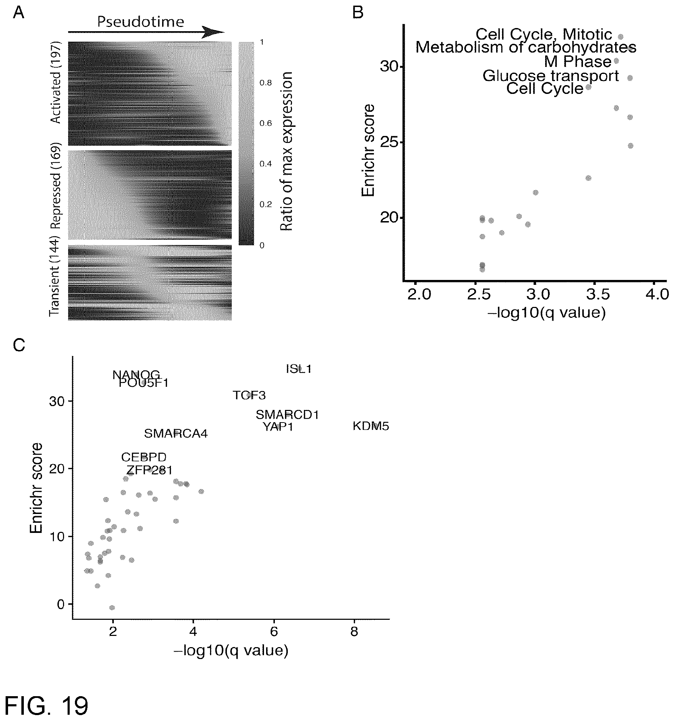

Fig. 19 shows dynamics of gene expression of limb apical ectodermal ridge (AER) cells across pseudotime. (A) Heatmap showing smoothed pseudotime-dependent differential gene expression (FDR of 1%) in AER cells, generated by a negative binomial regression and scaled as a percent of maximum gene expression. Each row indicates a different gene, and these are split into subsets that are activated (top), repressed (middle) or exhibit transient dynamics (bottom) between E9.5 and E13.5. (B-C) Plots showing the -log10 transformed q value and enrichR based combined score of enriched Reactome terms (B) and transcription factors (C) for genes whose expression significantly decreases in AER development. The top enriched pathway terms (Reactome2016) for significantly decreasing genes include cell cycle progression (Mitotic Cell Cycle, qval = 0.0002) and glucose metabolism (Metabolism of carbohydrates, qval = 0.0002). The top enriched TFs with targets from decreasing genes include pluripotent factors such as Isl1 (qval < 10-5), Pou5f1 (qval = 0.002) and Nanog (qval = 0.003).

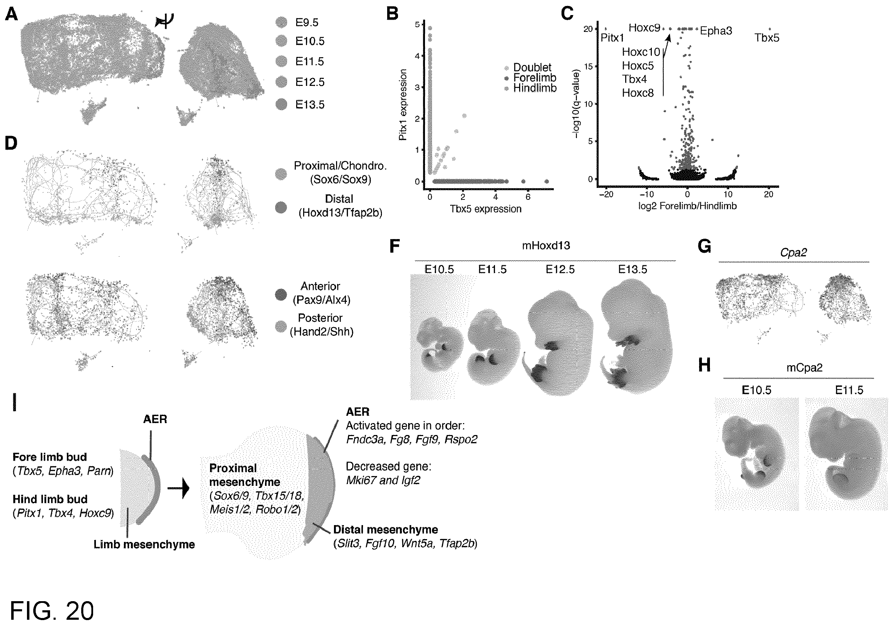

Fig. 20 shows characterizing cellular trajectories during limb mesenchyme differentiation. (A) UMAP 3D visualization of limb mesenchymal cells colored by development stage (left and right represent views from two directions). (B) Scatter plot showing the normalized expression of Pitx1 and Tbx5 in limb mesenchyme cells. Only cells in which Pitx1 and/or Tbx5 detected were shown. (C) Volcano plot showing the differentially expressed genes (FDR of 5%, colored by red) between forelimb and hindlimb. Top differentially expressed genes are labeled. X axis: log2 transformed fold change between forelimb and hindlimb for each gene. Y axis: -log10 transformed qval from differentiation test. (D) Same visualization as panel A, colored by normalized gene expression of proximal/chondrocyte (Sox6, Sox9), distal (Hoxd13, Tfap2b), anterior (Pax9, Alx4), or posterior (Hand2, Shh) markers. (F) In situ hybridization images of Hoxd13 in E10.5 to E13.5 embryos. (G) Same visualization as panels A & D, colored by normalized gene expression of Cpa2. Its expression pattern within this trajectory led us to predict that Cpa2 is a distal marker of the developing limb mesenchyme, like Hoxd13. (H) In situ hybridization images of Cpa2 in E10.5 and E11.5 embryos. (I) Combined summary of results for the AER and limb mesenchyme trajectories.

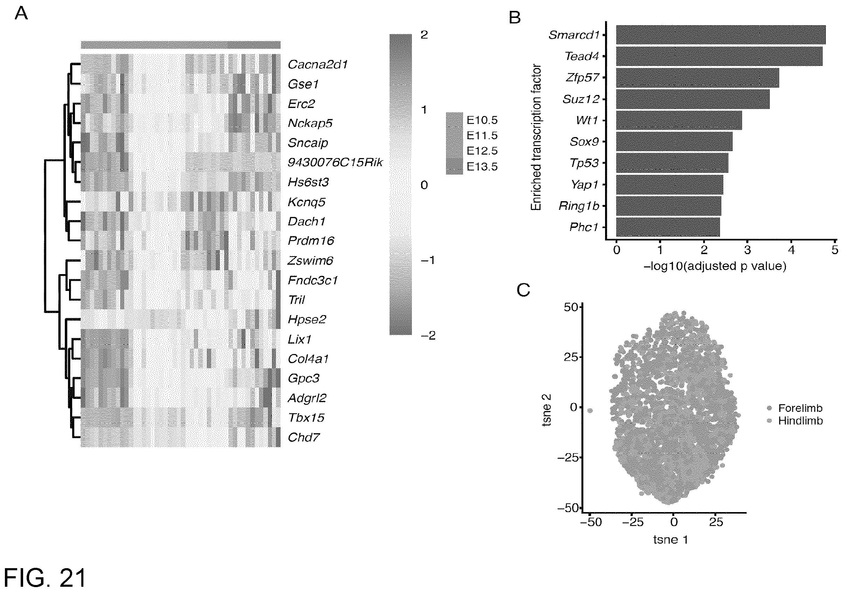

Fig. 21 shows characterizing cell fate trajectories during limb mesenchyme development. (A) Heatmap showing top differentially expressed genes between different development stages for limb mesenchyme cells. (B) Bar plot showing the -log10 transformed adjusted p value of enriched transcription factors for significantly up-regulated genes during limb mesenchyme development. (C) t-SNE visualization of limb mesenchyme cells colored by forelimb (Tbx5 +) and hindlimb (Pitx1+). Cells with no expression or both expression in Tbx5 and Pitx1 are not shown.

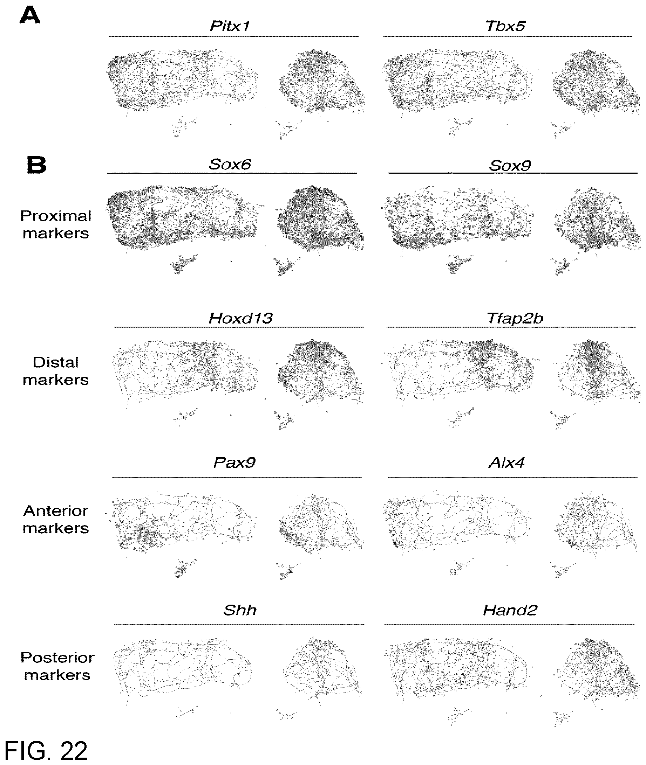

Fig. 22 shows expression of markers spatially restricted in the limbs. Each panel illustrates a different marker gene. Colors indicate UMI counts that have been scaled for library size, log-transformed, and then mapped to Z-scores to enable comparison between genes. Cells with no expression of a given marker are excluded to prevent overplotting. (A) Hindlimb marker Pitx1 and forelimb marker Tbx5. (B) First row: proximal limb markers Sox6 (which also marks chondrocytes) and Sox9. Second row: distal limb markers Hoxd13 and Tfap2b. Third row: Anterior limb markers68 Pax9 and Alx4. Fourth row: posterior limb markers Shh and Hand2.

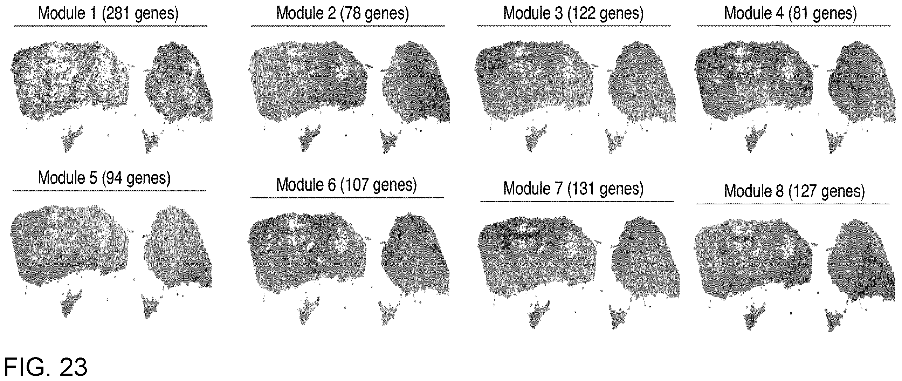

Fig. 23 shows modules spatially restricted genes in the limbs. A total of 1,191 genes were clustered via hierarchical clustering. The dendrogram was cut into 8 modules using the cutree function in R, and the aggregate expression of genes in each module was computed. Colors indicate aggregate UMI counts for each module that have been scaled for library size, log-transformed, and then mapped to Z-scores to enable comparison between modules. Cells with no expression of a given module are excluded to prevent overplotting.

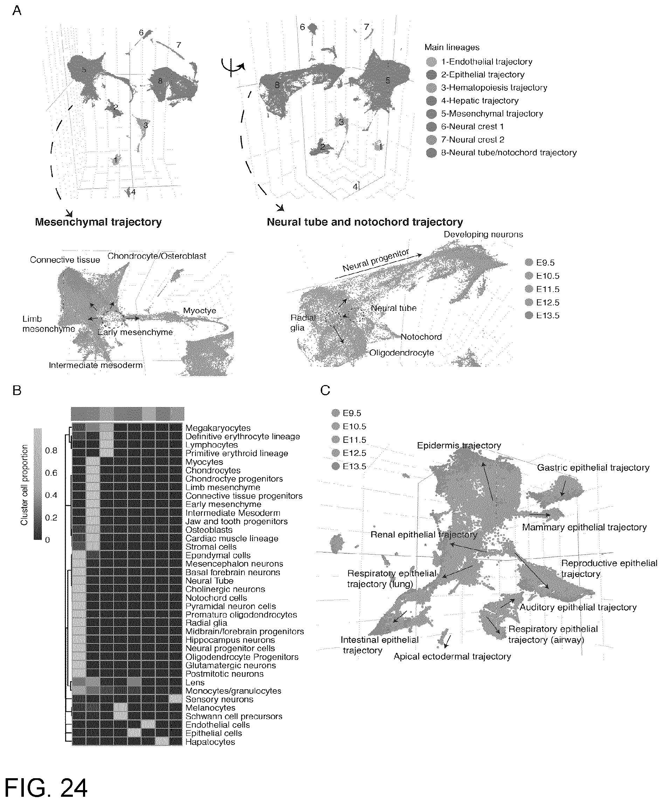

Fig. 24 shows characterization of eight major developmental trajectories present during mouse organogenesis. (A) UMAP 3D visualization of our overall dataset; top: views from two directions; bottom: zoomed view of mesenchymal (left) and neural tube/notochord (right) trajectories, colored by development stage. (B) Heatmap showing the proportion of cells from each of the 38 major cell types assigned to each of the 8 major trajectories. The columns represent the eight major lineages, labeled by the colors in the top bar (see key in panel A). (C) UMAP 3D visualization of epithelial sub-trajectories colored by development stage.

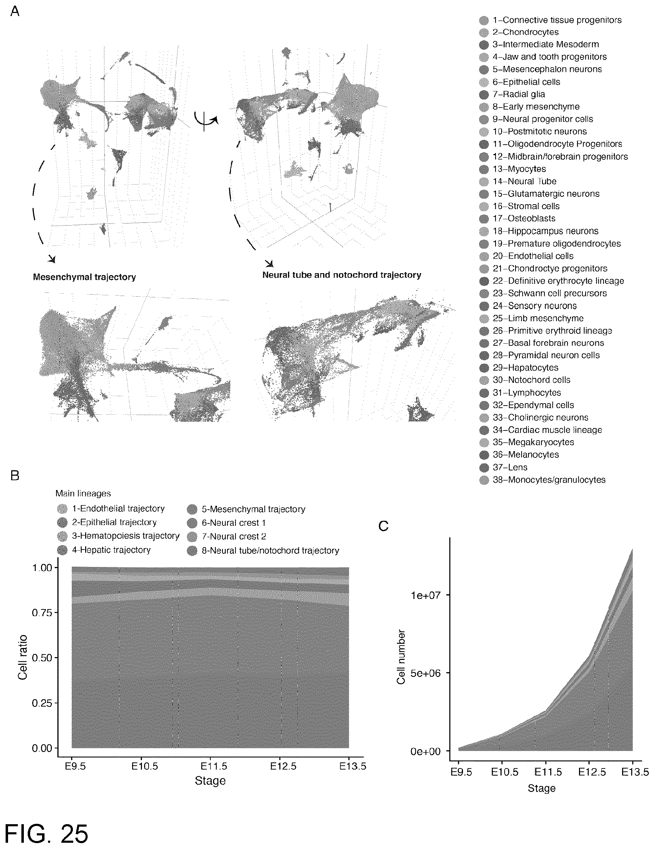

Fig. 25 shows characterization of eight major developmental trajectories present during mouse organogenesis. (A) Same to Fig. 24A, but with colors corresponding to the 38 main cell clusters. (B-C) Area plot showing the estimated proportion (B) and estimated absolute cell number (C) of cells per embryo derived from each of the eight main cell trajectories from E9.5 to E13.5.

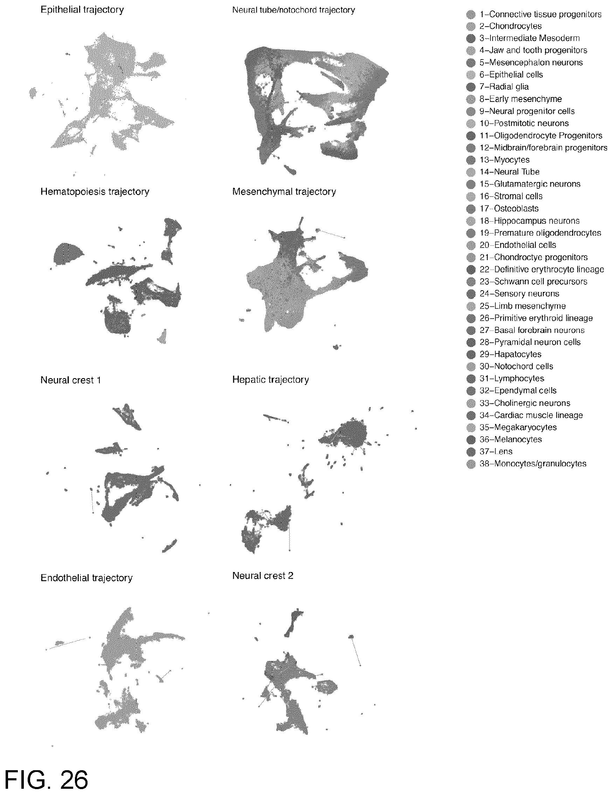

Fig. 26 shows UMAP visualization of the eight major cell trajectories colored by major cell cluster ID.

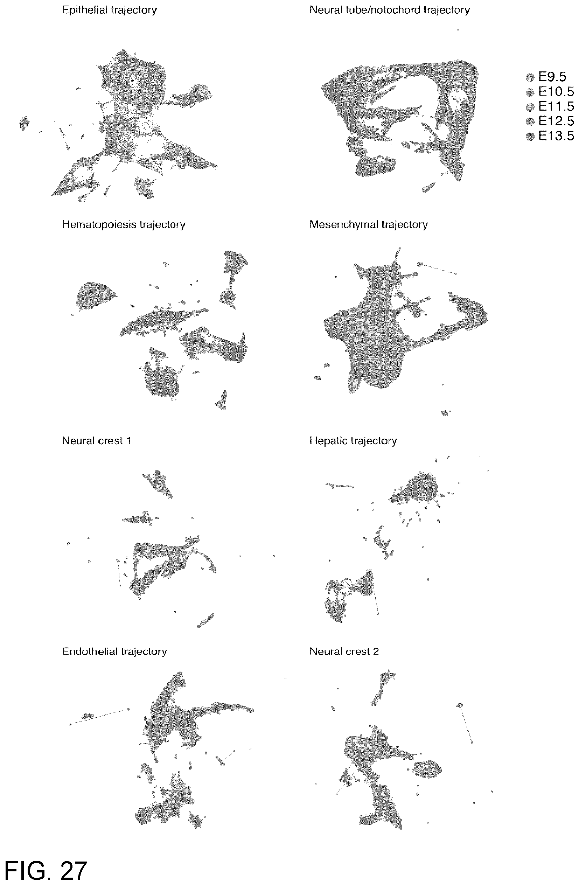

Fig. 27 shows UMAP visualization of the eight major cell trajectories colored by developmental stage.

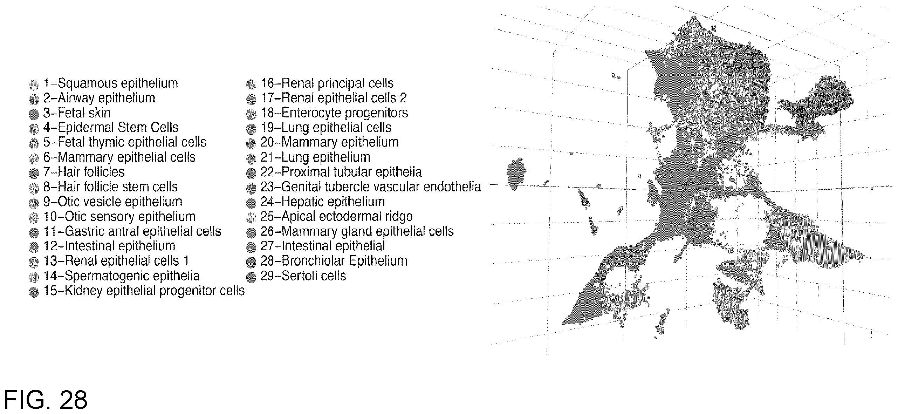

Fig. 28 shows UMAP visualization of epithelial cell subtypes. Colored as per the 29 epithelial subtypes shown in Fig. 17A.

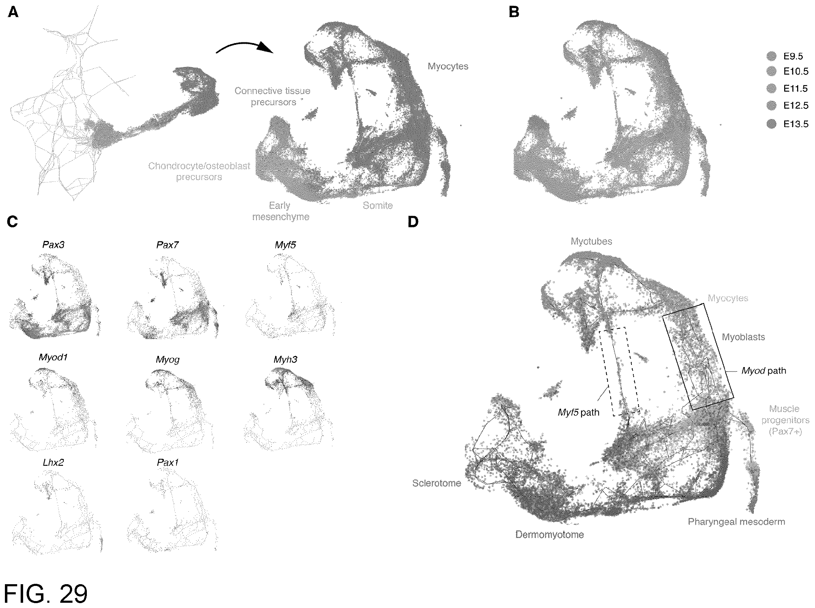

Fig. 29 shows resolving cellular trajectories in myogenesis. Edges in the principal graphs that define trajectories reported by Monocle 3 are shown as light blue line segments. (A) Cells putatively involved in myogenesis were isolated from the mesenchymal cell trajectory in silico and then used to construct a myocyte sub-trajectory (Methods). (B) Cells in the myocyte sub-trajectory colored by developmental stage. (C) Cells in the myocyte trajectory, colored by their expression of selected transcriptional regulators of myogenesis. Cells with no detectable expression for a given gene are omitted from its plot. (D) Cells classified by developmental stage according to the markers shown in panel C (Methods).

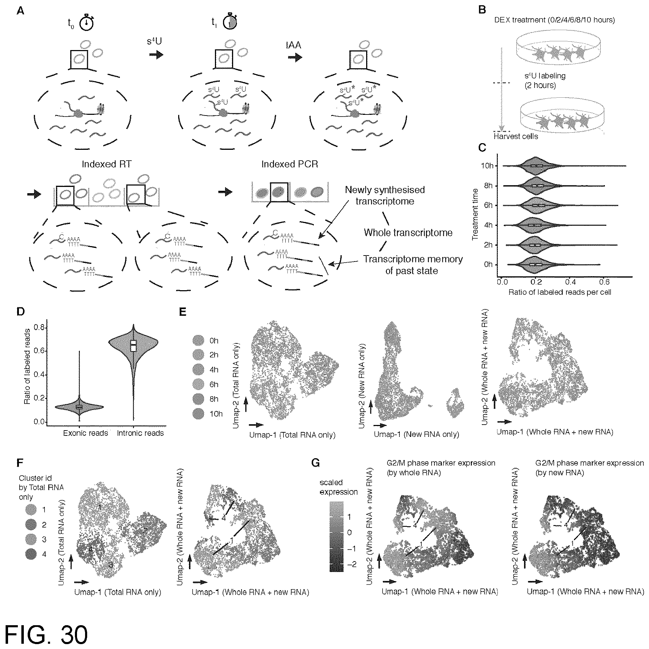

Fig. 30 shows joint profiling of total and newly synthesised transcriptome by sci-fate. (A) sci-fate workflow with key steps outlined in text. (B) Experiment scheme. A549 cells were treated with dexamethasone time dependently. Cells from all treatment conditions were labeled with S4U two hours before harvest for sci-fate. (C) Violin plot showing the ratio of S4U labeled reads per cell in six treatment time. (D) Violin plot showing the ratio of S4U labeled reads in exonic and intronic reads. For all box plots: thick horizontal lines, medians; upper and lower box edges, first and third quartiles, respectively; whiskers, 1.5 times the interquartile range; circles, outliers. (E) UMAP visualization of A549 cells by whole transcriptome (left), newly synthesised transcriptome (middle) and both (right). (F) Similar with (E), colored by cluster id identified by whole transcriptome. (G) UMAP visualization of A549 cells by joint information, colored by normalized expression of G2/M marker genes by RNA level (left) and newly synthesised RNA level (right). UMI counts for these genes are scaled by library size, log-transformed, aggregated and then mapped to Z-scores.

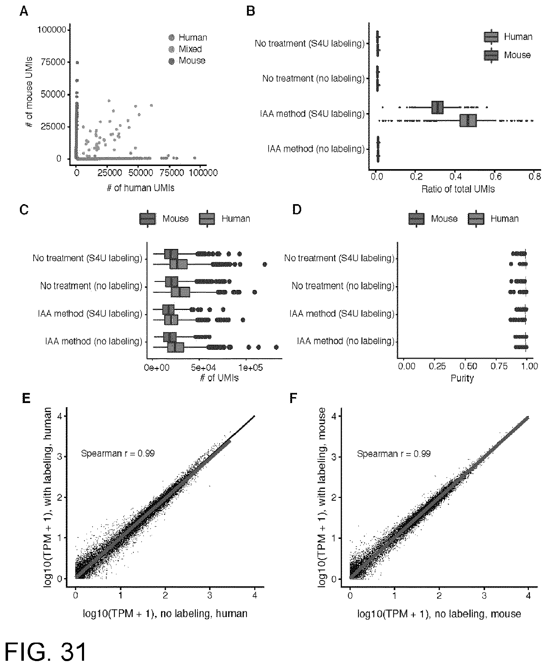

Fig. 31 shows performance and QC-related analyses for sci-fate. (A) Scatter plot of mouse (NIH/3T3) vs. human (HEK293T) UMI counts per cell in the condition of sci-fate. (B-D) Boxplot showing the ratio of S4U labeled reads, number of UMIs, and purity (proportion of reads mapping to the expected species) per cell from HEK293T (cell number n = 932) and NIH/3T3 cells (cell number n = 438). For all box plots: thick horizontal lines, medians; upper and lower box edges, first and third quartiles, respectively; whiskers, 1.5 times the interquartile range; circles, outliers. (E-F) Correlation (Spearman's correlation) between gene expression measurements in aggregated profiles of HEK293T (E) and NIH/3T3 cells (F) from sci-fate (y axis) vs. sci-RNA-seq cells (x axis).

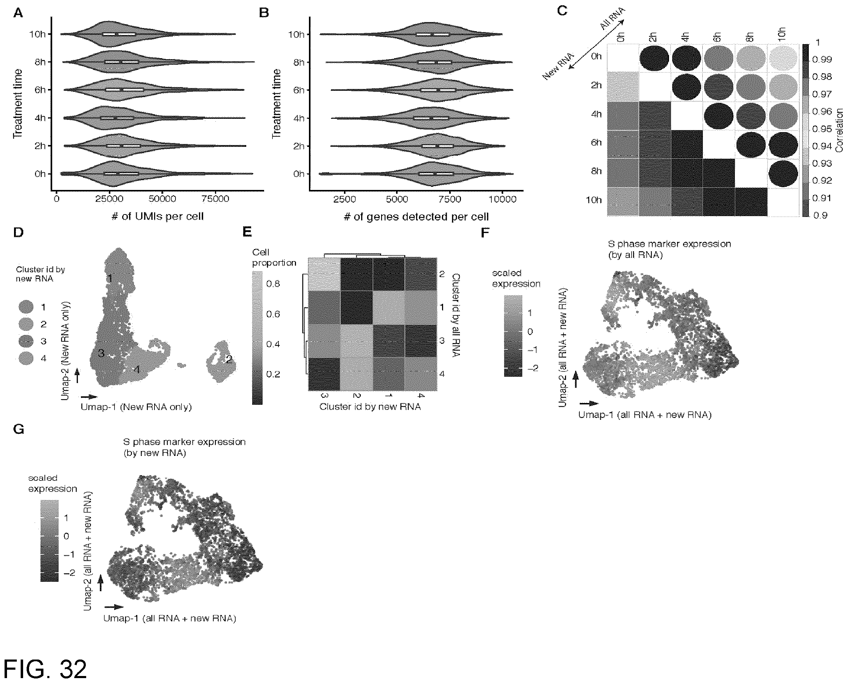

Fig. 32 shows performance of sci-fate on dexamethasone treated A549 cells. (A, B) Violin plot showing the number of UMIs (A) and genes (B) per cell in six treatment conditions. For all box plots: thick horizontal lines, medians; upper and lower box edges, first and third quartiles, respectively; whiskers, 1.5 times the interquartile range; circles, outliers. (C) Correlation plot showing the Pearson correlation coefficient between different treatment conditions for aggregated whole transcriptome (top right) and newly synthesised transcriptome (down left). (D) UMAP visualization of A549 cells by newly synthesised transcriptome, colored by cluster id identified by newly synthesised transcriptome.

(E) heatmap showing the proportion of cells from each clusters defined by whole transcriptome, falling into each cell cluster by newly synthesised transcriptome. (F-G) UMAP visualization of A549 cells by both total and newly synthesised transcriptome, colored by normalized expression of S phase marker genes by total RNA expression (F) and newly synthesised RNA (G). UMI counts for these genes are scaled for library size, log-transformed, aggregated and then mapped to Z-scores.

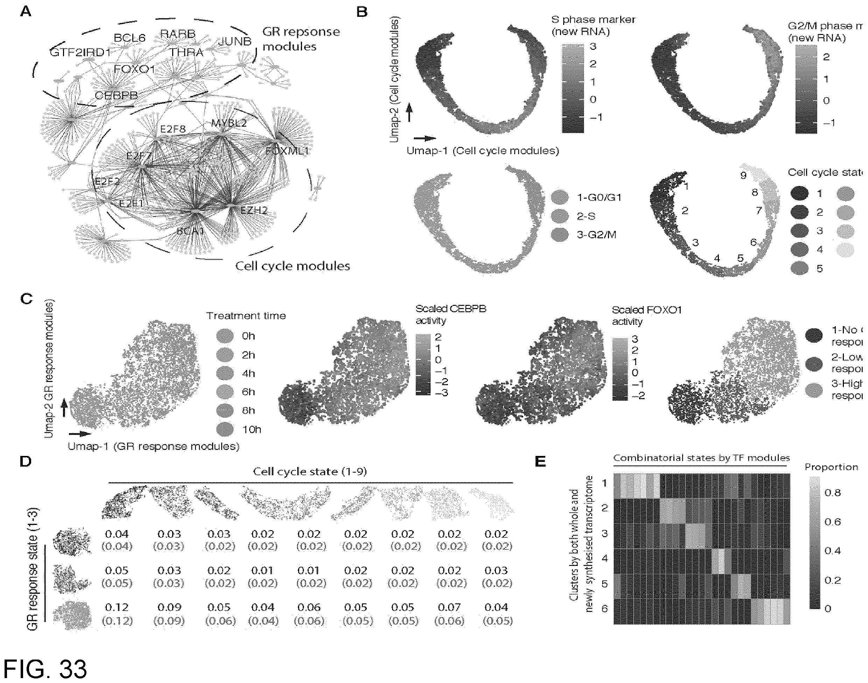

Fig. 33 shows characterizing TF modules driving cell state transition. (A) Identified links (blue) between transcription factors (orange) and regulated genes (grey). TF modules related with cell cycle progression or GR response are labeled. (B) UMAP visualization of A549 cells ordered by cell cycle TF modules, colored by newly synthesised mRNA of S phase and G2/M phase markers (top), three cell cycle phases (bottom left), and nine cell cycle states by unsupervised clustering analysis (bottom right). (C) UMAP visualization of A549 cells ordered by GR reponse TF modules, colored by DEX treatment time (left), CEBPB and FOXO1 activity (middle) and cluster id from unsupervised clustering analysis (right). To calculate TF activity, newly synthesised UMI counts for these genes are scaled by library size, log-transformed, aggregated and then mapped to Z-scores. (D) A table showing the observed ratio (black) of cell state by the combinatorial state of cell cycle modules (x axis) and GR response modules (y axis). The red number is the expected ratio assuming independent assortment. (E) Heatmap showing the proportion of cell states defined by the combinatorial states of TF modules in each main clusters identified by clustering analysis based on joint whole and newly synthesised transcriptome.

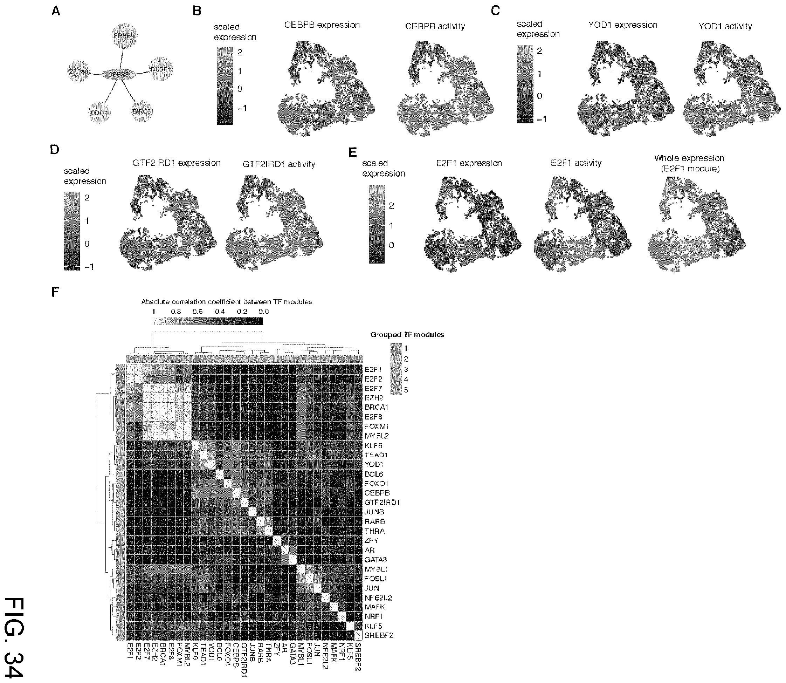

Fig. 34 shows TF modules driving cell state transition in DEX treated A549 cells. (A) Identified gene targets (grey) of CEBPB (orange). Only links with regularized correlation coefficient from LASSO > 0.6 are shown. (B) UMAP visualization of A549 cells by whole and newly synthesised transcriptome colored by CEBPB expression (left) and activity (right). (C) similar with (B), colored by the YOD1 expression (left), and YOD1 activity (right). (D) similar with (B), colored by the GTF2IRD1 expression (left), and GTF2IRD1 activity (right). (E) similar with (B), colored by the E2F1 expression (left), E2F1 activity (middle) and aggregated expression of whole transcriptome for E2F1 linked genes (right). (F) Heatmap showing the absolute value of Pearson's correlation coefficient between TF modules. 29 TF modules were grouped into five groups by hierarchical clustering analysis.

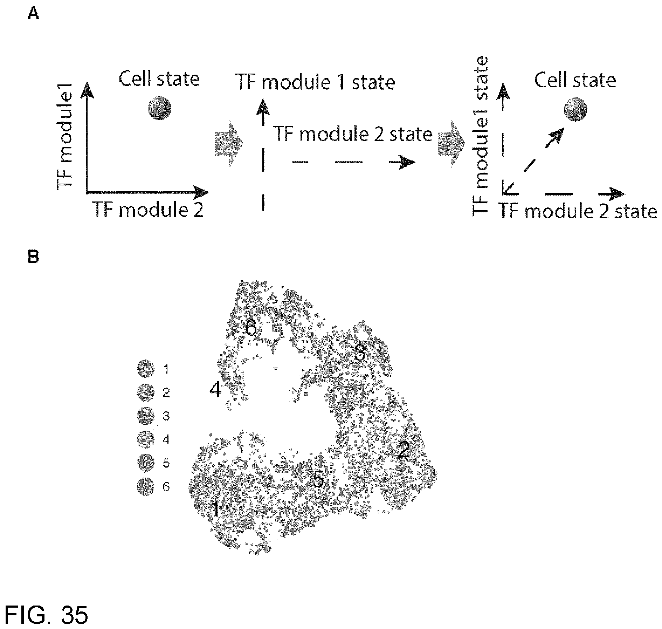

Fig. 35 shows cell states characterized by combinatorial states of functional TF modules. (A) Scheme showing the strategy for characterizing cell states by combinatorial states of functional TF modules. (B) Umap visualization of all cells by both whole and newly synthesized transcriptome, colored with main cluster id identified by density peak clustering algorithm on the UMAP space.

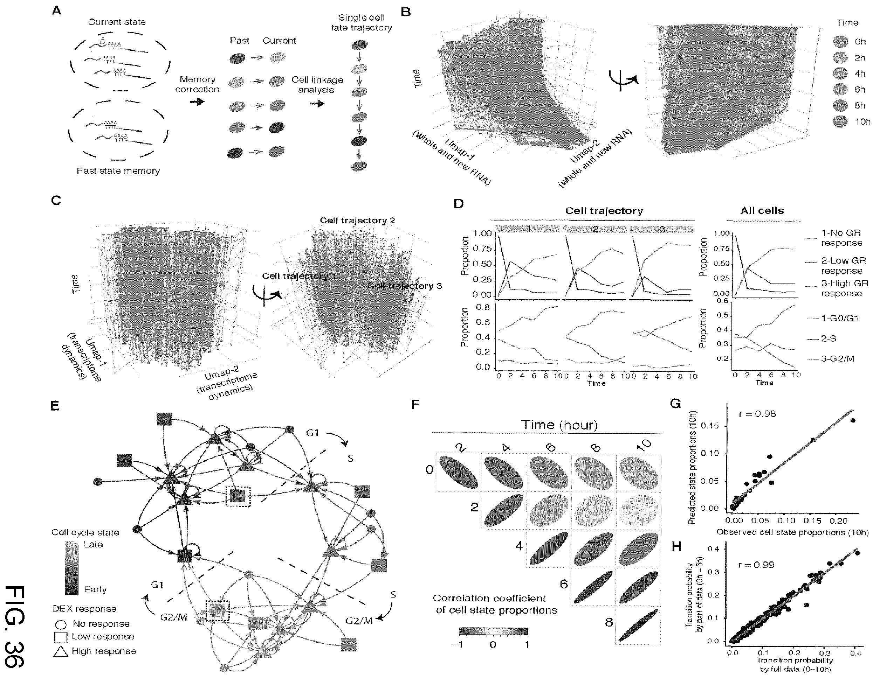

Fig. 36 shows characterizing of >6,000 single cell state transition trajectories. (A) Scheme showing memory correction and cell linkage analysis to construct single cell transition trajectory with details outlined in text and method. (B) 3D plot of cells colored by DEX treatment time (also as z coordinates). The x and y coordinates correspond to the UMAP space by whole and newly synthesised transcriptome in Fig. 30E (left). Linked parent and child cells are connected with grey lines. (C) Similar with (B), except the x and y coordinates correspond to the UMAP space by single cell transcriptome dynamics across six time points. (D) Line plots showing the cell state dynamics of different GR reponse states (top) and cell cycle states (bottom) in each cell trajectory clusters (left) or all cells (right) independent of cell linkage analysis. (E) Cell state transition network. The nodes are 27 cell states characterized in Fig. 33D and the links are identified transition paths between cell states. Links with low transition probabilities (< 0.1) are filtered out. Squares with dashed lines showing the example states with reversible transition dynamics. (F) Correlation plot showing the correlation of cell state proportions between treatment conditions. Positive correlations are displayed in blue and negative correlations in red color. The shape of the ellipse are correlated with the correlation coefficients (on the ellipse). (G) Scatter plot showing the correlation of cell state proportions between observed 10 hour DEX treatment groups and predicted cell state proportions. The prediction is based on the cell state transition probabilities and cell state proportion in no DEX treatment group. The blue line represent the linear regression line. (H) Scatter plot showing the correlation of cell state transition probabilities calculated by full data (0-10 hours) or part data (0-6 hours), along with the linear regression line.

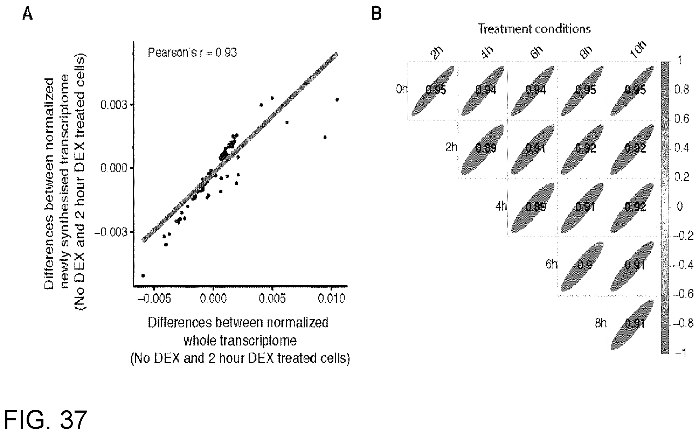

Fig. 37 shows new RNA detection rate and RNA degradation rate estimation. (A) scatter plot showing the correlation between x axis: differences of normalized whole transcriptome between no DEX and 2 hour DEX treated cells, and y axis: differences of normalized newly synthesised transcriptome between no DEX and 2 hour DEX treated cells. Blue line is the linear regression line. Both whole transcriptome and newly synthesised transcriptome of each time point are normalized by the library size of whole transcriptome of the time point. (B) Correlation plot showing the correlation of estimated gene degradation rate between treatment conditions. Positive correlations are displayed in blue and negative correlations in red color. The shape of the ellipse are correlated with the correlation coefficients (on the ellipse).

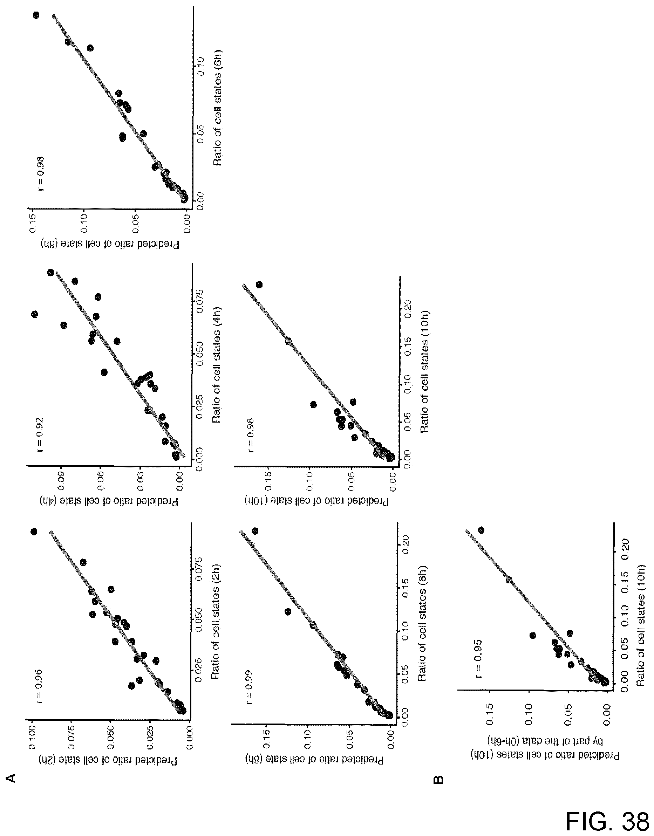

Fig. 38 shows cell state transition network for cell state prediction. (A) Scatter plot showing the correlation between observed cell states at each treatment time and predicted cell state by cell state transition probabilities and cell state proportion in no DEX treatment group. The blue line represent linear regression line. (B) Scatter plot showing the correlation of cell state proportions between observed 10 hour DEX treatment groups and predicted values. The predicted values is based on cell state transition probabilities estimated by part data (0-6 hours) and cell state proportion in no DEX treatment group. The blue line represent the linear regression line.

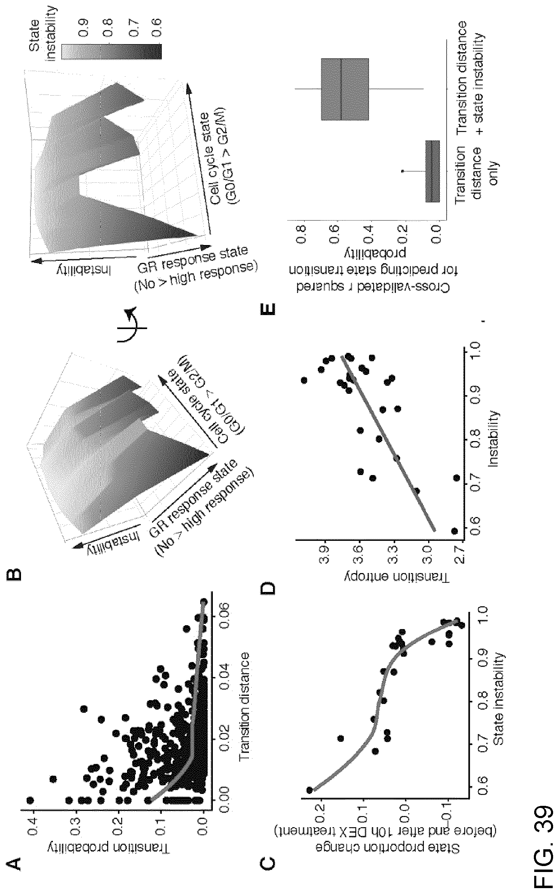

Fig. 39 shows cell state transition probabilities are regulated by nearby state stability landscape. (A) Scatter plot showing the relationship between transition distance (Pearson's distance) and transition probability between cell states, together with the red LOESS smooth line by ggplot2. (B) 3D plot showing the instability landscape of cell states. X-axis represents GR response states (from no to low to high response state). Y-axis represent the cell cycle states ordered from G0/G1 to G2/M states. Z-axis represent the cell state instability, defined by the probability of cells within each cell state jumping to other states after 2 hours. (C) Scatter plot showing the relationship between cell state instability and cell proportion change before and after 10 hour DEX treatment, together with the red LOESS smooth line by ggplot2. (D) Scatter plot showing the correlation between state instability and state transition entropy with the linear regression line (blue). (E) Box plot showing the cross-validated r squared for predicting inter-state transition probability by transition distance only or combining transition distance and state instability landscape by densely connected neural network.

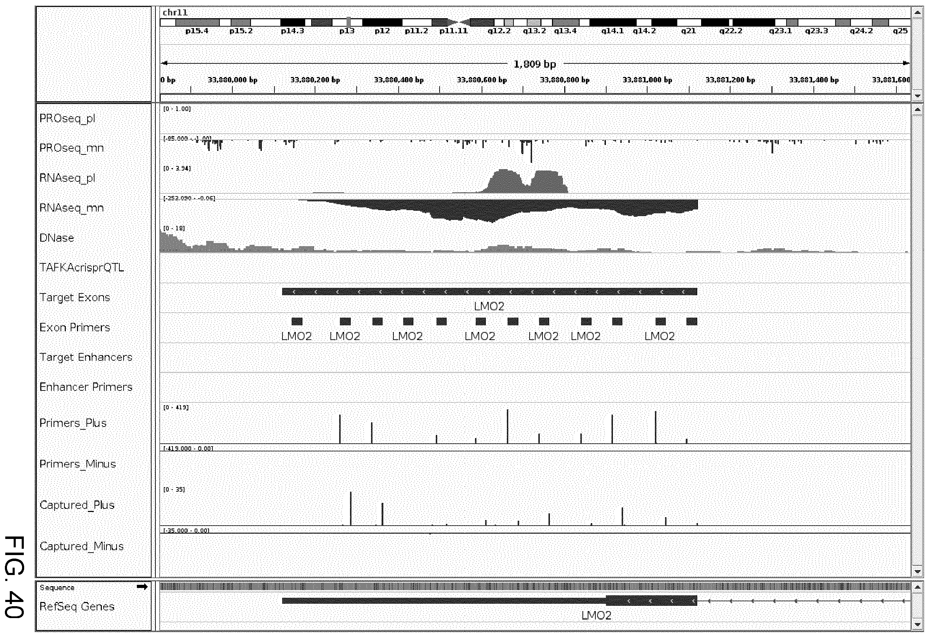

Fig. 40 shows a browser shot of a targeted exon in the LMO2 gene. The targeted exon is indicated in the 'Target Exons' track. Note that 12 RT primers that passed our filters span the exon as evenly as possible. The bottom two tracks, 'Primers_plus/minus' and 'Captured_plus/minus' show reads mapping to the RT primers and the corresponding captured transcripts. A difference in read count indicates the number of off target priming events.

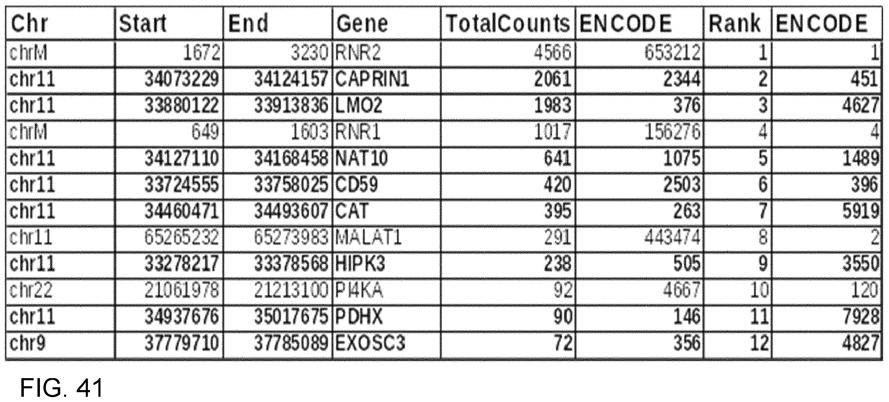

Fig. 41 show a table comparing rank of genes in an ENCODE total nuclear RNA-seq dataset and an in situ multiplex RT capture library. Targeted genes are indicated in bold. Of the 12 most abundant genes in the targeted library, 8 were genes that we targeted. Last two columns are 'Rank', rank in our dataset, 'ENCODE', rank in ENCODE dataset. Abundant nontargeted RNAs include mitochondrial ribosomal RNAs RNR2 and RNR1, and the highly abundant nuclear lncRNA MALAT1. Note that the LMO2 gene goes from the 4,627th most detected gene in the ENCODE dataset (out of 26,281 genes) to the 3rd most detected in our library.

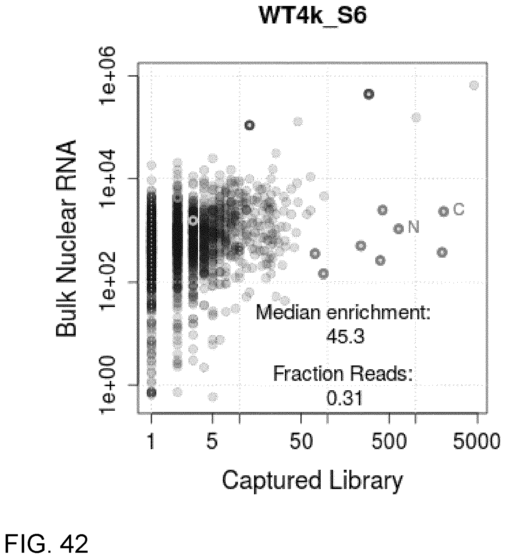

Fig. 42 shows enrichment of captured transcript over total RNA. Scatter plot of reads in a targeted library vs reads in ENCODE nuclear RNA. Genes targeted by RT are indicated in red; note that they generally fall off the diagonal, indicating that they were enriched over the level expected given expression in the reference dataset. The abundant nuclear lncRNAs MALAT1 and XIST are indicated in blue: they fall above the diagonal, indicating successful enrichment against these RNAs. An eRNA targeted is not enriched (orange). The median enrichment above expected level for the 9 genes targeted in this experiment is 45.3-fold. Overall, reads from targeted genes account for 31% of the total reads mapping to genes in this dataset.

The schematic drawings are not necessarily to scale. Like numbers used in the figures refer to like components, steps and the like. However, it will be understood that the use of a number to refer to a component in a given figure is not intended to limit the component in another figure labeled with the same number. In addition, the use of different numbers to refer to components is not intended to indicate that the different numbered components cannot be the same or similar to other numbered components. DETAILED DESCRIPTION OF ILLUSTRATIVE EMBODIMENTS

In one embodiment, the method provided herein can be used to produce single cell combinatorial indexing (sci) sequencing libraries that include transcriptomes of a plurality of single cells. For instance, the method can be used to obtain sequence information for whole cell transcriptomes, transcriptomes of newly synthesized RNA, or the combination. In another embodiment, the method provided herein can be used to produce sci sequencing libraries that include sequence information of a subpopulation of RNA nucleic acids. For instance, when a noncoding regulatory region is targeted for perturbation a coding region *cis* to the regulatory region can be tested for altered expression. In another example, cell atlas experiments can be conducted with the readout restricted to a limited number of mRNAs that are highly informative.

The method can include one or more of providing isolated nuclei or cells, distributing subsets of isolated nuclei or cells into compartments, processing the isolated nuclei or cells so they include nucleic acid fragments, and adding a compartment specific index to the nucleic acid fragments. Optionally, the method can include exposing cells to a predetermined condition and/or labeling newly synthesized RNA in the cells. The method can be directed to obtaining information that includes a cell's transcriptome, or a subpopulation of RNA nucleic acids. These steps can occur in essentially any order and can be combined in different ways. Optionally, nuclei can be isolated from the cells after exposing the cells to a predetermined condition and labeling newly synthesized RNA.

Providing isolated nuclei or cells

The method provided but not part of the invention herein can include providing the cells or isolated nuclei from a plurality of cells (FIG. 1, block 10; FIG. 2, block 22). The cells can be from any organism(s), and from any cell type or any tissue of the organism(s). In one embodiment, the cells can be embryonic cells, e.g., cells obtained from an embryo. In one embodiment, the cells or nuclei can be from cancer or a diseased tissue. The method can further include dissociating cells, and/or isolating the nuclei. The number of nuclei or cells can be at least two. The upper limit is dependent on the practical limitations of equipment (e.g., multi-well plates, number of indexes) used in other steps of the method as described herein. The number of nuclei or cells that can be used is not intended to be limiting, and can number in the billions. For instance, in one embodiment the number of nuclei or cells can be no greater than 100,000,000, no greater than 10,000,000, no greater than 1,000,000,000, no greater than 100,000,000, no greater than 10,000,000, no greater than 1,000,000, no greater than 100,000, no greater than 10,000, no greater than 1,000, no greater than 500, or no greater than 50. The skilled person will recognize that in some embodiments the nucleic acid molecules in each nucleus represent the entire transcriptome of that nucleus, e.g., the whole transcriptome, the newly synthesized transcriptome, or both.

In those embodiments using isolated nuclei, the nuclei can be obtained by extraction and fixation. Optionally and preferably, the method of obtaining isolated nuclei does not include enzymatic treatment. In those embodiments where the newly synthesized transcriptome is produced, nuclei are not isolated until after the cell has been exposed to conditions suitable for labeling the newly synthesized transcripts.

In one embodiment, nuclei are isolated from individual cells that are adherent or in suspension. Methods for isolating nuclei from individual cells are known to the person of ordinary skill in the art. Nuclei are typically isolated from cells present in a tissue. The method for obtaining isolated nuclei typically includes preparing the tissue, isolating the nuclei from the prepared tissue, and then fixing the nuclei. In one embodiment all steps are done on ice.

Tissue preparation includes snap freezing the tissue in liquid nitrogen, and then reducing the size of the tissue to pieces of 1 mm or less in diameter. Tissue can be reduced in size by subjecting the tissue to either mincing or a blunt force. Mincing can be accomplished with a blade to cut the tissue to small pieces. Applying a blunt force can be accomplished by smashing the tissue with a hammer or similar object, and the resulting composition of smashed tissue is referred to as a powder.

Nuclei isolation can be accomplished by incubating the pieces or powder in cell lysis buffer for at least 1 to 20 minutes, such as 5, 10, or 15 minutes. Useful buffers are those that promote cell lysis but retain nuclei integrity. An example of a cell lysis buffer includes 10 mM Tris-HCl, pH 7.4, 10 mM NaCl, 3 mM MgCl2, 0.1% IGEPAL CA-630, 1% SUPERase In RNase Inhibitor (20 U/$\mu$L, Ambion) and 1% BSA (20 mg/ml, NEB). Standard nuclei isolation methods often use one or more exogenous compounds, such as exogenous enzymes, to aid in the isolation. Examples of useful enzymes that can be present in a cell lysis buffer include, but are not limited to, protease inhibitors, DNase, lysozyme, Proteinase K, surfactants, lysostaphin, zymolase, cellulose, protease or glycanase, and the like (Islam et al. Micromachines (Basel), 2017, 8(3):83; www.sigmaaldrich.com/life-science/biochemicals/biochemical-products.html?TablePage=14573107). In one embodiment, one or more exogenous enzymes are not present in a cell lysis buffer useful in the method described herein. For instance, an exogenous enzyme, (i) is not added to the cells prior to mixing of cells and lysis buffer, (ii) is not present in a cell lysis buffer before it is mixed with cells, (iii) is not added to the mixture of cells and cell lysis buffer, or a combination thereof. The skilled person will recognize these levels of the components can be altered somewhat without reducing the usefulness of the cell lysis buffer for isolating nuclei. The extracted nuclei are then purified by one of more rounds of washing with a nuclei buffer. An example of a nuclei buffer includes 10 mM Tris-HCl, pH 7.4, 10 mM NaCl, 3 mM MgCl2, 1% SUPERase In RNase Inhibitor (20 U/$\mu$L, Ambion) and 1% BSA (20 mg/ml, NEB). Like a cell lysis buffer, exogenous enzymes can also be absent from a nuclei buffer used in a method of the present disclosure. The skilled person will recognize these levels of the components can be altered somewhat without reducing the usefulness of the nuclei buffer for isolating nuclei. The skilled person will recognize that BSA and/or surfactants can be useful in the buffers used for the isolation of nuclei.

Isolated nuclei are fixed by exposure to a cross-linking agent. A useful example of a cross-linking agent includes, but is not limited to, paraformaldehyde. The paraformaldehyde can be at a concentration of 1% to 8%, such as 4%. Treatment of nuclei with paraformaldehyde can include adding paraformaldehyde to a suspension of nuclei and incubating at 0°C. Optionally and preferably, fixation is followed by washing in a nuclei buffer.

Isolated fixed nuclei can be used immediately or aliquoted and flash frozen in liquid nitrogen for later use. When prepared for use after freezing, thawed nuclei can be permeabilized, for instance with 0.2% tritonX-100 for 3 minutes on ice, and briefly sonicated to reduce nuclei clumping.

Conventional tissue nuclei extraction techniques normally incubate tissues with tissue specific enzyme (e.g., trypsin) at high temperature (e.g., 37°C) for 30 minutes to several hours, and then lyse the cells with cell lysis buffer for nuclei extraction. The nuclei isolation method described herein has several advantages: (1) No artificial enzymes are introduced, and all steps are done on ice. This reduces potential perturbation to cell states (e.g., transcriptome state). (2) The new method has been validated across most tissue types including brain, lung, kidney, spleen, heart, cerebellum, and disease samples such as tumor tissues. Compared with conventional tissue nuclei extraction techniques that use different enzymes for different tissue types, the new technique can potentially reduce bias when comparing cell states from different tissues. (3) The new method also reduces cost and increases efficiency by removing the enzyme treatment step. (4) Compared with other nuclei extraction techniques (e.g., Dounce tissue grinder), the new technique is more robust for different tissue types (e.g., the Dounce method needs optimizing Dounce cycles for different tissues), and enables processing large pieces of samples in high throughput (e.g., the Dounce method is limited to the size of the grinder).

Optionally, the isolated nuclei can be nucleosome-free or can be subjected to conditions that deplete the nuclei of nucleosomes, generating nucleosome-depleted nuclei.

Distributing subsets

The method provided herein includes distributing subsets of the isolated nuclei or cells into a plurality of compartments (FIG. 1, block 11; FIG. 2, block 23; FIG. 3, block 32). The method can include multiple distribution steps, where a population of isolated nuclei or cells (also referred to herein as a pool) is split into subsets. Typically, subsets of isolated nuclei or cells, e.g., subsets present in a plurality of compartments, are indexed with compartment specific indexes and then pooled. Accordingly, the method typically includes at least one "split and pool" step of taking pooled isolated nuclei or cells and distributing them and adding a compartment specific index, where the number of "split and pool" steps can depend on the number of different indexes that are added to the nucleic acid fragments. Each initial subset of nuclei or cells prior to indexing can be unique from other subsets. For example, each first subset can be from a unique sample or exposed to a unique condition. After indexing, the subsets can be pooled after indexing, split into subsets, indexed, and pooled again as needed until a sufficient number of indexes are added to the nucleic acid fragments. This process assigns unique index or index combinations to each single cell or nucleus. After indexing is complete, e.g., after one, two, three, or more indexes are added, the isolated nuclei or cells can be lysed. In some embodiments, adding an index and lysing can occur simultaneously.

The number of nuclei or cells present in a subset, and therefore in each compartment, can be at least 1. In one embodiment, the number of nuclei or cells present in a subset is no greater than 100,000,000, no greater than 10,000,000, no greater than 1,000,000, no greater than 100,000, no greater than 10,000, no greater than 4,000, no greater than 3,000, no greater than 2,000, or no greater than 1,000, no greater than 500, or no greater than 50. In one embodiment, the number of nuclei or cells present in a subset can be 1 to 1,000, 1,000 to 10,000, 10,000 to 100,000, 100,000 to 1,000,000, 1,000,000 to 10,000,000, or 10,000,000 to 100,000,000. In one embodiment, the number of nuclei or cells present in each subset is approximately equal. The number of nuclei present in a subset, and therefor in each compartment, is based in part on the desire to reduce index collisions, which is the presence of two nuclei or cells having the same index combination ending up in the same compartment in this step of the method. Methods for distributing nuclei or cells into subsets are known to the person skilled in the art and are routine. While fluorescenceactivated cell sorting (FACS) cytometry can be used, use of simple dilution is preferred in some embodiments. In one embodiment, FACS cytometry is not used. Optionally, nuclei of different ploidies can be gated and enriched by staining, e.g., DAPI (4',6-diamidino-2-phenylindole) staining. Staining can also be used to discriminate single cells from doublets during sorting.

The number of compartments in the distribution steps (and subsequent addition of an index) can depend on the format used. For instance, the number of compartments can be from 2 to 96 compartments (when a 96-well plate is used), from 2 to 384 compartments (when a 384-well plate is used), or from 2 to 1536 compartments (when a 1536-well plate is used). In one embodiment, multiple plates can be used. In one embodiment, each compartment can be a droplet. When the type of compartment used is a droplet that contains two or more nuclei or cells, any number of droplets can be used, such as at least 10,000, at least 100,000, at least 1,000,000, or at least 10,000,000 droplets. Subsets of isolated nuclei or cells are typically indexed in compartments before pooling.

In some embodiments, the compartment is a droplet or well. The transcriptome, newly synthesized transcriptome, or subpopulation thereof of a cell or nucleus can be labeled with a unique index or index combination in a droplet or well. Indexed libraries derived from the droplet or well partitions can be pooled for further processing and sequencing. Examples of such methods include, but are not limited to, single cell analysis systems from 10X genomics (Pleasanton, CA), Biorad (Hercules, CA), and Cell See (Ann Arbor, MI).

Exposing to predetermined condition

In an optional embodiment, each subset of cells is exposed to an agent or perturbation (FIG. 1, block 12). An agent can be essentially anything that causes a change to a cell. For example, an agent can alter the transcriptome of a cell, alter the chromatin structure of a cell, alter the activity of a protein in the cell, alter the DNA of a cell, alter the methylation state, alter the DNA editing of a cell, or cause other changes. Examples of agents include, but are not limited to, a compound such as a protein (including an antibody), a nonribosomal protein, a polyketide, an organic molecule (including an organic molecule of 900 Daltons or less), an inorganic molecule, an RNA or RNAi molecule, a carbohydrate, a glycoprotein, a nucleic acid, or a combination thereof. In one embodiment, an agent causes a genetic perturbation, for instance a DNA editing protein such as CRISPR or Talen. In one embodiment, an agent is a therapeutic drug. In one embodiment, the cell can be a wild-type cell, and in another embodiment, the cell can be genetically modified to include a genetic perturbation, for instance, gene knock-in or gene knock-out (Szlachta et al., Nat Commun., 2018, 9:4275). Subsets of cells can be exposed to the same agent, but different variables can be altered across the compartments, permitting multiple variables to be tested in a single experiment. For instance, different dosages, different duration of exposure, and different cell types can be tested in a single multi-well plate. In one embodiment, the cells can express a protein having a known activity, and the effect of an agent on the activity evaluated under different conditions. The use of index sequences to label nucleic acid fragments permits later identification of the nucleic acids originating from a specific subset of nuclei or cells, e.g., from one well of a multi-well plate.

Labeling Nucleic Acids

In an optional embodiment, nucleic acids such as RNA, cDNA, or DNA, produced by a cell are labeled (FIG. 1, block 13). Current methods for single cell genomic techniques capture a snapshot of cell state, thus do not provide information on cell transition dynamics. The inventors have found that labeling newly synthesized RNA allows capture of both the whole transcriptome and the newly synthesized transcriptome at single cell level using split and pool indexing, combinatorial indexing, or any single cell indexing method. The whole transcriptome and newly synthesized RNA receive the same unique index or index combination allowing the current state (e.g., pre-existing) and newly synthesized nucleic acids to be assigned to the same cell. This allows the characterization of cell state transition dynamics regulated by intrinsic (e.g., a cell's intrinsic cell cycle program) and extrinsic (e.g., a cell's response to an external stimulus such as a therapeutic drug) factors. Additionally, in some embodiments capture of both whole transcriptome and newly synthesized transcriptome at single cell level is enabled, together with the degraded transcriptome information from its past state (past state memory). The past state memory of each cell can be corrected by mRNA degradation rate (memory correction), such that each cell can be characterized by transcriptome dynamics between two or more time points.

Various methods exist for labeling newly synthesized nucleic acid so it can be distinguished from previously existing nucleic acid, and essentially any method can be used. Typically, a label is incorporated into the nucleic acids as they are synthesized. One type of method includes incorporation of a nucleoside analog that adds an identifiable mutation. For instance, addition of the nucleoside analog 4-thiouridine (S4U) into a RNA molecule results in a point mutation during a reverse transcription step to result in mutated first strand cDNA having thymine-to-cytosine conversions (Sun and Chen, 2018, Metabolic Labeling of Newly Synthesized RNA with 4sU to in Parallel Assess RNA Transcription and Decay. In: Lamandé S. (eds) mRNA Decay. Methods in Molecular Biology, vol. 1720. Humana Press, New York, NY). This point mutation can be identified during the sequencing and analysis stages by comparison of the sequence with a reference. Another type of method includes incorporation of a hapten-labeled nucleotide that can be used to purify those RNAs containing the hapten. Examples include biotinylated nucleotides (Luo et al., 2011, Nucl. Acids Res., 39(19):8559-8571) and digoxigenin-modified nucleotides (available from Jena Bioscience GmbH). A third type of method includes incorporation of a nucleotide that can be modified with a chemical reaction, e.g., a click-functionalized nucleotide, and adding a hapten (Bharmal et al., 2010, J Biomol Tech., 21(3 Suppl):S43, and available from Jena Bioscience GmbH and available from Thermo Fisher Scientific). Another type of method includes incorporation of a mutagenic nucleotide such as, but not limited to, 8-oxo-dGTP and dPTP (available from Jena Bioscience GmbH).

Predetermined conditions are typically used on a cell and not isolated nuclei; however, the labeling of nucleic acid as it is synthesized can be done using cells or nuclei isolated from the cells.

In some embodiments, the labeling can include newly synthesized cDNA or DNA. Labeling can be used as an identifier for a specific condition or subset of cells or nuclei. For example, different amounts of label, e.g., nucleoside analog, hapten-labeled nucleotide, click-functionalized nucleotide, and/or mutagenic nucleotide and/or different ratios between labels can be used to specifically label the RNA, cDNA or DNA of a compartment. In another embodiment, a label can be added at different time points to capture the time dimension. Different labels or different ratios of labels can be added to differentially label RNA at different times. In some embodiments, the labeling can be part of the indexing scheme to resolve individual cells. For example, an extension step can contain a unique set of nucleotides for each compartment. Labeling can occur in a reverse transcription step, extension step, hybridization, or amplification step like PCR. In some embodiments, this allows the detection of doublets or multiples of cells or collisions between cells.

Processing to yield nucleic acid fragments

In one embodiment, processing isolated nuclei or cells can be used to fragment DNA nucleic acids in isolated nuclei or cells into nucleic acid fragments (FIG. 1, block 14). Fragmenting nucleic acids can be useful to obtain molecules having lengths that are suitable for sequencing with methods described herein. Processing can be necessary when the target nucleic acids to be sequenced are derived from DNA present in the nuclei or cells; however, in some embodiments processing is optional when the target nucleic acids to be sequenced are derived from RNA (e.g., mRNA and/or non-coding RNA) present in the nuclei or cells, because in some embodiments RNA molecules do not need to be fragmented. In other embodiments, nucleic acids derived from RNA molecules are fragmented. Fragmentation can occur at any stage of the method. For instance, the illustrative method shown in Figure 2 includes fragmentation after the addition of two indexes to nucleic acid molecules.

Processing nucleic acids in nuclei or cells typically adds a nucleotide sequence to one or both ends of the nucleic acid fragments generated by the processing, and the nucleotide sequence can, and typically does, include one or more universal sequences. A universal sequence can be used as, for instance, a "landing pad" in a subsequent step to anneal a nucleotide sequence that can be used as a primer for addition of another nucleotide sequence, such as an index, to a nucleic acid fragment. The nucleotide sequence of such a primer can optionally include an index sequence. Processing nucleic acids in nuclei or cells can add one or more unique molecular identifiers to one or both ends of the nuclei acid fragments generated by the processing.

Various methods for processing nucleic acids in nuclei or cells into nucleic acid fragments are known. Examples include CRISPR and Talen-like enzymes, and enzymes that unwind DNA (e.g. Helicases) that can make single stranded regions to which DNA fragments can hybridize and initiate extension or amplification. For example, helicase-based amplification can be used (Vincent et al., 2004, EMBO Rep., 5(8):795-800). In one embodiment, the extension or amplification is initiated with a random primer. In one embodiment, a transposome complex is used.

The transposome complex is a transposase bound to a transposase recognition site and can insert the transposase recognition site into a target nucleic acid within a nucleus in a process sometimes termed "tagmentation." In some such insertion events, one strand of the transposase recognition site may be transferred into the target nucleic acid. Such a strand is referred to as a "transferred strand." In one embodiment, a transposome complex includes a dimeric transposase having two subunits, and two non-contiguous transposon sequences. In another embodiment, a transposase includes a dimeric transposase having two subunits, and a contiguous transposon sequence. In one embodiment, the 5' end of one or both strands of the transposase recognition site may be phosphorylated.

Some embodiments can include the use of a hyperactive Tn5 transposase and a Tn5-type transposase recognition site (Gory shin and Reznikoff, J. Biol. Chem., 273:7367 (1998)), or MuA transposase and a Mu transposase recognition site comprising R1 and R2 end sequences (Mizuuchi, K., Cell, 35: 785, 1983; Savilahti, H, et al., EMBO J., 14: 4893, 1995). Tn5 Mosaic End (ME) sequences can also be used as optimized by a skilled artisan.

More examples of transposition systems that can be used with certain embodiments of the compositions and methods provided herein include *Staphylococcus aureus* Tn552 (Colegio et al., J. Bacteriol., 183: 2384-8, 2001; Kirby C et al., Mol. Microbiol., 43: 173-86, 2002), Ty1 (Devine & Boeke, Nucleic Acids Res., 22: 3765-72, 1994 and International Publication WO 95/23875), Transposon Tn7 (Craig, NL, Science. 271: 1512, 1996; Craig, NL, Review in: Curr Top Microbiol Immunol., 204:27-48, 1996), Tn/O and IS10 (Kleckner N, et al., Curr Top Microbiol Immunol., 204:49-82, 1996), Mariner transposase (Lampe D J, et al., EMBO J., 15: 5470-9, 1996), Tc1 (Plasterk R H, Curr. Topics Microbiol. Immunol., 204: 125-43, 1996), P Element (Gloor, GB, Methods Mol. Biol., 260: 97-114, 2004), Tn3 (Ichikawa & Ohtsubo, J Biol. Chem. 265:18829-32, 1990), bacterial insertion sequences (Ohtsubo & Sekine, Curr. Top. Microbiol. Immunol. 204: 1-26, 1996), retroviruses (Brown, et al., Proc Natl Acad Sci USA, 86:2525-9, 1989), and retrotransposon of yeast (Boeke & Corces, Annu Rev Microbiol. 43:403-34, 1989). More examples include IS5, Tn10, Tn903, IS911, and engineered versions of transposase family enzymes (Zhang et al., (2009) PLoS Genet. 5:e1000689. Epub 2009 Oct 16; Wilson C. et al (2007) J. Microbiol. Methods 71:332-5).

Other examples of integrases that may be used with the methods and compositions provided herein include retroviral integrases and integrase recognition sequences for such retroviral integrases, such as integrases from HIV-1, HIV-2, SIV, PFV-1, RSV.

Transposon sequences useful with the methods and compositions described herein are provided in U.S. Patent Application Pub. No. 2012/0208705, U.S. Patent Application Pub. No. 2012/0208724 and Int. Patent Application Pub. No. WO 2012/061832. In some embodiments, a transposon sequence includes a first transposase recognition site and a second transposase recognition site. In those embodiments where a transposome complex is used to introduce an index sequence, the index sequence can be present between the transposase recognition sites or in the transposon.

Some transposome complexes useful herein include a transposase having two transposon sequences. In some such embodiments, the two transposon sequences are not linked to one another, in other words, the transposon sequences are non-contiguous with one another. Examples of such transposomes are known in the art (see, for instance, U.S. Patent Application Pub. No. 2010/0120098).

Typically, tagmentation is used to produce nucleic acid fragments that include different nucleotide sequences at each end (e.g., an N5 primer sequence at one end and an N7 primer at the other end). This can be accomplished by using two types of transposome complexes, where each transposome complex includes a different nucleotide sequence that is part of the transferred strand. In some embodiments, tagmentation used herein inserts one nucleotide sequence into the nucleic acid fragments. Insertion of the nucleotide sequence results in nucleic acid fragments having a hairpin ligation duplex at one end and the transposome complex-inserted nucleotide sequence at the other end. The transposome complex-inserted nucleotide sequence includes a universal sequence. The universal sequence serves as a complementary sequence for hybridization in the amplification step described herein to introduce another index.

In some embodiments, a transposome complex includes a transposon sequence nucleic acid that binds two transposase subunits to form a "looped complex" or a "looped transposome." In one example, a transposome includes a dimeric transposase and a transposon sequence. Looped complexes can ensure that transposons are inserted into target DNA while maintaining ordering information of the original target DNA and without fragmenting the target DNA. As will be appreciated, looped structures may insert desired nucleic acid sequences, such as indexes, into a target nucleic acid, while maintaining physical connectivity of the target nucleic acid. In some embodiments, the transposon sequence of a looped transposome complex can include a fragmentation site such that the transposon sequence can be fragmented to create a transposome complex comprising two transposon sequences. Such transposome complexes are useful to ensuring that neighboring target DNA fragments, in which the transposons insert, receive barcode combinations that can be unambiguously assembled at a later stage of the assay.

In one embodiment, fragmenting nucleic acids is accomplished by using a fragmentation site present in the nucleic acids. Typically, fragmentation sites are introduced into target nucleic acids by using a transposome complex. In one embodiment, after nucleic acids are fragmented the transposase remains attached to the nucleic acid fragments, such that nucleic acid fragments derived from the same genomic DNA molecule remain physically linked (Adey et al., 2014, Genome Res., 24:2041-2049). For instance, a looped transposome complex can include a fragmentation site. A fragmentation site can be used to cleave the physical, but not the informational association between index sequences that have been inserted into a target nucleic acid. Cleavage may be by biochemical, chemical or other means. In some embodiments, a fragmentation site can include a nucleotide or nucleotide sequence that may be fragmented by various means. Examples of fragmentation sites include, but are not limited to, a restriction endonuclease site, at least one ribonucleotide cleavable with an RNAse, nucleotide analogues cleavable in the presence of a certain chemical agent, a diol linkage cleavable by treatment with periodate, a disulfide group cleavable with a chemical reducing agent, a cleavable moiety that may be subject to photochemical cleavage, and a peptide cleavable by a peptidase enzyme or other suitable means (see, for instance, U.S. Patent Application Pub. No. 2012/0208705, U.S. Patent Application Pub. No. 2012/0208724 and WO 2012/061832).

A transposome complex can optionally include an index sequence, also referred to as a transposase index. The index sequence is present as part of the transposon sequence. In one embodiment, the index sequence can be present on a transferred strand, the strand of the transposase recognition site that is transferred into the target nucleic acid.

Tagmentation of the nuclei and processing of the nuclei acid fragments can be followed by a clean-up process to enhance the purity of the molecules. Any suitable clean-up process may be used, such as electrophoresis, size exclusion chromatography, or the like. In some embodiments, solid phase reversible immobilization paramagnetic beads may be employed to separate the desired DNA molecules from, for instance, unincorporated primers, and to select nucleic acids based on size. Solid phase reversible immobilization paramagnetic beads are commercially available from Beckman Coulter (Agencourt AMPure XP), Thermofisher (MagJet), Omega Biotek (Mag-Bind), Promega Beads (Promega), and Kapa Biosystems (Kapa Pure Beads).

Adding a compartment specific index

An index sequence, also referred to as a tag or barcode, is useful as a marker characteristic of the compartment in which a particular nucleic acid was present. Accordingly, an index is a nucleic acid sequence tag which is attached to each of the target nucleic acids present in a particular compartment, the presence of which is indicative of, or is used to identify, the compartment in which a population of isolated nuclei or cells were present at a particular stage of the method. Addition of an index to nucleic acid fragments is accomplished with subsets of isolated nuclei or cells distributed to different compartments (FIG. 1, block 15; FIG. 2, blocks 24, 26, and 30; FIG. 3, block 33 and 37).

An index sequence can be any suitable number of nucleotides in length, e.g., 1, 2, 3, 4, 5, 6, 7, 8, 9, 10, 11, 12, 13, 14, 15, 16, 17, 18, 19, 20, or more. A four nucleotide tag gives a possibility of multiplexing 256 samples on the same array, and a six base tag enables 4096 samples to be processed on the same array.

In one embodiment, addition of an index is achieved during the processing of nucleic acids into nucleic acid fragments. For instance, a transposome complex that includes an index can be used. In some embodiments, an index is added after nucleic acid fragments containing a nucleotide sequence at one or both ends are generated by the processing. In other embodiments, processing is not needed to add an index. For instance, an index can be added directly to RNA nucleic acids without fragmenting the RNA nucleic acids. Accordingly, reference to "nucleic acid fragment" includes nucleic acids that result from processing and RNA nucleic acids, and the nucleic acids derived from these nucleic acids.

Methods for adding an index include, but are not limited to, ligation, extension (including extension using reverse transcriptase), hybridization, adsorption, specific or non-specific interactions of a primer, amplification, or transposition. The nucleotide sequence that is added to one or both ends of the nucleic acid fragments can also include one or more universal sequences and/or unique molecular identifiers. A universal sequence can be used as, for instance, a "landing pad" in a subsequent step to anneal a nucleotide sequence that can be used as a primer for addition of another nucleotide sequence, such as another index and/or another universal sequence, to a nucleic acid fragment. Thus, the incorporation of an index sequence can use a process that includes one, two, or more steps, using essentially any combination of ligation, extension, hybridization, adsorption, specific or non-specific interactions of a primer, amplification, or transposition.

For instance, in embodiments that include use of nucleic acid fragments that are derived from mRNA, various methods can be used to add an index to mRNA in one or two steps. For example, an index can be added using the types of methods used to produce cDNA. A primer with a poly-T sequence at the 3' end can be annealed to mRNA molecules and extended using a reverse transcriptase. Exposing the isolated nuclei or cells to these components under conditions suitable for reverse transcription results in a one step addition of the index to result in a population of indexed nuclei or cells, where each nucleus or cell contains indexed nucleic acid fragments. Alternatively, the primer with a poly-T sequence includes a universal sequence instead of an index, and the index is added by a subsequent step of ligation, primer extension, amplification, hybridization, or a combination thereof. In some embodiments, the barcode is added without the use of a universal sequence. The indexed nucleic acid fragments can, and typically do, include on the synthesized strand the index sequence indicative of the particular compartment.

In embodiments that include use of nucleic acid fragments derived from non-coding RNA, various methods can be used to add an index to the non-coding RNA in one or two steps. For example, an index can be added using a first primer that includes a random sequence and a template-switch primer, where either primer can include an index. A reverse transcriptase having a terminal transferase activity to result in addition of non-template nucleotides to the 3' end of the synthesized strand can be used, and the template-switch primer includes nucleotides that anneal with the non-template nucleotides added by the reverse transcriptase. An example of a useful reverse transcriptase enzyme is a Moloney murine leukemia virus reverse transcriptase. In a particular embodiment, the SMARTer™ reagent available from Takara Bio USA, Inc. (Cat. No. 634926) is used for the use of template-switching to add an index to non-coding RNA, and mRNA if desired. Alternatively, the first primer and/or the template-switch primer can include a universal sequence instead of an index, and the index is added by a subsequent step of ligation, primer extension, amplification, hybridization, or a combination thereof. The indexed nucleic acid fragments can, and typically do, include on the synthesized strand the index sequence indicative of the particular compartment. Other embodiments include 5' or 3' profiling of RNA or full-length RNA profiling.

In another embodiment, specific mRNA and/or non-coding RNA can be targeted for amplification. Targeting permits production of sequencing libraries enriched for sequences that are more likely to yield useful information, result in a large reduction in the sequencing depth and the associated costs, and increase the power to detect subtle differences between cells. RNA molecules including one or more mRNA and/or one or more non-coding RNA can be selected as likely to yield useful information, and primers can be used to selectively anneal to the predetermined RNA nucleic acids and amplify a subpopulation of the total RNA molecules present in a cell or nucleus. The skilled person will recognize that the appropriate RNA molecules to select depends on the experiment. For instance, in the evaluation of noncoding perturbations, only coding regions *cis* to the regulatory element being disrupted can be tested for changes in expression. This approach may reduce background of ribosomal reads more than the use of random hexamer or poly-T primers. This approach also permits targeting splice junctions and exons resulting from alternative transcription start site events, thus providing isoform information not readily detected with conventional sci methods.

The targeted amplification of RNA molecules can occur at several steps during library production. In one embodiment, targeted amplification of multiple targets occurs during the reverse transcription of RNA molecules. An experiment can include multiple different primers targeting different RNA molecules. In one embodiment, multiple primers targeting different regions of the same RNA molecule can be used. The use of multiple primers directed to different regions of the same RNA molecule allows multiple opportunities for the RNA molecule to be reverse transcribed into cDNA, increasing the likelihood of detection of the RNA molecule.

In one embodiment, the primers used for targeted amplification do not include an index. When an index is not being added during the amplification reaction the distribution of cells or nuclei into different compartments is not necessary, and the amplification can occur as a single reaction with all RNA molecules and all primers present. In embodiments where an index is being added during the amplification reaction the distribution of the cells or nuclei is useful, and the amplification can occur as a single reaction in each compartment with all RNA molecules and all primers present, but each primer in a compartment having the same compartment specific index.

In one embodiment, the design of primers for multiplex target capture can include one or more of the following considerations. After a RNA is selected for targeted amplification the sequence of the RNA can be collected and all possible reverse transcriptase primers - the candidate primers - determined. The length of any primer should be long enough to function in a reverse transcription reaction and can be, for instance, between 20 and 30 nucleotides in length.

The candidate primers can be filtered by various criteria, including, but not limited to, GC content, location of GC bases in the primer, likelihood of offsite targeting, and mappability. A useful GC content is from 40-60%, corresponding to melting temperatures that are roughly between 55 and 70°C. It is preferred to have two guanine or cytosine bases in the last 5 nucleotides of the 3' end of the primer to increase the likelihood that the annealed primer will be a good substrate for extension by the reverse transcriptase enzyme.

Regarding the likelihood of off target priming, the inventors found that while the target RNAs were highly enriched, a large fraction of reads were still derived from other RNAs that were abundant within cells. Most of these off target priming events were the result of approximately 5 to 8 base pairs of complementarity between the 3' end of the primer and the off target RNA. The inventors found it useful to consider the abundance of the final hexamer of the candidate primer within total cellular RNA. It was determined that useful primers included a last hexamer that was either (i) not present within ribosomal RNA or (ii) represented at a low level within total cellular RNA.

Examples of hexamers not present within ribosomal RNA are described (the 'Not So Random' or NSR hexamers of Armour et al., 2009, Nature Methods, 6(9):647-49). Primers having this characteristic were found to be much less likely to have off target priming within ribosomal RNA. One method to determine whether a hexamer is represented at a low level within total cellular RNA can include identifying the abundance of each hexamer in RNA molecules within a cell, for instance all nascent transcription, including ribosomal transcription, within the type of cell to be analyzed according to the methods described herein. The use of candidate primers that are at a low level of abundance, e.g., within the lowest quartile of abundance, can reduce off-site targeting.

Candidate primers can also be evaluated by mappability. For instance, each candidate can be aligned to the targets using a bowtie-type of algorithm, and allowing 3 mismatches. This step helps to ensure that each primer will have only one target site in the genome.

In some embodiments, amplification of multiple targets in the same reaction, also referred to as multiplex target capture, control of annealing temperatures of reverse transcriptase primers is helpful in maintaining specific reverse transcription and amplification of the desired target RNAs. For instance, typical reverse transcription protocols denature a mixture of RNA and reverse transcription primer and cool to 4°C to allow annealing. A low annealing temperature is too permissive and results in undesirable off target annealing events. To increase the likelihood that the only annealing events that extend are those where the entire targeted reverse transcription primers are annealed to the correct targets, a high temperature is maintained during the entire process of reverse transcription. In one embodiment, the components - e.g., mixture of fixed cells, reverse transcription primer pool, and dNTPs - at 65°C, anneal at 53°C, add a reverse transcription enzyme/buffer mixture that is pre-equilibrated at 53°C to the annealing reaction, and extend at 53°C for 20 minutes. Thus, the possibility of the reverse transcription primers to anneal at a low temperature between the denaturing and extension steps is reduced. The skilled person will recognize that modifications can be made somewhat, for instance altering the temperature or time, without reducing the specificity of the reverse transcription.

Other methods can be used for the addition of an index to a nucleic acid fragment, and how an index is added is not intended to be limiting. For instance, in one embodiment the incorporation of an index sequence includes ligating a primer to one or both ends of the nucleic acid fragments. The ligation of the ligation primer can be aided by the presence of a universal sequence at the ends of the nucleic acid fragments. An example of a primer is a hairpin ligation duplex. The ligation duplex can be ligated to one end or preferably both ends of nucleic acid fragments.

In another embodiment the incorporation of an index sequence includes use of single stranded nucleic acid fragments and synthesis of the second DNA strand. In one embodiment, the second DNA strand is produced using a primer that includes sequences complementary to nucleotides present at the ends of the single stranded nucleic acid fragments.

In another embodiment, the incorporation of an index occurs in one, two, three, or more rounds of split and pool barcoding resulting in single, dual, triple, or multiple (e.g., four or more) indexed single cell libraries.

In another embodiment, the incorporation of indices and amplification mediator (e.g., a universal sequence) is beneficial, allowing targeted single cell sequencing libraries and/or targeted single cell sequencing libraries to be prepared.

Addition of universal sequences for immobilization

In one embodiment, the addition of nucleotides during the processing and/or indexing steps add universal sequences useful in the immobilizing and sequencing the fragments. In another embodiment, the indexed nucleic acid fragments can be further processed to add universal sequences useful in immobilizing and sequencing the nucleic acid fragments. The skilled person will recognize that in embodiments where the compartment is a droplet sequences for immobilizing nucleic acid fragments are optional. In one embodiment, the incorporation of universal sequences useful in immobilizing and sequencing the fragments includes ligating identical universal adapters (also referred to as 'mismatched adaptors,' the general features of which are described in Gormley et al., US 7,741,463, and Bignell et al., US 8,053,192,) to the 5' and 3' ends of the indexed nucleic acid fragments. In one embodiment, the universal adaptor includes all sequences necessary for sequencing, including sequences for immobilizing the indexed nucleic acid fragments on an array.

In one embodiment, blunt-ended ligation can be used. In another embodiment, the nucleic acid fragments are prepared with single overhanging nucleotides by, for example, activity of certain types of DNA polymerase such as Taq polymerase or Klenow exo minus polymerase which has a non-template-dependent terminal transferase activity that adds one or more deoxynucleotides, for example, deoxyadenosine (A) to the 3' ends of the indexed nucleic acid fragments. In some cases, the overhanging nucleotide is more than one base. Such enzymes can be used to add a single nucleotide 'A' to the blunt ended 3' terminus of each strand of the nucleic acid fragments. Thus, an 'A' could be added to the 3' terminus of each strand of the double-stranded target fragments by reaction with Taq or Klenow exo minus polymerase, while the additional sequences to be added to each end of the nucleic acid fragment can include a compatible 'T' overhang present on the 3' terminus of each region of double stranded nucleic acid to be added. This end modification also prevents self-ligation of the nucleic acids such that there is a bias towards formation of the indexed nucleic acid fragments flanked by the sequences that are added in this embodiment.

In another embodiment, when the universal adapter ligated to the indexed nucleic acid fragments does not include all sequences necessary for sequencing, then an amplification step, such as PCR, can be used to further modify the universal adapters present in each indexed nucleic acid fragment prior to immobilizing and sequencing. For instance, an initial primer extension reaction can be carried out using a universal anchor sequence complementary to a universal sequence present in the indexed nucleic acid fragment, in which extension products complementary to both strands of each individual indexed nucleic acid fragment are formed. Typically, the PCR adds additional universal sequences, such as a universal capture sequence.

After the universal adapters are added, either by a single step method of ligating or hybridizating a universal adaptor including all sequences necessary for sequencing, or by a two-step method of ligating a universal adapter and then an amplification to further modify the universal adapter, the final index fragments will include a universal capture sequence and an anchor sequence. The result of adding universal adapters to each end is a plurality or library of indexed nucleic acid fragments.

The resulting indexed fragments collectively provide a library of nucleic acids that can be immobilized and then sequenced. The term library, also referred to herein as a sequencing library, refers to the collection of nucleic acid fragments from single nuclei or cells containing known universal sequences at their 3' and 5' ends. The library includes nucleic acids from the whole transcriptome, nucleic acids from newly synthesized RNA molecules, or a combination of both, and can be used to perform sequencing of the whole transcriptome, the transcriptome of the newly synthesized RNA, or a combination of both.

The indexed nucleic acid fragments can be subjected to conditions that select for a predetermined size range, such as from 150 to 400 nucleotides in length, such as from 150 to 300 nucleotides. The resulting indexed nucleic acid fragments are pooled, and optionally can be subjected to a clean-up process to enhance the purity to the DNA molecules by removing at least a portion of unincorporated universal adapters or primers. Any suitable clean-up process may be used, such as electrophoresis, size exclusion chromatography, or the like. In some embodiments, solid phase reversible immobilization paramagnetic beads may be employed to separate the desired DNA molecules from unattached universal adapters or primers, and to select nucleic acids based on size. Solid phase reversible immobilization paramagnetic beads are commercially available from Beckman Coulter (Agencourt AMPure XP), Thermofisher (MagJet), Omega Biotek (Mag-Bind), Promega Beads (Promega), and Kapa Biosystems (Kapa Pure Beads).

A non-limiting illustrative embodiment of the present disclosure is shown in FIG. 1. In this embodiment, the method includes providing a plurality of cells (FIG. 1, block 10). The method further includes distributing subsets of cells into a plurality of compartments (FIG. 1, block 11), and exposing the cells to a predetermined condition (FIG. 1, block 12). The predetermined conditions can, and typically do, vary between different compartments. For instance, different compartments can include different dosages of an agent, different perturbations, different duration of exposure, different cell types, and the like. Newly synthesized RNA is then labeled (FIG. 1, block 13). The labeling can occur with cells, or nuclei can be isolated from the cells and the labeling occur with nuclei. In some embodiments the cellular RNA is processed to result in fragments (FIG. 1, block 14). RNA present in the cells or nuclei is then indexed (FIG. 1, block 15). Various embodiments for indexing RNA are possible. For instance, in one embodiment all mRNA present in a cell are indexed by using a primer that includes a poly-T region. In another embodiment, specific RNA nucleic acids can be indexed.

Another non-limiting illustrative embodiment of the present disclosure is shown in FIG. 2 and described in Example 1. In this embodiment, the method includes providing isolated nuclei from a plurality of cells (FIG. 2, block 22). The method further includes distributing subsets of the isolated nuclei into a first plurality of compartments (FIG. 2, block 23). The number of compartments in the first distribution step (FIG. 2, block 23) can depend on the format used. For instance, the number of compartments can be from 2 to 96 compartments (when a 96-well plate is used), from 2 to 384 compartments (when a 384-well plate is used), or from 2 to 1536 compartments (when a 1536-well plate is used). Alternatively, other compartments can be used, such as droplets.

The method also includes generating indexed nuclei (FIG. 2, block 24). In one embodiment, generating indexed nuclei includes the use of reverse transcriptase with an oligo-dT primer to add an index, a random nucleotide sequence, and a universal sequence. The index in each compartment is unique, e.g., each index is compartment specific. The random sequence is used as a unique molecular identifier (UMI) to label unique nuclei acid fragments. The random sequence can also be used to aid in removal of duplicates in downstream processing. The universal sequence serves as a complementary sequence for hybridization in the ligation step described herein. In another embodiment, generating indexed nuclei includes the use of reverse transcriptase with specific primers to target predetermined RNA molecules. The reverse transcription can result in the addition of an index, a random nucleotide sequence, and a universal sequence to the targeted RNA molecules. Exposing the nuclei to these components under conditions suitable for reverse transcription results in a population of indexed nuclei, where each nucleus contains indexed nucleic acid fragments. The indexed nucleic acid fragments can, and typically do, include on the synthesized strand the index sequence indicative of the particular compartment. An example of an indexed nucleic acid fragment is shown in FIG. 1A of Example 1 (see "Indexed reverse transcription").

The indexed nuclei from multiple compartments can be combined (FIG. 2, block 25). Subsets of these combined indexed nuclei, referred to herein as pooled indexed nuclei, are then distributed into a second plurality of compartments (FIG. 2, block 25). Distribution of nuclei into subsets is followed by incorporating into the indexed nucleic acid fragments in each compartment a second index sequence to generate dual-indexed fragments. This results in the further indexing of the indexed nucleic acid fragments (FIG. 2, block 26).

In this illustrative embodiment, the incorporation of the second index sequence includes ligating a hairpin ligation duplex to the indexed nucleic acid fragments in each compartment. The use of hairpin ligation duplex to introduce a universal sequence, an index, or a combination thereof, to the end of a target nucleic acid fragment typically uses one end of the duplex as a primer for a subsequent amplification. In contrast, a hairpin ligation duplex used in this embodiment does not act as a primer. An advantage of using a hairpin ligation duplex described herein is a reduction of the self-self ligation observed with many hairpin ligation duplexes described in the art. In one embodiment, the ligation duplex includes five elements: 1) a universal sequence that is a complement of the universal sequence present on the oligo-dT primer, 2) a second index, 3) an ideoxyU, 4) a nucleotide sequence that can form a hairpin, and 5) the reverse complement of the second index. The second index sequences are unique for each compartment in which the distributed indexed nuclei were placed (FIG. 2, block 25) after the first index was added by reverse transcription. An example of a dual-indexed nucleic acid fragment is shown in FIG. 1A of Example 1 (see "Indexed hairpin ligation").

Removal of the ideoxyU present in the hairpin region of the hairpin ligation duplex incorporated into the nucleic acid fragments can occur before, during, or after clean-up. Removal of the uracil residue can be accomplished by any available method, and in cone embodiment the Uracil-Specific Excision Reagent (USER) available from NEB is used.

Subsets of these combined dual-indexed nuclei, referred to herein as pooled dual-indexed nuclei, are then distributed into a third plurality of compartments (FIG. 2, block 27). In one embodiment, 100 to 30,000 nuclei are distributed to each well. In one embodiment, the number of nuclei in a well is at least 100, at least 500, at least 1,000, or at least 5,000. In one embodiment, the number of nuclei in a well is no greater than 30,000, no greater than 25,000, no greater than 20,000, or no greater than 15,000. In one embodiment, the number of nuclei present in a subset can be 100 to 1,000, 1,000 to 10,000, 10,000 to 20,000, or 20,000 to 30,000._ In one embodiment, 2,500 nuclei are distributed to each well. In one embodiment, the number of nuclei present in each subset is approximately equal.

Distribution of dual-indexed nuclei into subsets is followed by synthesis of the second DNA strand (FIG. 2, block 28). The nucleic acids in the nuclei are processed by subjecting them to tagmentation (FIG. 2, block 29). Each compartment containing the dual-indexed nuclei includes a transposome complex. In this embodiment, tagmentation is used to produce nucleic acid fragments that include different nucleotide sequences at each end (e.g., an N5 primer sequence at one end and an N7 primer at the other end).

Tagmentation of nuclei is followed by incorporating into the dual-indexed nucleic acid fragments in each compartment a third index sequence to generate triple-indexed fragments, where the third index sequence in each compartment is different from first and second index sequences in the compartments. This results in the further indexing of the indexed nucleic acid fragments (FIG. 2, block 30; see also FIG. 1A of the Example ("USER treatment, Indexed PCR")) prior to immobilizing and sequencing. In one embodiment, the universal sequences present at ends of the dual-indexed nucleic acid fragments (e.g., the hairpin ligation duplex-inserted nucleotides sequence at one end and the transposome complex-inserted nucleotide sequence at the other end) can be used for the binding of primers and be extended in an amplification reaction. Typically, two different primers are used. One primer hybridizes with universal sequences at the 3' end of one strand of the dual-indexed nucleic acid fragments, and a second primer hybridizes with universal sequences at the 3' end of the other strand of the dual-indexed nucleic acid fragments. Thus, the anchor sequence (e.g., the site to which a universal primer such as a sequencing primer for read 1 or read 2 anneals for sequencing) present on each primer can be different. Suitable primers can each include additional universal sequences, such as a universal capture sequence (e.g., the site to which a capture oligonucleotide hybridizes, where the capture oligonucleotide can be immobilized on a surface of a solid substrate). Because each primer includes an index, this step results in the addition of another index sequence, one at each end of the nucleic acid fragments to result in triple-indexed fragments. In one embodiment, indexed primers, such as an indexed P5 primer and an indexed P7 primer, can be used to add the third index. The triple-indexed fragments are pooled and they can be subjected to a clean-up step as described herein.

The plurality of triple-indexed fragments can be prepared for sequencing. After the triple-indexed fragments are pooled and subjected to clean-up they are enriched, typically by immobilization and/or amplification, prior to sequencing (FIG. 2, block 31).

Another non-limiting illustrative embodiment of the present disclosure is shown in FIG. 3 and described in Example 4. In this embodiment, the method includes providing isolated nuclei or cells (FIG. 3, block 30). The nuclei or cells can be exposed to reverse transcriptase and specific primers to target and enrich predetermined RNA molecules (FIG. 3, block 31). Exposing the nuclei or cells to these components under conditions suitable for reverse transcription results in a population of nuclei or cells, where each nucleus contains nucleic acid fragments that are enriched for sequences present in the predetermined RNA molecules. The method further includes distributing subsets of the nuclei or cells into a first plurality of compartments (FIG. 3, block 32). The number of compartments in the first distribution step (FIG. 3, block 32) can depend on the format used. For instance, the number of compartments can be from 2 to 96 compartments (when a 96-well plate is used), from 2 to 384 compartments (when a 384-well plate is used), or from 2 to 1536 compartments (when a 1536-well plate is used). Alternatively, other compartments can be used, such as droplets.

The method also includes generating indexed nuclei or cells (FIG. 3, block 33) by incorporating into the nucleic acid fragments in each compartment an index sequence to generate indexed fragments.

In one embodiment, the incorporation of the index sequence includes ligating a hairpin ligation duplex to the indexed nucleic acid fragments in each compartment. The nuclei or cells containing the indexed fragments are pooled and subsets of these combined indexed nuclei or cells are then distributed into a second plurality of compartments (FIG. 3, block 34).

Distribution of indexed nuclei or cells into subsets can be followed by synthesis of the second DNA strand (FIG. 3, block 35). The nucleic acids in the nuclei or cells are processed by subjecting them to tagmentation (FIG. 3, block 36). Each compartment containing the indexed nuclei includes a transposome complex. In this embodiment, tagmentation is used to produce nucleic acid fragments that include different nucleotide sequences at each end (e.g., an N5 primer sequence at one end and an N7 primer at the other end).

Tagmentation of nuclei can be followed by incorporating into the indexed nucleic acid fragments in each compartment a second index sequence to generate dual-indexed fragments, where the second index sequence in each compartment is different from first index sequences in the compartments. This results in the further indexing of the indexed nucleic acid fragments (FIG. 3, block 37) prior to immobilizing and sequencing.

The plurality of dual-indexed fragments can be prepared for sequencing, where the sequencing data is enriched for sequences present in the predetermined RNA molecules. After the dual-indexed fragments are pooled and subjected to clean-up they are enriched, typically by immobilization and/or amplification, prior to sequencing (FIG. 3, block 38).

Preparation of Immobilized Samples for Sequencing

Methods for attaching indexed fragments from one or more sources to a substrate are known in the art. In one embodiment, indexed fragments are enriched using a plurality of capture oligonucleotides having specificity for the indexed fragments, and the capture oligonucleotides can be immobilized on a surface of a solid substrate. For instance, capture oligonucleotides can include a first member of a universal binding pair, and wherein a second member of the binding pair is immobilized on a surface of a solid substrate. Likewise, methods for amplifying immobilized dual-indexed fragments include, but are not limited to, bridge amplification and kinetic exclusion. Methods for immobilizing and amplifying prior to sequencing are described in, for instance, Bignell et al. (US 8,053,192), Gunderson et al. (WO2016/130704), Shen et al. (US 8,895,249), and Pipenburg et al. (US 9,309,502).

A pooled sample can be immobilized in preparation for sequencing. Sequencing can be performed as an array of single molecules or can be amplified prior to sequencing. The amplification can be carried out using one or more immobilized primers. The immobilized primer(s) can be, for instance, a lawn on a planar surface, or on a pool of beads. The pool of beads can be isolated into an emulsion with a single bead in each "compartment" of the emulsion. At a concentration of only one template per "compartment," only a single template is amplified on each bead.

The term "solid-phase amplification" as used herein refers to any nucleic acid amplification reaction carried out on or in association with a solid support such that all or a portion of the amplified products are immobilized on the solid support as they are formed. In particular, the term encompasses solid-phase polymerase chain reaction (solid-phase PCR) and solid phase isothermal amplification which are reactions analogous to standard solution phase amplification, except that one or both of the forward and reverse amplification primers is/are immobilized on the solid support. Solid phase PCR covers systems such as emulsions, wherein one primer is anchored to a bead and the other is in free solution, and colony formation in solid phase gel matrices wherein one primer is anchored to the surface, and one is in free solution.

In some embodiments, the solid support comprises a patterned surface. A "patterned surface" refers to an arrangement of different regions in or on an exposed layer of a solid support. For example, one or more of the regions can be features where one or more amplification primers are present. The features can be separated by interstitial regions where amplification primers are not present. In some embodiments, the pattern can be an x-y format of features that are in rows and columns. In some embodiments, the pattern can be a repeating arrangement of features and/or interstitial regions. In some embodiments, the pattern can be a random arrangement of features and/or interstitial regions. Exemplary patterned surfaces that can be used in the methods and compositions set forth herein are described in US Pat. Nos. 8,778,848, 8,778,849 and 9,079,148, and US Pub. No. 2014/0243224.

In some embodiments, the solid support includes an array of wells or depressions in a surface. This may be fabricated as is generally known in the art using a variety of techniques, including, but not limited to, photolithography, stamping techniques, molding techniques and microetching techniques. As will be appreciated by those in the art, the technique used will depend on the composition and shape of the array substrate.

The features in a patterned surface can be wells in an array of wells (e.g. microwells or nanowells) on glass, silicon, plastic or other suitable solid supports with patterned, covalently-linked gel such as poly(N-(5-azidoacetamidylpentyl)acrylamide-co-acrylamide) (PAZAM, see, for example, US Pub. No. 2013/184796, WO 2016/066586, and WO 2015/002813). The process creates gel pads used for sequencing that can be stable over sequencing runs with a large number of cycles. The covalent linking of the polymer to the wells is helpful for maintaining the gel in the structured features throughout the lifetime of the structured substrate during a variety of uses. However, in many embodiments the gel need not be covalently linked to the wells. For example, in some conditions silane free acrylamide (SFA, see, for example, US Pat. No. 8,563,477) which is not covalently attached to any part of the structured substrate, can be used as the gel material.

In particular embodiments, a structured substrate can be made by patterning a solid support material with wells (e.g. microwells or nanowells), coating the patterned support with a gel material (e.g. PAZAM, SFA or chemically modified variants thereof, such as the azidolyzed version of SFA (azido-SFA)) and polishing the gel coated support, for example via chemical or mechanical polishing, thereby retaining gel in the wells but removing or inactivating substantially all of the gel from the interstitial regions on the surface of the structured substrate between the wells. Primer nucleic acids can be attached to gel material. A solution of indexed fragments can then be contacted with the polished substrate such that individual indexed fragments will seed individual wells via interactions with primers attached to the gel material; however, the target nucleic acids will not occupy the interstitial regions due to absence or inactivity of the gel material. Amplification of the indexed fragments will be confined to the wells since absence or inactivity of gel in the interstitial regions prevents outward migration of the growing nucleic acid colony. The process can be conveniently manufactured, being scalable and utilizing conventional micro- or nanofabrication methods.

Although the disclosure encompasses "solid-phase" amplification methods in which only one amplification primer is immobilized (the other primer usually being present in free solution), in one embodiment it is preferred for the solid support to be provided with both the forward and the reverse primers immobilized. In practice, there will be a "plurality" of identical forward primers and/or a "plurality" of identical reverse primers immobilized on the solid support, since the amplification process requires an excess of primers to sustain amplification. References herein to forward and reverse primers are to be interpreted accordingly as encompassing a "plurality" of such primers unless the context indicates otherwise.

As will be appreciated by the skilled reader, any given amplification reaction requires at least one type of forward primer and at least one type of reverse primer specific for the template to be amplified. However, in certain embodiments the forward and reverse primers may include template-specific portions of identical sequence, and may have entirely identical nucleotide sequence and structure (including any non-nucleotide modifications). In other words, it is possible to carry out solid-phase amplification using only one type of primer, and such single-primer methods are encompassed within the scope of the disclosure. Other embodiments may use forward and reverse primers which contain identical template-specific sequences but which differ in some other structural features. For example, one type of primer may contain a non-nucleotide modification which is not present in the other.

In all embodiments of the disclosure, primers for solid-phase amplification are preferably immobilized by single point covalent attachment to the solid support at or near the 5' end of the primer, leaving the template-specific portion of the primer free to anneal to its cognate template and the 3' hydroxyl group free for primer extension. Any suitable covalent attachment means known in the art may be used for this purpose. The chosen attachment chemistry will depend on the nature of the solid support, and any derivatization or functionalization applied to it. The primer itself may include a moiety, which may be a non-nucleotide chemical modification, to facilitate attachment. In a particular embodiment, the primer may include a sulphur-containing nucleophile, such as phosphorothioate or thiophosphate, at the 5' end. In the case of solid-supported polyacrylamide hydrogels, this nucleophile will bind to a bromoacetamide group present in the hydrogel. A more particular means of attaching primers and templates to a solid support is via 5' phosphorothioate attachment to a hydrogel comprised of polymerized acrylamide and N-(5-bromoacetamidylpentyl) acrylamide (BRAPA), as described in WO 05/065814.

Certain embodiments of the disclosure may make use of solid supports that include an inert substrate or matrix (e.g. glass slides, polymer beads, etc.) which has been "functionalized," for example by application of a layer or coating of an intermediate material including reactive groups which permit covalent attachment to biomolecules, such as polynucleotides. Examples of such supports include, but are not limited to, polyacrylamide hydrogels supported on an inert substrate such as glass. In such embodiments, the biomolecules (e.g. polynucleotides) may be directly covalently attached to the intermediate material (e.g. the hydrogel), but the intermediate material may itself be non-covalently attached to the substrate or matrix (e.g. the glass substrate). The term "covalent attachment to a solid support" is to be interpreted accordingly as encompassing this type of arrangement.

The pooled samples may be amplified on beads wherein each bead contains a forward and reverse amplification primer. In a particular embodiment, the library of indexed fragments is used to prepare clustered arrays of nucleic acid colonies, analogous to those described in U.S. Pub. No. 2005/0100900, U.S. Pat. No. 7,115,400, WO 00/18957 and WO 98/44151 by solid-phase amplification and more particularly solid phase isothermal amplification. The terms 'cluster' and 'colony' are used interchangeably herein to refer to a discrete site on a solid support including a plurality of identical immobilized nucleic acid strands and a plurality of identical immobilized complementary nucleic acid strands. The term "clustered array" refers to an array formed from such clusters or colonies. In this context, the term "array" is not to be understood as requiring an ordered arrangement of clusters.

The term "solid phase" or "surface" is used to mean either a planar array wherein primers are attached to a flat surface, for example, glass, silica or plastic microscope slides or similar flow cell devices; beads, wherein either one or two primers are attached to the beads and the beads are amplified; or an array of beads on a surface after the beads have been amplified.

Clustered arrays can be prepared using either a process of thermocycling, as described in WO 98/44151, or a process whereby the temperature is maintained as a constant, and the cycles of extension and denaturing are performed using changes of reagents. Such isothermal amplification methods are described in patent application numbers WO 02/46456 and U.S. Pub. No. 2008/0009420. Due to the lower temperatures useful in the isothermal process, this is particularly preferred in some embodiments.

It will be appreciated that any of the amplification methodologies described herein or generally known in the art may be used with universal or target-specific primers to amplify immobilized DNA fragments. Suitable methods for amplification include, but are not limited to, the polymerase chain reaction (PCR), strand displacement amplification (SDA), transcription mediated amplification (TMA) and nucleic acid sequence based amplification (NASBA), as described in U.S. Pat. No. 8,003,354. The above amplification methods may be employed to amplify one or more nucleic acids of interest. For example, PCR, including multiplex PCR, SDA, TMA, NASBA and the like may be utilized to amplify immobilized DNA fragments. In some embodiments, primers directed specifically to the polynucleotide of interest are included in the amplification reaction.

Other suitable methods for amplification of polynucleotides may include oligonucleotide extension and ligation, rolling circle amplification (RCA) (Lizardi et al., Nat. Genet. 19:225-232 (1998)) and oligonucleotide ligation assay (OLA) (See generally U.S. Pat. Nos. 7,582,420, 5,185,243, 5,679,524 and 5,573,907; EP 0 320 308 B1; EP 0 336 731 B1; EP 0 439 182 B1; WO 90/01069; WO 89/12696; and WO 89/09835) technologies. It will be appreciated that these amplification methodologies may be designed to amplify immobilized DNA fragments. For example, in some embodiments, the amplification method may include ligation probe amplification or oligonucleotide ligation assay (OLA) reactions that contain primers directed specifically to the nucleic acid of interest. In some embodiments, the amplification method may include a primer extension-ligation reaction that contains primers directed specifically to the nucleic acid of interest. As a non-limiting example of primer extension and ligation primers that may be specifically designed to amplify a nucleic acid of interest, the amplification may include primers used for the GoldenGate assay (Illumina, Inc., San Diego, CA) as exemplified by U.S. Pat. No. 7,582,420 and 7,611,869.

DNA nanoballs can also be used in combination with methods and compositions as described herein. Methods for creating and utilizing DNA nanoballs for genomic sequencing can be found at, for example, US patents and publications U.S. Pat. No. 7,910,354, 2009/0264299, 2009/0011943, 2009/0005252, 2009/0155781, 2009/0118488 and as described in, for example, Drmanac et al., 2010, Science 327(5961): 78-81. Briefly, following genomic library DNA fragmentation adaptors are ligated to the fragments, the adapter ligated fragments are circularized by ligation with a circle ligase and rolling circle amplification is carried out (as described in Lizardi et al., 1998. Nat. Genet. 19:225-232 and US 2007/0099208 A1). The extended concatameric structure of the amplicons promotes coiling thereby creating compact DNA nanoballs. The DNA nanoballs can be captured on substrates, preferably to create an ordered or patterned array such that distance between each nanoball is maintained thereby allowing sequencing of the separate DNA nanoballs. In some embodiments such as those used by Complete Genomics (Mountain View, Calif.), consecutive rounds of adapter ligation, amplification and digestion are carried out prior to circularization to produce head to tail constructs having several genomic DNA fragments separated by adapter sequences.

Exemplary isothermal amplification methods that may be used in a method of the present disclosure include, but are not limited to, Multiple Displacement Amplification (MDA) as exemplified by, for example Dean et al., Proc. Natl. Acad. Sci. USA 99:5261-66 (2002) or isothermal strand displacement nucleic acid amplification exemplified by, for example U.S. Pat. No. 6,214,587. Other non-PCR-based methods that may be used in the present disclosure include, for example, strand displacement amplification (SDA) which is described in, for example Walker et al., Molecular Methods for Virus Detection, Academic Press, Inc., 1995; U.S. Pat. Nos. 5,455,166, and 5,130,238, and Walker etal., Nucl. Acids Res. 20:1691-96 (1992) or hyper-branched strand displacement amplification which is described in, for example Lage et al., Genome Res. 13:294-307 (2003). Isothermal amplification methods may be used with, for instance, the strand-displacing Phi 29 polymerase or Bst DNA polymerase large fragment, 5'->3' exo- for random primer amplification of genomic DNA. The use of these polymerases takes advantage of their high processivity and strand displacing activity. High processivity allows the polymerases to produce fragments that are 10-20 kb in length. As set forth above, smaller fragments may be produced under isothermal conditions using polymerases having low processivity and strand-displacing activity such as Klenow polymerase. Additional description of amplification reactions, conditions and components are set forth in detail in the disclosure of U.S. Patent No. 7,670,810.

Another polynucleotide amplification method that is useful in the present disclosure is Tagged PCR which uses a population of two-domain primers having a constant 5' region followed by a random 3' region as described, for example, in Grothues et al. Nucleic Acids Res. 21(5):1321-2 (1993). The first rounds of amplification are carried out to allow a multitude of initiations on heat denatured DNA based on individual hybridization from the randomly-synthesized 3' region. Due to the nature of the 3' region, the sites of initiation are contemplated to be random throughout the genome. Thereafter, the unbound primers may be removed and further replication may take place using primers complementary to the constant 5' region.

In some embodiments, isothermal amplification can be performed using kinetic exclusion amplification (KEA), also referred to as exclusion amplification (ExAmp). A nucleic acid library of the present disclosure can be made using a method that includes a step of reacting an amplification reagent to produce a plurality of amplification sites that each includes a substantially clonal population of amplicons from an individual target nucleic acid that has seeded the site. In some embodiments, the amplification reaction proceeds until a sufficient number of amplicons are generated to fill the capacity of the respective amplification site. Filling an already seeded site to capacity in this way inhibits target nucleic acids from landing and amplifying at the site thereby producing a clonal population of amplicons at the site. In some embodiments, apparent clonality can be achieved even if an amplification site is not filled to capacity prior to a second target nucleic acid arriving at the site. Under some conditions, amplification of a first target nucleic acid can proceed to a point that a sufficient number of copies are made to effectively outcompete or overwhelm production of copies from a second target nucleic acid that is transported to the site. For example, in an embodiment that uses a bridge amplification process on a circular feature that is smaller than 500 nm in diameter, it has been determined that after 14 cycles of exponential amplification for a first target nucleic acid, contamination from a second target nucleic acid at the same site will produce an insufficient number of contaminating amplicons to adversely impact sequencing-by-synthesis analysis on an Illumina sequencing platform.

In some embodiments, amplification sites in an array can be, but need not be, entirely clonal. Rather, for some applications, an individual amplification site can be predominantly populated with amplicons from a first indexed fragment and can also have a low level of contaminating amplicons from a second target nucleic acid. An array can have one or more amplification sites that have a low level of contaminating amplicons so long as the level of contamination does not have an unacceptable impact on a subsequent use of the array. For example, when the array is to be used in a detection application, an acceptable level of contamination would be a level that does not impact signal to noise or resolution of the detection technique in an unacceptable way. Accordingly, apparent clonality will generally be relevant to a particular use or application of an array made by the methods set forth herein. Exemplary levels of contamination that can be acceptable at an individual amplification site for particular applications include, but are not limited to, at most 0.1%, 0.5%, 1%, 5%, 10% or 25% contaminating amplicons. An array can include one or more amplification sites having these exemplary levels of contaminating amplicons. For example, up to 5%, 10%, 25%, 50%, 75%, or even 100% of the amplification sites in an array can have some contaminating amplicons. It will be understood that in an array or other collection of sites, at least 50%, 75%, 80%, 85%, 90%, 95% or 99% or more of the sites can be clonal or apparently clonal.

In some embodiments, kinetic exclusion can occur when a process occurs at a sufficiently rapid rate to effectively exclude another event or process from occurring. Take for example the making of a nucleic acid array where sites of the array are randomly seeded with indexed fragments from a solution and copies of the indexed fragments are generated in an amplification process to fill each of the seeded sites to capacity. In accordance with the kinetic exclusion methods of the present disclosure, the seeding and amplification processes can proceed simultaneously under conditions where the amplification rate exceeds the seeding rate. As such, the relatively rapid rate at which copies are made at a site that has been seeded by a first target nucleic acid will effectively exclude a second nucleic acid from seeding the site for amplification. Kinetic exclusion amplification methods can be performed as described in detail in the disclosure of US Application Pub. No. 2013/0338042.

Kinetic exclusion can exploit a relatively slow rate for initiating amplification (e.g. a slow rate of making a first copy of an indexed fragment) vs. a relatively rapid rate for making subsequent copies of the indexed fragment (or of the first copy of the indexed fragment). In the example of the previous paragraph, kinetic exclusion occurs due to the relatively slow rate of indexed fragment seeding (e.g. relatively slow diffusion or transport) vs. the relatively rapid rate at which amplification occurs to fill the site with copies of the indexed fragment seed. In another exemplary embodiment, kinetic exclusion can occur due to a delay in the formation of a first copy of an indexed fragment that has seeded a site (e.g. delayed or slow activation) vs. the relatively rapid rate at which subsequent copies are made to fill the site. In this example, an individual site may have been seeded with several different indexed fragments (e.g. several indexed fragments can be present at each site prior to amplification). However, first copy formation for any given indexed fragment can be activated randomly such that the average rate of first copy formation is relatively slow compared to the rate at which subsequent copies are generated. In this case, although an individual site may have been seeded with several different indexed fragments, kinetic exclusion will allow only one of those indexed fragments to be amplified. More specifically, once a first indexed fragment has been activated for amplification, the site will rapidly fill to capacity with its copies, thereby preventing copies of a second indexed fragment from being made at the site.

In one embodiment, the method is carried out to simultaneously (i) transport indexed fragments to amplification sites at an average transport rate, and (ii) amplify the indexed fragments that are at the amplification sites at an average amplification rate, wherein the average amplification rate exceeds the average transport rate (U.S. Pat. No. 9,169,513). Accordingly, kinetic exclusion can be achieved in such embodiments by using a relatively slow rate of transport. For example, a sufficiently low concentration of indexed fragments can be selected to achieve a desired average transport rate, lower concentrations resulting in slower average rates of transport. Alternatively or additionally, a high viscosity solution and/or presence of molecular crowding reagents in the solution can be used to reduce transport rates. Examples of useful molecular crowding reagents include, but are not limited to, polyethylene glycol (PEG), ficoll, dextran, or polyvinyl alcohol. Exemplary molecular crowding reagents and formulations are set forth in U.S. Pat. No. 7,399,590. Another factor that can be adjusted to achieve a desired transport rate is the average size of the target nucleic acids.

An amplification reagent can include further components that facilitate amplicon formation and in some cases increase the rate of amplicon formation. An example is a recombinase. Recombinase can facilitate amplicon formation by allowing repeated invasion/extension. More specifically, recombinase can facilitate invasion of an indexed fragment by the polymerase and extension of a primer by the polymerase using the indexed fragment as a template for amplicon formation. This process can be repeated as a chain reaction where amplicons produced from each round of invasion/extension serve as templates in a subsequent round. The process can occur more rapidly than standard PCR since a denaturation cycle (e.g. via heating or chemical denaturation) is not required. As such, recombinase-facilitated amplification can be carried out isothermally. It is generally desirable to include ATP, or other nucleotides (or in some cases non-hydrolyzable analogs thereof) in a recombinase-facilitated amplification reagent to facilitate amplification. A mixture of recombinase and single stranded binding (SSB) protein is particularly useful as SSB can further facilitate amplification. Exemplary formulations for recombinase-facilitated amplification include those sold commercially as TwistAmp kits by TwistDx (Cambridge, UK). Useful components of recombinase-facilitated amplification reagent and reaction conditions are set forth in US 5,223,414 and US 7,399,590.

Another example of a component that can be included in an amplification reagent to facilitate amplicon formation and in some cases to increase the rate of amplicon formation is a helicase. Helicase can facilitate amplicon formation by allowing a chain reaction of amplicon formation. The process can occur more rapidly than standard PCR since a denaturation cycle (e.g. via heating or chemical denaturation) is not required. As such, helicase-facilitated amplification can be carried out isothermally. A mixture of helicase and single stranded binding (SSB) protein is particularly useful as SSB can further facilitate amplification. Exemplary formulations for helicase-facilitated amplification include those sold commercially as IsoAmp kits from Biohelix (Beverly, MA). Further, examples of useful formulations that include a helicase protein are described in US 7,399,590 and US 7,829,284.

Yet another example of a component that can be included in an amplification reagent to facilitate amplicon formation and in some cases increase the rate of amplicon formation is an origin binding protein.

Use in Sequencing/Methods of Sequencing

Following attachment of indexed fragments to a surface, the sequence of the immobilized and amplified indexed fragments is determined. Sequencing can be carried out using any suitable sequencing technique, and methods for determining the sequence of immobilized and amplified indexed fragments, including strand re-synthesis, are known in the art and are described in, for instance, Bignell et al. (US 8,053,192), Gunderson et al. (WO2016/130704), Shen et al. (US 8,895,249), and Pipenburg et al. (US 9,309,502).

The methods described herein can be used in conjunction with a variety of nucleic acid sequencing techniques. Particularly applicable techniques are those wherein nucleic acids are attached at fixed locations in an array such that their relative positions do not change and wherein the array is repeatedly imaged. Embodiments in which images are obtained in different color channels, for example, coinciding with different labels used to distinguish one nucleotide base type from another are particularly applicable. In some embodiments, the process to determine the nucleotide sequence of an indexed fragment can be an automated process. Preferred embodiments include sequencing-by-synthesis ("SBS") techniques.

SBS techniques generally involve the enzymatic extension of a nascent nucleic acid strand through the iterative addition of nucleotides against a template strand. In traditional methods of SBS, a single nucleotide monomer may be provided to a target nucleotide in the presence of a polymerase in each delivery. However, in the methods described herein, more than one type of nucleotide monomer can be provided to a target nucleic acid in the presence of a polymerase in a delivery.

In one embodiment, a nucleotide monomer includes locked nucleic acids (LNAs) or bridged nucleic acids (BNAs). The use of LNAs or BNAs in a nucleotide monomer increases hybridization strength between a nucleotide monomer and a sequencing primer sequence present on an immobilized indexed fragment.

SBS can use nucleotide monomers that have a terminator moiety or those that lack any terminator moieties. Methods using nucleotide monomers lacking terminators include, for example, pyrosequencing and sequencing using γ-phosphate-labeled nucleotides, as set forth in further detail herein. In methods using nucleotide monomers lacking terminators, the number of nucleotides added in each cycle is generally variable and dependent upon the template sequence and the mode of nucleotide delivery. For SBS techniques that utilize nucleotide monomers having a terminator moiety, the terminator can be effectively irreversible under the sequencing conditions used as is the case for traditional Sanger sequencing which utilizes dideoxynucleotides, or the terminator can be reversible as is the case for sequencing methods developed by Solexa (now Illumina, Inc.).

SBS techniques can use nucleotide monomers that have a label moiety or those that lack a label moiety. Accordingly, incorporation events can be detected based on a characteristic of the label, such as fluorescence of the label; a characteristic of the nucleotide monomer such as molecular weight or charge; a byproduct of incorporation of the nucleotide, such as release of pyrophosphate; or the like. In embodiments where two or more different nucleotides are present in a sequencing reagent, the different nucleotides can be distinguishable from each other, or alternatively the two or more different labels can be the indistinguishable under the detection techniques being used. For example, the different nucleotides present in a sequencing reagent can have different labels and they can be distinguished using appropriate optics as exemplified by the sequencing methods developed by Solexa (now Illumina, Inc.).

Preferred embodiments include pyrosequencing techniques. Pyrosequencing detects the release of inorganic pyrophosphate (PPi) as particular nucleotides are incorporated into the nascent strand (Ronaghi, M., Karamohamed, S., Pettersson, B., Uhlen, M. and Nyren, P. (1996) "Real-time DNA sequencing using detection of pyrophosphate release." Analytical Biochemistry 242(1), 84-9; Ronaghi, M. (2001) "Pyrosequencing sheds light on DNA sequencing." Genome Res. 11(1), 3-11; Ronaghi, M., Uhlen, M. and Nyren, P. (1998) "A sequencing method based on real-time pyrophosphate." Science 281(5375), 363; U.S. Pat. Nos. 6,210,891; 6,258,568 and 6,274,320). In pyrosequencing, released PPi can be detected by being immediately converted to adenosine triphosphate (ATP) by ATP sulfurase, and the level of ATP generated is detected via luciferase-produced photons. The nucleic acids to be sequenced can be attached to features in an array and the array can be imaged to capture the chemiluminescent signals that are produced due to incorporation of a nucleotides at the features of the array. An image can be obtained after the array is treated with a particular nucleotide type (e.g. A, T, C or G). Images obtained after addition of each nucleotide type will differ with regard to which features in the array are detected. These differences in the image reflect the different sequence content of the features on the array. However, the relative locations of each feature will remain unchanged in the images. The images can be stored, processed and analyzed using the methods set forth herein. For example, images obtained after treatment of the array with each different nucleotide type can be handled in the same way as exemplified herein for images obtained from different detection channels for reversible terminator-based sequencing methods.

In another exemplary type of SBS, cycle sequencing is accomplished by stepwise addition of reversible terminator nucleotides containing, for example, a cleavable or photobleachable dye label as described, for example, in WO 04/018497 and U.S. Pat. No. 7,057,026. This approach is being commercialized by Solexa (now Illumina Inc.), and is also described in WO 91/06678 and WO 07/123,744. The availability of fluorescently-labeled terminators in which both the termination can be reversed and the fluorescent label cleaved facilitates efficient cyclic reversible termination (CRT) sequencing. Polymerases can also be co-engineered to efficiently incorporate and extend from these modified nucleotides.

In some reversible terminator-based sequencing embodiments, the labels do not substantially inhibit extension under SBS reaction conditions. However, the detection labels can be removable, for example, by cleavage or degradation. Images can be captured following incorporation of labels into arrayed nucleic acid features. In particular embodiments, each cycle involves simultaneous delivery of four different nucleotide types to the array and each nucleotide type has a spectrally distinct label. Four images can then be obtained, each using a detection channel that is selective for one of the four different labels. Alternatively, different nucleotide types can be added sequentially and an image of the array can be obtained between each addition step. In such embodiments, each image will show nucleic acid features that have incorporated nucleotides of a particular type. Different features will be present or absent in the different images due the different sequence content of each feature. However, the relative position of the features will remain unchanged in the images. Images obtained from such reversible terminator-SBS methods can be stored, processed and analyzed as set forth herein. Following the image capture step, labels can be removed and reversible terminator moieties can be removed for subsequent cycles of nucleotide addition and detection. Removal of the labels after they have been detected in a particular cycle and prior to a subsequent cycle can provide the advantage of reducing background signal and crosstalk between cycles. Examples of useful labels and removal methods are set forth herein.

In particular embodiments some or all of the nucleotide monomers can include reversible terminators. In such embodiments, reversible terminators/cleavable fluorophores can include fluorophores linked to the ribose moiety via a 3' ester linkage (Metzker, Genome Res. 15:1767-1776 (2005)). Other approaches have separated the terminator chemistry from the cleavage of the fluorescence label (Ruparel et al., Proc Natl Acad Sci USA 102: 5932-7 (2005)). Ruparel et al. described the development of reversible terminators that used a small 3' allyl group to block extension, but could easily be deblocked by a short treatment with a palladium catalyst. The fluorophore was attached to the base via a photocleavable linker that could easily be cleaved by a 30 second exposure to long wavelength UV light. Thus, either disulfide reduction or photocleavage can be used as a cleavable linker. Another approach to reversible termination is the use of natural termination that ensues after placement of a bulky dye on a dNTP. The presence of a charged bulky dye on the dNTP can act as an effective terminator through steric and/or electrostatic hindrance. The presence of one incorporation event prevents further incorporations unless the dye is removed. Cleavage of the dye removes the fluorophore and effectively reverses the termination. Examples of modified nucleotides are also described in U.S. Pat. Nos. 7,427,673, and 7,057,026.

Additional exemplary SBS systems and methods which can be utilized with the methods and systems described herein are described in U.S. Pub. Nos. 2007/0166705, 2006/0188901, 2006/0240439, 2006/0281109, 2012/0270305, and 2013/0260372, U.S. Pat. No. 7,057,026, PCT Publication No. WO 05/065814, U.S. Patent Application Publication No. 2005/0100900, and PCT Publication Nos. WO 06/064199 and WO 07/010,251.

Some embodiments can use detection of four different nucleotides using fewer than four different labels. For example, SBS can be performed using methods and systems described in the incorporated materials of U.S. Pub. No. 2013/0079232. As a first example, a pair of nucleotide types can be detected at the same wavelength, but distinguished based on a difference in intensity for one member of the pair compared to the other, or based on a change to one member of the pair (e.g. via chemical modification, photochemical modification or physical modification) that causes apparent signal to appear or disappear compared to the signal detected for the other member of the pair. As a second example, three of four different nucleotide types can be detected under particular conditions while a fourth nucleotide type lacks a label that is detectable under those conditions, or is minimally detected under those conditions (e.g., minimal detection due to background fluorescence, etc.). Incorporation of the first three nucleotide types into a nucleic acid can be determined based on presence of their respective signals and incorporation of the fourth nucleotide type into the nucleic acid can be determined based on absence or minimal detection of any signal. As a third example, one nucleotide type can include label(s) that are detected in two different channels, whereas other nucleotide types are detected in no more than one of the channels. The aforementioned three exemplary configurations are not considered mutually exclusive and can be used in various combinations. An exemplary embodiment that combines all three examples, is a fluorescent-based SBS method that uses a first nucleotide type that is detected in a first channel (e.g. dATP having a label that is detected in the first channel when excited by a first excitation wavelength), a second nucleotide type that is detected in a second channel (e.g. dCTP having a label that is detected in the second channel when excited by a second excitation wavelength), a third nucleotide type that is detected in both the first and the second channel (e.g. dTTP having at least one label that is detected in both channels when excited by the first and/or second excitation wavelength) and a fourth nucleotide type that lacks a label that is not, or minimally, detected in either channel (e.g. dGTP having no label).

Further, as described in the incorporated materials of U.S. Pub. No. 2013/0079232, sequencing data can be obtained using a single channel. In such so-called one-dye sequencing approaches, the first nucleotide type is labeled but the label is removed after the first image is generated, and the second nucleotide type is labeled only after a first image is generated. The third nucleotide type retains its label in both the first and second images, and the fourth nucleotide type remains unlabeled in both images.

Some embodiments can use sequencing by ligation techniques. Such techniques use DNA ligase to incorporate oligonucleotides and identify the incorporation of such oligonucleotides. The oligonucleotides typically have different labels that are correlated with the identity of a particular nucleotide in a sequence to which the oligonucleotides hybridize. As with other SBS methods, images can be obtained following treatment of an array of nucleic acid features with the labeled sequencing reagents. Each image will show nucleic acid features that have incorporated labels of a particular type. Different features will be present or absent in the different images due the different sequence content of each feature, but the relative position of the features will remain unchanged in the images. Images obtained from ligation-based sequencing methods can be stored, processed and analyzed as set forth herein. Exemplary SBS systems and methods which can be utilized with the methods and systems described herein are described in U.S. Pat. Nos. 6,969,488, 6,172,218, and 6,306,597.

Some embodiments can use nanopore sequencing (Deamer, D. W. & Akeson, M. "Nanopores and nucleic acids: prospects for ultrarapid sequencing." Trends Biotechnol. 18, 147-151 (2000); Deamer, D. and D. Branton, "Characterization of nucleic acids by nanopore analysis", Acc. Chem. Res. 35:817-825 (2002); Li, J., M. Gershow, D. Stein, E. Brandin, and J. A. Golovchenko, "DNA molecules and configurations in a solid-state nanopore microscope" Nat. Mater. 2:611-615 (2003)). In such embodiments, the indexed fragment passes through a nanopore. The nanopore can be a synthetic pore or biological membrane protein, such as α-hemolysin. As the indexed fragment passes through the nanopore, each base-pair can be identified by measuring fluctuations in the electrical conductance of the pore. (U.S. Pat. No. 7,001,792; Soni, G. V. & Meller, "A. Progress toward ultrafast DNA sequencing using solid-state nanopores." Clin. Chem. 53, 1996-2001 (2007); Healy, K. "Nanopore-based single-molecule DNA analysis." Nanomed. 2, 459-481 (2007); Cockroft, S. L., Chu, J., Amorin, M. & Ghadiri, M. R. "A single-molecule nanopore device detects DNA polymerase activity with single-nucleotide resolution." J. Am. Chem. Soc. 130, 818-820 (2008)). Data obtained from nanopore sequencing can be stored, processed and analyzed as set forth herein. In particular, the data can be treated as an image in accordance with the exemplary treatment of optical images and other images that is set forth herein.

Some embodiments can use methods involving the real-time monitoring of DNA polymerase activity. Nucleotide incorporations can be detected through fluorescence resonance energy transfer (FRET) interactions between a fluorophore-bearing polymerase and γ-phosphate-labeled nucleotides as described, for example, in U.S. Pat. Nos. 7,329,492 and 7,211,414, or nucleotide incorporations can be detected with zero-mode waveguides as described, for example, in U.S. Pat. No. 7,315,019, and using fluorescent nucleotide analogs and engineered polymerases as described, for example, in U.S. Pat. No. 7,405,281 and U.S. Pub. No. 2008/0108082. The illumination can be restricted to a zeptoliter-scale volume around a surface-tethered polymerase such that incorporation of fluorescently labeled nucleotides can be observed with low background (Levene, M. J. et al. "Zero-mode waveguides for single-molecule analysis at high concentrations." Science 299, 682-686 (2003); Lundquist, P. M. et al. "Parallel confocal detection of single molecules in real time." Opt. Lett. 33, 1026-1028 (2008); Korlach, J. et al. "Selective aluminum passivation for targeted immobilization of single DNA polymerase molecules in zero-mode waveguide nano structures." Proc. Natl. Acad. Sci. USA 105, 1176-1181 (2008)). Images obtained from such methods can be stored, processed and analyzed as set forth herein.

Some SBS embodiments include detection of a proton released upon incorporation of a nucleotide into an extension product. For example, sequencing based on detection of released protons can use an electrical detector and associated techniques that are commercially available from Ion Torrent (Guilford, CT, a Life Technologies subsidiary) or sequencing methods and systems described in U.S. Pub. Nos. 2009/0026082; 2009/0127589; 2010/0137143; and 2010/0282617. Methods set forth herein for amplifying target nucleic acids using kinetic exclusion can be readily applied to substrates used for detecting protons. More specifically, methods set forth herein can be used to produce clonal populations of amplicons that are used to detect protons.

The above SBS methods can be advantageously carried out in multiplex formats such that multiple different indexed fragments are manipulated simultaneously. In particular embodiments, different indexed fragments can be treated in a common reaction vessel or on a surface of a particular substrate. This allows convenient delivery of sequencing reagents, removal of unreacted reagents and detection of incorporation events in a multiplex manner. In embodiments using surface-bound target nucleic acids, the indexed fragments can be in an array format. In an array format, the indexed fragments can be typically bound to a surface in a spatially distinguishable manner. The indexed fragments can be bound by direct covalent attachment, attachment to a bead or other particle or binding to a polymerase or other molecule that is attached to the surface. The array can include a single copy of an indexed fragment at each site (also referred to as a feature) or multiple copies having the same sequence can be present at each site or feature. Multiple copies can be produced by amplification methods such as, bridge amplification or emulsion PCR as described in further detail herein.

The methods set forth herein can use arrays having features at any of a variety of densities including, for example, at least about 10 features/cm$^2$, 100 features/ cm$^2$, 500 features/ cm$^2$, 1,000 features/ cm$^2$, 5,000 features/ cm$^2$, 10,000 features/ cm$^2$, 50,000 features/ cm$^2$, 100,000 features/ cm$^2$, 1,000,000 features/ cm$^2$, 5,000,000 features/ cm$^2$, or higher.

An advantage of the methods set forth herein is that they provide for rapid and efficient detection of a plurality of cm$^2$, in parallel. Accordingly, the present disclosure provides integrated systems capable of preparing and detecting nucleic acids using techniques known in the art such as those exemplified herein. Thus, an integrated system of the present disclosure can include fluidic components capable of delivering amplification reagents and/or sequencing reagents to one or more immobilized indexed fragments, the system including components such as pumps, valves, reservoirs, fluidic lines and the like. A flow cell can be configured and/or used in an integrated system for detection of target nucleic acids. Exemplary flow cells are described, for example, in U.S. Pub. No. 2010/0111768 and US Ser. No. 13/273,666. As exemplified for flow cells, one or more of the fluidic components of an integrated system can be used for an amplification method and for a detection method. Taking a nucleic acid sequencing embodiment as an example, one or more of the fluidic components of an integrated system can be used for an amplification method set forth herein and for the delivery of sequencing reagents in a sequencing method such as those exemplified above. Alternatively, an integrated system can include separate fluidic systems to carry out amplification methods and to carry out detection methods. Examples of integrated sequencing systems that are capable of creating amplified nucleic acids and also determining the sequence of the nucleic acids include, without limitation, the MiSeqTM platform (Illumina, Inc., San Diego, CA) and devices described in US Ser. No. 13/273,666.

Also provided herein but not part of the invention are compositions. During the practice of the methods described herein various compositions can result. For example, a composition including indexed nucleic acid fragments, wherein the indexed nucleic acid fragments are derived from newly synthesized RNA, can result. In one embodiment, newly synthesized RNA is labeled. Also provided is a multi-well plate, wherein a well of the multi-well plate includes indexed nucleic acid fragments.

Also provided herein but not part of the invention are kits. In one embodiment, a kit is for preparing a sequencing library where newly synthesized RNA is labeled. In one embodiment, the kit includes a nucleotide label described herein. In another embodiment, the kit includes one or more primers for annealing to RNA, where at least one primer is for targeted amplification of one or more predetermined nucleic acid. In a further embodiment, the kit includes the components to add at least three indexes to nucleic acids. A kit can also include other components useful in producing a sequencing library. For instance, the kit can include at least one enzyme that mediates ligation, primer extension, or amplification for processing RNA molecules to include an index. The kit can include nucleic acids with index sequences. The kit can also include other components useful for adding an index to a nucleic acid, such as a transposome complex. The kit can also include one or more primers for annealing to RNA. The primers can be for the production of a whole transcriptome (e.g., a primer that includes a poly-T sequence) or for targeted amplification of one or more predetermined nucleic acid.

The components of a kit are typically in a suitable packaging material in an amount sufficient for at least one assay or use. Optionally, other components can be included, such as buffers and solutions. Instructions for use of the packaged components are also typically included. As used herein, the phrase "packaging material" refers to one or more physical structures used to house the contents of the kit. The packaging material is constructed by routine methods, generally to provide a sterile, contaminant-free environment. The packaging material may have a label which indicates that the components can be used producing a sequencing library. In addition, the packaging material contains instructions indicating how the materials within the kit are employed. As used herein, the term "package" refers to a container such as glass, plastic, paper, foil, and the like, capable of holding within fixed limits the components of the kit. "Instructions for use" typically include a tangible expression describing the reagent concentration or at least one assay method parameter, such as the relative amounts of reagent and sample to be admixed, maintenance time periods for reagent/sample admixtures, temperature, buffer conditions, and the like.

EXAMPLES

The present disclosure is illustrated by the following examples.

Example 1

The dynamic transcriptional landscape of mammalian organogenesis at single cell resolution

During mammalian organogenesis, the cells of the three germ layers transform into an embryo that includes most major internal and external organs. The key regulators of developmental defects can be studied during this crucial period, but current technologies lack the throughput and resolution to obtain a global view of the molecular states and trajectories of a rapidly diversifying and expanding number of cell types. Here we set out to investigate the transcriptional dynamics of mouse development during organogenesis at single cell resolution. With an improved single cell combinatorial indexing-based protocol ('sci-RNA-seq3'), we profiled over 2 million cells derived from 61 mouse embryos staged between 9.5 and 13.5 days of gestation (E9.5 to E13.5; 10 to 15 replicates per timepoint). We identify hundreds of expanding, contracting and transient cell types, many of which are only detected because of the depth of cellular coverage obtained here, and define the corresponding sets of cell type-specific marker genes, several of which we validate by whole mount in situ hybridization. We explore the dynamics of proliferation and gene expression within cell types overtime, including focused analyses of the apical ectodermal ridge, limb mesenchyme and skeletal muscle. With a new algorithm, we identify the major single cell developmental trajectories of mouse organogenesis, and within these discover examples of distinct paths to the same endpoint, i.e. branching and convergence. These data comprise a foundational resource for mammalian developmental biology, and are made available in a way that will facilitate their ongoing annotation by the research community.

Introduction

Mammalian organogenesis is an astonishing process. Within a short window of time, the cells of the three germ layers transform into a proper embryo that includes most of its major internal and external organs. Although very early human embryos can be cultivated and studied in vitrol, there is limited access to material corresponding to later stages of human embryonic development. Consequently, most studies of mammalian organogenesis rely on model organisms, and in particular, the mouse.

Compared with humans, mice develop quickly, with just 21 days between fertilization and the birth of pups. The implantation of the mouse blastocyst (32-64 cells) occurs at embryonic day 4 (E4.0). This is followed by gastrulation and the formation of primary germ layers (E6.5-E7.5; 660-15K cells)2,3. During this time, the primitive streak forms and the allocation of the distinct lineages of the embryo in an anterior-to-posterior sequence takes place4. At the early-somite stages (E8.0-E8.5) the embryo transits from gastrulation to early organogenesis associated with the neural plate and heart tube formation (60K-90K cells). Classical organogenesis begins at E9.5. In the ensuing four days (E9.5-E13.5), the mouse embryo expands from a few hundred thousand cells to over ten million cells, and concurrently develops sensory organs, gastrointestinal and respiratory organs, its spinal cord, skeletal system, and haematopoietic system. Unsurprisingly, this critical period of mouse development has been intensively studied. Indeed, most of the key regulators of developmental defects can be studied during this window5,6.

A conventional paradigm for studies of mouse organogenesis involves focusing on an individual organ system at a restricted stage of development and combining gene knockout studies with phenotyping by anatomic morphology, in situ hybridization, immunohistochemistry7,8, or more recently, transcriptome or epigenome profiling9. Although such focused studies have generated fundamental insights into mammalian development, the underlying technologies lack the throughput and resolution to obtain a global view of the dynamic molecular processes underway in the diverse and rapidly expanding populations and subpopulations of cells during organogenesis.

The 'shotgun profiling' of the molecular contents of single cells represents a promising avenue for addressing these shortcomings and further advancing our understanding of mammalian development. For example, the application of single cell RNA-seq methods have recently revealed tremendous heterogeneity in neurons and myocardiocytes during mouse development10,11. Although two single cell transcriptional atlases of the mouse were recently released and represent important resources for the field12,13, they are mostly restricted to adult organs, and do not attempt to characterize the emergence and temporal dynamics of mammalian cell types during development.

Single cell combinatorial indexing (' sci-') is a methodological framework that employs split-pool barcoding to uniquely label the nucleic acid contents of large numbers of single cells or nuclei 14-21. We recently developed a 'sci-' protocol for transcriptomes ('sci-RNA-seq') and applied it to generate 50-fold 'shotgun cellular coverage' of the nematode Caenorhabditis elegans at L2 stage19. Although the throughput of 'sci-' methods increases exponentially with the number of rounds of indexing, this potential has yet to be fully realized because of other factors such as the rate of cell loss and the limited reaction efficiency of some steps19,21. To address this, we developed and extensively optimized 3-level sci-RNA-seq (sci-RNA-seq3), resulting in a workflow that can profile over one million cells per experiment. As previously19, multiple samples (e.g. replicates, timepoints, etc.) can be barcoded during the first round of indexing and concurrently processed.

Here we set out to investigate the transcriptional dynamics of mouse development during organogenesis at single cell resolution using sci-RNA-seq3. In one experiment, we profiled over 2 million single cells derived from 61 mouse embryos between E9.5 and E13.5 (10 to 15 replicates per timepoint). From these data, we identify 38 major cell types, as well as over 600 more granular cell types (termed 'subtypes' here to distinguish them from the 38 major cell types). Altogether, we discover thousands of new candidate marker genes for cell types and subtypes, and validate representative examples by whole mount in situ hybridization. We quantify the dynamics of proliferation and gene expression in expanding and transient cell types during midgestation, including focused analyses of the apical ectodermal ridge, limb mesenchyme and skeletal muscle. With a new algorithm, we define the major single-cell developmental trajectories of mouse organogenesis, and within these discover examples of distinct paths to the same endpoint, i.e. branching and convergence. All data are made freely available in a way that will facilitate their ongoing annotation by the research community.

Results

Profiling 2 million cells from 61 mouse embryos across 5 developmental stages with sci-RNA-seq3

To increase the throughput of sci-RNA-seq, we explored over 1,000 experimental conditions. Relative to our original description of the method[19], the major improvements introduced by sci-RNA-seq3 (Fig. 4A, Methods) are: (i) We developed a new nuclear extraction and fixation strategy, wherein nuclei are extracted directly from fresh tissues without any enzymatic treatment. Subsequent to their extraction, nuclei are fixed in 4% paraformaldehyde and can be stored in liquid nitrogen prior to further processing. (ii) As compared with our previous description of 3-level indexing[19], we switched from indexed Tn5 tagmentation to indexed hairpin ligation. (iii) Several individual reactions, e.g. reverse transcription, have been further optimized for efficiency. (iv) The FACS sorting step is omitted, and sonication and filtration steps added to minimize the aggregation of nuclei. The library preparation steps of sci-RNA-seq3 can be completed by a single individual in one week, and greatly exceeds the 'per experiment' throughput of alternative sc-RNA-seq protocols (Fig. 4B).

We collected C57BL/6 mouse embryos between E9.5-E13.5 and snap froze them in liquid nitrogen, including 10 to 15 embryos from at least three independent litters per stage. We subsequently isolated nuclei from 61 individual whole embryos and performed sci-RNA-seq3 (Fig. 4A). Nuclei derived from each embryo were deposited to different wells during the first round of indexing, such that the RNA-seq profiles of individual nuclei could be linked to the embryos from which they were derived (Fig. 5A). As internal control, we also spiked a mixture of HEK293T and NIH/3T3 cells into two wells during the first round of indexing. After completing the sci-RNA-seq3 protocol, the resulting library was sequenced in one NovaSeq run, yielding 11 billion reads (Fig. 5B).

From this one experiment, we recovered 2,072,011 single cell transcriptomes (unique molecular identifier or UMI count $\geq$ 200), including 2,058,652 cells from the 61 mouse embryos and 13,359 cells from HEK293T or NIH/3T3 cells. Reassuringly, the transcriptomes of HEK293T and NIH/3T3 cells overwhelmingly mapped to the genome of one species or the other, with 420 (3%) collisions (Fig. 4C). At a sequencing depth of 23,207 reads per cell, we observed a median of 3,676 UMIs per HEK293T cell and 5,163 UMIs per NIH/3T3 cell, with 3.9% and 2.9% of reads per cell mapping to incorrect species, respectively (Fig. 5C-D). We compared our original sci-RNA-seq protocol with sci-RNA-seq3 by downsampling a previously collected dataset[19] to an equivalent sequencing depth per HEK293T or NIH/3T3 cell. The sci-RNA-seq3 protocol, while garnering a 40-fold increase in throughput, exhibited comparable efficiency in terms of the number of UMIs detected per cell (Fig. 5E). Furthermore, the aggregated transcriptomes of HEK293T single cell profiles derived from sci-RNA-seq3 and sci-RNA-seq were highly correlated (Pearson: 0.98, Fig. 5F).

The 2,058,652 embryo-derived cells were mapped to the 61 individual embryos based on their first-round barcode (median 35,272 cells per embryo; Fig. 4D). The number of cells recovered from each embryo was well correlated with the number of first-round wells assigned to it (Spearman: 0.75, Fig. 6A). At a relatively shallow sequencing depth (~5,000 reads per cell), we identified a median of 519 genes (671 UMIs) per cell (Fig. 4E). This is comparable or higher than other scRNA-seq studies from which diverse cell types has been distinguished and annotated[19,21,22], despite less than one-third as many raw sequencing reads per cell (Fig. 6B-D). Later stage embryos (E12.5 and E13.5) exhibited somewhat fewer UMI counts per cell, suggesting decreasing mRNA content per nucleus during development (Fig. 6E).

Based on our rough estimates of the number of cells per embryo at each timepoint (Methods), and summing together all 10 to 15 replicates per timepoint, we estimate our 'shotgun cellular coverage' of the mouse embryo to be 0.8x at E9.5 (200K cells per embryo; 152K profiled here), 0.3x at E10.5 (1.1M cells; 378K profiled), 0.2x at E11.5 (2M cells; 616K profiled), 0.08x at E12.5 (6M cells; 475K profiled), and 0.03x at E13.5 (13M cells; 437K profiled). Thus, although we are not yet 'oversampling', the number of cells that we are profiling at each stage are equivalent to a substantial percentage of the cellular content of an individual mouse embryo (3-80%).

As a check on data quality, we aggregated the single cell transcriptomes of each individual, resulting in 61 'pseudo-bulk profiles' of mouse embryos. By counting the number of UMIs mapping to the Xist transcript (only expressed in females) or to Y chromosome transcripts, the mouse embryos are readily separated to male (x = 31) and female (n = 30) groups (Fig. 4F) with balanced representation in terms of the number of male vs. female replicates at each stage (Fig. 6F).

As a further quality check, we subjected the 'pseudo-bulk' transcriptomes of the 61 embryos to t-stochastic neighbor embedding (t-SNE), which resulted in five tightly clustered groups perfectly matching their developmental stages (Fig. 7A). We also ordered the mouse embryos along a 'pseudotime' trajectory using Monocle[23], based on the top 1,000 genes differentially expressed across timepoints, and the resulting ordering also matches expectation (Fig. 4G). There are notably two prominent gaps in the embryo-level pseudotime trajectory, one between E9.5 and E10.5 and the other between E11.5 and E12.5, suggesting dramatic changes in the global transcriptome during these windows. We assigned each embryo a pseudotime, which potentially reflects a more fine-grained assessment of developmental stage (Fig. 7B). For example, E10.5 embryos positioned earlier versus later in developmental pseudotime were morphologically distinct (Fig. 7C).

We also examined changes in the global transcriptome during development. 12,236 genes were differentially expressed across different developmental stages (Data not shown); we plot some of most dynamic genes in Fig. 4H. As expected, we observe increased expression of adult hemoglobin genes such as *Hbb-bt* and *Hbb-bs,* and decreased expression of embryonic hemoglobin genes such as *Hbb-bhl* and *Hbb-x.* Genes with known roles in neuronal differentiation, including *Cntn4*[24], *Neurod2*[25] and *Neurod6*[26], exhibit increased expression at later stages. However, many highly dynamic genes have not been previously characterized, *e.g. Slc35f4, Prtg* and *Trim30a.* Regardless, our supposition -- and indeed, the motivation for collecting single cell data -- is that the dynamics of 'whole embryo' gene expression are mostly driven by dramatic changes in the relative proportions of distinct cell types, rather than changes within any single cell type.

Identification and annotation of the major cell types and subtypes present during mouse organogenesis

To identify major cell types, we subjected the 2,058,652 single cell transcriptomes (*i.e.*, all embryos from all timepoints altogether) to Louvain clustering, which identified 40 distinct groups, and t-SNE visualization (Fig. 8A). Reassuringly, although we observe clear differences between cells derived from different timepoints (Fig. 9A), cells derived from replicate embryos of the same timepoint are similarly distributed (Fig. 10). Based on the sets of genes specific to each of these 40 clusters, we manually curated cell type assignments by comparison to published marker genes (Data not shown). For 37 clusters, we could confidently assign them to exactly one literature-defined cell type, while two clusters both corresponded to the definitive erythroid lineage. One cluster had abnormally high UMI counts but no strongly cluster-specific genes, suggesting that it may be a technical artifact of cell doublets. Merging the definitive erythroid lineage clusters and discarding this putative doublet cluster resulted in 38 major cell types (Fig. 8A). For many clusters, highly specific marker genes made cell type identification straightforward (Fig. 8B, Fig. 9B-C, Data not shown). For example, cluster 6 (epithelial cells) specifically expressed the well-characterized marker genes *Epcam* and *Trp63*[27,28], while cluster 29 (hepatocytes) were specifically marked by *Afp* and *Alb* expression[12]. Smaller clusters, including some corresponding to highly specialized cell types, could be readily annotated as well. For example, cluster 36 was enriched for transcripts highly expressed during retinal development such as *Tyr* and *Trpm1,* strongly suggesting that these are melanocytes[29,30]. Cluster 37 was enriched for transcripts exclusively expressed in the developing lens. For clusters corresponding to the embryonic mesenchyme and connective tissue, cell type identification was more challenging, largely because fewer highly specific marker genes are available in the current literature.

Out of 26,183 genes, 17,789 genes (68%) were differentially expressed (FDR of 5%) across the 38 major cell types (Fig. 9B, Data not shown). Amongst these, we identified 2,863 cell type-specific markers, the vast majority of which have not previously to our knowledge been associated with the respective cell type (mean 75 markers per cluster; Fig. 8B, Fig. 9C). As an example of how these data are useful for defining new, developmentally and cell type-specific markers of gene expression, consider sonic hedgehog (*Shh*), which has been shown to play a critical role during development of many organ systems including the limb, midline structures in the brain, the thalamus, the spinal cord, and the lungs[31]. We detect the highest expression of *Shh* in cluster 30 (notochord; Data not shown), together with *Ntn1*, *Slit1*, *and Spon1,* all known to be expressed in the cells of the notochord and floor plate during development[32-34]. However, the genes *Tox2, Stxbp6, Schip1, Frmd4b,* not previously been described as markers of the notochord, were also highly specific to cluster 30.

As expected, we observed marked changes in the proportions of cell types during organogenesis. While most of the 38 major cell types proliferated exponentially, a few were transient and eventually disappeared at E13.5 (Fig. 11A-B). For example, the primitive erythroid lineage originating from the yolk sack represented by cluster 26 was characterized by *Hbb-bh1* expression, while the definitive erythroid lineage originating from the fetal liver was marked by *Hbb-bs* expression in cluster 22 (Data not shown). At E9.5, we detected mainly cells corresponding to the primitive erythroid lineage (Fig. 8A). Over the next 5 days, the definitive erythroid lineage became the predominant cell type in the fetal circulation and ultimately the exclusive red cell lineage by E13.5 (Fig. 8A). The corresponding gene markers exhibited similar dynamics (Fig. 11C).

The 38 major cell types identified here have a median of 47,073 cells, with the largest cluster containing 144,648 cells (connective tissue progenitors; 7.0% of the overall dataset), and the smallest cluster only 1,000 cells (monocytes/granulocytes; 0.05% of the overall dataset). As cell type heterogeneity was readily apparent within many of these 38 clusters, we adopted an iterative strategy, repeating Louvain clustering on each main cell type to identify subclusters (Figs. 12-13). After subclusters dominated by one or two embryos were removed and highly similar subclusters merged (Methods), a total of 655 subclusters were identified (termed 'subtypes' here to distinguish them from the 38 major cell types; Figs. 12-13). Of note, our sensitivity to detect cell types and subtypes in this study was a direct function of the large number of cells profiled. For example, repeating Louvain clustering on 2.5% of our data (50,000 cells) identified only a subset of cell types and subtypes (Fig. 14).

The 655 subtypes consist of a median of 1,869 cells, and range from 51 cells (a subtype of notochord cells) to 65,894 cells (a subtype of connective tissue progenitor cells) (Fig. 15A).

Nearly all subtypes (99%) are comprised of contributions from multiple embryos, with no single embryo dominating (Fig. 15B-C). In support of the view that these subtypes constitute bona fide transcriptional programs that are distinct from related subtypes, we identified a median of 55 specific markers per subtype (Fig. 15D; note that subtype-specific markers are defined by virtue of being specific *within* the corresponding major cell type, rather than in the overall dataset). More so than the 38 major cell types, individual subtypes exhibited variable dynamics between E9.5 and E13.5. The majority of subtypes (64%) increased in estimated cell number, while 12% decreased and 24% showed more complex patterns (Fig. 16A-B). Interestingly, we could readily separate embryos of various developmental stages based solely on the proportions of cells assigned to each of the subtypes (Fig. 16C).

Characterizing gene expression trajectories during limb apical ectodermal ridge (AER) development

As an example of what can be accomplished with detailed subtype annotation and exploration, we focused on the epithelium (cluster 6), and in particular the apical ectodermal ridge (subcluster 6.25). Based on subtype-specific marker genes, we annotated the 29 subtypes of epithelium (cluster 6; Fig. 17A; Fig. 18A, Data not shown). For example, epithelial cells in subtype 6.10 were marked by *Oc90*, a gene exclusively expressed in the epithelium of the otic vesicle[35], whereas epithelial cells in subtype 6.25 showed increased expression of the well-characterized maker genes *Fgf8, Msx2,* and *Rspo2,* specific to the apical ectodermal ridge (AER), a highly specialized epithelium involved in digit development[36]. For all epithelial subtypes, we identified genes not previously known to be markers. For example, the AER was also distinguished by expression of *Fndc3a, Adamts3, Slc16a10, Snap91,* and *Pou6f2.* Whole-mount *in situ* hybridization (WISH) of *Fgf8* (a known marker) and *Fndc3a* (a novel marker) confirmed that both genes are expressed in the most distal tip of the limb bud representing the AER at E10.5 (Fig. 17B-E).

We next examined the dynamics of cell proliferation and gene expression during AER development. We identified a total of 1,237 AER cells, representing only 0.06% of our overall dataset but contributed to by nearly every embryo (45 of 61 with over 5 AER cells profiled). Although AER cells are detected at all timepoints, we observe them to be at their peak in terms of cellular proportion per embryo at E9.5 and to decline thereafter (Fig. 17F), consistent with previous reports[37] and our own *in situ* validation studies (Fig. 17C). To characterize the dynamics of gene expression within the AER during development, we performed pseudotemporal ordering of AER cells based on the top 500 differentially expressed genes among developmental stages, yielding a simple early-to-late trajectory (Fig. 17G). 710 protein-coding genes were differentially expressed along developmental pseudotime (FDR of 5%) (Data not shown). For example, *Fgf9,* known to exhibit AER-specific expression in the limb bud[38], showed delayed activation dynamics compared with *Fgf8* and *Fndc3a* (Fig. 17H). Significantly activated genes may play important roles in AER cell differentiation. For example, the activated genes include *Rspo2,* known to be critical for maintenance of AER and for growth and patterning in limb development[39] (Fig. 17H).

We also identified genes whose expression significantly decreased within AER cells between E9.5 and E13.5 (169 genes at an FDR of 1%; Fig. 19A). These include Ki67 (*Mki67*) and Insulin-like growth factor 2 (*Igf2*), both of which have roles in promoting cellular proliferation[40,41] (Fig. 17H). Indeed, consistent with the cessation of proliferation by the AER during this developmental window, pathway-level analyses of significantly decreasing genes highlight terms related to cell cycle progression and glucose metabolism, as well as transcription factors associated with pluripotency (*Isl1*, *Pou5f1*, *Nanog*) (Fig. 19B-C).

Characterizing cell fate trajectories during limb mesenchyme development

We next sought to investigate the developmental trajectories that cell types traverse during this critical period of mammalian development, including transitions between cell types and subtypes. Most contemporary algorithms for pseudotemporal trajectory reconstruction suffer from two major limitations. First, they assume that cells reside on a single continuous manifold, *i.e.* with no discontinuities between subsets of cells. However, because our earliest embryos derive from E9.5, our dataset does not contain cells corresponding to at least some ancestral states. Second, they assume that the underlying trajectory is a tree in which branch points correspond to fate decisions. However, some tissues are known to contain transcriptionally indistinguishable cells contributed by transcriptionally distinct lineages, *i.e.* the convergence of trajectories separated by one or several branching events.

To address these limitations, we developed a new algorithm, incorporated in the Monocle package[42], for resolving multiple disjoint trajectories while also allowing for both branching and convergence within trajectories. Monocle 3 begins by projecting the cells onto a low-dimensional space encoding transcriptional state using Uniform Manifold Approximation and Projection (UMAP)[43]. Monocle 3 then detects communities of mutually similar cells using the Louvain clustering, and merges adjacent communities using a statistical test introduced in the approximate graph abstraction (AGA) algorithm[44]. Importantly, these procedures allow for the maintenance of multiple, disjoint communities of cells. The final step in Monocle 3 aims to resolve the paths that individual cells can take during development, pinpointing the locations of not only branches but also convergences within the set of cells that comprise each community, *i.e.* trajectories. We previously described a procedure called 'L1-graph' for embedding a 'principal graph' within a projection of single-cell RNA-seq profiles, such that every cell is near some point on the graph[45]. Although L1-graph was able to learn trajectories with closed loops and branches, it could only run on datasets with a few hundred cells. To enable the algorithm to process thousands or even millions of cells, we implemented two enhancements. First, we run it on several hundred centroids of the data rather than the cells themselves. Second, we constrain the algorithm's linear programming procedure to respect boundaries between the disjoint trajectories defined by the AGA test.

We first sought to apply this new algorithm to a single major cell type, cluster 25, whose 26,559 cells we annotate as limb bud mesenchyme on the basis of *Hoxd13, Fgf10* and *Lmx1b* expression (Data not shown). Visualizing the trajectory of cells of this cluster with Monocle 3 illustrates the dramatic expansion of limb mesenchymal cells over developmental time, with the main outgrowth between E10.5 and E12.5 (Fig. 20A). Gene expression is anything but static during this expansion, with the levels of 4,763 protein-coding genes significantly changing (FDR of 1%; Data not shown). The early stages of limb mesenchyme development are characterized by some expected genes such as *Tbx15*[46], and *Gpc3*[47] and the later stages by *Msx1*[48], *Epha4*[49] and *Dachl*[50] (Fig. 21A), but the vast majority of dynamically expressed genes are novel. Transcription factors significantly upregulated during limb mesenchyme development included those with roles in chondrocyte differentiation *(e.g. Sox9*[51] *and Yap1*[52]*)*, muscle differentiation *(e.g. Tead4*[53]*)*, and wound healing and limb regeneration *(e.g. Smarcd1*[54]*)* (Fig. 21B).

Interestingly, forelimb and hindlimb cells were not readily separated by unsupervised clustering (Fig. 21C) or trajectory analysis (Fig. 22A), but could be distinguished by the mutually exclusive expression of *Tbx5* in forelimb (2,085 cells, 7.9% of all limb mesenchyme cells) and *Pitx1* in hindlimb (1,885 cells, 7.1% of all limb mesenchyme cells) with only 22 cells expressing both markers (0.08% of all limb mesenchyme cells vs. ~0.6% expected if they were independent; Fig. 20B)[55]. 285 genes were differentially expressed between cells assigned to the forelimb and hindlimb in this way (Fig. 20C, Data not shown). Known marker genes such as *Tbx4* and the genes of the Hoxc cluster *(Hoxc4-10)*[56] were upregulated in hindlimb cells as expected, but novel markers were also identified. For example, we observed *Epha3* and *Hs3st3b1* to be 5-fold enriched in forelimb, and *Pcdh17* and *Igf1* to be 3-fold enriched in hindlimb.

Although developmental time is a major axis of variation in the Monocle 3 limb mesenchyme trajectory (Fig. 20A), there is clearly additional structure. At least some of this appears to correspond to the two main spatial axes of limb development: the proximal-distal axis (the primary direction of outgrowth) and the anterior-posterior axis (corresponding to the five digits)[55]. For example, *Sox6* and *Sox9* (proximal)[57,58], *Hoxd13* and *Tfap2b* (distal)[36], *Pax9* and *Alx4* (anterior), and *Shh* and *Hand2* (posterior), were differentially distributed over the Monocle 3 trajectory (Fig. 20D; Fig. 22B). Whole-mount *in situ* hybridization of *Hoxd13* (a known distal marker) and *Cpa2* (a novel marker whose distribution in the Monocle 3 trajectory was similar to that of known distal markers), confirmed that both genes are expressed in the distal limb mesenchyme between E10.5 and E13.5 (Fig. 20F-H). Applying Moran's test for detecting spatial autocorrelation to the limb mesenchymal trajectory revealed 1,191 significantly varying genes (FDR of 1%; Moran's I > 10). These genes clustered into eight patterns of expression, several of which matched the distributions of markers for the proximal-distal and anterior-posterior axes (Fig. 23, Data not shown).

A combined summary of our results for the AER and limb mesenchyme trajectories is shown in Fig. 20I. Although limb development is defined by relatively simple trajectories, our analyses illustrate how this single cell atlas of mouse organogenesis can be used to characterize the spatiotemporal dynamics of gene expression in specific systems.

Delineation and characterization of the major cell lineages of mouse organogenesis

We next sought to identify major developmental lineages and cellular trajectories across the entire dataset. Monocle 3 organized sampled 100,000 high quality cells (UMI > 400) into eight well-separated lineages (Fig. 24A, Fig. 25A). Nearly all of the 38 major cell types fall almost exclusively in one of these eight groups (Fig. 24B). The exceptions are three of the four smallest clusters, monocytes/granulocytes (36 cells), lens (125 cells) and megakaryocytes (287 cells), probably consequent to their low numbers. The two most complex structures are clearly the *mesenchymal trajectory,* which includes all mesenchymal and muscle cell types (left of Fig. 24A and Fig. 25A), and the *neural tube/notochord trajectory,* which includes the notochord, neural tube, progenitor and developing neuronal and glial cell types (right of Fig. 24A and Fig. 25A). A first neural crest trajectory ("*neural crest 1*") includes melanocytes and Schwann cell precursors, while a second neural crest trajectory ("*neural crest 2*"), is comprised of sensory neurons. The *hematopoietic trajectory* includes megakaryocytes, erythrocytes and lymphocytes, while the remaining three trajectories (*hepatic, endothelial, epithelial)* each correspond to a single major cell type. Although the estimated number of cells per embryo in each of these lineages increases exponentially from E9.5 to E13.5, their proportions remain relatively stable, with the exception of hepatocytes which expand their contribution by nearly ten-fold during this developmental window (0.3% at E9.5 → 2.8% at E13.5) (Fig. 25B-C).

UMAP projects cells of the same type to defined regions, but unlike t-SNE, also places related cell types near one another. For example, early mesenchymal cells appeared to radiate from a defined region into myocytes, limb mesenchyme, chondrocytes/osteoblasts and connective tissues (Fig. 24A, left). Similarly, cell types found at later developmental timepoints such as glutamatergic neurons are separated from early CNS precursors (e.g. radial glia) by a "bridge" of neural progenitor cells (Fig. 24A, right). On the other hand, discontinuities (*e.g.* between the eight major lineages), likely reflect the lack of representation of intermediate or ancestral states between these groups, consequent to restriction of our study to E9.5-E13.5.

When we separately subjected each of the eight major lineages to trajectory analysis as above, analogous to iterative sub-clustering, the mesenchymal and neural tube/notochord trajectories were again organized as described above (Fig. 26-27, top row), while the other major lineages (epithelial, endothelial, etc.) exhibited multiple discontinuous sublineages, potentially revealing detailed trajectories for subtypes (Fig. 26-27, remaining rows). For example, if we annotate the epithelial trajectory by its 29 subtypes (Fig. 17A), we observe several distinct sub-trajectories, each emanating from a focal concentration of E9.5-derived cells, underlying subsets of epithelial subtypes (Fig. 24C, Fig. 28). For example, the epithelial cells of the apical ectodermal ridge (Fig. 17G) form a linear sub-trajectory from E9.5 to E13.5 cells that is well separated from other epithelial sub-trajectories (Fig. 24C, bottom center).

Reconstructing cellular trajectories during skeletal myogenesis

Considerable further work is necessary to fully elucidate the relationships between cell types and subtypes that comprise the trajectories represented in Fig. 24, particularly the more complex ones. As a representative example of what may be possible, we sought to examine in greater detail developing muscle tissue, which is comprised of distinct mesodermal lineages that form prior to the onset of organogenesis. For example, the extraocular muscles are contributed by the prechordal mesoderm, while others of the face and jaw are generated by the pharyngeal mesoderm. Skeletal myogenesis is driven by a core set of myogenic regulatory factors (MRFs) which are activated by distinct sets of upstream genes[59]. For example, *Pax3* activates *Myod1* in the trunk muscles, whereas in the head, *Pax3* is dispensable and the MRFs are activated by *Pitx2* and *Tbx1*[60-62]. *Myod1* or *Myf5* in turn activate myogenin, which drives the expression of numerous genes required by contractile skeletal muscle. We hypothesized that the myogenic trajectory, when viewed at the scale of a whole embryo, would feature multiple entry points that feed cells into a common path corresponding to activation of the core gene expression program shared by myotubes.

To test this hypothesis, we isolated myocytes and their putative "ancestral" cells from the mesenchyme trajectory by first quantifying the fraction of cells at each principal graph node that were classified as myocytes (cluster 13). We collected all 'majority myocyte' nodes and then used the principal graph's edges to expand this set of nodes into wider "neighborhood" of cells (Fig. 29A). Next, we re-ran Monocle 3 on this subset of cells to construct a myogenesis-specific trajectory. This trajectory featured multiple focal concentrations of cells from E9.5, with cells from later stages distributed over several paths radiating outward (Fig. 29B) *Pax3* and *Pax7,* which mark skeletal muscle progenitors, were expressed in cells distributed over a broad swath of the principal graph (Fig. 29C). Two parallel linear segments emanate from this region of the graph, on which cells expressed either *Myf5* or *Myod.* Both paths feed into a common region occupied by cells expressing *Myog* or *Myhi,* markers of myocytes and myotubes, respectively. An additional path traversed by cells from E9.5, which expressed *Lhx2, Tbx1,* and *Pitx2* but very low levels of *Pax3,* feeds into the trajectory just upstream of the *Myf5* and *Myod1* segments, possibly corresponding to pharyngeal mesoderm. Thus, dynamics of the MRFs and their upstream activators over the trajectory was consistent with the view that different mesodermal lineages use distinct factors to converge on core program of muscle genes (Fig. 29D).

Discussion

In this study, we sought to characterize mammalian development by profiling the transcriptomes of single cells at the scale of the whole mouse embryo, focusing on window that corresponds to classic organogenesis. By profiling over 2,000,000 cells from 61 individual embryos in a single experiment with sci-RNA-seq3, we also provide the technical framework for small labs to generate single cell RNA-seq datasets with unprecedented throughput. To resolve branching, convergence, and discontinuities in developmental trajectories, we present Monocle 3, a novel algorithm for trajectory inference that scales to millions of cells.

In mid-gestational mouse embryos, we identify 38 major cell types and over 600 subtypes. Each of these types and subtypes are characterized by the expression of sets of marker genes, the vast majority of which are novel, and representative examples of which we validate by whole mount *in situ* hybridization. As an illustration of the utility of deep shotgun cellular coverage to characterize rare cell types, we highlight markers and dynamically expressed genes in the apical ectodermal ridge (AER), a specialized epithelium with a critical role in digit development but only 0.06% of the cells profiled here. The 38 major cell types broadly resolve into 8 trajectories, including mesenchymal, neural tube/notochord, hematopoietic, hepatic, endothelial, epithelial, and two neural crest trajectories. The discontinuity between these eight trajectories is likely a consequence of the lack of representation of ancestral or intermediate states in our dataset, which begins at E9.5. Trajectory analysis of the limb mesenchyme revealed correlates of developmental heterogeneity corresponding both temporal and multiple spatial axes. Focusing on the subset of the mesenchymal trajectory corresponding to myocytes and their progenitors, we identify multiple sub-trajectories that feed into a common endpoint corresponding to myotubes. This example of 'convergence' of expression programs stands in contrast to the branching structure assumed by most algorithms for developmental trajectory inference.

Our study has several limitations that need to be considered. First, as with other single cell atlases, individual cell transcriptome data are sparse. However, previous research have shown that transcriptional programs can be readily distinguished within single cell transcriptome datasets at surprisingly shallow sequencing depths[63]. That we are able to define 655 transcriptionally distinct subtypes with a median of 671 UMIs per cell is consistent with this view, and aggregating transcriptomes with each cell type or subtype enables us to construct representative expression profiles. Second, although we are reasonably confident in most of the cell type assignments made here, they should nonetheless be regarded as preliminary. A key challenge is that mid-gestational mouse development (E9.5-E13.5) has not previously been studied before at single cell resolution nor at a whole organism scale. Existing single cell transcriptional atlases have profiled individual organs of adult mice or later embryonic stages[12,13]. Although we have made significant progress to date, the comprehensive annotation of these 655 cell subtypes is an ongoing project, and one that we anticipate will benefit from community input and domain expertise to arrive at a stable consensus. To that end, we created a wiki to facilitate their annotation by us and the community (available on the world-wide web at atlas.gs.washington.edu/mouse-rna/). A unique page for each subtype includes a downloadable matrix of the cells that comprise it, a list of the marker genes specific to that subtype, and a description of the dynamics of that subtype over the developmental window examined here.

A long-standing goal of the field, perhaps at long last within sight from a technical perspective, is to produce a comprehensive, spatiotemporally-resolved molecular atlas of mammalian development at single cell resolution. Towards this end, focusing on the mouse has several advantages, including its small size, the accessibility of early developmental timepoints, an inbred genetic background, and genetic manipulability. By profiling a number of cells corresponding to a substantial percentage of cellular content of an individual mouse embryo (3 to 80% 'shotgun cellular coverage' per stage), these data constitute a powerful resource for the developmental biology field, and may also help to further advance the development of computational methods for resolving and interpreting cell types or development trajectories. Looking ahead, we anticipate that the integrated measurement of the transcriptome, additional molecular phenotypes[64], lineage history[65] and spatial information will further give shape to a global view of mammalian development.

We close by noting that single cell atlases of the development of wild type mice also represent a first step towards understanding pleiotropic developmental disorders at the organismal scale, as well as for detailed investigations of subtle roles for genes and regulatory sequences in development. For example, whereas ~35% of gene knockouts in mouse are lethal[5], many knockouts, and in particular those of conserved regulatory sequences, do not show any abnormalities with conventional phenotyping[66]. We anticipate that organism-scale sc-RNA-seq will empower reverse genetics, e.g. potentially enabling the discovery of previously missed phenotypes with subtle defects in the molecular programs or the relative proportions of specific cell types[67].

METHODS

Embryo dissection

The C57BL/6 mice were obtained from The Jackson Laboratory (Bar Harbor, ME) and plug matings were set up. Day of plugging was considered as embryonic day (E) 0.5. Dissections were done as previously described[69] and all embryos were immediately snap frozen in liquid nitrogen. All animal procedures were in accordance with institutional, state, and government regulations (IACUC protocol 4378-01).

Whole-mount in situ hybridization

The mRNA expression in E9.5-E11.5 mouse embryos was assessed by whole mount in situ hybridisation (WISH) using a digoxigenin-labeled antisense riboprobe transcribed from a cloned gene specific probes (PCR DIG Probe Synthesis Kit, Roche). Whole embryos were fixed overnight in 4% PFA/PBS. The embryos were washed in PBST (0.1% Tween), and dehydrated stepwise in 25%, 50% and 75% methanol/PBST and finally stored at -20°C in 100% methanol. The WISH protocol was as follows: Day 1) Embryos were rehydrated on ice in reverse methanol/PBST steps, washed in PBST, bleached in 6% $H_2O_2$/PBST for 1 hour and washed in PBST. Embryos were then treated in 10 $\mu$g/ml Proteinase K/PBST for 3 minutes, incubated in glycine/PBST, washed in PBST and finally re-fixed for 20 minutes with 4% PFA/PBS, 0.2% glutaraldehyde and 0.1% Tween 20. After further washing steps with PBST, embryos were incubated at 68°C in L1 buffer (50% deionised formamide, 5x SSC, 1% SDS, 0.1% Tween 20 in DEPC; pH 4.5) for 10 minutes. Next, embryos were incubated for 2 hours at 68°C in hybridisation buffer 1 (L1 with 0.1% tRNA and 0.05% heparin). Afterwards, embryos were incubated o.n. at 68°C in hybridisation buffer 2 (hybridisation buffer 1 with 0.1% tRNA and 0.05% heparin and 1:500 DIG probe). Day 2) Removal of unbound probe was done through a series of washing steps 3x30 minutes each at 68°C: L1, L2 (50% deionised formamide, 2x SSC pH 4.5, 0.1% Tween 20 in DEPC; pH 4.5) and L3 (2x SSC pH 4.5, 0.1% Tween 20 in DEPC; pH 4.5). Subsequently, embryos were treated for 1 hour with RNase solution (0.1 M NaCl, 0.01 M Tris pH 7.5, 0.2% Tween 20, 100 $\mu$g/ml RNase A in $H_2O$), followed by washing in TBST 1 (140mM NaCl, 2.7mM KCl, 25mM Tris-HCl, 1% Tween 20; pH 7.5). Next, embryos were blocked for 2 hours at RT in blocking solution (TBST 1 with 2% calf-serum and 0.2% BSA), followed by incubation at 4°C o.n. in blocking solution containing 1:5000 Anti-Digoxigenin-AP. Day 3) Removal of unbound antibody was done through a series of washing steps 8x 30 min at RT with TBST 2 (TBST with 0.1% Tween 20, and 0.05% levamisole/tetramisole) and left o.n. at 4°C. Day 4) Staining of the embryos was initiated by washing at RT with alkaline phosphatate buffer (0.02 M NaCl, 0.05 M $MgCl_2$, 0.1% Tween 20, 0.1 M Tris-HCl, and 0.05% levamisole/tetramisole in $H_2O$) 3x 20 minutes, followed by staining with BM Purple AP Substrate (Roche). The stained embryos were imaged using a Zeiss Discovery V.12 microscope and Leica DFC420 digital camera.

Mammalian cell culture

All mammalian cells were cultured at 37°C with 5% CO2, and were maintained in high glucose DMEM (Gibco cat. no. 11965) for HEK293T and NIH/3T3 cells, both supplemented with 10% FBS and 1X Pen/Strep (Gibco cat. no. 15140122; 100U/ml penicillin, 100μg/ml streptomycin). Cells were trypsinized with 0.25% typsin-EDTA (Gibco cat. no. 25200-056) and split 1: 10 three times a week.

Mouse embryo nuclei extraction and fixation

Mouse embryos from different development stages were processed together to reduce batch effect. Each mouse embryo was minced into small pieces by blade in 1 mL ice-cold cell lysis buffer (10 mM Tris-HCl, pH 7.4, 10 mM NaCl, 3 mM MgCl2 and 0.1% IGEPAL CA-630 from[70], modified to also include 1% SUPERase In and 1% BSA) and transferred to the top of a 40um cell strainer (Falcon). Tissues were homogenized with the rubber tip of a syringe plunger (5ml, BD) in 4ml cell lysis buffer. The filtered nuclei were then transferred to a new 15ml tube (Falcon) and pelleted by centrifuge at 500xg for 5min and washed once with 1ml cell lysis buffer. The nuclei were fixed in 4ml ice cold 4% paraformaldehyde (EMS) for 15min on ice. After fixation, the nuclei were washed twice in 1ml nuclei wash buffer (cell lysis buffer without IGEPAL), and re-suspended in 500ul nuclei wash buffer. The samples were split to two tubes with 250ul in each tube and flash frozen in liquid nitrogen.

As quality control, HEK293T and NIH/3T3 cells were trypsinized, spun down at 300xg for 5 min (4°C) and washed once in 1XPBS. Equal cell number of HEK293T and NIH/3T3 cells were combined and lysed using 1 mL ice-cold cell lysis buffer followed by the same fixation and storage condition as in mouse embryo.

sci-RNA-seq3 library preparation and sequencing

Thawed nuclei are permeabilized with 0.2% tritonX-100 (in nuclei wash buffer) for 3 minutes on ice, and briefly sonicated (Diagenode, 12s on low power mode) to reduce nuclei clumping. The nuclei were then washed once with nuclei wash buffer and filtered through 1ml Flowmi cell strainer (Flowmi). Filtered nuclei were spun down at 500xg for 5min and resuspended in nuclei wash buffer.

Nuclei from each mouse embryo were then distributed into several individual wells in four 96-well plates. The links between well id and mouse embryo were recorded for downstream data processing. For each well, 80,000 nuclei (16 μL) were mixed with 8 μl of 25 μM anchored oligo-dT primer (5'- /5Phos/CAGAGCNNNNNNNN[10bp bar-code]TTTTTTTTTTTTTTTTTTTTTTTTTTTTTTVN-3' (SEQ ID NO:1), where "N" is any base; IDT) and 2 μL 10 mM dNTP mix (Thermo), denatured at 55°C for 5 min and immediately placed on ice. 14 μL of first-strand reaction mix, containing 8 μL 5X Superscript IV First-Strand Buffer (Invitrogen), 2 μl 100 mM DTT (Invitrogen), 2 μl SuperScript IV reverse transcriptase (200 U/μl, Invitrogen), 2 μL RNaseOUT Recombinant Ribonuclease Inhibitor (Invitrogen), was then added to each well. Reverse transcription was carried out by incubating plates by gradient temperature (4°C 2 minutes, 10°C 2 minutes, 20°C 2 minutes, 30°C 2 minutes, 40°C 2 minutes, 50°C 2 minutes and 55°C 10 minutes).

After RT reaction, 60μL nuclei dilution buffer (10 mM Tris-HCl, pH 7.4, 10 mM NaCl, 3 mM MgCl2 and 1% BSA) was added into each well. Nuclei from all wells were pooled together and spun down at 500xg for 10min. Nuclei were then resuspended in nuclei wash buffer and redistributed into another four 96-well plates with each well including 4μL T4 ligation buffer (NEB), 2μL T4 DNA ligase (NEB), 4μL Betaine solution (5M, Sigma-Aldrich), 6μL nuclei in nuclei wash buffer, 8μL barcoded ligation adaptor (100uM, 5'-GCTCTG[9bp or 10bp barcode A]/ideoxyU/ACGACGCTCTTCCGATCT[reverse complement of barcode A]-3') (SEQ ID NO:2) and 16μL 40% PEG 8000 (Sigma-Aldrich). The ligation reaction was done at 16°C for 3 hours.

After RT reaction, 60μL nuclei dilution buffer (10 mM Tris-HCl, pH 7.4, 10 mM NaCl, 3 mM MgCl2 and 1% BSA) was added into each well. Nuclei from all wells were pooled together and spun down at 600xg for 10min. Nuclei were washed once with nuclei wash buffer and filtered with 1ml Flowmi cell strainer (Flowmi) twice, counted and redistributed into eight 96-well plates with each well including 2,500 nuclei in 5μL nuclei wash buffer and 5μL elution buffer (Qiagen). 1.33 μl mRNA Second Strand Synthesis buffer (NEB) and 0.66 μl mRNA Second Strand Synthesis enzyme (NEB) were then added to each well, and second strand synthesis was carried out at 16°C for 180 min.

For tagmentation, each well was mixed with 11 μL Nextera TD buffer (Illumina) and 1 μL i7 only TDE1 enyzme (62.5nM, Illumina), and then incubated at 55°C for 5 min to carry out tagmentation. The reaction was then stopped by adding 24 μL DNA binding buffer (Zymo) per well and incubating at room temperature for 5 min. Each well was then purified using 1.5x AMPure XP beads (Beckman Coulter). In the elution step, each well was added with 8μL nuclease free water, 1μL 10X USER buffer (NEB), 1μL USER enzyme (NEB) and incubated at 37°C for 15 min. Another 6.5μL

elution buffer was added into each well. The AMPure XP beads were removed by magnetic stand and the elution product was transferred into a new 96-well plate.

For PCR amplification, each well (16μL product) was mixed with 2μL of 10 μMindexed P5 primer (5'-AATGA-TACGGCGACCACCGAGATCTACAC[i5]ACACTCTTTCCCTACACGACGC TCTTCCGATCT-3 ; IDT) (SEQ ID NO:3), 2 μL of 10 μM P7 primer (5'-CAAGCAGAAGACGGCATACGAGAT[i7]GTCTCGTGGGCTCGG-3', IDT) (SEQ ID NO:4), and 20 μL NEBNext High-Fidelity 2X PCR Master Mix (NEB). Amplification was carried out using the following program: 72°C for 5 min, 98°C for 30 sec, 12-14 cycles of (98°C for 10 sec, 66°C for 30 sec, 72°C for 1 min) and a final 72°C for 5 min.

After PCR, samples were pooled and purified using 0.8 volumes of AMPure XP beads. Library concentrations were determined by Qubit (Invitrogen) and the libraries were visualized by electrophoresis on a 6% TBE-PAGE gel. All libraries were sequenced on one NovaSeq platform (Illumina) (Read 1: 34 cycles, Read 2: 52 cycles, Index 1: 10 cycles, Index 2: 10 cycles).

Sequencing reads processing

Base calls were converted to fastq format using Illumina's bcl2fastq and demultiplexed based on PCR i5 and i7 barcodes using maximum likelihood demultiplexing package deML[71] with default settings. Downstream sequence processing and single cell digital expression matrix generation were similar with sci-RNA-seq[19] except that RT index was combined with hairpin adaptor index, and thus the mapped reads were split into constituent cellular indices by demultiplexing reads using both the RT index and ligation index (ED < 2, including insertions and deletions). Briefly, demultiplexed reads were filtered based on RT index and ligation index (ED < 2, including insertions and deletions) and adaptor clipped using trim_galore/0.4.1 with default settings. Trimmed reads were mapped to the mouse reference genome (mm10) for mouse embryo nuclei, or a chimeric reference genome of human hg19 and mouse mm10 for HEK293T and NIH/3T3 mixed nuclei, using STAR/v 2.5.2b[72] with default settings and gene annotations (GENCODE V19 for human; GENCODE VM11 for mouse). Uniquely mapping reads were extracted, and duplicates were removed using the unique molecular identifier (UMI) sequence, reverse transcription (RT) index, hairpin ligation adaptor index and read 2 end-coordinate (*i.e.* reads with identical UMI, RT index, ligation adaptor index and tagmentation site were considered duplicates). Finally, mapped reads were split into constituent cellular indices by further demultiplexing reads using the RT index and ligation hairpin (ED < 2, including insertions and deletions). For mixed-species experiment, the percentage of uniquely mapping reads for genomes of each species was calculated. Cells with over 85% of UMIs assigned to one species were regarded as species-specific cells, with the remaining cells classified as mixed cells or "collisions". To generate digital expression matrices, we calculated the number of strand-specific UMIs for each cell mapping to the exonic and intronic regions of each gene with python HTseq package[73]. For multi-mapped reads, reads were assigned to the closest gene, except in cases where another intersected gene fell within 100 bp to the end of the closest gene, in which case the read was discarded. For most analyses we included both expected-strand intronic and exonic UMIs in per-gene single-cell expression matrices.

Whole mouse embryo analysis

After the single cell gene count matrix was generated, each cell was assigned to its original mouse embryo based on the RT barcode. Reads mapping to each embryo were aggregated to generate "bulk RNA-seq" for each embryo. For sex separation of embryos, we counted reads mapping to female specific non-coding RNA(Xist) or chr Y genes (except gene Erdr1 which is in both chr X and chr Y). Embryos were readily separated into female population (more reads mapping to Xist than chr Y genes) and male group (more reads mapping to chr Y genes than Xist).

Pseudotemporal ordering of whole mouse embryos was done by Monocle 2[74]. Briefly, an aggregated gene expression matrix was constructed as described above. Differentially expressed genes across different development conditions were identified with differentialGeneTest function of Monocle 2[74]. The top 2,000 genes with the lowest q value were used to construct the pseudotime trajectory using Monocle 2[74]. Each embryo was assigned a pseudo-time value based on its position along the trajectory tree.

Cell clustering, t-SNE visualization and marker gene identification

A digital gene expression matrix was constructed from the raw sequencing data as described above. Cells with less than 200 UMIs were discarded. Downstream analysis were performed with Monocle2[74] and python package scanpy[75]. Briefly, gene count mapping to sex chromosomes were removed before clustering and dimension reduction. Preprocessing step is similar to the approach used by Zheng *et al*[22] by "zheng17 recipe" function (n_top_genes = 2,000) in scanpy[75]. The dimension of the data was reduced by PCA (30 components) first and then with t-SNE, followed by Louvain clustering performed on the 30 principal components (resolution=1.5). 40 clusters were identified. We then sampled 1,000 cells from each cluster and differentially expressed genes across different clusters were identified with differentialGeneTest function of Monocle 2[74]. Genes specific to each cluster were identified similar s before[76]. clusters were assigned to known cell types based on cluster specific markers (Table 1). One cluster had abnormally high UMI counts but no strongly cluster-specific genes, suggesting that it may be a technical artifact of cell doublets and thus get removed. Another two clusters both appeared to correspond to definitive erythroid lineage and are merged. Consensus expression profiles for each cell type were constructed as in [76]. To identify cell type specific gene marker, we selected gene that were differentially expressed across different cell types (FDR of 5%, likelihood ratio test) and also has maximum expression in each cell type with at least 2-fold increase compared to other cell type with the second maximum expression.

Table 1

| Cluster Cell type | Markers used for cell type identification | Literature |
| --- | --- | --- |
| 1 Connective tissue progenitors | Il 1rapl2, Meox2, Tgfb2, Adamts9, Postn, Ror1 | (available on the world-wide web at www.ncbi.nlm.nih.gov/ pubmed/25249460) |
| 2 Chondrocytes | Runx2, Twist2, Prrx1 | (available on the world-wide web at www.ncbi.nlm.nih.gov/pmc/ articles/PMC4990491/) |
| 3 Intermediate Mesoderm | Wt1, Mylk, Ednra | (available on the world-wide web at www.ncbi.nlm.nih.gov/ pubmed/25480331) |
| 4 Jaw and tooth progenitors | Sox9, Foxp2, Col2a1,Col9a1, Col11a1, Pax9 | (available on the world-wide web at www.ncbi.nlm.nih.gov/ pubmed/26969076) |
| 5 Mesencephalon neurons | Car10, Mapt, Ntng1, Snhg11 | (available on the world-wide web at www.cell.com/cell/ comments/S0092-8674(18)30116-8) |
| 6 Epithelial cells | Epcam, Trp63, Grh12, | (available on the world-wide web at www.cell.com/cell/ comments/S0092-8674(18)30116-8) |
| 7 Radial glia | Pth2r, Fabp7, Pax3, Fzd10, Hes5 | (available on the world-wide web at www.cell.com/cell/ comments/S0092-8674(18)30116-8) |
| 8 Early mesenchyme | Gpc5, Smoc1 | (available on the world-wide web at www.ncbi.nlm.nih.gov/ pubmed/21194678) |
| 9 Neural progenitor cells | Prmt8, Gadd45g, Cdkn1c, Btg2 | (available on the world-wide web at www.cell.com/cell/ comments/S0092-8674(18)30116-8) |
| 10 Postmitotic neurons | Tmem163, Slc17a6 | (available on the world-wide web at www.cell.com/cell/ comments/S0092-8674(18)30116-8) |
| 11 Oligodendrocyte Progenitors | Slc17a6, Sox1, Olig2, Nkx2-1 | (available on the world-wide web at www.ncbi.nlm.nih.gov/pmc/ articles/PMC3472585/) |
| 12 Midbrain/ forebrain progenitors | Egfem1, EN2, Fgf15 | (available on the world-wide web at www.ncbi.nlm.nih.gov/ pubmed/10640701?dopt=Abstract) |
| 13 Myocytes | Neb, Myh3, Tpm2, Acta2 (smooth muscle) | (available on the world-wide web at www.ncbi.nlm.nih.gov/ pubmed/24509862) |
| 14 Neural Tube | Foxb1, Scube2, Prtg | (available on the world-wide web at www.ncbi.nlm.nih.gov/ pubmed/18064677) |
| 15 Glutamatergic neurons | Grm5, Nxph1, Ndn, Pcsk1n, Cdh23, Grin2b | (available on the world-wide web at www.cell.com/cell/ comments/S0092-8674(18)30116-8) |
| 16 Stromal cells | Il23a, Bmpr1a, Prtg | (available on the world-wide web at www.ncbi.nlm.nih.gov/pmc/ articles/PMC4307917/) |
| 17 Osteoblasts | Col1a1, Camk1d, Rbm8a | (available on the world-wide web at www.ncbi.nlm.nih.gov/ pubmed/15042706?dopt=Abstract) |

(continued)

| Cluster Cell type | Markers used for cell type identification | Literature |
|---|---|---|
| 18 Hippocampus neurons | Pax2, Lhx1, Cdh22, Asic4 | (available on the world-wide web at www.cell.com/cell/comments/S0092-8674(18)30116-8) |
| 19 Premature oligodendrocytes | Id4, Gpc1 | (available on the world-wide web at www.ncbi.nlm.nih.gov/pmc/articles/PMC305683/) (available on the world-wide web at www.ncbi.nlm.nih.gov/pmc/articles/PMC5650988/) |
| 20 Endothelial cells | Ptprb, Pecam1, Vwf, Klhl4, Hbegf, Egfl7 (Kidney) | (available on the world-wide web at www.ncbi.nlm.nih.gov/pmc/articles/PMC305683/) |
| 21 Chondroctye progenitors | ITGA11, ATP1A2, Lamc3, Epha7 | (available on the world-wide web at journals.plos.org/plosone/article?id=10.1371(journal.pone.0082035) |
| 22 Definitive erythrocyte lineage | Snca, Hbb-bs, Abcb4, Slc4a1, Kel, HBB-y, Hba-x, | (available on the world-wide web at www.cell.com/cell/comments/S0092-8674(18)30116-8) |
| 23 Schwann cell precursors | Plp1, Cdh19 | (available on the world-wide web at www.ncbi.nlm.nih.gov/pmc/articles/PMC4484967/) |
| 24 Sensory neurons | Syt13, Shox2, Ptprr, Pcbp3 | (available on the world-wide web at www.cell.com/cell/comments/S0092-8674(18)30116-8) |
| 25 Limb mesenchyme | Msx1, Fgf10, Wnt5a, Lmx1b | (available on the world-wide web at www.ncbi.nlm.nih.gov/pubmed/9106164?dopt=Abstract) |
| 26 Primitive erythroid lineage | Hba-a1, HBB-y, Hba-x, Hbb-bh1, | (available on the world-wide web at www.cell.com/cell/comments/S0092-8674(18)30116-8) |
| 27 Basal forebrain neurons | Arx, DLX1, DLX2 | (available on the world-wide web at www.ncbi.nlm.nih.gov/pubmed/9256348?dopt=Abstract) |
| 28 Pyramidal neuron cells | Neurod2, Neurod6, Tiam2, Kcnq3 | (available on the world-wide web at www.cell.com/cell/comments/S0092-8674(18)30116-8) |
| 29 Hapatocytes | Afp,Alb, Apoa2, Afp29, Pik3c2g, Hoga1, TFEB | (available on the world-wide web at www.cell.com/cell/comments/S0092-8674(18)30116-8) |
| 30 Notochord cells | Shh, Slit1, Ntn1 | (available on the world-wide web at www.cell.com/trends/genetics/pdf/50168-9525(04)00016-2.pdf |
| 31 Lymphocytes | Apoe, Lyz2, Selenop, Ptprc, Ly86, CTSS | (available on the world-wide web at www.cell.com/cell/comments/S0092-8674(18)30116-8) |
| 32 Ependymal cells | Sostdc1, Htr2c, Kcne2, Ttr | (available on the world-wide web at www.cell.com/cell/comments/S0092-8674(18)30116-8) |
| 33 Cholinergic neurons | Slit2, Slit3, Chat | (available on the world-wide web at www.ncbi.nlm.nih.gov/pubmed/10349621?dopt=Abstract) |
| 34 Cardiac muscle lineage | Myl2, Myocd, Hcn4, Ctnna3, Ryr2, Tbx20 | (available on the world-wide web at onlinelibrary.wiley.com/doi/10.1002/dvg.22819/epdf) |
| 35 Megakaryocytes | Pf4, Itgb3, Itga2b, Ppbp, Cd226 | (available on the world-wide web at www.cell.com/cell/comments/S0092-8674(18)30116-8) |
| 36 Melanocytes | Tyr,TRPM1, Pmel | (available on the world-wide web at www.ncbi.nlm.nih.gov/pubmed/1557387?dopt=Abstract) |
| 37 Lens | GJA8, Cryba1, CRYAA | (available on the world-wide web at ghr.nlm.nih.gov/gene/GJA8) |
| 38 Monocytes/ granulocytes | Ngp, S100A8 | (available on the world-wide web at www.cell.com/cell/comments/S0092-8674(18)30116-8) |

For sub cluster identification, we selected high quality cells (UMI > 400) in each main cell type and applied PCA, t-SNE, Louvain clustering similarly with the general cluster analysis. Highly biased subclusters were filtered out if most cells (>50%) of the cluster were from a single embryo. Highly similar subclusters were merged if their aggregated transcriptomes were highly correlated (Pearson correlation coefficient > 0.95) and the two clusters were close with each other on t-SNE space. Differentially expressed genes across sub clusters were identified for each main cell type as described above.

For cell number estimation of each cell type (or sub cell types), we first calculated the proportion of each cell type in individual embryo, and then multiplied the proportion with estimated total cell number for each embryo (E9.5: 200,000; E10.5: 1,100,000; E11.5: 2,600,000; E12.5: 6,100,000; E13.5: 13,000,000).

To identify sex specific cell types (or sub cell types), we first calculated cell number in each cell type (sub cell type) for male and female across five developmental stages. The cell type specific ratio between male and female was compared with overall cell number ratio between male and female in each developmental stage. We then applied binomial test in R to identify cell types or sub cell types with significant difference between male and female in each cell type (x and n are the number of female cells and total cells in each cell types from each developmental stage, p is the female cell ratio in each development stage). The p-value is converted into adjusted q-value by Benjamini & Hochberg method with p.adjust function in R.

AER and limb mesenchyme pseudo-time analysis

Pseudotemporal ordering of AER cells, forelimb or hidnlimb was done by Monocle 2[74]. Briefly, differentially expressed genes across five development stages were identified with the differentialGeneTest function of Monocle 2[74]. The top 500 genes with the lowest q value were used to construct the pseudotime trajectory using Monocle 2[74], with UMI count per cell as a covariate in the tree construction. Each cell was assigned a pseudotime value based on its position along the trajectory tree. Smoothed gene marker expression change along pseudotime were generated by plot_genes_in_pseudotim function in Monocle 2[74]. Cells in the trajectory were grouped in the same method as[77]. Briefly, cells were grouped first at similar positions in pseudotime by k-means clustering along the pseudotime axis (k = 10). These clusters were subdivided into groups containing at least 50 and no more than 100 cells. We then aggregated the transcriptome profiles of cells within each group. The gene expression along pseudotime was calculated in the same approach as[77]. Briefly, genes passing significant test (FDR of 5%) across different treatment conditions were selected and a natural spline was used to fit the gene expression along pseudotime, with mean_number_genes included as a covariate. The gene expression for each gene was subtracted by the lowest expression and then divided by the highest expression. Genes with max expression within the early 20% of pseudotime were labeled as activated genes. Genes with max expression in the last 20% of pseudotime were labeled as repressed genes. Other genes were labeled as transient genes. Enriched reactome terms (Reactome_2016) and transcription factors (ChEA_2016) were identified using EnrichR package[78].

Trajectory inference with Monocle 3

The Monocle 3 workflow consists of 3 core steps to organize cells into potentially discontinuous trajectories, followed by optional statistical tests to find genes that vary in expression over those trajectories. Monocle 3 also includes visualization tools to help explore trajectories in three dimensions.

Dimensionality reduction with Uniform Manifold Approximation and Projection (UMAP)

Monocle 3 first projects the data into a low-dimensional space, which facilitates learning a principal graph that describes how cells transit between transcriptomic states. Monocle 3 does so with UMAP, a recently proposed algorithm based on Riemannian geometry and algebraic topology to perform dimension reduction and data visualization [79]. Its visualization quality is competitive with the popular t-SNE (t-stochastic neighbor embedding) method used widely in single-cell transcriptomics. However, where t-SNE mainly aims to place highly similar cells in the same regions of a low-dimensional space, UMAP also preserves longer-range distance relationships. The UMAP algorithm itself is also more efficient (the algorithm complexity of UMAP is $O(N)$ vs. $O(Nlog(N))$ for t-SNE). Briefly, UMAP first constructs a topological representation of the high dimensional data with local manifold approximations and patches together their local fuzzy simplicial set representations. UMAP then optimizes the lower dimension embedding, minimizing the cross-entropy between the low dimensional representation and the high dimensional one.

The computational efficiency of UMAP dramatically accelerated the analysis of the mouse embryo data. We found that UMAP finishes analyzing two million cells dataset in 3 hours while t-SNE takes more than 10 hours with 10 cores (the multi-core bh-t-SNE is used). A few implementation details leads to the effectiveness of UMAP. Two major steps are involved in both UMAP and t-SNE algorithms: firstly, an intermediate structure from the high dimension space (normally the top PCA reduced space) is built and then a low dimensional embedding is found to represent the intermediate structure. For the second step, both methods used stochastic grid descent approach with differing loss functions to embed the data into low dimension space. While t-SNE needs a loss function for global normalization, UMAP uses a different objective function that avoids that need. This step essentially enables UMAP scales linear with the number of data samples. In Monocle 3, we interact with the UMAP python implementation (available on the world-wide web atgithub.com/lmcinnes/umap) from Leland McInnes and John Healy through the *reticulate* package (available on the world-wide web atcran.r-project.org/web/packages/reticulate/index.html).

Partitioning cells into discontinuous trajectories

Recently, Wolf and colleagues proposed the idea to organize single-cell transcriptome data into an "abstract partition graph" (AGA) that relates clusters of cells that might be developmentally related to one another. Briefly, their algorithm constructs a k-nearest neighbor graph on cells and then identifies "communities" of cell via the Louvain method, similar to previous methods for analyzing CyTOF or single-cell RNA-seq data [80]. AGA then constructs a graph in which the vertices are Louvain communities. Two vertices are linked with an edge in the AGA graph when the cells in the respective communities are neighbors in the kNN graph more frequently than would be expected under a simple binomial model[81]. Similar methods were also recently developed and applied in analyzing zebrafish and xenopus cell atlas datasets[82,83].

Monocle 3 draws from these ideas, first constructing a kNN graph on cells in the UMAP space, then grouping them into Louvain communities, and testing each pair of communities for a significant number of links between their respective cells. Those communities that have more links than expected under the null hypothesis of spurious linkage (FDR < 10%) remain connected in the AGA graph, and those links that fail this test are severed. The resulting AGA graph will have one or more components, each of which is passed to the next step (L1-graph) as a separate group of cells that will be organized in a trajectory. The AGA algorithm essentially stops at this stage, presenting the AGA graph as a kind of coarse-grained trajectory in each community reflects a different state cells can adopt as they develop. In contrast, as described in the next section, Monocle 3 uses the AGA graph to constrain the space of principal graphs that can form the final trajectory. That is, Monocle 3 uses the coarse-grained AGA graph to learn a fine-grained trajectory.

Monocle 3's implementation of the above procedures scale to millions of cells. Briefly, it uses the *clustering_louvain* function from the igraph package to perform community detection. Next, the core AGA calculations from Wolf *et al* are computed via a series of sparse matrix operations. Let A be a (sparse) matrix representing the community membership of the cells. Each column of $X$ represents a Louvain community and each row of $X$ corresponds to a particular cell. $X_{ij}$ = 1 if cell $i$ belongs to Louvain community $j$, otherwise 0. We can further obtain the adjacency matrix A of the kNN graph used to perform the louvain clustering where $A_{ij}$ = 1 if cell $i$ connects to $j$ in the kNN graph. Then the connection matrix $M$ between each cluster is calculated as,

$$M = X^{\mathsf{T}} A X$$

Once $M$ is constructed, we can then follow *Supplemental Note 3.1* from ref. [81] to calculate the significance of the connection between each louvain clustering and consider any clusters with p-value larger than 0.05 by default as not disconnected.

Learning the principal graph

Monocle 3 learns a principal graph that resides in the same low-dimensional space as the data to represent the possible paths cells can take as they develop. Monocle 3 uses an enhanced implementation of the L1-graph algorithm[84] to learn the principal graph. Mao *et al.* described two versions of the L1-graph approach[84]. In the first ("Algorithm 1"), they optimize with respect to all the individual data points in the dataset. Previously, we showed that although L1-graph can be applied to single-cell RNA-seq data, it tends to learn very noisy graphs that are not robust to downsampling and the approach does not effectively scale to datasets beyond a few hundred cells[85]. In Qiu *et al.,* we did not explore "Algorithm 2", which first selects a set of "landmark" data points using the K-means clustering algorithm. The algorithm then optimizes against this much smaller sample of the data. Monocle 3 uses this approach, which when applied to cells in the UMAP space, is both robust and with some key modifications can scale to millions of cells.

Our implementation of L1-graph has a few key features that support analyzing large datasets and robust recovery of the principal graph. First, we learn the L1 graph in the (by default, 3 dimensional) UMAP space. We use K-medioids clustering to select landmark cells to accelerate the optimization. The number of landmark cells chosen has an impact on the algorithm's running time and the quality of the solution: too many landmarks will lead to an infeasible linear programming problem. We therefore determine the number of landmarks in a datadependent manner by setting K to be three times the number of Louvain communities detected amongst the cells, which in practice leads to fast, stable solutions.

The second major optimization to L1-graph is that we impose constraints on the "feasible" space of all possible graphs W considered by the optimization. Mao *et al.* considered all possible edges between landmark datapoints. However, even with as few as a thousand landmark cells, the linear programming problem can quickly become infeasible, because the number of variables is a function of the number of edges in the graph. In Monocle 3, we only admit edges into the feasible space that are either in the minimum spanning tree (MST) constructed on the landmark points, or which are in the kNN graph (by default k=3) constructed on the vertices that have odd degree in the MST. Finally, we exclude edges that would link cells in different connected components of the AGA graph built as described in the previous section.

Identifying genes with trajectory-dependent expression

In order to identify genes that vary in expression over a developmental trajectory, we borrow a statistical test commonly used in analyzing spatial data. Moran's *I* statistic is a measure of multi-directional and multi-dimensional spatial autocorrelation. The statistic encodes spatial relationships between datapoints via a nearest neighbor graph, making it particularly well suited for analyzing large single-cell RNA-seq datasets.

Moran's *I* test [86] is defined as

$$I = \frac{N}{W} \frac{\sum_i \sum_j w_{ij}(x_i - \bar{x})(x_j - \bar{x})}{\sum_i (x_i - \bar{x})^2}$$

where $N$ is the number of cells indexed by $i$ and $j$; $x$ is the expression value of gene of interest; $\bar{x}_i$ ($\bar{x}_j$) is the mean of the gene expression for cell $i$'s (or $j$'s) nearest neighbors; $w_{ij}$ is a matrix of weights defined by a nearest neighbor graph with zero on the diagonal (i.e., $w_{ii} = 0$) and $w_{ij} = 1/k_i$ where $k_i$ is the number of nearest neighbors; and $W$ is the sum of all $w_{ij}$.

To identify the nearest neighbors used for creating the weight matrix $W$, we first build a k (default to be 25) nearest neighbor graph (kNN) for all cells in the UMAP space. We also project each cell to its nearest node in the principal graph. Then we remove all edges from the kNN graph that connect cells that project onto principal graph nodes do not share an edge.

In Monocle 3, we implemented the *manifoldTest* function to identify manifold correlated genes which relies on modified versions of routines from *spdep* package for performing the Moran's I test.

REFERENCES

1. Fogarty, N. M. E. et al. Genome editing reveals a role for OCT4 in human embryogenesis. Nature 550, 67-73 (2017).

2. Kojima, Y., Tam, O. H. & Tam, P. P. L. Timing of developmental events in the early mouse embryo. Semin. Cell Dev. Biol. 34, 65-75 (2014).

3. Tam, P. P. L. & Loebel, D. A. F. Gene function in mouse embryogenesis: get set for gastrulation. Nat. Rev. Genet. 8, 368-381 (2007).

4. Rivera-Pérez, J. A. & Hadjantonakis, A.-K. The Dynamics of Morphogenesis in the Early Mouse Embryo. Cold Spring Harb. Perspect. Biol. 7, a015867 (2014).

5. Dickinson, M. E. et al. High-throughput discovery of novel developmental phenotypes. Nature 537, 508-514 (2016).

6. Meehan, T. F. et al. Disease model discovery from 3,328 gene knockouts by The International Mouse Phenotyping Consortium. Nat. Genet. 49, 1231-1238 (2017).

7. Shyer, A. E., Huycke, T. R., Lee, C., Mahadevan, L. & Tabin, C. J. Bending gradients: how the intestinal stem cell gets its home. Cell 161, 569-580 (2015).

8. Uygur, A. et al. Scaling Pattern to Variations in Size during Development of the Vertebrate Neural Tube. Dev. Cell 37, 127-135 (2016).

9. Gorkin, D. et al. Systematic mapping of chromatin state landscapes during mouse development. (2017). doi:10.1101/166652

10. Mayer, C. et al. Developmental diversification of cortical inhibitory interneurons. Nature 555, 457-462 (2018).

11. Lescroart, F. et al. Defining the earliest step of cardiovascular lineage segregation by single-cell RNA-seq. Science (2018). doi:10.1126/science.aao4174

12. Han, X. et al. Mapping the Mouse Cell Atlas by Microwell-Seq. Cell 172, 1091-1107.e17 (2018).

13. The Tabula Muris Consortium, Quake, S. R., Wyss-Coray, T. & Darmanis, S. Transcriptomic characterization of 20 organs and tissues from mouse at single cell resolution creates a Tabula Muris. (2017). doi:10.1101/237446

14. Amini, S. et al. Haplotype-resolved whole-genome sequencing by contiguity-preserving transposition and combinatorial indexing. Nat. Genet. 46, 1343-1349 (2014).

15. Adey, A. et al. In vitro, long-range sequence information for de novo genome assembly via transposase contiguity. Genome Res. 24, 2041-2049 (2014).

16. Cusanovich, D. A. et al. Multiplex single cell profiling of chromatin accessibility by combinatorial cellular indexing. Science 348, 910-914 (2015).

17. Vitak, S. A. et al. Sequencing thousands of single-cell genomes with combinatorial indexing. Nat. Methods 14, 302-308 (2017).

18. Ramani, V. et al. Massively multiplex single-cell Hi-C. Nat. Methods 14, 263-266 (2017).

19. Cao, J. et al. Comprehensive single-cell transcriptional profiling of a multicellular organism. Science 357, 661-667 (2017).

20. Mulqueen, R. M. et al. Scalable and efficient single-cell DNA methylation sequencing by combinatorial indexing. (2017). doi:10.1101/157230

21. Rosenberg, A. B. et al. Single-cell profiling of the developing mouse brain and spinal cord with split-pool barcoding. Science (2018). doi:10.1126/science.aam8999

22. Zheng, G. X. Y. et al. Massively parallel digital transcriptional profiling of single cells. Nat. Commun. 8, 14049 (2017).

23. Qiu, X. et al. Reversed graph embedding resolves complex single-cell developmental trajectories. (2017). doi:10.1101/110668

24. Fernandez, T. et al. Disruption of contactin 4 (CNTN4) results in developmental delay and other features of 3p deletion syndrome. Am. J. Hum. Genet. 74, 1286-1293 (2004).

25. Olson, J. M. et al. NeuroD2 is necessary for development and survival of central nervous system neurons. Dev. Biol. 234, 174-187 (2001).

26. Uittenbogaard, M., Baxter, K. K. & Chiaramello, A. NeuroD6 Genomic Signature Bridging Neuronal Differentiation to Survival via the Molecular Chaperone Network. J. Neurosci. Res. 88, 33 (2010).

27. Yang, A. et al. p63 is essential for regenerative proliferation in limb, craniofacial and epithelial development. Nature 398, 714-718 (1999).

28. McQualter, J. L., Yuen, K., Williams, B. & Bertoncello, I. Evidence of an epithelial stem/progenitor cell hierarchy in the adult mouse lung. Proc. Natl. Acad. Sci. U. S. A. 107, 1414-1419 (2010).

29. Cichorek, M., Wachulska, M., Stasiewicz, A. & Tyminska, A. Skin melanocytes: biology and development. *Advances in Dermatology and Allergology* 1, 30-41 (2013).

30. Tomihari, M., Hwang, S.-H., Chung, J.-S., Cruz, P. D., Jr. & Ariizumi, K. Gpnmb is a melanosome-associated glycoprotein that contributes to melanocyte/keratinocyte adhesion in a RGD-dependent fashion. Exp. Dermatol. 18, 586-595 (2009).

31. Varjosalo, M. & Taipale, J. Hedgehog: functions and mechanisms. Genes Dev. 22, 2454-2472 (2008).

32. Strähle, U., Lam, C. S., Ertzer, R. & Rastegar, S. Vertebrate floor-plate specification: variations on common themes. Trends Genet. 20, 155-162 (2004).

33. Holmes, G. P. et al. Distinct but overlapping expression patterns of two vertebrate slit homologs implies functional roles in CNS development and organogenesis. Mech. Dev. 79, 57-72 (1998).

34. Akle, V. et al. F-spondin/spon1b expression patterns in developing and adult zebrafish. PLoS One 7, e37593 (2012).

35. Hartman, B. H., Durruthy-Durruthy, R., Laske, R. D., Losorelli, S. & Heller, S. Identification and characterization of mouse otic sensory lineage genes. Front. Cell. Neurosci. 9, 79 (2015).

36. Petit, F., Sears, K. E. & Ahituv, N. Limb development: a paradigm of gene regulation. Nat. Rev. Genet. 18, 245-258 (2017).

37. Guo, Q., Loomis, C. & Joyner, A. L. Fate map of mouse ventral limb ectoderm and the apical ectodermal ridge. Dev. Biol. 264, 166-178 (2003).

38. Lewandoski M, E. al. Fgf8 signalling from the AER is essential for normal limb development. - PubMed - NCBI. Available at: https://www.ncbi.nlm.nih.gov/pubmed/11101846. (Accessed: 22nd April 2018)

39. Aoki M, E. al. R-spondin2 expression in the apical ectodermal ridge is essential for outgrowth and patterning in mouse limb development. - PubMed - NCBI. Available at: https://www.ncbi.nlm.nih.gov/pubmed/18067586. (Accessed: 22nd April 2018)

40. Gerdes, J., Schwab, U., Lemke, H. & Stein, H. Production of a mouse monoclonal antibody reactive with a human nuclear antigen associated with cell proliferation. Int. J. Cancer 31, 13-20 (1983).

41. Bergman, D., Halje, M., Nordin, M. & Engström, W. Insulin-like growth factor 2 in development and disease: a mini-review. Gerontology 59, 240-249 (2013).

42. Trapnell, C. et al. The dynamics and regulators of cell fate decisions are revealed by pseudotemporal ordering of single cells. Nat. Biotechnol. 32, 381-386 (2014).

43. McInnes, L. & Healy, J. UMAP: Uniform Manifold Approximation and Projection for Dimension Reduction. (2018).

44. Alexander Wolf, F. et al. Graph abstraction reconciles clustering with trajectory inference through a topology preserving map of single cells. bioRxiv 208819 (2017). doi:10.1101/208819

45. Qiu, X. et al. Reversed graph embedding resolves complex single-cell trajectories. Nat. Methods 14, 979-982 (2017).

46. Singh, M. K. et al. The T-box transcription factor Tbx15 is required for skeletal development. Mech. Dev. 122, 131-144 (2005).

47. Paine-Saunders, S., Viviano, B. L., Zupicich, J., Skarnes, W. C. & Saunders, S. glypican-3 controls cellular responses to Bmp4 in limb patterning and skeletal development. Dev. Biol. 225, 179-187 (2000).

48. Hara, K. & Ide, H. Msx1 expressing mesoderm is important for the apical ectodermal ridge (AER)-signal transfer in chick limb development. Dev. Growth Differ. 39, 705-714 (1997).

49. Lupiáñez, D. G. et al. Disruptions of Topological Chromatin Domains Cause Pathogenic Rewiring of Gene-Enhancer Interactions. Cell 161, 1012-1025 (2015).

50. Davis, R. J. et al. Dach1 mutant mice bear no gross abnormalities in eye, limb, and brain development and exhibit postnatal lethality. Mol. Cell. Biol. 21, 1484-1490 (2001).

51. Akiyama, H., Chaboissier, M.-C., Martin, J. F., Schedl, A. & de Crombrugghe, B. The transcription factor Sox9 has essential roles in successive steps of the chondrocyte differentiation pathway and is required for expression of Sox5 and Sox6. Genes Dev. 16, 2813-2828 (2002).

52. Deng, Y. et al. Yap1 Regulates Multiple Steps of Chondrocyte Differentiation during Skeletal Development and Bone Repair. Cell Rep. 14, 2224-2237 (2016).

53. Joshi, S. et al. TEAD transcription factors are required for normal primary myoblast differentiation in vitro and muscle regeneration in vivo. PLoS Genet. 13, e1006600 (2017).

54. Knapp, D. et al. Comparative transcriptional profiling of the axolotl limb identifies a tripartite regeneration-specific gene program. PLoS One 8, e61352 (2013).

55. Zeller, R., López-Ríos, J. & Zuniga, A. Vertebrate limb bud development: moving towards integrative analysis of organogenesis. Nat. Rev. Genet. 10, 845-858 (2009).

56. Nishimoto, S., Minguillon, C., Wood, S. & Logan, M. P. O. A combination of activation and repression by a colinear Hox code controls forelimb-restricted expression of Tbx5 and reveals Hox protein specificity. PLoS Genet. 10, e1004245 (2014).

57. Vargesson, N., Luria, V., Messina, I., Erskine, L. & Laufer, E. Expression patterns of Slit and Robo family members during vertebrate limb development. Mech. Dev. 106, 175-180 (2001).

58. Chimal-Monroy, J. et al. Analysis of the molecular cascade responsible for mesodermal limb chondrogenesis: Sox genes and BMP signaling. Dev. Biol. 257, 292-301 (2003).

59. Braun, T. & Gautel, M. Transcriptional mechanisms regulating skeletal muscle differentiation, growth and homeostasis. Nat. Rev. Mol. Cell Biol. 12, 349-361 (2011).

60. Tajbakhsh, S., Rocancourt, D., Cossu, G. & Buckingham, M. Redefining the genetic hierarchies controlling skeletal myogenesis: Pax-3 and Myf-5 act upstream of MyoD. Cell 89, 127-138 (1997).

61. Harel, I. et al. Distinct origins and genetic programs of head muscle satellite cells. Dev. Cell 16, 822-832 (2009).

62. Sambasivan, R. et al. Distinct regulatory cascades govern extraocular and pharyngeal arch muscle progenitor cell fates. Dev. Cell 16, 810-821 (2009).

63. Heimberg, G., Bhatnagar, R., El-Samad, H. & Thomson, M. Low Dimensionality in Gene Expression Data Enables the Accurate Extraction of Transcriptional Programs from Shallow Sequencing. Cell Syst 2, 239-250 (2016).

64. Cusanovich, D. A. et al. The cis-regulatory dynamics of embryonic development at single cell resolution. (2017). doi:10.1101/166066

65. McKenna, A. et al. Whole-organism lineage tracing by combinatorial and cumulative genome editing. Science 353, aaf7907 (2016).

66. Osterwalder, M. et al. Enhancer redundancy provides phenotypic robustness in mammalian development. Nature 554, 239-243 (2018).

67. Dickel, D. E. et al. Ultraconserved Enhancers Are Required for Normal Development. Cell 172, 491-499.e15 (2018).

68. Li, D. et al. Formation of proximal and anterior limb skeleton requires early function of Irx3 and Irx5 and is negatively regulated by Shh signaling. Dev. Cell 29, 233-240 (2014).

69. Kraft, K. et al. Deletions, Inversions, Duplications: Engineering of Structural Variants using CRISPR/Cas in Mice. Cell Rep. (2015). doi:10.1016/j.celrep.2015.01.016

70. Buenrostro, J. D., Giresi, P. G., Zaba, L. C., Chang, H. Y. & Greenleaf, W. J. Transposition of native chromatin for fast and sensitive epigenomic profiling of open chromatin, DNA-binding proteins and nucleosome position. Nat. Methods 10, 1213-1218 (2013).

71. Renaud, G., Stenzel, U., Maricic, T., Wiebe, V. & Kelso, J. deML: robust demultiplexing of Illumina sequences using a likelihood-based approach. Bioinformatics 31, 770-772 (2015).

72. Dobin, A. et al. STAR: ultrafast universal RNA-seq aligner. Bioinformatics 29, 15-21 (2013).

73. Anders, S., Pyl, P. T. & Huber, W. HTSeq--a Python framework to work with high-throughput sequencing data. Bioinformatics btu638 (2014).

74. Qiu, X. et al. Reversed graph embedding resolves complex single-cell developmental trajectories. (2017). doi:10.1101/110668

75. Wolf, F. A., Angerer, P. & Theis, F. J. SCANPY: large-scale single-cell gene expression data analysis. Genome Biol. 19, 15 (2018).

76. Cao, J. et al. Comprehensive single-cell transcriptional profiling of a multicellular organism. Science 357, 661-667 (2017).

77. Pliner, H. et al. Chromatin accessibility dynamics of myogenesis at single cell resolution. (2017). doi:10.1101/155473

78. Kuleshov, M. V. et al. Enrichr: a comprehensive gene set enrichment analysis web server 2016 update. Nucleic Acids Res. 44, W90-7 (2016).

79. McInnes, L. & Healy, J. UMAP: Uniform Manifold Approximation and Projection for Dimension Reduction. (2018).

80. Levine, J. H. et al. Data-Driven Phenotypic Dissection of AML Reveals Progenitor-like Cells that Correlate with Prognosis. Cell 162, 184-197 (2015).

81. Wolf, F. A. et al. Graph abstraction reconciles clustering with trajectory inference through a topology preserving map of single cells. (2017). doi: 10.1101/208819

82. Wagner, D. E. et al. Single-cell mapping of gene expression landscapes and lineage in the zebrafish embryo. Science eaar4362 (2018).

83. Briggs, J. A. et al. The dynamics of gene expression in vertebrate embryogenesis at single-cell resolution. Science eaar5780 (2018).

84. Mao, Q., Wang, L., Tsang, I. & Sun, Y. Principal Graph and Structure Learning Based on Reversed Graph Embedding. IEEE Trans. Pattern Anal. Mach. Intell. (2016). doi:10.1109/TPAMI.2016.2635657

85. Qiu, X. et al. Reversed graph embedding resolves complex single-cell trajectories. Nat. Methods 14, 979-982 (2017).

86. Moran, P. A. P. Notes on continuous stochastic phenomena. Biometrika 37, 17-23 (1950).

Example 2

A new technique for tissue nuclei extraction and fixation (sc-RNA-seq)

Reagents. BSA (Molecular biology grade, NEB, #B9000S); SuperRnase Inhibitor (Thermo, #AM2696); EMS 157-4-100 4% Paraformaldehyde (Formaldehyde) Aqueous Solution, EM Grade, 100 mL (Amazon).
Buffers. Nuclei Buffer (stored at 4°C): 10 mM Tris-HCl, pH 7.4, 10 mM NaCl, 3 mM $MgCl_2$. 10% IGEPAL CA-630 (stored in 4°C). Nuclei wash buffer (made fresh each time): 980ul nuclei buffer with 10ul BSA and 10ul SuperRnaseIn, mix well and store on ice. Nuclei lysis buffer (made fresh each time): Nuclei wash buffer with 0.1% IGEPAL CA-630.

Nuclei extraction directly from tissue

Tissues are minced into small pieces by blade in 1 mL ice-cold cell lysis buffer (10 mM Tris-HCl, pH 7.4, 10 mM NaCl, 3 mM MgCl2 and 0.1% IGEPAL CA-630, 1% SUPERase In and 1% BSA) and transferred to the top of a 40um cell strainer (Falcon).
Tissues were homogenized with the rubber tip of a syringe plunger (5ml, BD) in 4ml cell lysis buffer.
The filtered nuclei were then transferred to a new 15ml tube (Falcon) and pelleted by centrifuge at 500xg for 5min and washed once with 1ml cell lysis buffer.

Nuclei fixation

The nuclei were fixed in 4ml ice cold 4% paraformaldehyde (EMS) for 15min on ice.
After fixation, the nuclei were washed twice in 1ml nuclei wash buffer (cell lysis buffer without IGEPAL), and re-suspended in 500ul nuclei wash buffer.
The samples were split to several and flash frozen in liquid nitrogen. The frozen samples can be transported on dry ice.

Example 3

Characterizing single cell state transition dynamics by sci-fate

The beauty of development lies in the generation of diverse cell states in strictly organized temporal order. Despite of the proliferation in single cell genomic techniques, it has remained challenging to quantitatively determine cell state transition dynamics. Here we introduce sci-fate, a combinatorial indexing-based high throughput assay for profiling both whole and newly synthesized transcriptome in each of thousands of single cells. As a proof of concept, we applied sci-fate to a model system of cortisol response, and characterized over 6,000 single cell state transition events, consistent with known cell cycle dynamics upon glucocorticoid receptor activation. From the analysis, we showed the cell state transition direction and probabilities are regulated by inter-state distances and state instability landscape. The technique and computational approaches are readily applicable to other biological systems to quantitatively characterize cell state dynamics, and decipher the internal mechanism for cell fate determination.
Cell transits across functional and molecularly distinct state during multicellular organism development. Characterizing the cell state transition path, or cell fate, is the core in understanding development and applications such as cell engineer. While methods for single cell genomic techniques have proliferated, they only capture a snapshot of cell state, thus cannot provide information on cell transition dynamics (7). Although time-lapse microscopy based single cell tracing can be used to characterize cell state transitions (2, 3), they are limited in throughput and can only track the changes of several genes, and thus has low capacity to decipher complex systems.
Here we describe a novel strategy to infer quantitative cell state transition dynamics at the level of whole transcriptome. This strategy depends on a new combinatorial indexing based single cell RNA-seq technique, sci-fate. By labeling newly synthesized mRNA with 4-thiouridine (4, 5) which will generate C > T point mutations during reverse transcription, sci-fate captures both whole transcriptome and newly synthesized transcriptome at single cell level, together with the degraded transcriptome information from its past state (past state memory). The past state memory of each cell is then corrected by mRNA degradation rate (memory correction technique), such that each cell can be characterized by transcriptome dynamics between two time points.
To characterize cell state transition dynamics regulated by intrinsic and extrinsic factors, we applied sci-fate to a model system of cortisol response, in which cell fate was driven by two major forces: intrinsic cell cycle program and extrinsic drug induced glucocorticoid receptor (GR) activation. GR activation influences the activity of almost every cell in the body, and regulates genes controlling development, metabolism and immune response (6). With sci-fate, we profiled whole transcriptome dynamics for over 6,000 single cells. Based on the similarity between past and current transcriptome states, we built thousands of cell state transition trajectories spanning five time points, which can be clustered into three types of cell fates consistent with known cell cycle progress patterns in GR activation. We further characterized cellular hidden states by functional TF modules activity, and inferred a cell transition network for cell state prediction. Finally we showed the cell state transition direction and probability are regulated by transcriptome similarity and instability landscape of its nearby states. The theoretical, computational and experimental approaches developed here should be readily applicable to other biological systems in which cell transition dynamics are still unknown.

Overview of sci-fate

sci-fate relies on the following steps (Fig. 30A): (i) cells were first incubated with 4-thiouridine (S4U), a widely used thymidine analog to label newly synthesized RNA(7-13). (ii) Cells are harvested, fixed by 4% paraformaldehyde, followed by thiol(SH)-linked alkylation reaction which covalently attaches a carboxyami-domethyl group to S4U by nucleophilic substitution(4). (iii) Cells were distributed in bulk to each well of 4x96 well plates. The first RNA-seq molecular index is introduced to the mRNA of cells in each well via *in situ* reverse transcription (RT) with a poly(T) primer bearing both a well-specific barcode and a degenerate unique molecular identifier (UMI). During cDNA synthesis, the mRNA labeled with modified S4U mimic thymine-to-cytosine (T > C) conversions and result in mutated first strand cDNA. (iv) Cells from all wells are pooled and then redistributed by fluorescence-activated cell sorting (FACS) to multiple 96-well plates. Cells are gated on DAPI (4',6-diamidino-2-phenylindole) staining to discriminate single cell from doublets during sorting. Double-stranded cDNA is generated by RNA degradation and second-strand synthesis, and is subjected to transposition with Tn5. cDNA is then amplified via the polymerase chain reaction (PCR) with a combination of primers recognizing the Tn5 adaptor on the 5' end and the RT primer on the 3' end. These primers also bear a well-specific barcode that introduces the second RNA-seq molecular index. (v) Amplicons from the PCR are pooled and subjected to massively parallel sequencing. As with other "sci-" protocols(*14-21*), most nuclei pass through a unique combination of wells and therefore each cell's contents are marked by a unique combination of barcodes that can be used to group reads that derive from the same cell. Newly synthesized mRNA out of the whole transcriptome is identified by background error corrected "T > C" conversions (Method).

As quality control, we first tested the technique in a mixture of HEK293T (human) and NIH/3T3 (mouse) cells under four conditions: with or without S4U labeling (200nM, 6 hrs), and with or without IAA treatment (Fig. 31A-D). With S4U labeling and IAA treatment (sci-fate condition), transcriptomes from human/mouse cells were overwhelmingly species-coherent (> 99% purity for both human and mouse cells, 2.6% collisions) with high ratio of T > C mutated reads detected (46% for human and 31% for mouse cells in sci-fate condition vs. 0.8% for human and 0.8% for mouse cells in no treatment condition). We obtained roughly equivalent cell purity across four conditions, albeit slightly lower UMIs detected in IAA treatment groups. Aggregated transcriptomes of sci-fate vs. normal sci-RNA-seq were highly-correlated (Spearman's correlation r = 0.99; Fig. 31E-F), suggesting the short term labeling and conversion process have minimal effect on cell state.

Joint profiling of total and newly synthesized transcriptome in dexamethasone treated A549 cells

We then applied sci-fate to a model of cortisol response, wherein dexamethasone (DEX), a synthetic mimic of cortisol, activates glucocorticoid receptor (GR), which binds to thousands of locations across the genome, and significantly alters cell state within a short term (22-25). We treated lung adenocarcinoma-derived A549 cells for 0, 2, 4, 6, 8 or 10 hrs with 100nM DEX. In each condition, cells were incubated with S4U (200nM) for the last two hours before harvest for 384 x 192 well sci-fate (Fig. 30B). The six conditions were each represented in 64 wells during the first round of indexing so that the treatment condition could be recovered based on the first index of each cell.

After filtering out low quality cells, potential doublets and a small subgroup of differentiated cells (Method), we obtained single cell profiles for 6,680 cells (median of 26,176 mRNAs detected per cell) with a median of 20% labeled UMIs per cell (Fig. 30C, Fig. 32A-B). The intronic reads shows significantly higher newly synthesized rate than exonic reads (65% in intronic reads vs. 13% in exonic reads, p-value < 2.2e-16, Wilcoxon signed rank test; Fig. 30D), consistent with the expectation that the intronic reads are enriched in newly synthesized transcriptome.

We first asked if the whole transcriptome and newly synthesized transcriptome convey different information in cell state characterization. We aggregated the the whole transcriptome and newly synthesized transcriptome for each treatment conditions and checked their correlations. Different from the whole transcriptome, the newly synthesized transcriptome showed a sharp difference between no DEX treatment (0h) and treated groups (Fig. 32C). Consistent with this, dimension reduction with Uniform Manifold Approximation and Projection (UMAP)(26) on whole or newly synthesized transcriptome gives different results (Fig. 30E): whole transcriptome cannot separate no DEX treatment (0h) and early DEX treatment (2h) cells while newly synthesized transcriptome aggregates all DEX treated cells into a single group. Cell clusters identified by whole or newly synthesized transcriptome do not fully match with each other (Fig. 30F, Fig. 32D-E). This is expected as the newly synthesized transcriptome directly reflects the gene promoter activity, or epigenetic response to external environment, while the whole transcriptome is mostly determined by the leftover mRNA from its past state.

To characterize cell states with joint information, we combined the top principal components (PCs) from whole and newly synthesized transcriptome for UMAP analysis. Joint information separates cells into no DEX treatment (0h), early treatment (2h) and late treatment (>2h) (Fig. 30E). Interestingly, two clusters (cluster 1 and 4) characterized by whole transcriptome were split into four separate groups by joint information (Fig. 30F). We checked the expression level and newly synthesis rate of cell cycle related gene markers (27) (Fig. 30G, Fig. 32F-G): the newly separated clusters by joint information correspond to G2/M phase (high expression and high synthesis rate of G2/M markers) and early G0/G1 phase cells (high expression and low synthesis rate of G2/M markers). This suggests newly synthesized transcriptome convey different cell state information compared with the whole transcriptome, and joint information potentially enables higher resolution in cellular state characterization.

Characterizing functional TF modules driving cell fate determination

We next sought to characterize TF modules driving cell state transition. The links between transcription factors (TF) and their regulated genes were identified by two steps: for each gene, we computed correlations between mRNA synthesis rate during the last two hours and TF expression level across over 6,000 cells using LASSO (least absolute shrinkage and selection operator). These identified links were further filtered by either published CHIP-seq data(28) or motif enrichment analysis(29) (Method). In total we identified 986 links between 29 TFs and 532 genes (Fig. 33A, Table S1), based on TF-gene covariance and validated by DNA binding data. To evaluate the possibility that the links were artifacts of regularized regression, we permuted the sample IDs of the TF expression matrix and performed the same analysis. No links were identified after this permutation.

TF modules driving GR response are identified, including known GR response effectors such as CEBPB(30) (Fig. 34A-B), FOXO1(31), and JUNB(32) (Fig. 33A). We also found several novel GR response related TF modules including YOD1 and GTF2IRD1, with both upregulated expression and activity in DEX treated cells (Fig. 34C-D). Main TF modules driving cell cycle progression are identified, and these include E2F1, E2F2, E2F7, BRCA1, and MYBL2 (33). Compared with total expression level, the new RNA synthesis rate of regulated genes by cell cycle TF modules displays higher correlation with the target TF expression (Fig. 34E). Additionally, we also found TF modules related with cell differentiation such as GATA3, mostly expressed in a group of quiescent population of cells (34), and TF modules related with oxidative stress response such as NRF1 (35) and NFE2L2 (NRF2) (36).

We next characterized TF activity by aggregating the new RNA synthesis rate of genes within each TF module, and computed the absolute correlation coefficient between each TF pairs (Fig. 34F). Highly correlated TF activity suggests they may function in a linked process. Hierarchical clustering segregate these 29 TF modules into five major modules (Fig. 34F): the first module are all cell cycle related TF modules such as E2F1 and FOXM1 (33), and represents the driving force for cell cycle progression. The third module are all GR response related TF modules such as FOXO1, CEBPB, JUNE and RARB(30)(31)(32). The other TF module groups include three TFs (KLF6, TEAD1, and YOD1) co-regulated by both cell cycle and GR response (module 2), an internal differentiation pathway including GATA3 and AR (module 3), and stress response related TFs such as NRF1 and NFE2L2 (module 5).

To identify different cell cycle states, we first ordered cells by cell cycle linked TF module activity. Cells are ordered into a smooth trajectory of cell cycle, validated by the synthesis rate of known cell cycle markers (27) (Fig. 33B). We observed a gap between G2/M phase and G0/G1 phase, consistent with the dramatic cell state change during cell division. By unsupervised clustering, we identified nine cell cycle states spanning G0/G1, S and G2/M cell cycle phases based on cell cycle marker expression (Fig. 33B). Cells can be ordered into another smooth trajectory by GR reponse linked TF modules. The trajectory correlates well with DEX treatment time and dynamics of known GR activation regulated TF activity (Fig. 33C). By unsupervised clustering analysis, we identified three cell clusters along GR response, corresponding to no/low/high GR response state (Fig. 33C).

We next sought to quantitatively characterize hidden cell states in the system (Fig. 35A). Nine cell cycle states and three GR reponse states were identified in Fig. 33B-C. All possible combinatorial states were identified, with the smallest group including 1.1% (74) of all cells (Fig. 33D). The observed cell state proportion is close to the expected proportion assuming independent assortment. This is consistent with the low correlation coefficient (Pearson's correlation r = 0.004) between the activity of these two functional TF modules across over 6,000 cells. For comparison, by dimension reduction and clustering analysis on whole and newly synthesized transcriptome, we identified 6 main clusters (Fig. 35B). These main clusters can be readily defined by combined groups of these 27 cell states (Fig. 33E).

Characterizing single cell transition trajectory and state transition network

With both whole transcriptome and newly synthesized transcriptome characterized for each cell, we can infer the single cell transcriptome state before S4U labeling (Fig. 36A). The recovery of past cellular transcriptome depends on two parameters: the detection rate of newly synthesized reads in sci-fate, and the degradation rate (or half time) of each mRNA (Method). Both two parameters can both be estimated from the same experiment in sci-fate.

We first estimated the detection rate of sci-fate. We assume the mRNA half life is stable across different DEX treatmentment conditions. This assumption is further validated by self-consistency check later. Under this assumption, the partly degraded bulk transcriptome before the 2 hour S4U labeling should be the same between no DEX and 2 hour DEX treated cells. Thus their differences in whole transcriptome (bulk) should equal with their differences in the newly synthesized transcriptome (bulk) corrected by technique detection rate. As whole and newly synthesized transcriptome are both profiled in our experiment, we can directly compute the detection rate of sci-fate. The differences in newly synthesized mRNA correlates well with the differences in mRNA expression level (Pearson's r = 0.93, Fig. 37A), suggesting the new RNA detection rate is rather stable across genes. We thus used the median of new RNA capture rate (82% ) for downstream analysis.

We next computed the mRNA degradation rate in 2 hours. As A549 cell population can be regarded stable without external perturbation, for cells after 2 hour DEX treatment, its past state (before 2 hour S4U labeling) should be the same with the 0 hour DEX treated cells. Similarly, the past state (before S4U labeling) for T = 0/2/4/6/8/10 hour DEX treated cells should be similar to the profiled T = 0/0/2/4/6/8 hour cells. With whole transcriptome and newly synthesized transcriptome profiled for all treatment conditions, mRNA degradation rate across thousands of genes in each 2 hour time interval can be estimated. As a self-consistency check mentioned above, the gene degradation rates are highly correlated across different DEX treatment time (Fig. 37B). We then used the averaged gene degradation rate for downstream analysis. With both new mRNA detection rate and gene degradation rate available, we estimated single cell past transcriptome state so that each cell can be characterized by transcriptome dynamics in a two-hour interval.

To recover cell state dynamics for a longer interval (i.e. 10 hours), we developed a cell linkage pipeline to link parent and child cells in the same cell state transition trajectory (Fig. 36A): for each cell A (e.g. 2 hour DEX treated cells), we identified a cell B profiled in the earlier time point (e.g. no DEX treated cells) and B had its current state similar with A's past state, based on a recently developed alignment strategy to identify common cell states between two data sets (27). B can be regarded as the parent state of A. Similarly, we also identified another cell C profiled in the later time point (e.g. 4 hour DEX treated cells) and C had its past state similar with A's current state. Cell C can be regarded as the A's future state. By extending the same strategy to all past and future state identified for each cell, we constructed 6,680 single cell transition trajectory across 10 hours and five time points (Fig. 36A-B). Of note, this analysis is based on an assumption that the past and current state of each cell (except cells at the start and end time points) are comprehensively detected, which holds true in our data sets as over 6,000 cells are profiled (over 1,000 cells per condition), or a cell for less than one min during cell cycle. Multiple cells (>50) are profiled at each cell state, thus stochastic cell state transition process can also be captured.

To validate the result, we applied dimension reduction and unsupervised clustering analysis to these 6,680 single cell trajectories, which grouped into three trajectory clusters. We checked the dynamics of cell states characterized in Fig. 36C. As expected, all three trajectories showed cell state transition from no GR response to low/high GR response states over time (Fig. 36D). We observed distinct cell cycle dynamics across these three trajectories(Fig. 36D): trajectory 1 showed decreased G2/M phase and consistently increased G0/G1 phase, and represented cell state transition from G2/M and G1 intermediate states to G1 phase. Trajectory 2 showed cell state transition from S and G2/M intermediate states to G2/M phase. In the trajectory 3, we observed cell state transition from G1 and S intermediate phase to early S phase during early DEX treatment (0-2 hour), but the transition is inhibited in late DEX treatment conditions (>2 hour DEX treatment), suggesting long term DEX treatment results in G1 phase arrest. This is consistent with cell state proportion changes along treatment time and previous research (37, 38)(Fig. 36D). These suggests the single cell transition paths characterized by sci-fate can recover general cell state transition directions.

With multiple cells (>70) profiled at each state, we computed the cell state transition probability across all 27 hidden states. Cell state transitions with low transition probabilities (< 0.1) are potentially due to rare events or noise, and thus filtered out. The cell state transition network can be defined by 27 cell states as nodes, and links showing the potential transition paths (Fig. 36E). The direction of cell cycle progression is readily characterized by at least three transition stages with irreversible transition directions along cell cycle (Fig. 36E). In late G1 phase and late G2/M phase, we also found several states showing reversible transitions dynamics, which potentially reflect two cell cycle checkpoints in G1/S and G2/M phases(33). As expected, cells on similar cell cycle but different GR responses states showed dramatically different transition dynamics, and cells with high GR response state tend to be arrested in G1 or G2/M phase.

As a consistency check to validate whether the cell state transition network captures cell state transition dynamics, we evaluated if the transition probabilities can recover the real cell state distributions across different time points. Indeed, although cell state proportions are dynamically changed across 10 hours (Fig. 36F), the state transition network accurate predicts the 27 cell state ratios across all five later time points from cell state proportion in 0 hour DEX treated cells (Fig. 36G, Fig. 38A). We also computed the cell state transition network with only part of the data (0 hour to 6 hour), which gave highly correlated transition probabilities with the full data, and accurately predict cell states at 10 hours (Fig. 36H, Fig. 38B).

Characterizing factors regulating cell state transition directions

To characterize the factors regulating cell state transition probability, we first calculated cell state distance, by the pearson's distance of aggregated transcriptome (whole and newly synthesized) between each state pairs. As expected, cell state transition probability negatively correlates with transition distance (Spearman's correlation coefficient = -0.38, Fig. 39A). We also computed state instability, defined by the proportion of cells moving out of the state within two hours (Fig. 39B). The state instability landscape matches well with cell transition directions (Fig. 39B): states in no GR response show higher instability compared with high GR response states. In high GR response states, cells at early G1 phase has the lowest instability, while cells at G1/S intermediate states showed a high unstable peak, consistent with the G1 phase arrest in late DEX treatment.

The cell state proportion changes after 10 hours correlates well with cell state instability (Spearman's correlation coefficient = -0.88, Fig. 39C), suggesting cell state dynamics are regulated by the cell state instability landscape. The state instability also correlates well with state transition probability entropy, which reflects the diversity of state transition targets (Pearson's correlation r = 0.73, Fig. 39D). To validate whether the inter-state transition probability can be inferred by nearby state instability, we fit nearby state instability and distance into a neural network model, to predict state transition probability from each state to the other states. Combining both nearby state instability and distances achieved more than ten folds higher performance in predicting inter-state transition probability, compared with using state distances alone (median cross validated r squared is 0.58 by using both information vs. 0.046 by using state distance only, p-value = 4.5e-10, two sided wilcoxon rank sum test, Fig. 39E), suggesting the cell state transition directions and probabilities are regulated by nearby state stability landscape. And cells prefer to moving to a more stable nearby state over just the nearest position.

Discussion

Here we developed the first strategy to characterize cell state transition dynamics on whole transcriptome level. The strategy depends on sci-fate, a novel combinatorial indexing based high throughput single cell RNA-seq technique, capable of profiling both whole and newly synthesized transcriptome in thousands of cells. Similar with other "sci-" techniques, sci-fate is readily scaled up to millions of cells(39), and potentially compatible with profiling both transcriptome and epigenome(40). This enables sci-fate to characterize cell state dynamics in a much complexed system (i.e. whole embryo development) where the real cell transition path to hundreds of cell types are still unknown. We further developed a computation pipeline to estimate newly synthesized RNA capture rate and gene degradation rate from sci-fate data (memory correction), and infer thousands of differential trajectories for each single cell, linked by shared past and current transcriptome state at each time point.

To validate the techniques and examine how cell state dynamic are regulated by internal and external factors, we applied the strategy to a model system of cortisol response, in which cell fate were dynamically regulated by internal cell cycle and extrinsic drug induced GR activation. We showed the newly synthesized transcriptome directly links to the epigenome response to environmental stimuli, and joint analysis of both whole and newly synthesized transcriptome enables higher resolution in cell state separation. By co-variance between TF expression and new RNA synthesis rate across thousands of cells, we identified up to one thousand links between TFs and regulated genes, validated by DNA binding data. We further identified 27 "hidden cell states" characterized by the combinatorial state of functional TF modules in cell cycle progression and GR response, compared with only 6 states by conventional clustering analysis.

By memory correction and cell linkage analysis, we built over 6,000 single cell transition trajectories spanning 10 hours, with the main trajectories consistent with known cell state dynamics in cell cycle and GR response. Cell state transition network are characterized by the transition probability across all cell states, validated by the recovery of 27 cell state dynamics across all five time points. Finally, we found the cell state transition probabilities are regulated by two key features of cell state transition network: inter-state distance and state instability landscape, both of which can be potentially estimated by conventional single cell RNA-seq techniques.

While powerful, this strategy has several limitations. First, to faithfully build single cell trajectory, we need comprehensive cell state characterization at each time point. Also multiple observations for each states are needed to robustly estimate the transition probability. These limitations can be readily resolved by the combinatorial strategy of sci-fate, which is capable of profiling millions of cells in a single experiment. Another caveat is that most S4U labeling experiments are applied to in vitro systems. However, recent research has shown that S4U can stably label cell type specific RNA transcription in multiple mouse tissues (i.e. brain, intestine and adipose tissue)(41, 42), suggesting sci-fate, with further optimizations to enhance S4U incorporation and detection rate, can be applied to profile in vivo single cell transcriptome dynamics.

sci-fate opens a new avenue for applying "static" single cell genomic techniques to characterizing dynamic systems. Compared with traditional imaging based techniques, sci-fate profiles cell state dynamics at whole transcriptome level, and enables comprehensive cell state characterization without marker selection and discovery of key driving force in cell differentiation. Finally, we anticipate that sci-fate can be readily combined with alternative lineage tracing techniques(*43-45*), to decode the detailed cell state transition dynamics to every final cell state within hundreds of developmental lineages.

Materials and Methods:

Mammalian cell culture

All mammalian cells were cultured at 37°C with 5% $CO_2$, and were maintained in high glucose DMEM (Gibco cat. no. 11965) for HEK293T and NIH/3T3 cells or DMEM/F12 medium for A549 cells, both supplemented with 10% FBS and 1X Pen/Strep (Gibco cat. no. 15140122; 100U/ml penicillin, 100 µg/ml streptomycin). Cells were trypsinized with 0.25% typsin-EDTA (Gibco cat. no. 25200-056) and split 1:10 three times per week.

Sample processing for sci-fate

A549 cells were treated with 100 nM DEX for 0 hrs, 2 hrs, 4 hrs, 6 hrs, 8 hrs and 10 hrs. Cells in all treatment conditions were incubated with 200uM S4U for the last two hours before cell harvest. For HEK293T and NIH/3T3 cells, cells were incubated with 200uM S4U for 6 hours before cell harvest.

All cell lines (A549, HEK293T and NIH/3T3 cells) were trypsinized, spun down at 300xg for 5 min (4°C) and washed once in 1X ice-cold PBS. All cells were fixed with 4ml ice cold 4% paraformaldehyde (EMS) for 15 min on ice. After fixation, cells were pelleted at 500xg for 3 min (4°C) and washed once with 1ml PBSR (1 x PBS, pH 7.4, 1% BSA, 1% SuperRnaseIn, 1% 10mM DTT). After wash, cells were resuspended in PBSR at 10 million cells per ml, and flash frozen and stored in liquid nitrogen. Paraformaldehyde fixed cells were thawed on 37 degree water bath, spun down at 500xg for 5 min, and incubated with 500ul PBSR including 0.2% Triton X-100 for 3min on ice. Cells were pelleted and resuspended in 500ul nuclease free water including 1% SuperRnaseIn. 3ml 0.1N HCl were added into the cells for 5min incubation on ice (21). 3.5ml Tris-HCl (pH = 8.0) and 35ul 10% Triton X-100 were added into cells to neutralize HCl. Cells were pelleted and washed with 1ml PBSR. Cells were resuspended in 100ul PBSR. 100ul PBSR with fixed cells were incubated with mixture including 40ul Iodoacetamide (IAA, 100mM), 40ul sodium phosphate buffer (500mM, pH = 8.0), 200ul DMSO and 20ul H2O, at 50°C for 15min. The reaction was quenched by 8ul DTT (1M) and 8.5ml PBS(47). Cells were pelleted and resuspended in 100ul PBSI (1 × PBS, pH 7.4, 1% BSA, 1% SuperRnaseIn). For all later washes, nuclei were pelleted by centrifugation at 500xg for 5 min (4°C).

The following steps are similar with sci-RNA-seq protocol with paraformaldehyde fixed nuclei (*15, 16*). Briefly, cells were distributed into four 96-well plates. For each well, 5,000 nuclei (2 µL) were mixed with 1 µl of 25 µM anchored oligo-dT primer (5'-ACGACGCTCTTCCGATCTNNNNNNNNN[10bp index]TTTTTTTTTTTTTTTTTTTTTTTTTTTTTTVN-3') (SEQ ID NO:5), where "N" is any base and "V" is either "A", "C" or "G"; IDT) and 0.25 µL 10 mM dNTP mix (Thermo), denatured at 55°C for 5 min and immediately placed on ice. 1.75 µL of first-strand reaction mix, containing 1 µL 5X Superscript IV First-Strand Buffer (Invitrogen), 0.25 µl 100 mM DTT (Invitrogen), 0.25 µl SuperScript IV reverse transcriptase (200 U/µl, Invitrogen), 0.25 µL RNaseOUT Recombinant Ribonuclease Inhibitor (Invitrogen), was then added to each well. Reverse transcription was carried out by incubating plates at the following temperature gradient: 4°C 2 minutes, 10°C 2 minutes, 20°C 2 minutes, 30°C 2 minutes, 40°C 2 minutes, 50°C 2 minutes and 55°C 10 minutes. All cells (or nuclei) were then pooled, stained with 4',6-diamidino-2-phenylindole (DAPI, Invitrogen) at a final concentration of 3 µM, and sorted at 25 nuclei per well into 5 µL EB buffer. Cells were gated based on DAPI stain such that singlets were discriminated from doublets and sorted into each well. 0.66 µl mRNA Second Strand Synthesis buffer (NEB) and 0.34 µl mRNA Second Strand Synthesis enzyme (NEB) were then added to each well, and second strand synthesis was carried out at 16°C for 180 min. Each well was then mixed with 5 µL Nextera TD buffer (Illumina) and 1 µL i7 only TDE1 enzyme (25 nM, Illumina, diluted in Nextera TD buffer), and then incubated at 55°C for 5 min to carry out tagmentation. The reaction was stopped by adding 10 µL DNA binding buffer (Zymo) and incubating at room temperature for 5 min. Each well was then purified using 30 uL AMPure XP beads (Beckman Coulter), eluted in 16 µL of buffer EB (Qiagen), then transferred to a fresh multi-well plate.

For PCR reactions, each well was mixed with 2µL of 10 µM P5 primer (5'-AATGATACGGCGACCACCGAGATCTACAC[i5]ACACTCTTTCCCTACACGACGC TCTTCCGATCT-3 ; IDT) (SEQ ID NO:6), 2 µL of 10 µM P7 primer (5'-CAAGCAGAAGACGGCATACGAGAT[i7]GTCTCGTGGGCTCGG-3'; IDT) (SEQ ID NO:7), and 20 µL NEBNext High-Fidelity 2X PCR Master Mix (NEB). Amplification was carried out using the following program: 72°C for 5 min, 98°C for 30 sec, 18-22 cycles of (98°C for 10 sec, 66°C for 30 sec, 72°C for 1 min) and a final 72°C for 5 min. After PCR, samples were pooled and purified using 0.8 volumes of AMPure XP beads. Library concentrations were determined by Qubit (Invitrogen) and the libraries were visualized by electrophoresis on a 6% TBE-PAGE gel. Libraries were sequenced on the NextSeq 500 platform (Illumina) using a V2 150 cycle kit (Read 1: 18 cycles, Read 2: 130 cycles, Index 1: 10 cycles, Index 2: 10 cycles).

Read alignments and downstream processing

Read alignment and gene count matrix generation for the single cell RNA-seq was performed using the pipeline that we developed for sci-RNA-seq (*48*) with minor modifications. Reads were first mapped to a reference genome with STAR/v2.5.2b (*49*), with gene annotations from GENCODE V19 for human, and GENCODE VM11 for mouse. For experiments with HEK293T and NIH/3T3 cells, we used an index combining chromosomes from both human (hg19) and mouse (mm10). For the A549 experiment, we used human genome build hg19.

The single cell sam files were first converted into alignment tsv file using sam2tsv function in jvarkit(*50*). Next, for each single cell alignment file, mutations matching the background SNPs were filtered out. For background SNP reference of A549 cells, we downloaded the paired-end bulk RNA-seq data for A549 cells from ENCODE (*28*) (sampled name: ENCFF542FVG, ENCFF538ZTA, ENCFF214JEZ, ENCFF629LOL, ENCFF149CJD, ENCFF006WNO, ENCFF828WTU, ENCFF380VGD). Each paired-end fastq files were first adaptor-clipped using trim_galore/0.4.1(*51*) with default settings, aligned to human hg19 genome build with STAR/v2.5.2b (*49*). Unmapped and multiple mapped reads were removed by samtools/v1.3 (52). Duplicated reads were filtered out by MarkDuplicates function in picard/1.105(*53*). De-duplicated reads from all samples were combined and sorted with samtools/v1.3 (52). Background SNPs were called by mpileup function in samtools/v1.3(*52*) and mpileup2snp function in VarScan/2.3.9(*54*). For HEK293T and NIH/3T3 test experiment, background SNP reference was generated in a similar pipeline above, with the aggregated single cell sam data from control condition (no S4U labeling and no IAA treatment condition).

For each single cell alignment file, all mutations with quality score <= 13 were removed. Mutations at the both ends of each reads were mostly due to sequencing errors, and thus also got filtered out. For each read, we checked if there are T > C mutations (for sense strand) or A > G mutations (for antisense strand), and labeled these mutated reads as newly synthesized reads.

Each cell was characterized by two digital gene expression matrixes from the full sequencing data and newly synthesized RNA data as described above. Genes with expression in equal or less than 5 cells were filtered out. Cells with fewer than 2000 UMIs or more than 80,000 UMIs were discarded. Cells with doublet score > 0.2 by doublet analysis pipeline Scrublet/0.2(*55*) were removed.

The dimensionality of the data was first reduced with PCA (after selecting the top 2,000 genes with highest variance) on digital gene expression matrixes on either full gene expression data or the newly synthesized gene expression data by Monocle 3 (*56, 57*). The top 10 PCs were selected for dimension reduction analysis with uniform manifold approximation and projection (UMAP/0.3.2), a recently proposed algorithm based on Riemannian geometry and algebraic topology to perform dimension reduction and data visualization (*26*). For joint analysis, we combined top 10 PCs calculated on the whole transcriptome and top 10 PCs on the newly synthesized transcriptome for each single cell before dimension reduction with UMAP. Cell clusters were done via densityPeak algorithm implemented in Monocle 3 (*56, 57*). We first performed UMAP analysis on joint information of all processed cells, and identified an outlier cluster (724 out of 7,404 cells). These cells were marked by high level expression of GATA3, a marker of differentiated cells (*34*), and were filtered out before downstream analysis.

Analysis for linking transcription factor (TF) to regulated genes

We aimed to identify links between TFs and regulated genes based on their covariance. Cells with more than 10,000 UMI detected, and genes with newly synthesis reads detected in more than 10% of all cells were selected. The full gene expression and newly synthesized gene count per cell were normalized by cell-specific library size factors computed on the full gene expression matrix by estimateSizeFactors in Monocle 3 (*56, 57*), log transformed, centered, then scaled by scale() function in R. For each gene detected, a LASSO regression model was constructed with package glmnet (*58*) to predict the normalized expression levels, based on the normalized expression of 853 TFs annotated in the "motifAnnotations_hgnc" data from package RcisTarget(*29*), by fitting the following model:

$$G_i = \beta_0 + \beta_t T_i$$

where $G_i$ is the adjusted gene expression value for gene i. It is calculated by the newly synthesized mRNA count for each cell, normalized by cell specific size factor ($SG_i$) estimate by estimateSizeFactors in Monocle 3 (*56, 57*) on the full expression matrix of each cell, and log transformed:

$$G_i = ln(\frac{g_i}{SG_i} + 0.1)$$

To simplify downstream comparison between genes, we standardize the response $G_i$ prior to fitting the model for each gene i with the scale() function in R.

Similar with $G_i$, $T_i$ is the adjusted TF expression value for each cell. It is calculated by the full TF expression count for each cell, normalized by cell specific size factor ($SG_i$) estimate by estimateSizeFactors in Monocle 3 (*56, 57*) on the full expression matrix of each cell, and log transformed:

$$T_i = ln(\frac{t_i}{SG_i} + 0.1)$$

Prior to fitting, $T_i$ are are standardized with the scale() function in R.

Our approach aims to TFs that may regulate each gene, by finding the subset that can be used to predict its expression in a regression model. However, a TF with expression correlated with a gene's expression does not guarantee it is regulating that gene: if gene A is specifically expressed in cell state 1 and TF B is specifically expressed in cell type 2. Although negative correlations between a TF's expression and a gene's newly synthesis rate could reflect the activity of a transcriptional repressor, we felt that the more likely explanation for negative links reported by glmnet was mutually exclusive patterns of cell-state specific expression and TF activity. Thus during prediction, we excluded TFs with negative correlated expression with the gene's synthesis rate and also low correlation coefficient (<= 0.03) links. We identified a total of 6,103 links between TFs and regulated genes.

To identify putative direct-binding targets" we intersected the links with TFs profiled in ENCODE Chip-seq experiment(*28*). Out of 1,086 links with TFs characterized in ENCODE, 807 links were validated by TF binding sites near gene promoters (*59*), a 4.3 folds enrichment in odd ratio (number of validated links over non-validated links) compared with background (odd ratio = 2.89 in links identified in LASSO regression vs. 0.67 in background, p-value < 2.2e-16, Fisher's Exact test). Only gene sets with significantly enrichment of the correct TF Chip-seq binding sites are retained (Fish's Exact test, False discovery rate of 5%), and pruned to remove indirect target genes without TF binding data support. 591 links were retained in this approach.

To expand the validated TF-gene links, we further applied package SCENIC(*29*), a pipeline to construct gene regulatory networks based on the enrichment of target TF motifs around genes' promoters (10kb). Each co-expression module identified by LASSO regression was analyzed using cis-regulatory motif analysis using RcisTarget(*29*). Only modules with significant motif enrichment of the correct TF regulator were retained, and pruned to remove indirect target genes without motif support. We filtered the TF-gene links by three correlation coefficient threshold (0.3, 0.4 and 0.5), and combined all links validated by RcisTarget(*29*). In total, there were 509 links validated by motif analysis approach. Combining both approaches, we identified a total 986 TF-gene regulatory links by the covariance between TF expression and gene synthesis rate, validated by DNA binding data or motif analysis. To evaluate the possibility that the links were artifacts of regularized regression, we permuted the sample IDs of the TF expression matrix and performed the same analysis. No links were identified after this permutation.

Ordering cells by functional TF modules

To calculate TF activity in each cell, newly synthesized UMI counts for genes within the target TF module were scaled by library size, log-transformed, aggregated and then mapped to Z-scores. As TFs with highly correlated or anti-correlated activity suggest they may function in linked biological process, we calculated the absolute Pearson's correlation coefficient between each pair of TF activity, and based on this we clustered TFs by ward.d2 clustering method in package pheatmap/1.0.12(*60*). Five functional TF modules were identified and annotated based on their functions.

To characterize cell states on the dimension of each functional TF modules, cells were ordered by the activity of cell cycle related TFs (TF module 1) or GR response related TFs (TF module 3) with UMAP (metric = "cosine", n_neighbors = 30, min_dist = 0.01). The cell cycle progression trajectory were validated by cell cycle gene markers in Seurat/2.3.4(*27*). Three cell cycle phases were identified by densityPeak algorithm implemented in Monocle 3 (*56, 57*), on the UMAP coordinates ordered by cell cycle TF modules. As each main cell cycle phase still showed variable TF activity and cell cycle marker expression, we segmented each phase to early/middle/late states by k-means clustering (k = 3), and recovered a total of nine cell cycle states. Three GR reponse states were identified by densityPeak algorithm implemented in Monocle 3 (*56, 57*).

Past transcriptome state recovery from sci-fate

To identify the past transcriptome state (the cell state before S4U labeling), we assume the mRNA half life is stable across different DEX treatmentment conditions. This assumption is further validated by self-consistency check later. Under this assumption, the partly degraded bulk transcriptome before the 2 hour S4U labeling should be the same between no DEX and 2 hour DEX treated cells. Thus their differences in whole transcriptome (bulk) should equal with their differences in the newly synthesized transcriptome (bulk) corrected by technique detection rate:

$$A_{0h} / S_{0h} - (N_{0h} / S_{0h}) / \alpha = A_{2h} / S_{2h} - (N_{2h} / S_{2h}) / \alpha$$

$A_{0h}$ is the aggregated UMI count for all cells in no DEX treatment group; $S_{0h}$ is the library size (total UMI count of cells) at no DEX treatment; $N_{0h}$ is the aggregated newly synthesised UMI count for all cells in no DEX treatment group; $A_{2h}$ is the aggregated UMI count for all cells in 2 hour DEX treatment group; $S_{2h}$ is the library size (total UMI count of cells) in 2 hour DEX treatment group; $N_{2h}$ is the aggregated newly synthesized UMI count for all cells in 2 hour DEX treatment group; $\alpha$ is the detection rate for sci-fate. In theory, one detection rate can be calculated for each gene. However, for genes with minor differences of newly synthesis rate between two conditions, the estimated $\alpha$ is dominated by noise. We thus selected genes showing higher differences in normalized newly synthesis rate between two conditions: we first tested a series of threshold for gene filtering and calculated the $\alpha$ for each gene. We then plotted the relationship between threshold and the ratio of genes with out-range $\alpha$ values (< 0 or > 1). We selected the threshold that was at the knee point of the plot with 186 genes selected. The differences in newly synthesized mRNA of these genes highly correlates with the differences in mRNA expression level (Pearson's r = 0.93, Fig. 35A), suggesting the new RNA detection rate is rather stable across genes. There is a median of 82% newly synthesized RNA captured by sci-fate.

We next computed the mRNA degradation rate across each 2 hours. As A549 cell population can be regarded stable without external perturbation, for 2 hour DEX treated cells, its past state (before 2 hour S4U labeling) should be the same with the 0 hour DEX treated cells. Similarly, the past state (before S4U labeling) for T = 0/2/4/6/8/10 hour DEX treated cells should be similar to the profiled T = 0/0/2/4/6/8 hour cells:

$$A_{t1} / S_{t1} - (N_{t1} / S_{t1}) / \alpha = A_{t0} / S_{t0} * \beta$$

$A_{t1}$ is the aggregated UMI count for all cells in t1; $S_{t1}$ is is the library size (the total UMI count of cells) at t1; $N_{t1}$ is the aggregated newly synthesized UMI count for all cells at t1; tris the estimated detection rate of sci-fate; $A_{t0}$ is the aggregated UMI count for all cells in t0; $S_{t0}$ is is the library size (the total UMI count of cells) at t0; $\beta$ is 1 - gene specific degradation rate between t0 and t1, and is related with the mRNA half life $\gamma$ by:

$$\beta = 1 - (1 / 2)^{(t1 - t0) / \gamma}$$

The gene degradation rate $\beta$ can be calculated on each 2 hour interval of DEX treatment. As a self-consistency check mentioned above, the gene degradation rates are highly correlated across different DEX treatment time (Fig. 35B). We then used the averaged gene degradation rate for downstream analysis.

With the detection rate and gene degradation rate estimated, the past transcriptome state of each cell can be estimated by:

$$a_{t1} - n_{t1} / \alpha = a_{t0} * \beta$$

$a_{t1}$ is the single cell UMI count in t1; $n_{t1}$ is the single cell newly synthesized UMI count at t1; $\alpha$ is the estimated detection rate of sci-fate; $\beta$ is 1 - gene specific degradation rate between t0 and t1. $a_{t0}$ is the estimated single cell UMI count in a past time point t0, with all negative values converted to 0.

Linkage analysis to build single cell state trajectory

By linkage analysis, we aim to identify linked parent and child cells in the same cell trajectory. Technically, for cells at t1, we combines their past state transcriptome state (before S4U labeling, 2 hours before t1 in our experiment) as one group 1, and the full transcriptome state of t0 (2 hours before t1) as another group 2. Assuming there is no apparent cell apoptosis, these two groups should have similar cell state distribution. We applied a manifold alignment strategy to identify common cell states between two data sets, based on common sources of variation(*27*). This analysis is based on another assumption that the past and current state of each cell (except cells at the start and end time points) are comprehensively detected, which holds true in our data sets as over 6,000 cells are profiled (over 1,000 cells per condition), or a cell for less than one min during cell cycle. As a result of the pipeline, cell states from t0 and past cell states from t1 are aligned in the same UMAP space. Violation of the assumptions above can be detected by outliers during alignment of the two data sets. For each cell A in t1, we selected its nearest neighbour in t0 as its parent state in the alignment UMAP space. Similarly, for each cell in t0, we selected its nearest neighbour in t1 as its child cell state. Of note, the link is not necessary to be bi-directional: the parent state of one cell may be linked to a different child cell. As the parent state and child state was identified for each cell (except the cells at 0 hour and 10 hour), we then identified the linked parent cell of each cell's parent, and similarly the linked child cell of each cell's child. Thus each single cell can be characterized by a single cell state transition path across all five time points spanning 10 hours. As multiple cells (>50) are profiled at each cell state, stochastic cell state transition process can also be captured.

Dimension reduction and clustering analysis for single cell transcriptome dynamics

For dimension reduction on single cell transcriptome dynamics, top 5 PCs for full transcriptome and top 5 PCs for newly synthesized transcriptome were selected for each state, and combined in temporal order along single cell state trajectory for UMAP analysis. Main cell trajectory types were identified by density peak clustering algorithm(*61*).

With cell state proportion at the beginning time point (0 hour treatment) and cell state transition probabilities estimated from the data, we first predicted the cell state distribution after 2 hours, assuming the cell state transition process in DEX treatment is a cell-autonomous, time-independent, Markovian dynamics. Similarly, the cells state distribution at later time point can be calculated based on the predicted cell state distribution 2 hours before.

Inter-state transition probability prediction by state instability

Cell state instability is defined as the probability of each state moving to other states after 2 hours. To calculate cell state distance, we first sampled equal number (n = 50) of cells at each state, and aggregated the full transcriptome and newly synthesized transcriptome of all cells within the state. Each cell state can be defined by the joint information combining the whole and newly synthesized transcriptome. The cell state distance is calculated as the Pearson's correlation coefficient of the joint information between two states.

To predict inter-state transition probability, we constructed a 3 layer neural network (units number: 128, 128, 26 with relu activation at each layer; loss function: cosine_proximity, batch size: 128, epochs: 80) with Keras/2.2.4(*62*). For input, we used state instability of current state, the normalized state instability of the other 26 states (scaled by the instability of current state), and transition distance (squared) from current state to the other 26 states (in the same order of states in state instability vector). To avoid over-fitting, we permuted the state orders in state instability 200 times for each input, while still keeping the state order of state transition distance the same with the state instability. To evaluate the model performance, we apply leave-one-out validation by training the model on 26 states, and validate the model on the left state on predicting the state transition probabilities to all the other 26 states. For predicting the inter-state probability with state transition distance only, the same model is used for training and validation with all input state instabilities replaced with 1.

References

1. N. Moris, C. Pina, A. M. Arias, Transition states and cell fate decisions in epigenetic landscapes. Nat. Rev. Genet. 17, 693-703 (2016).

2. A. Filipczyk et al., Network plasticity of pluripotency transcription factors in embryonic stem cells. Nat. Cell Biol. 17, 1235-1246 (2015).

3. S. Hormoz et al., Inferring Cell-State Transition Dynamics from Lineage Trees and Endpoint Single-Cell Measurements. Cell Syst. 3, 419-433.e8 (2016).

4. V. A. Herzog et al., Thiol-linked alkylation of RNA to assess expression dynamics. Nat. Methods. 14, 1198-1204 (2017).

5. J. A. Schofield, E. E. Duffy, L. Kiefer, M. C. Sullivan, M. D. Simon, TimeLapse-seq: adding a temporal dimension to RNA sequencing through nucleoside recoding. Nat. Methods. 15, 221-225 (2018).

6. J. C. Buckingham, Glucocorticoids: exemplars of multi-tasking. Br. J. Pharmacol. 147, S258 (2006).

7. M. D. Cleary, C. D. Meiering, E. Jan, R. Guymon, J. C. Boothroyd, Biosynthetic labeling of RNA with uracil phosphoribosyltransferase allows cell-specific microarray analysis of mRNA synthesis and decay. Nat. Biotechnol. 23, 232-237 (2005).

8. L. Dolken et al., High-resolution gene expression profiling for simultaneous kinetic parameter analysis of RNA synthesis and decay. RNA. 14, 1959-1972 (2008).

9. C. Miller et al., Dynamic transcriptome analysis measures rates of mRNA synthesis and decay in yeast. Mol. Syst. Biol. 7, 458-458 (2014).

10. E. E. Duffy et al., Tracking Distinct RNA Populations Using Efficient and Reversible Covalent Chemistry. Mol. Cell. 59, 858-866 (2015).

11. B. Schwalb et al., TT-seq maps the human transient transcriptome. Science. 352, 1225-1228 (2016).

12. M. Rabani et al., Metabolic labeling of RNA uncovers principles of RNA production and degradation dynamics in mammalian cells. Nat. Biotechnol. 29, 436-442 (2011).

13. M. R. Miller, K. J. Robinson, M. D. Cleary, C. Q. Doe, TU-tagging: cell type-specific RNA isolation from intact complex tissues. Nat. Methods. 6, 439-441 (2009).

14. D. A. Cusanovich et al., Multiplex single cell profiling of chromatin accessibility by combinatorial cellular indexing. Science. 348, 910-914 (2015).

15. J. Cao et al., Comprehensive single-cell transcriptional profiling of a multicellular organism. Science. 357, 661-667 (2017).

16. J. Cao et al., Joint profiling of chromatin accessibility and gene expression in thousands of single cells. Science. 361, 1380-1385 (2018).

17. V. Ramani et al., Massively multiplex single-cell Hi-C (2016), , doi:10.1101/065052.

18. R. M. Mulqueen et al., Highly scalable generation of DNA methylation profiles in single cells. Nat. Biotechnol. 36, 428-431 (2018).

19. S. A. Vitak et al., Sequencing thousands of single-cell genomes with combinatorial indexing. Nat. Methods. 14, 302-308 (2017).

20. Y. Yin et al., High-throughput mapping of meiotic crossover and chromosome mis-segregation events in interspecific hybrid mice (2018), , doi:10.1101/338053.

21. A. B. Rosenberg et al., Single-cell profiling of the developing mouse brain and spinal cord with split-pool barcoding. Science. 360, 176-182 (2018).

22. T. E. Reddy et al., Genomic determination of the glucocorticoid response reveals unexpected mechanisms of gene regulation. Genome Res. 19, 2163-2171 (2009).

23. S. John et al., Chromatin accessibility pre-determines glucocorticoid receptor binding patterns. Nat. Genet. 43, 264-268 (2011).

24. T. E. Reddy, J. Gertz, G. E. Crawford, M. J. Garabedian, R. M. Myers, The Hypersensitive Glucocorticoid Response Specifically Regulates Period 1 and Expression of Circadian Genes. Mol. Cell. Biol. 32, 3756-3767 (2012).

25. C. M. Vockley et al., Direct GR Binding Sites Potentiate Clusters of TF Binding across the Human Genome. Cell. 166, 1269-1281.e19 (2016).

26. L. McInnes, J. Healy, N. Saul, L. Großberger, UMAP: Uniform Manifold Approximation and Projection. Journal of Open Source Software. 3, 861 (2018).

27. A. Butler, P. Hoffman, P. Smibert, E. Papalexi, R. Satija, Integrating single-cell transcriptomic data across different conditions, technologies, and species. Nat. Biotechnol. 36, 411-420 (2018).

28. The ENCODE Project Consortium, The ENCODE (ENCyclopedia Of DNA Elements) Project. Science. 306, 636-640 (2004).

29. S. Aibar et al., SCENIC: single-cell regulatory network inference and clustering. Nat. Methods. 14, 1083-1086 (2017).

30. M. Boruk, J. G. A. Savory, R. J. G. Haché, AF-2-Dependent Potentiation of CCAAT Enhancer Binding Proteinβ -Mediated Transcriptional Activation by Glucocorticoid Receptor. Mol. Endocrinol. 12, 1749-1763 (1998).

31. W. Qin et al., Identification of functional glucocorticoid response elements in the mouse FoxO1 promoter. Biochem. Biophys. Res. Commun. 450, 979-983 (2014).

32. C. S. Sheela Rani, N. Elango, S.-S. Wang, K. Kobayashi, R. Strong, Identification of an Activator Protein-1-Like Sequence as the Glucocorticoid Response Element in the Rat Tyrosine Hydroxylase Gene. Mol. Pharmacol. 75, 589 (2009).

33. M. Fischer, G. A. Müller, Cell cycle transcription control: DREAM/MuvB and RB-E2F complexes. Crit. Rev. Biochem. Mol. Biol. 52, 638-662 (2017).

34. J. Chou, S. Provot, Z. Werb, GATA3 in development and cancer differentiation: cells GATA have it! J. Cell. Physiol. 222, 42-49 (2010).

35. J. Y. C. Madhurima Biswas, Role of Nrf1 in antioxidant response element-mediated gene expression and beyond. Toxicol. Appl. Pharmacol. 244, 16 (2010).

36. I.-G. Ryoo, M.-K. Kwak, Regulatory crosstalk between the oxidative stress-related transcription factor Nfe212/Nrf2 and mitochondria. Toxicol. Appl. Pharmacol. 359, 24-33 (2018).

37. J. M. Harmon, M. R. Norman, B. J. Fowlkes, E. B. Thompson, Dexamethasone induces irreversible G1 arrest and death of a human lymphoid cell line. J. Cell. Physiol. 98, 267-278 (1979).

38. A. K. Greenberg et al., Glucocorticoids inhibit lung cancer cell growth through both the extracellular signal-related kinase pathway and cell cycle regulators. Am. J. Respir. Cell Mol. Biol. 27, 320-328 (2002).

39. J. Cao et al., Comprehensive single-cell transcriptional profiling of a multicellular organism. Science. 357, 661-667 (2017).

40. J. Cao et al., Joint profiling of chromatin accessibility and gene expression in thousands of single cells. Science. 361, 1380-1385 (2018).

41. W. Matsushima et al., SLAM-ITseq: sequencing cell type-specific transcriptomes without cell sorting. Development. 145 (2018), doi:10.1242/dev.164640.

42. U. Sharma et al., Small RNAs are trafficked from the epididymis to developing mammalian sperm (2017), , doi:10.1101/194522.

43. A. McKenna et al., Whole-organism lineage tracing by combinatorial and cumulative genome editing. Science. 353, aaf7907 (2016).

44. B. Raj et al., Simultaneous single-cell profiling of lineages and cell types in the vertebrate brain. Nat. Biotechnol. 36, 442-450 (2018).

45. K. L. Frieda et al., Synthetic recording and in situ readout of lineage information in single cells. Nature. 541, 107-111 (2017).

46. H. Wickham, ggplot2: Elegant Graphics for Data Analysis (Springer, 2016).

47. M. Muhar et al., SLAM-seq defines direct gene-regulatory functions of the BRD4-MYC axis. Science. 360, 800-805 (2018).

48. J. Cao et al., Comprehensive single-cell transcriptional profiling of a multicellular organism. Science. 357, 661-667 (2017).

49. A. Dobin et al., STAR: ultrafast universal RNA-seq aligner. Bioinformatics. 29, 15-21 (2013).

50. P. Lindenbaum, JVarkit: java-based utilities for Bioinformatics. figshare (2015).

51. FelixKrueger, FelixKrueger/TrimGalore. GitHub, (available at https://github.com/FelixKrueger/TrimGalore).

52. H. Li et al., The Sequence Alignment/Map format and SAMtools. Bioinformatics. 25, 2078-2079 (2009).

53. Picard Tools - By Broad Institute, (available at http://broadinstitute.github.io/picard/).

54. D. C. Koboldt et al., VarScan 2: somatic mutation and copy number alteration discovery in cancer by exome sequencing. Genome Res. 22, 568-576 (2012).

55. S. L. Wolock, R. Lopez, A. M. Klein, Scrublet: computational identification of cell doublets in single-cell transcriptomic data (2018), , doi:10.1101/357368.

56. X. Qiu et al., Reversed graph embedding resolves complex single-cell trajectories. Nat. Methods. 14, 979-982 (2017).

57. cole-trapnell-lab, cole-trapnell-lab/monocle-release. *GitHub,* (available at https://github.com/cole-trapnell-lab/monocle-release).\

58. J. Friedman, T. Hastie, R. Tibshirani, Regularization Paths for Generalized Linear Models via Coordinate Descent. J. Stat. Softw. 33 (2010), doi:10.18637/jss.v033.i01.

59. Dataset - ENCODE Transcription Factor Binding Site Profiles, (available at http://amp.pharm.mssm.edu/Harmonizome/dataset/ENCODE+Transcription+Factor+Bindi ng+Site+Profiles).

60. raivokolde, raivokolde/pheatmap. *GitHub,* (available at https://github.com/raivokolde/pheatmap).

61. A. Rodriguez, A. Laio, Clustering by fast search and find of density peaks. Science. 344, 1492-1496 (2014).

62. keras-team, keras-team/keras. *GitHub,* (available at https://github.com/keras-team/keras).

Example 4

Multiplex Transcript Capture

Most single cell RNA sequencing methods saturate at a coverage of 15,000 to 50,000 unique reads per cell (Ziegenhain et al. 2017), while the total mRNA content of single cells can range from 50,000 to 300,000 molecules (Marinov et al. 2014). Furthermore, most of these methods use oligo(dT) priming for reverse transcription (RT), which focuses sequencing at the 3' end of RNAs. This means that these methods have limited power to detect changes in the abundance of any given transcript. Recent studies that profiled large numbers of cells (Gasperini et al. 2019; Cao et al. 2019) have necessitated very high sequencing depth: the Illumina NovaSeq runs utilized in these studies cost $30,000 each, placing such experiments firmly out of reach for most groups.

However, in both cases, the number of reads required to glean biological insights from the data is relatively small. In single cell readouts of noncoding perturbations, only genes cis to the regulatory element being disrupted are tested for changes in expression (Xie et al. 2017; Gasperini et al. 2018). In cell atlas experiments, while global expression patterns are used to cluster similar cells, cell type assignment was done using a small number of key transcription factor genes. Thus, the ability to focus readout to gene transcripts that are most informative in these experiments would result in a large reduction in the sequencing depth required, and an increase in power to detect subtle differences between cells.

We focused single-cell sequencing on mRNAs of interest by using specific RT primers rather than oligo(dT) priming. A similar method was recently used in bulk to specifically sequence all known splice junctions in yeast, resulting in a 100 fold enrichment for targeted regions over non-targeted (Xu et al., 2018). A pool of RT primers tiling across transcripts of interest will allow the reduction of a transcriptome library (sciRNA-seq) readout to hundreds of captured transcripts per experiment.

This sciRNA-seq gerrymandering has multiple advantages over oligo(dT) priming. First, it will direct sequencing to regions of the genome that we have determined to be most informative for each experiment. Second, it allows each RNA molecule multiple opportunities to be reverse transcribed into cDNA, increasing the likelihood of detection per RNA molecule. Third, this approach allows us to target only amplicons that are uniquely mappable and could reduce background of ribosomal reads more than the alternatives of random hexamer or oligo(dT) priming. Fourth, it allows us to target informative regions of mRNAs such as splice junctions and exons resulting from alternative transcription start site events, thus providing isoform information not readily detected with conventional sciRNA-seq.

sciRNA-seq is uniquely suited to modification with multiple RT primers. Most single cell RNA-seq methods use beads bound with unique identifier oligos to append cell identifying barcodes to each cell's transcriptome, usually capturing mRNAs by hybridizing to their poly(A) tail. While such beads have been modified to add a handful of specific RT primers to increase coverage of a few transcripts (Saikia et al. 2018), this strategy would be difficult to scale to hundreds of targeted transcripts or rapidly change between experiments. Thus, the adaptability of single cell combinatorial indexing will be helpful in the development of multiplex RT single cell RNA-seq.

The workflow for this aspect is similar to the three level sciRNA-seq protocol described at Examples 1 and 3, but in some versions does not include the RT step.

1. Design a pool of RT primers. In one aspect, these will be synthesized individually and pooled. For targeting >384 amplicons, a library of primers can be synthesized, propagated as double stranded DNA, and processed to produce single stranded primers as described (Xu et al. 2018). This second strategy allows the addition of many unique indexes to the RT primers (allowing sciRNA-seq indexing at RT and final PCR).

2. Multiplex RT, using the pool of primers. This will be either a single reaction with thousands of cells (if no indexing is done at this step), or many parallel reactions that add a well specific index when reverse transcribing.

3. Ligate a hairpin adapter to add a well specific index.

4. Pool all cells and carry out second strand synthesis.

5. Distribute cells amongst many wells, and carry out tagmentation to add a second constant PCR handle.

6. PCR amplification, adding a final well specific index.

7. Sequence.

Primer design workflow:

1. Collect sequence for all exons from the genes being targeted.

2. Parse out all possible 25 bp RT primers.

3. Filter candidate RT primers by:

a. GC content between 40-60%, corresponding to melting temperatures that are roughly between 55 and 70 degrees.

b. At least 2 G or C in the last 5 nt of the primer, increasing likelihood that the annealed RT primer will be a good substrate for extension by the reverse transcriptase enzyme

c. Likelihood of off target priming. In our first experiment, we found that while our target genes were highly enriched, a large fraction of reads were still derived from other RNAs that are abundant within cells. Most of these off target priming events were the result of ~5-8 bp of complementarity between the end 3' end of the primer and the off target RNA. Thus, our latest primer design pipeline takes into account the abundance of the final hexamer of the RT primer within total cellular RNA. We only include RT primers where this last hexamer is either:

i. Not present at all within ribosomal RNA. From the set of 'Not So Random' or NSR hexamers described previously(Armour et al. 2009). Primers that pass this filter will be much less likely to have off target priming within ribosomal RNA.

ii. Lowly represented within total cellular RNA. We counted the abundance of all 4,096 possible hexamers within PRO-seq reads mapped to the human genome(Core et al. 2014). PRO-seq measures all nascent transcription within cells, including ribosomal transcription. We only use RT primers that end in hexamers that are within the bottom quartile of abundance in this dataset. This rescues some hexamers that, though present within ribosomal RNA, are not not abundant as RNA within cells.

This abundance filter drastically changes primer choice. There is only ~17% overlap between primers chosen by our pipeline with or without this filter. Future versions of our design pipeline will refine this off target filter. As we collect data for more primers, we should be able to evaluate more off target priming events.

4. Filter candidates by mappability. We aligned each candidate to hg19 using bowtie, allowing 3 mismatches. This step ensures that each primer will have only one target site in the genome.

5. Of the possible primers that have made it through these filters, pick the set that tiles most evenly across the gene.

For each gene we are targeting, we decide how many primer to design per exon. We include the first and last primer that passes filters for each exon, and then pick internal primers that cover the exon most evenly by minimizing the distance from the primer locations that would exactly split the exon in to n chunks.

For example, for a 300 bp exon, where we are searching for 3 primers, we take the primers closest to positions 1, 150, and 300 that passed all filters up to this point.

6. For our pilot experiment, RT primers were ordered in 384 well plates, and pooled to create an equimolar mixture of all primers. This mixture was then phosphorylated with T4 polynucleotide kinase, to allow for ligation of an indexed hairpin oligo during the sciRNA-seq library generation (Cao et al. 2019). This is much more cost effective than ordering phosphorylated oligos. The 25 bp RT primers also add an 8 bp unique molecular identifier (UMI) and a 6 bp handle for annealing of a hairpin oligo that will add a well specific index (for combinatorial indexing) and a PCR handle.

This process can be iterative when each RT primer is ordered separately: a lower off target ratio was achieved in later experiments by selectively repooling primers that were found to have favorable capture rates in the first experiment. Each Illumina sequencing read spans the 25 bp RT primer, and the captured RNA molecule, allowing us map RT primers and captured molecules separately to calculate an on-target rate for each primer.

Later rounds could incorporate more RT primers by having them array synthesized. The primer library can be propagated by PCR, and made single stranded by selective exonucleolytic degradation of the strand that does not include a blocking group in the PCR primer (Xu et al. 2018). A large array could be used to synthesize multiple pools of primers: if each pool has a specific PCR handle, one array could be used to generate dozens of pools of thousands of primers each that could be selectively amplified.

Multiplex Reverse Transcription:

Multiplex target capture could conceivably be done at several steps during the RNA-seq library generation protocol. However, we believe that reverse transcription is the easiest to parallelize. Highly multiplex PCR reactions are very difficult to carry out successfully. PCR reactions include many (10-20) cycles. This means that issues with off target annealing are exacerbated after exponential growth through these cycles that often outpaces that of the desired target. In multiplex PCR, each target is afforded two specific PCR primers. The goal is for these two primers to specifically amplify their target only. However, in a large pool of primers, there will be several combinations that anneal to other primers with in the pool. Because the concentration of primers is much higher than that of the template molecules, these primer dimers will dominate the pool by the end of the PCR. The infeasibility of highly multiplexed PCR is why many targeted amplification protocols, such as exome sequencing, often utilize molecular inversion probes to capture targets(Hiatt et al. 2013). In such protocols, target specificity is achieved through a single annealing step between probe and target. The target specific probes add PCR handles, that are then used in a target generic PCR amplification. Single cell combinatorial indexing methods rely upon indexing at several steps during library generation: an inversion probe method for capturing targets from cDNA would not allow for enough indexing steps.

For multiplex target capture, we use a specific reverse transcription primer, followed by a PCR reaction that amplifies all molecules that we reverse transcribed. Thus, our strategy is analogous to using molecular inversion probes for targeted DNA amplification: a single step (reverse transcription) selectively targets transcripts of interest, and adds a general PCR handle that can be used to amplify all targeted molecules during PCR. Thus, high specificity during reverse transcription is critical. Maintaining a high temperature after annealing of RT primers is helpful for multiplex specific priming. Normal reverse transcription protocols denature a mixture of RNA and reverse transcription primer, and cool to 4 degrees to allow annealing. This low annealing temperature is too permissive to off target annealing events. We need to ensure that the only annealing events that are able to extend are those where the whole of the highly specific RT primers that we have designed have found their targets. Thus, we maintain a high temperature during the entire protocol, as inspired by other multiplex specific reverse transcription methods (Xu et al. 2018). We denature a mixture of fixed cells, RT primer pool, and dNTPs at 65°C, anneal at 53°C, and then add a reverse transcription enzyme/buffer mixture that is pre-equilibrated at 53°C to the annealing reaction, and extend at 53°C for 20 minutes. Thus, the RT primers do not have the opportunity to anneal at a low temperature between the denaturing and extension steps.

The rest of the method follows the methods described in Examples 1 and 3. A hairpin adapter is ligated in situ, adding a cell index. Cells are pooled, washed, and split into new wells for the last indexing step. In these wells, second strand synthesis is carried out. Double stranded cDNA is then tagemented, to add a second general PCR handle (the first handle is from ligation, second is from tagmentation). DNA is purified from cells by Ampure bead binding, and then PCR is carried out, adding a second index.

Preliminary results:

All results, shown in Figs 40-42, are from a bulk (no single cell combinatorial indexing) in situ (all steps carried out in paraformaldehyde fixed nuclei) library made using a pool of RT primer targeting genes in the LMO2 locus in K562 cells.

References:

Armour, Christopher D., John C. Castle, Ronghua Chen, Tomas Babak, Patrick Loerch, Stuart Jackson, Jyoti K. Shah, et al. 2009. "Digital Transcriptome Profiling Using Selective Hexamer Priming for cDNA Synthesis." Nature Methods 6 (9): 647-49.

Cao, Junyue, Malte Spielmann, Xiaojie Qiu, Xingfan Huang, Daniel M. Ibrahim, Andrew J. Hill, Fan Zhang, et al. 2019. "The Single-Cell Transcriptional Landscape of Mammalian Organogenesis." Nature 566 (7745): 496-502.

Core, Leighton J., André L. Martins, Charles G. Danko, Colin T. Waters, Adam Siepel, and John T. Lis. 2014. "Analysis of Nascent RNA Identifies a Unified Architecture of Initiation Regions at Mammalian Promoters and Enhancers." Nature Genetics 46 (12): 1311-20.

Gasperini, Molly, Andrew J. Hill, José L. McFaline-Figueroa, Beth Martin, Seungsoo Kim, Melissa D. Zhang, Dana Jackson, et al. 2019. "A Genome-Wide Framework for Mapping Gene Regulation via Cellular Genetic Screens." Cell 176 (6): 1516.

Gasperini, Molly, Andrew Hill, José L. McFaline-Figueroa, Beth Martin, Cole Trapnell, Nadav Ahituv, and Jay Shendure. 2018. "crisprQTL Mapping as a Genome-Wide Association Framework for Cellular Genetic Screens." bioRxiv. https://doi.org/10.1101/314344.

Hiatt, Joseph B., Colin C. Pritchard, Stephen J. Salipante, Brian J. O'Roak, and Jay Shendure. 2013. "Single Molecule Molecular Inversion Probes for Targeted, High-Accuracy Detection of Low-Frequency Variation." Genome Research 23 (5): 843-54.

Marinov, Georgi K., Brian A. Williams, Ken McCue, Gary P. Schroth, Jason Gertz, Richard M. Myers, and Barbara J. Wold. 2014. "From Single-Cell to Cell-Pool Transcriptomes: Stochasticity in Gene Expression and RNA Splicing." Genome Research 24 (3): 496-510.

Saikia, Mridusmita, Philip Burnham, Sara H. Keshavjee, Michael F. Z. Wang, Pablo Moral-Lopez, Meleana M. Hinchman, Charles G. Danko, John S. L. Parker, and Iwijn De Vlaminck. 2018. "Simultaneous Multiplexed Amplicon Sequencing and Transcriptome Profiling in Single Cells." bioRxiv. https://doi.org/10.1101/328328.

Xie, Shiqi, Jialei Duan, Boxun Li, Pei Zhou, and Gary C. Hon. 2017. "Multiplexed Engineering and Analysis of Combinatorial Enhancer Activity in Single Cells." Molecular Cell 66 (2): 285-99.e5.

Xu, Hansen, Benjamin J. Fair, Zach Dwyer, Michael Gildea, and Jeffrey A. Pleiss. 2018. "Multiplexed Primer Extension Sequencing Enables High Precision Detection of Rare Splice Isoforms." bioRxiv. https://doi.org/10.1101/331629.

Ziegenhain, Christoph, Beate Vieth, Swati Parekh, Björn Reinius, Amy Guillaumet-Adkins, Martha Smets, Heinrich Leonhardt, Holger Heyn, Ines Hellmann, and Wolfgang Enard. 2017. "Comparative Analysis of Single-Cell RNA Sequencing Methods." Molecular Cell 65 (4): 631-43.e4.

The foregoing detailed description and examples have been given for clarity of understanding only. No unnecessary limitations are to be understood therefrom. The disclosure is not limited to the exact details shown and described, for variations obvious to one skilled in the art will be included within the disclosure defined by the claims.

Unless otherwise indicated, all numbers expressing quantities of components, molecular weights, and so forth used in the specification and claims are to be understood as being modified in all instances by the term "about." Accordingly, unless otherwise indicated to the contrary, the numerical parameters set forth in the specification and claims are approximations that may vary depending upon the desired properties sought to be obtained by the present disclosure. At the very least, and not as an attempt to limit the doctrine of equivalents to the scope of the claims, each numerical parameter should at least be construed in light of the number of reported significant digits and by applying ordinary rounding techniques.

Notwithstanding that the numerical ranges and parameters setting forth the broad scope of the disclosure are approximations, the numerical values set forth in the specific examples are reported as precisely as possible. All numerical values, however, inherently contain a range necessarily resulting from the standard deviation found in their respective testing measurements.

All headings are for the convenience of the reader and should not be used to limit the meaning of the text that follows the heading, unless so specified.

## Claims

1. A method for preparing a sequencing library comprising nucleic acids from a plurality of single nuclei or cells, the method comprising:

   (a) providing a plurality of nuclei or cells in a first plurality of compartments, wherein each compartment comprises a subset of nuclei or cells;
   (b) labeling newly synthesized RNA in the subsets of cells or nuclei obtained from the cells;
   (c) processing RNA molecules in each subset of nuclei or cells to generate indexed nuclei or cells,

      wherein the processing comprises adding to RNA nucleic acids present in each subset of nuclei or cells a first compartment specific index sequence to result in indexed nucleic acids present in indexed nuclei or cells, wherein the processing comprises ligation, primer extension, hybridization, or amplification; and

   (d) combining the indexed nuclei or cells to generate pooled indexed nuclei or cells.

2. The method of claim 1, wherein the processing comprises:
   contacting subsets with reverse transcriptase and a primer that anneals to RNA nucleic acids, resulting in double stranded nucleic acids comprising the primer and the corresponding DNA nucleotide sequence of the RNA molecules.

3. The method of claim 2, wherein the primer comprises a poly-T nucleotide sequence that anneals to a mRNA poly(A) tail.

4. The method of claim 3, wherein the processing further comprises contacting subsets with a second primer, wherein the second primer comprises a sequence that anneals to a predetermined DNA nucleic acid; and optionally, the second primer comprises a compartment specific index.

5. The method of claim 2, wherein the primer comprises a sequence that anneals to a predetermined RNA nucleic acid, the method comprises primers in different compartments that anneal to different nucleotides of the same predetermined RNA nucleic acid.

6. The method of claim 1, wherein the processing to add the first compartment specific index sequence comprises a two-step process of adding a nucleotide sequence comprising a universal sequence to the RNA nucleic acids to result in nucleic acids comprising the universal sequence, and then adding the first compartment specific index sequence to the nucleic acids comprising the universal sequence.

7. The method of claim 1, where pre-existing RNA nucleic acids and newly synthesized RNA nucleic acids are labeled with the same index in the same compartment.

8. The method of claim 1, wherein the labeling comprises incubating the plurality of nuclei or cells in a composition comprising a nucleotide label, wherein the nucleotide label is incorporated into the newly synthesized RNA.

9. The method of claim 1, further comprising exposing subsets of nuclei or cells to a predetermined condition before the labeling, wherein preferably the predetermined condition comprises exposure to an agent comprising a protein, a non-ribosomal protein, a polyketide, an organic molecule, an inorganic molecule, an RNA or RNAi molecule, a carbohydrate, a glycoprotein, a nucleic acid, or a combination thereof; but most preferably the agent comprises a therapeutic drug.

10. The method of claim 9, wherein the predetermined condition of two or more compartments is different when the method comprises exposing subsets of nuclei or cells to a predetermined condition before the labeling.

11. The method of claim 1, further comprising:

distributing subsets of the pooled indexed nuclei or cells into a second plurality of compartments and adding to indexed nucleic acids present in subsets of nuclei or cells a second index sequence to generate dual-indexed nuclei or cells comprising dual-indexed nucleic acid fragments, wherein the adding comprises ligation, primer extension, hybridization, amplification, or transposition;
combining the dual-indexed nuclei or cells to generate pooled dual-indexed nuclei or cells.

12. The method of claim 11, further comprising:

distributing subsets of the pooled dual-indexed nuclei or cells into a third plurality of compartments and adding to indexed nucleic acids present in subsets of nuclei or cells a third index sequence to generate triple-indexed nuclei or cells comprising triple-indexed nucleic acid fragments, wherein the adding comprises ligation, hybridization, primer extension, amplification, or transposition;
combining the triple-indexed nuclei or cells to generate pooled triple-indexed nuclei or cells.

13. The method of claim 1, wherein the adding comprises ligation of the first compartment specific index sequence, further comprising adding a second index sequence to generate dual-indexed nuclei or cells comprising dual-indexed nucleic acid fragments, wherein the adding comprises transposition.

14. The method of claim 11, wherein the adding comprises ligation of the second compartment specific index sequence, further comprising adding a third index sequence to generate dual-indexed nuclei or cells comprising triple-indexed nucleic acid fragments, wherein the adding comprises transposition.

15. The method of claim 1, further comprising obtaining the indexed nucleic acids from the pooled indexed nuclei or cells, thereby producing a sequencing library from the plurality of nuclei or cells.

16. The method of claim 11, further comprising obtaining the dual-indexed nucleic acids from the pooled dual-indexed nuclei or cells, thereby producing a sequencing library from the plurality of nuclei or cells.

17. The method of claim 12, further comprising obtaining the triple-indexed nucleic acids from the pooled triple -indexed nuclei or cells, thereby producing a sequencing library from the plurality of nuclei or cells.

18. The method of claim 2, wherein the primer comprises a sequence that anneals to a predetermined RNA nucleic acid, and preferably wherein the predetermined RNA nucleic acid is a mRNA.

19. The method of claim 2, wherein the primer comprises a template-switch primer.

20. The method of claim 8, wherein the nucleotide label comprises a nucleotide analog, a hapten-labeled nucleotide, mutagenic nucleotide, or a nucleotide that can be modified by a chemical reaction.

21. The method of claim 8, wherein more than one nucleotide label is incorporated into the newly synthesized RNA, and preferably the ratio of the nucleotide label or labels is different for different compartments or time points

22. The method of claim 9, wherein the exposing and the labeling occur at the same time or the exposing occurs before the labeling.

**Patentansprüche**

1. Ein Verfahren zur Herstellung einer Sequenzierungsbibliothek, die Nukleinsäuren aus einer Vielzahl von einzelnen Zellkernen oder Zellen beinhaltet, wobei das Verfahren Folgendes beinhaltet:

(a) Bereitstellen einer Vielzahl von Zellkernen oder Zellen in einer ersten Vielzahl von Abteilen, wobei jedes Abteil eine Untermenge von Zellkernen oder Zellen beinhaltet;
(b) Markieren neu synthetisierter RNA in den Untermengen von Zellen oder Zellkernen, die aus den Zellen erhalten wurden;
(c) Verarbeiten von RNA-Molekülen in jeder Untermenge von Zellkernen oder Zellen, um indizierte Zellkerne oder Zellen zu erzeugen,

wobei das Verarbeiten das Hinzugeben einer ersten abteilspezifischen Indexsequenz zu RNA-Nukleinsäuren, die in jeder Untermenge von Zellkernen oder Zellen vorhanden sind, beinhaltet, um indizierte Nukleinsäuren, die in indizierten Zellkernen oder Zellen vorhanden sind, zu ergeben, wobei das Verarbeiten Ligation, Primerverlängerung, Hybridisierung oder Amplifikation beinhaltet; und

(d) Kombinieren der indizierten Zellkerne oder Zellen, um gepoolte indizierte Zellkerne oder Zellen zu erzeugen.

2. Verfahren gemäß Anspruch 1, wobei das Verarbeiten Folgendes beinhaltet:
In-Kontakt-Bringen von Untermengen mit reverser Transkriptase und einem Primer, der sich an RNA-Nukleinsäuren anlagert, was doppelsträngige Nukleinsäuren ergibt, die den Primer und die entsprechende DNA-Nukleotidsequenz der RNA-Moleküle beinhalten.

3. Verfahren gemäß Anspruch 2, wobei der Primer eine Poly-T-Nukleotidsequenz, die sich an einen mRNA-Poly(A)-Schwanz anlagert, beinhaltet.

4. Verfahren gemäß Anspruch 3, wobei das Verarbeiten ferner das In-Kontakt-Bringen von Untermengen mit einem zweiten Primer beinhaltet, wobei der zweite Primer eine Sequenz beinhaltet, die sich an eine vorbestimmte DNA-Nukleinsäure anlagert; und wobei der zweite Primer optional einen abteilspezifischen Index beinhaltet.

5. Verfahren gemäß Anspruch 2, wobei der Primer eine Sequenz beinhaltet, die sich an eine vorbestimmte RNA-Nukleinsäure anlagert, wobei das Verfahren Primer in verschiedenen Abteilen beinhaltet, die sich an verschiedene Nukleotide der gleichen vorbestimmten RNA-Nukleinsäure anlagern.

6. Verfahren gemäß Anspruch 1, wobei das Verarbeiten zum Hinzugeben der ersten abteilspezifischen Indexsequenz den folgenden zweistufigen Prozess beinhaltet:
Hinzugeben einer Nukleotidsequenz, die eine Universalsequenz beinhaltet, zu den RNA-Nukleinsäuren, um Nukleinsäuren zu ergeben, die die Universalsequenz beinhalten, und dann Hinzugeben der ersten abteilspezifischen Indexsequenz zu den Nukleinsäuren, die die Universalsequenz beinhalten.

7. Verfahren gemäß Anspruch 1, wobei vorbestehende RNA-Nukleinsäuren und neu synthetisierte RNA-Nukleinsäuren in dem gleichen Abteil mit dem gleichen Index markiert werden.

8. Verfahren gemäß Anspruch 1, wobei das Markieren das Inkubieren der Vielzahl von Zellkernen oder Zellen in einer Zusammensetzung beinhaltet, die eine Nukleotidmarkierung beinhaltet, wobei die Nukleotidmarkierung in die neu synthetisierte RNA eingebaut wird.

9. Verfahren gemäß Anspruch 1, das ferner vor dem Markieren das Exponieren von Untermengen von Zellkernen oder Zellen gegenüber einer vorbestimmten Bedingung beinhaltet, wobei die vorbestimmte Bedingung bevorzugt das Exponieren gegenüber einem Mittel beinhaltet, das ein Protein, ein nicht-ribosomales Protein, ein Polyketid, ein organisches Molekül, ein anorganisches Molekül, ein RNA- oder RNAi-Molekül, ein Kohlenhydrat, ein Glykoprotein, eine Nukleinsäure oder eine Kombination davon beinhaltet; wobei das Mittel am bevorzugtesten einen therapeutischen Wirkstoff beinhaltet.

10. Verfahren gemäß Anspruch 9, wobei die vorbestimmte Bedingung von zwei oder mehr Abteilen verschieden ist, wenn das Verfahren vor dem Markieren das Exponieren von Untermengen von Zellkernen oder Zellen gegenüber einer vorbestimmten Bedingung beinhaltet.

11. Verfahren gemäß Anspruch 1, das ferner Folgendes beinhaltet:
Verteilen von Untermengen der gepoolten indizierten Zellkerne oder Zellen auf eine zweite Vielzahl von Abteilen und Hinzugeben einer zweiten Indexsequenz zu indizierten Nukleinsäuren, die in Untermengen von Zellkernen oder Zellen vorhanden sind, um doppelt indizierte Zellkerne oder Zellen zu erzeugen, die doppelt indizierte Nukleinsäurefragmente beinhalten, wobei das Hinzugeben Ligation, Primerverlängerung, Hybridisierung, Amplifikation oder Transposition beinhaltet; Kombinieren der doppelt indizierten Zellkerne oder Zellen, um gepoolte doppelt indizierte Zellkerne oder Zellen zu erzeugen.

12. Verfahren gemäß Anspruch 11, das ferner Folgendes beinhaltet:

Verteilen von Untermengen der gepoolten doppelt indizierten Zellkerne oder Zellen auf eine dritte Vielzahl von Abteilen und Hinzugeben einer dritten Indexsequenz zu indizierten Nukleinsäuren, die in Untermengen von Zellkernen oder Zellen vorhanden sind, um dreifach indizierte Zellkerne oder Zellen zu erzeugen, die dreifach indizierte Nukleinsäurefragmente beinhalten, wobei das Hinzugeben Ligation, Hybridisierung, Primerverlängerung, Amplifikation oder Transposition beinhaltet;
Kombinieren der dreifach indizierten Zellkerne oder Zellen, um gepoolte dreifach indizierte Zellkerne oder Zellen zu erzeugen.

13. Verfahren gemäß Anspruch 1, wobei das Hinzugeben die Ligation der ersten abteilspezifischen Indexsequenz beinhaltet, wobei es ferner das Hinzugeben einer zweiten Indexsequenz beinhaltet, um doppelt indizierte Zellkerne oder Zellen zu erzeugen, die doppelt indizierte Nukleinsäurefragmente beinhalten, wobei das Hinzugeben Transposition beinhaltet.

14. Verfahren gemäß Anspruch 11, wobei das Hinzugeben Ligation der zweiten abteilspezifischen Indexsequenz beinhaltet, wobei es ferner das Hinzugeben einer dritten Indexsequenz beinhaltet, um doppelt indizierte Zellkerne oder Zellen zu erzeugen, die dreifach indizierte Nukleinsäurefragmente beinhalten, wobei das Hinzugeben Transposition beinhaltet.

15. Verfahren gemäß Anspruch 1, das ferner das Erhalten der indizierten Nukleinsäuren aus den gepoolten indizierten Zellkernen oder Zellen beinhaltet, wodurch eine Sequenzierungsbibliothek aus der Vielzahl von Zellkernen oder Zellen produziert wird.

16. Verfahren gemäß Anspruch 11, das ferner das Erhalten der doppelt indizierten Nukleinsäuren aus den gepoolten doppelt indizierten Zellkernen oder Zellen beinhaltet, wodurch eine Sequenzierungsbibliothek aus der Vielzahl von Zellkernen oder Zellen produziert wird.

17. Verfahren gemäß Anspruch 12, das ferner das Erhalten der dreifach indizierten Nukleinsäuren aus den gepoolten dreifach indizierten Zellkernen oder Zellen beinhaltet, wodurch eine Sequenzierungsbibliothek aus der Vielzahl von Zellkernen oder Zellen produziert wird.

18. Verfahren gemäß Anspruch 2, wobei der Primer eine Sequenz beinhaltet, die sich an eine vorbestimmte RNA-Nukleinsäure anlagert, und wobei die vorbestimmte RNA-Nukleinsäure bevorzugt eine mRNA ist.

19. Verfahren gemäß Anspruch 2, wobei der Primer einen Matrizenwechselprimer beinhaltet.

20. Verfahren gemäß Anspruch 8, wobei die Nukleotidmarkierung ein Nukleotidanalogon, ein haptenmarkiertes Nukleotid, ein mutagenes Nukleotid oder ein Nukleotid, das durch eine chemische Reaktion modifiziert werden kann, beinhaltet.

21. Verfahren gemäß Anspruch 8, wobei mehr als eine Nukleotidmarkierung in die neu synthetisierte RNA eingebaut

70

wird und das Verhältnis der Nukleotidmarkierung oder -markierungen bevorzugt für verschiedene Abteile oder Zeitpunkte verschieden ist.

**22.** Verfahren gemäß Anspruch 9, wobei das Exponieren und das Markieren gleichzeitig erfolgen oder das Exponieren vor dem Markieren erfolgt.

**Revendications**

**1.** Un procédé pour préparer une banque de séquençage comprenant des acides nucléiques provenant d'une pluralité de noyaux ou de cellules uniques, le procédé comprenant :

(a) la mise à disposition d'une pluralité de noyaux ou de cellules dans une première pluralité de compartiments, dans lequel chaque compartiment comprend un sous-ensemble de noyaux ou de cellules ;
(b) le marquage de l'ARN nouvellement synthétisé dans les sous-ensembles de cellules ou de noyaux obtenus à partir des cellules ;
(c) le traitement de molécules d'ARN dans chaque sous-ensemble de noyaux ou de cellules afin de générer des noyaux ou des cellules indexés,

dans lequel le traitement comprend l'ajout à des acides nucléiques ARN présents dans chaque sous-ensemble de noyaux ou de cellules d'une première séquence d'index spécifique de compartiment afin de donner lieu à des acides nucléiques indexés présents dans des noyaux ou des cellules indexés, dans lequel le traitement comprend une ligature, une extension d'amorce, une hybridation, ou une amplification ; et

(d) la combinaison des noyaux ou des cellules indexés afin de générer des noyaux ou des cellules indexés regroupés.

**2.** Le procédé de la revendication 1, dans lequel le traitement comprend :
la mise en contact de sous-ensembles avec une transcriptase inverse et une amorce qui s'annèle à des acides nucléiques ARN, donnant lieu à des acides nucléiques double brin comprenant l'amorce et la séquence nucléotidique d'ADN correspondante des molécules d'ARN.

**3.** Le procédé de la revendication 2, dans lequel l'amorce comprend une séquence nucléotidique poly-T qui s'annèle à une queue poly(A) d'ARNm.

**4.** Le procédé de la revendication 3, dans lequel le traitement comprend en outre la mise en contact de sous-ensembles avec une deuxième amorce, dans lequel la deuxième amorce comprend une séquence qui s'annèle à un acide nucléique ADN prédéterminé ; et facultativement, la deuxième amorce comprend un index spécifique de compartiment.

**5.** Le procédé de la revendication 2, dans lequel l'amorce comprend une séquence qui s'annèle à un acide nucléique ARN prédéterminé, le procédé comprenant des amorces dans différents compartiments qui s'annèlent à différents nucléotides du même acide nucléique ARN prédéterminé.

**6.** Le procédé de la revendication 1, dans lequel le traitement visant à ajouter la première séquence d'index spécifique de compartiment comprend un processus en deux étapes d'ajout d'une séquence nucléotidique comprenant une séquence universelle aux acides nucléiques ARN afin de donner lieu à des acides nucléiques comprenant la séquence universelle, puis d'ajout de la première séquence d'index spécifique de compartiment aux acides nucléiques comprenant la séquence universelle.

**7.** Le procédé de la revendication 1, où les acides nucléiques ARN préexistants et les acides nucléiques ARN nouvellement synthétisés sont marqués avec le même index dans le même compartiment.

**8.** Le procédé de la revendication 1, dans lequel le marquage comprend l'incubation de la pluralité de noyaux ou de cellules dans une composition comprenant un marqueur nucléotidique, dans lequel le marqueur nucléotidique est incorporé dans l'ARN nouvellement synthétisé.

9. Le procédé de la revendication 1, comprenant en outre l'exposition de sous-ensembles de noyaux ou de cellules à une condition prédéterminée avant le marquage, dans lequel de préférence la condition prédéterminée comprend une exposition à un agent comprenant une protéine, une protéine non ribosomique, un polycétide, une molécule organique, une molécule inorganique, une molécule d'ARN ou d'ARNi, un glucide, une glycoprotéine, un acide nucléique, ou une combinaison de ceux-ci ; mais de préférence encore l'agent comprend un médicament thérapeutique.

10. Le procédé de la revendication 9, dans lequel la condition prédéterminée de deux compartiments ou plus est différente lorsque le procédé comprend l'exposition de sous-ensembles de noyaux ou de cellules à une condition prédéterminée avant le marquage.

11. Le procédé de la revendication 1, comprenant en outre :

    la distribution de sous-ensembles de noyaux ou de cellules indexés regroupés dans une deuxième pluralité de compartiments et l'ajout à des acides nucléiques indexés présents dans des sous-ensembles de noyaux ou de cellules d'une deuxième séquence d'index afin de générer des noyaux ou des cellules doublement indexés comprenant des fragments d'acide nucléique doublement indexés, dans lequel l'ajout comprend une ligature, une extension d'amorce, une hybridation, une amplification, ou une transposition ;
    la combinaison des noyaux ou des cellules doublement indexés afin de générer des noyaux ou des cellules doublement indexés regroupés.

12. Le procédé de la revendication 11, comprenant en outre :

    la distribution de sous-ensembles des noyaux ou des cellules doublement indexés regroupés dans une troisième pluralité de compartiments et l'ajout à des acides nucléiques indexés présents dans des sous-ensembles de noyaux ou de cellules d'une troisième séquence d'index afin de générer des noyaux ou des cellules triplement indexés comprenant des fragments d'acide nucléique triplement indexés, dans lequel l'ajout comprend une ligature, une hybridation, une extension d'amorce, une amplification ou une transposition ;
    la combinaison des noyaux ou des cellules triplement indexés afin de générer des noyaux ou des cellules triplement indexés regroupés.

13. Le procédé de la revendication 1, dans lequel l'ajout comprend une ligature de la première séquence d'index spécifique de compartiment, comprenant en outre l'ajout d'une deuxième séquence d'index afin de générer des noyaux ou des cellules doublement indexés comprenant des fragments d'acide nucléique doublement indexés, dans lequel l'ajout comprend une transposition.

14. Le procédé de la revendication 11, dans lequel l'ajout comprend une ligature de la deuxième séquence d'index spécifique de compartiment, comprenant en outre l'ajout d'une troisième séquence d'index afin de générer des noyaux ou des cellules doublement indexés comprenant des fragments d'acide nucléique triplement indexés, dans lequel l'ajout comprend une transposition.

15. Le procédé de la revendication 1, comprenant en outre l'obtention des acides nucléiques indexés à partir des noyaux ou des cellules indexés regroupés, produisant ainsi une banque de séquençage à partir de la pluralité de noyaux ou de cellules.

16. Le procédé de la revendication 11, comprenant en outre l'obtention des acides nucléiques doublement indexés à partir des noyaux ou des cellules doublement indexés regroupés, produisant ainsi une banque de séquençage à partir de la pluralité de noyaux ou de cellules.

17. Le procédé de la revendication 12, comprenant en outre l'obtention des acides nucléiques triplement indexés à partir des noyaux ou des cellules triplement indexés regroupés, produisant ainsi une banque de séquençage à partir de la pluralité de noyaux ou de cellules.

18. Le procédé de la revendication 2, dans lequel l'amorce comprend une séquence qui s'annèle à un acide nucléique ARN prédéterminé, et de préférence dans lequel l'acide nucléique ARN prédéterminé est un ARNm.

19. Le procédé de la revendication 2, dans lequel l'amorce comprend une amorce de commutation de matrice (*template-switch primer*).

**20.** Le procédé de la revendication 8, dans lequel le marqueur nucléotidique comprend un analogue nucléotidique, un nucléotide marqué par un haptène, un nucléotide mutagène, ou un nucléotide qui peut être modifié par une réaction chimique.

**21.** Le procédé de la revendication 8, dans lequel plus d'un marqueur nucléotidique est incorporé dans l'ARN nouvellement synthétisé, et de préférence le rapport du marqueur ou des marqueurs nucléotidiques est différent pour différents compartiments ou différents points temporels.

**22.** Le procédé de la revendication 9, dans lequel l'exposition et le marquage se produisent en même temps ou l'exposition se produit avant le marquage.

FIG. 1

10      | Provide cells |

11      | Distribute subsets of cells |

12      | Expose cells to a predetermined condition |

13      | Label newly synthesized RNA |

14      | Process cellular RNA |

15      | Index cells |

FIG. 2

```
22        ┌─────────────────────────────────┐
          │      Provide isolated nuclei     │
          └─────────────────────────────────┘
                           │
                           ▼
23        ┌─────────────────────────────────┐
          │      Distribute subsets of nuclei │
          └─────────────────────────────────┘
                           │
                           ▼
24        ┌─────────────────────────────────┐
          │  Index nuclei by reverse transcription │
          └─────────────────────────────────┘
                           │
                           ▼
25        ┌─────────────────────────────────┐
          │   Pool and distribute indexed nuclei │
          └─────────────────────────────────┘
                           │
                           ▼
26        ┌─────────────────────────────────┐
          │      Index nuclei by ligation     │
          └─────────────────────────────────┘
                           │
                           ▼
27        ┌─────────────────────────────────┐
          │  Pool and distribute dual-indexed │
          │              nuclei               │
          └─────────────────────────────────┘
                           │
                           ▼
28        ┌─────────────────────────────────┐
          │      Second strand synthesis      │
          └─────────────────────────────────┘
                           │
                           ▼
29        ┌─────────────────────────────────┐
          │           Tagmentation            │
          └─────────────────────────────────┘
                           │
                           ▼
30        ┌─────────────────────────────────┐
          │   Index nuclei by amplification   │
          └─────────────────────────────────┘
                           │
                           ▼
31        ┌─────────────────────────────────┐
          │   Immobilize library and sequence │
          └─────────────────────────────────┘
```

FIG. 3

30 | Provide isolated nuclei or cells |

31 | Enrich predetermined RNA molecules by reverse transcription |

32 | Distribute subsets of nuclei or cells |

33 | Index nuclei by ligation |

34 | Pool and distribute indexed nuclei |

35 | Second strand synthesis |

36 | Tagmentation |

37 | Index nuclei by amplification |

38 | Immobilize library and sequence |

FIG. 4

FIG. 5

FIG. 6

79

FIG. 7

FIG. 8

A

B

C

FIG. 9

FIG. 10

FIG. 11

FIG. 12

FIG. 13

FIG. 14

FIG. 15

FIG. 16

FIG. 17

FIG. 18

FIG. 19

FIG. 20

FIG. 21

**A**

Pitx1

Tbx5

**B**

Sox6

Sox9

Proximal
markers

Hoxd13

Tfap2b

Distal
markers

Pax9

Alx4

Anterior
markers

Shh

Hand2

Posterior
markers

FIG. 22

Module 1 (281 genes)    Module 2 (78 genes)    Module 3 (122 genes)    Module 4 (81 genes)

Module 5 (94 genes)    Module 6 (107 genes)    Module 7 (131 genes)    Module 8 (127 genes)

FIG. 23

FIG. 24

FIG. 25

Epithelial trajectory

Neural tube/notochord trajectory

Hematopoiesis trajectory

Mesenchymal trajectory

Neural crest 1

Hepatic trajectory

Endothelial trajectory

Neural crest 2

1–Connective tissue progenitors
2–Chondrocytes
3–Intermediate Mesoderm
4–Jaw and tooth progenitors
5–Mesencephalon neurons
6–Epithelial cells
7–Radial glia
8–Early mesenchyme
9–Neural progenitor cells
10–Postmitotic neurons
11–Oligodendrocyte Progenitors
12–Midbrain/forebrain progenitors
13–Myocytes
14–Neural Tube
15–Glutamatergic neurons
16–Stromal cells
17–Osteoblasts
18–Hippocampus neurons
19–Premature oligodendrocytes
20–Endothelial cells
21–Chondroctyc progenitors
22–Definitive erythrocyte lineage
23–Schwann cell precursors
24–Sensory neurons
25–Limb mesenchyme
26–Primitive erythroid lineage
27–Basal forebrain neurons
28–Pyramidal neuron cells
29–Hapatocytes
30–Notochord cells
31–Lymphocytes
32–Ependymal cells
33–Cholinergic neurons
34–Cardiac muscle lineage
35–Megakaryocytes
36–Melanocytes
37–Lens
38–Monocytes/granulocytes

FIG. 26

Epithelial trajectory

Neural tube/notochord trajectory

- E9.5
- E10.5
- E11.5
- E12.5
- E13.5

Hematopoiesis trajectory

Mesenchymal trajectory

Neural crest 1

Hepatic trajectory

Endothelial trajectory

Neural crest 2

FIG. 27

1–Squamous epithelium
2–Airway epithelium
3–Fetal skin
4–Epidermal Stem Cells
5–Fetal thymic epithelial cells
6–Mammary epithelial cells
7–Hair follicles
8–Hair follicle stem cells
9–Otic vesicle epithelium
10–Otic sensory epithelium
11–Gastric antral epithelial cells
12–Intestinal epithelium
13–Renal epithelial cells 1
14–Spermatogenic epithelia
15–Kidney epithelial progenitor cells

16–Renal principal cells
17–Renal epithelial cells 2
18–Enterocyte progenitors
19–Lung epithelial cells
20–Mammary epithelium
21–Lung epithelium
22–Proximal tubular epithelia
23–Genital tubercle vascular endothelia
24–Hepatic epithelium
25–Apical ectodermal ridge
26–Mammary gland epithelial cells
27–Intestinal epithelial
28–Bronchiolar Epithelium
29–Sertoli cells

FIG. 28

101

FIG. 29

FIG. 30

FIG. 31

FIG. 32

FIG. 33

FIG. 34

FIG. 35

FIG. 36

FIG. 37

FIG. 38

FIG. 39

FIG. 40

| Chr | Start | End | Gene | TotalCounts | ENCODE | Rank | ENCODE |
|---|---|---|---|---|---|---|---|
| chrM | 1672 | 3230 | RNR2 | 4566 | 653212 | 1 | 1 |
| chr11 | 34073229 | 34124157 | CAPRIN1 | 2061 | 2344 | 2 | 451 |
| chr11 | 33880122 | 33913836 | LMO2 | 1983 | 376 | 3 | 4627 |
| chrM | 649 | 1603 | RNR1 | 1017 | 156276 | 4 | 4 |
| chr11 | 34127110 | 34168458 | NAT10 | 641 | 1075 | 5 | 1489 |
| chr11 | 33724555 | 33758025 | CD59 | 420 | 2503 | 6 | 396 |
| chr11 | 34460471 | 34493607 | CAT | 395 | 263 | 7 | 5919 |
| chr11 | 65265232 | 65273983 | MALAT1 | 291 | 443474 | 8 | 2 |
| chr11 | 33278217 | 33378568 | HIPK3 | 238 | 505 | 9 | 3550 |
| chr22 | 21061978 | 21213100 | PI4KA | 92 | 4667 | 10 | 120 |
| chr11 | 34937676 | 35017675 | PDHX | 90 | 146 | 11 | 7928 |
| chr9 | 37779710 | 37785089 | EXOSC3 | 72 | 356 | 12 | 4827 |

FIG. 41

FIG. 42

## REFERENCES CITED IN THE DESCRIPTION

### Patent documents cited in the description

- US 2003082584 A1
- WO 2016130704 A, Gunderson
- WO 2007010251 A
- WO 2006064199 A
- WO 2005065814 A
- WO 2015106941 A
- WO 1998044151 A
- WO 2000018957 A
- US 4683195 A
- US 4683202 A
- WO 04018497 A
- US 7057026 B
- WO 9106678 A
- WO 07123744 A
- US 7329492 B
- US 7211414 B
- US 7315019 B
- US 7405281 B
- US 20080108082 A
- WO 9523875 A
- US 20120208705
- US 20120208724
- WO 2012061832 A
- US 20100120098
- US 7741463 B, Gormley
- US 8053192 B, Bignell
- US 8895249 B, Shen
- US 9309502 B, Pipenburg
- US 8778848 B
- US 8778849 B
- US 9079148 B
- US 20140243224 A
- US 2013184796 A
- WO 2016066586 A
- WO 2015002813 A
- US 8563477 B
- WO 05065814 A
- US 20050100900 A
- US 7115400 B
- WO 0018957 A
- WO 9844151 A
- WO 0246456 A
- US 20080009420 A
- US 8003354 B
- US 7582420 B
- US 5185243 A
- US 5679524 A
- US 5573907 A

- EP 0320308 B1
- EP 0336731 B1
- EP 0439182 B1
- WO 9001069 A
- WO 8912696 A
- WO 8909835 A
- US 7611869 B
- US 7910354 B
- US 20090264299 A
- US 20090011943 A
- US 20090005252 A
- US 20090155781 A
- US 20090118488 A
- US 20070099208 A1
- US 6214587 B
- US 5455166 A
- US 5130238 A
- US 7670810 B
- US 20130338042
- US 9169513 B
- US 7399590 B
- US 5223414 A
- US 7829284 B
- US 6210891 B
- US 6258568 B
- US 6274320 B
- US 7427673 B
- US 20070166705 A
- US 20060188901 A
- US 20060240439 A
- US 20060281109 A
- US 20120270305 A
- US 20130260372 A
- US 20050100900
- WO 06064199 A
- WO 07010251 A
- US 20130079232 A
- US 6969488 B
- US 6172218 B
- US 6306597 B
- US 7001792 B
- US 20090026082 A
- US 20090127589 A
- US 20100137143 A
- US 20100282617 A
- US 20100111768 A
- US 273666

**Non-patent literature cited in the description**

- **RAPOLAS ZILLIONIS et al.** Single-cell barcoding and sequencing using droplet microfluidics. *NATURE PROTOCOLS,* 2016, vol. 12 (1), 44-73
- **SUBRAMANIAN et al.** *Cell,* 2017, vol. 171, 1437-1452
- **BENTLEY et al.** *Nature,* 2008, vol. 456, 53-59
- **SHAHI et al.** *Scientific Reports,* 2017, vol. 7 (44447
- **STOECKIUS et al.** *Nature Methods,* 2017, vol. 14, 865-868
- **ISLAM et al.** *Micromachines (Basel),* 2017, vol. 8 (3), 83, www.sigmaaldrich.com/life-science/biochemicals/biochemical-products.html?TablePage=14573107
- **SZLACHTA et al.** *Nat Commun.,* 2018, vol. 9, 4275
- mRNA Decay. Methods in Molecular Biology. Humana Press, vol. 1720
- **LUO et al.** *Nucl. Acids Res.,* 2011, vol. 39 (19), 8559-8571
- **BHARMAL et al.** *J Biomol Tech.,* 2010, vol. 21 (3), S43
- **VINCENT et al.** *EMBO Rep.,* 2004, vol. 5 (8), 795-800
- **GORY SHIN ; REZNIKOFF.** *J. Biol. Chem.,* 1998, vol. 273, 7367
- **MIZUUCHI, K.** *Cell,* 1983, vol. 35, 785
- **SAVILAHTI, H et al.** *EMBO J.,* 1995, vol. 14, 4893
- **COLEGIO et al.** *J. Bacteriol.,* 2001, vol. 183, 2384-8
- **KIRBY C et al.** *Mol. Microbiol.,* 2002, vol. 43, 173-86
- **DEVINE ; BOEKE.** *Nucleic Acids Res.,* 1994, vol. 22, 3765-72
- **CRAIG.** *Science.,* 1996, vol. 271, 1512
- *Curr Top Microbiol Immunol.,* 1996, vol. 204, 27-48
- **KLECKNER N et al.** *Curr Top Microbiol Immunol.,* 1996, vol. 204, 49-82
- **LAMPE D J et al.** *EMBO J.,* 1996, vol. 15, 5470-9
- **PLASTERK R H.** *Curr. Topics Microbiol. Immunol.,* 1996, vol. 204, 125-43
- **GLOOR, GB.** *Methods Mol. Biol.,* 2004, vol. 260, 97-114
- **ICHIKAWA ; OHTSUBO.** *J Biol. Chem.,* 1990, vol. 265, 18829-32
- **OHTSUBO ; SEKINE.** *Curr. Top. Microbiol. Immunol.,* 1996, vol. 204, 1-26
- **BROWN et al.** *Proc Natl Acad Sci USA,* 1989, vol. 86, 2525-9
- **BOEKE ; CORCES.** *Annu Rev Microbiol.,* 1989, vol. 43, 403-34
- **ZHANG et al.** *PLoS Genet.,* 16 October 2009, vol. 5, e1000689

- **WILSON C. et al.** *J. Microbiol. Methods,* 2007, vol. 71, 332-5
- **ADEY et al.** *Genome Res.,* 2014, vol. 24, 2041-2049
- **ARMOUR et al.** *Nature Methods,* 2009, vol. 6 (9), 647-49
- **LIZARDI et al.** *Nat. Genet.,* 1998, vol. 19, 225-232
- **DRMANAC et al.** *Science,* 2010, vol. 327 (5961), 78-81
- **DEAN et al.** *Proc. Natl. Acad. Sci. USA,* 2002, vol. 99, 5261-66
- **WALKER et al.** Molecular Methods for Virus Detection. Academic Press, Inc, 1995
- **WALKER et al.** *Nucl. Acids Res.,* 1992, vol. 20, 1691-96
- **LAGE et al.** *Genome Res.,* 2003, vol. 13, 294-307
- **GROTHUES et al.** *Nucleic Acids Res.,* 1993, vol. 21 (5), 1321-2
- **RONAGHI, M. ; KARAMOHAMED, S. ; PETTERSSON ; B., UHLEN ; M. AND NYREN, P.** Real-time DNA sequencing using detection of pyrophosphate release. *Analytical Biochemistry,* 1996, vol. 242 (1), 84-9
- **RONAGHI, M.** Pyrosequencing sheds light on DNA sequencing. *Genome Res.,* 2001, vol. 11 (1), 3-11
- **RONAGHI, M. ; UHLEN, M. ; NYREN, P.** A sequencing method based on real-time pyrophosphate. *Science,* 1998, vol. 281 (5375), 363
- **METZKER.** *Genome Res.,* 2005, vol. 15, 1767-1776
- **RUPARE et al.** *Proc Natl Acad Sci USA,* 2005, vol. 102, 5932-7
- **DEAMER, D. W. ; AKESON, M.** Nanopores and nucleic acids: prospects for ultrarapid sequencing. *Trends Biotechnol.,* 2000, vol. 18, 147-151
- **DEAMER, D. ; D. BRANTON.** Characterization of nucleic acids by nanopore analysis. *Acc. Chem. Res.,* 2002, vol. 35, 817-825
- **LI, J. ; M. GERSHOW ; D. STEIN ; E. BRANDIN ; J. A. GOLOVCHENKO.** DNA molecules and configurations in a solid-state nanopore microscope. *Nat. Mater.,* 2003, vol. 2, 611-615
- **SONI, G. V. ; MELLER.** A. Progress toward ultrafast DNA sequencing using solid-state nanopores. *Clin. Chem.,* 2007, vol. 53, 1996-2001
- **HEALY, K.** Nanopore-based single-molecule DNA analysis. *Nanomed.,* 2007, vol. 2, 459-481
- **COCKROFT, S. L. ; CHU, J. ; AMORIN, M. ; GHADIRI, M. R.** A single-molecule nanopore device detects DNA polymerase activity with single-nucleotide resolution. *J. Am. Chem. Soc.,* 2008, vol. 130, 818-820

- **LEVENE, M. J. et al.** Zero-mode waveguides for single-molecule analysis at high concentrations. *Science,* 2003, vol. 299, 682-686
- **LUNDQUIST, P. M. et al.** Parallel confocal detection of single molecules in real time. *Opt. Lett.,* 2008, vol. 33, 1026-1028
- **KORLACH, J. et al.** Selective aluminum passivation for targeted immobilization of single DNA polymerase molecules in zero-mode waveguide nano structures. *Proc. Natl. Acad. Sci. USA,* 2008, vol. 105, 1176-1181
- **FOGARTY, N. M. E. et al.** Genome editing reveals a role for OCT4 in human embryogenesis. *Nature,* 2017, vol. 550, 67-73
- **KOJIMA, Y. ; TAM, O. H. ; TAM, P. P. L.** Timing of developmental events in the early mouse embryo. *Semin. Cell Dev. Biol.,* 2014, vol. 34, 65-75
- **TAM, P. P. L. ; LOEBEL, D. A. F.** Gene function in mouse embryogenesis: get set for gastrulation. *Nat. Rev. Genet.,* 2007, vol. 8, 368-381
- **RIVERA-PÉREZ, J. A. ; HADJANTONAKIS, A.-K.** The Dynamics of Morphogenesis in the Early Mouse Embryo. *Cold Spring Harb. Perspect. Biol.,* 2014, vol. 7, a015867
- **DICKINSON, M. E. et al.** High-throughput discovery of novel developmental phenotypes. *Nature,* 2016, vol. 537, 508-514
- **MEEHAN, T. F. et al.** Disease model discovery from 3,328 gene knockouts by The International Mouse Phenotyping Consortium. *Nat. Genet.,* 2017, vol. 49, 1231-1238
- **SHYER, A. E. ; HUYCKE, T. R. ; LEE, C. ; MAHADEVAN, L. ; TABIN, C. J.** Bending gradients: how the intestinal stem cell gets its home. *Cell,* 2015, vol. 161, 569-580
- **UYGUR, A. et al.** Scaling Pattern to Variations in Size during Development of the Vertebrate Neural Tube. *Dev. Cell,* 2016, vol. 37, 127-135
- **GORKIN, D. et al.** *Systematic mapping of chromatin state landscapes during mouse development.,* 2017
- **MAYER, C. et al.** Developmental diversification of cortical inhibitory interneurons. *Nature,* 2018, vol. 555, 457-462
- **LESCROART, F. et al.** Defining the earliest step of cardiovascular lineage segregation by single-cell RNA-seq. *Science,* 2018
- **HAN, X. et al.** Mapping the Mouse Cell Atlas by Microwell-Seq. *Cell,* 2018, vol. 172, 1091-1107.e17
- **QUAKE, S. R. ; WYSS-CORAY, T. ; DARMANIS, S.** Transcriptomic characterization of 20 organs and tissues from mouse at single cell resolution creates a Tabula Muris. *The Tabula Muris Consortium,* 2017
- **AMINI, S. et al.** Haplotype-resolved whole-genome sequencing by contiguity-preserving transposition and combinatorial indexing. *Nat. Genet.,* 2014, vol. 46, 1343-1349
- **ADEY, A. et al.** In vitro, long-range sequence information for de novo genome assembly via transposase contiguity. *Genome Res.,* 2014, vol. 24, 2041-2049
- **CUSANOVICH, D. A. et al.** Multiplex single cell profiling of chromatin accessibility by combinatorial cellular indexing. *Science,* 2015, vol. 348, 910-914
- **VITAK, S. A. et al.** Sequencing thousands of single-cell genomes with combinatorial indexing. *Nat. Methods,* 2017, vol. 14, 302-308
- **RAMANI, V. et al.** Massively multiplex single-cell Hi-C. *Nat. Methods,* 2017, vol. 14, 263-266
- **CAO, J. et al.** Comprehensive single-cell transcriptional profiling of a multicellular organism. *Science,* 2017, vol. 357, 661-667
- **MULQUEEN, R. M. et al.** *Scalable and efficient single-cell DNA methylation sequencing by combinatorial indexing.,* 2017
- **ROSENBERG, A. B. et al.** Single-cell profiling of the developing mouse brain and spinal cord with split-pool barcoding. *Science,* 2018
- **ZHENG, G. X. Y. et al.** Massively parallel digital transcriptional profiling of single cells. *Nat. Commun.,* 2017, vol. 8, 14049
- **QIU, X. et al.** *Reversed graph embedding resolves complex single-cell developmental trajectories,* 2017
- **FERNANDEZ, T. et al.** Disruption of contactin 4 (CNTN4) results in developmental delay and other features of 3p deletion syndrome. *Am. J. Hum. Genet.,* 2004, vol. 74, 1286-1293
- **OLSON, J. M. et al.** NeuroD2 is necessary for development and survival of central nervous system neurons. *Dev. Biol.,* 2001, vol. 234, 174-187
- **UITTENBOGAARD, M. ; BAXTER, K. K.** Chiaramello, A. NeuroD6 Genomic Signature Bridging Neuronal Differentiation to Survival via the Molecular Chaperone Network. *J. Neurosci. Res.,* 2010, vol. 88, 33
- **YANG, A. et al.** p63 is essential for regenerative proliferation in limb, craniofacial and epithelial development. *Nature,* 1999, vol. 398, 714-718
- **MCQUALTER, J. L. ; YUEN, K. ; WILLIAMS, B. ; BERTONCELLO, I.** Evidence of an epithelial stem/progenitor cell hierarchy in the adult mouse lung. *Proc. Natl. Acad. Sci. U. S. A.,* 2010, vol. 107, 1414-1419
- **TOMIHARI, M. ; HWANG, S.-H. ; CHUNG, J.-S. ; CRUZ, P. D. ; JR. & ARIIZUMI ; K. GPNMB.** is a melanosome-associated glycoprotein that contributes to melanocyte/keratinocyte adhesion in a RGD-dependent fashion. *Exp. Dermatol.,* 2009, vol. 18, 586-595

- **VARJOSALO, M. ; TAIPALE, J.** Hedgehog: functions and mechanisms. *Genes Dev.,* 2008, vol. 22, 2454-2472
- **STRÄHLE, U. ; LAM, C. S. ; ERTZER, R. ; RASTEGAR, S.** Vertebrate floor-plate specification: variations on common themes. *Trends Genet.,* 2004, vol. 20, 155-162
- **HOLMES, G. P. et al.** Distinct but overlapping expression patterns of two vertebrate slit homologs implies functional roles in CNS development and organogenesis. *Mech. Dev.,* 1998, vol. 79, 57-72
- **AKLE, V. et al.** F-spondin/spon1b expression patterns in developing and adult zebrafish. *PLoS One,* 2012, vol. 7, e37593
- **HARTMAN, B. H. ; DURRUTHY-DURRUTHY, R. ; LASKE, R. D. ; LOSORELLI, S. ; HELLER, S.** Identification and characterization of mouse otic sensory lineage genes. *Front. Cell. Neurosci.,* 2015, vol. 9, 79
- **PETIT, F. ; SEARS, K. E. ; AHITUV, N. LIMB.** development: a paradigm of gene regulation. *Nat. Rev. Genet.,* 2017, vol. 18, 245-258
- **GUO, Q. ; LOOMIS, C. ; JOYNER, A. L.** Fate map of mouse ventral limb ectoderm and the apical ectodermal ridge. *Dev. Biol.,* 2003, vol. 264, 166-178
- **LEWANDOSKI M, E.** Fgf8 signalling from the AER is essential for normal limb development. *NCBI,* 22 April 2018, https://www.ncbi.nlm.nih.gov/pubmed/11101846
- **AOKI M, E.** R-spondin2 expression in the apical ectodermal ridge is essential for outgrowth and patterning in mouse limb development. *NCBI,* 22 April 2018, https://www.ncbi.nlm.nih.gov/pubmed/18067586
- **GERDES, J. ; SCHWAB, U. ; LEMKE, H. ; STEIN, H.** Production of a mouse monoclonal antibody reactive with a human nuclear antigen associated with cell proliferation. *Int. J. Cancer,* 1983, vol. 31, 13-20
- **BERGMAN, D. ; HALJE, M. ; NORDIN, M. ; ENGSTRÖM, W.** Insulin-like growth factor 2 in development and disease: a mini-review. *Gerontology,* 2013, vol. 59, 240-249
- **TRAPNELL, C. et al.** The dynamics and regulators of cell fate decisions are revealed by pseudotemporal ordering of single cells. *Nat. Biotechnol.,* 2014, vol. 32, 381-386
- **MCINNES, L. ; HEALY, J.** *UMAP: Uniform Manifold Approximation and Projection for Dimension Reduction.,* 2018
- **ALEXANDER WOLF, F. et al.** Graph abstraction reconciles clustering with trajectory inference through a topology preserving map of single cells. *bioRxiv,* 2017, 208819
- **QIU, X. et al.** Reversed graph embedding resolves complex single-cell trajectories. *Nat. Methods,* 2017, vol. 14, 979-982
- **SINGH, M. K. et al.** The T-box transcription factor Tbx15 is required for skeletal development. *Mech. Dev.,* 2005, vol. 122, 131-144
- **PAINE-SAUNDERS, S. ; VIVIANO, B. L. ; ZUPICICH, J. ; SKARNES, W. C. ; SAUNDERS, S.** glypican-3 controls cellular responses to Bmp4 in limb patterning and skeletal development. *Dev. Biol.,* 2000, vol. 225, 179-187
- **HARA, K. ; IDE, H.** Msx1 expressing mesoderm is important for the apical ectodermal ridge (AER)-signal transfer in chick limb development. *Dev. Growth Differ.,* 1997, vol. 39, 705-714
- **LUPIÁÑEZ, D. G. et al.** Disruptions of Topological Chromatin Domains Cause Pathogenic Rewiring of Gene-Enhancer Interactions. *Cell,* 2015, vol. 161, 1012-1025
- **DAVIS, R. J. et al.** Dach1 mutant mice bear no gross abnormalities in eye, limb, and brain development and exhibit postnatal lethality. *Mol. Cell. Biol.,* 2001, vol. 21, 1484-1490
- **AKIYAMA, H. ; CHABOISSIER, M.-C. ; MARTIN, J. F. ; SCHEDL, A. ; DE CROMBRUGGHE, B.** The transcription factor Sox9 has essential roles in successive steps of the chondrocyte differentiation pathway and is required for expression of Sox5 and Sox6. *Genes Dev.,* 2002, vol. 16, 2813-2828
- **DENG, Y. et al.** Yap1 Regulates Multiple Steps of Chondrocyte Differentiation during Skeletal Development and Bone Repair. *Cell Rep.,* 2016, vol. 14, 2224-2237
- **JOSHI, S. et al.** TEAD transcription factors are required for normal primary myoblast differentiation in vitro and muscle regeneration in vivo. *PLoS Genet.,* 2017, vol. 13, e1006600
- **KNAPP, D. et al.** Comparative transcriptional profiling of the axolotl limb identifies a tripartite regeneration-specific gene program. *PLoS One,* 2013, vol. 8, e61352
- **ZELLER, R. ; LÓPEZ-RÍOS, J. ; ZUNIGA, A.** Vertebrate limb bud development: moving towards integrative analysis of organogenesis. *Nat. Rev. Genet.,* 2009, vol. 10, 845-858
- **NISHIMOTO, S. ; MINGUILLON, C. ; WOOD, S. ; LOGAN, M. P. O.** A combination of activation and repression by a colinear Hox code controls forelimb-restricted expression of Tbx5 and reveals Hox protein specificity. *PLoS Genet.,* 2014, vol. 10, e1004245
- **VARGESSON, N. ; LURIA, V. ; MESSINA, I. ; ERSKINE, L. ; LAUFER, E.** Expression patterns of Slit and Robo family members during vertebrate limb development. *Mech. Dev.,* 2001, vol. 106, 175-180
- **CHIMAL-MONROY, J. et al.** Analysis of the molecular cascade responsible for mesodermal limb chondrogenesis: Sox genes and BMP signaling. *Dev. Biol.,* 2003, vol. 257, 292-301

- **BRAUN, T. ; GAUTEL, M.** Transcriptional mechanisms regulating skeletal muscle differentiation, growth and homeostasis. *Nat. Rev. Mol. Cell Biol.,* 2011, vol. 12, 349-361
- **TAJBAKHSH, S. ; ROCANCOURT, D. ; COSSU, G. ; BUCKINGHAM, M.** Redefining the genetic hierarchies controlling skeletal myogenesis: Pax-3 and Myf-5 act upstream of MyoD. *Cell,* 1997, vol. 89, 127-138
- **HAREL, I. et al.** Distinct origins and genetic programs of head muscle satellite cells. *Dev. Cell,* 2009, vol. 16, 822-832
- **SAMBASIVAN, R. et al.** Distinct regulatory cascades govern extraocular and pharyngeal arch muscle progenitor cell fates. *Dev. Cell,* 2009, vol. 16, 810-821
- **HEIMBERG, G. ; BHATNAGAR, R. ; EL-SAMAD, H. ; THOMSON, M.** Low Dimensionality in Gene Expression Data Enables the Accurate Extraction of Transcriptional Programs from Shallow Sequencing. *Cell Syst,* 2016, vol. 2, 239-250
- **CUSANOVICH, D. A. et al.** *The cis-regulatory dynamics of embryonic development at single cell resolution.,* 2017
- **MCKENNA, A. et al.** Whole-organism lineage tracing by combinatorial and cumulative genome editing. *Science,* 2016, vol. 353, aaf7907
- **OSTERWALDER, M. et al.** Enhancer redundancy provides phenotypic robustness in mammalian development. *Nature,* 2018, vol. 554, 239-243
- **DICKEL, D. E. et al.** Ultraconserved Enhancers Are Required for Normal Development. *Cell,* 2018, vol. 172, 491-499.e15
- **LI, D. et al.** Formation of proximal and anterior limb skeleton requires early function of Irx3 and Irx5 and is negatively regulated by Shh signaling. *Dev. Cell,* 2014, vol. 29, 233-240
- **KRAFT, K. et al.** Deletions, Inversions, Duplications: Engineering of Structural Variants using CRISPR/Cas in Mice. *Cell Rep.,* 2015
- **BUENROSTRO, J. D. ; GIRESI, P. G. ; ZABA, L. C. ; CHANG, H. Y. ; GREENLEAF, W. J.** Transposition of native chromatin for fast and sensitive epigenomic profiling of open chromatin, DNA-binding proteins and nucleosome position. *Nat. Methods,* 2013, vol. 10, 1213-1218
- **RENAUD, G. ; STENZEL, U. ; MARICIC, T. ; WIEBE, V. ; KELSO, J.** deML: robust demultiplexing of Illumina sequences using a likelihood-based approach. *Bioinformatics,* 2015, vol. 31, 770-772
- **DOBIN, A. et al.** STAR: ultrafast universal RNA-seq aligner. *Bioinformatics,* 2013, vol. 29, 15-21
- **ANDERS, S. ; PYL, P. T. ; HUBER, W.** HTSeq--a Python framework to work with high-throughput sequencing data. *Bioinformatics btu638,* 2014
- **WOLF, F. A. ; ANGERER, P. ; THEIS, F. J.** SCAN-PY: large-scale single-cell gene expression data analysis. *Genome Biol.,* 2018, vol. 19, 15
- **CAO, J. et al.** Comprehensive single-cell transcriptional profiling of a multicellular organism. *Science,* 2017, vol. 357, 661-667
- **PLINER, H. et al.** *Chromatin accessibility dynamics of myogenesis at single cell resolution,* 2017
- **KULESHOV, M. V. et al.** Enrichr: a comprehensive gene set enrichment analysis web server 2016 update. *Nucleic Acids Res.,* 2016, vol. 44, W90-7
- **LEVINE, J. H. et al.** Data-Driven Phenotypic Dissection of AML Reveals Progenitor-like Cells that Correlate with Prognosis. *Cell,* 2015, vol. 162, 184-197
- **WOLF, F. A. et al.** *Graph abstraction reconciles clustering with trajectory inference through a topology preserving map of single cells.,* 2017
- **WAGNER, D. E. et al.** Single-cell mapping of gene expression landscapes and lineage in the zebrafish embryo. *Science,* 2018, eaar4362
- **BRIGGS, J. A. et al.** The dynamics of gene expression in vertebrate embryogenesis at single-cell resolution. *Science,* 2018, eaar5780
- **MAO, Q. ; WANG, L. ; TSANG, I. ; SUN, Y.** Principal Graph and Structure Learning Based on Reversed Graph Embedding. *IEEE Trans. Pattern Anal. Mach. Intell.,* 2016
- **MORAN, P. A. P.** Notes on continuous stochastic phenomena. *Biometrika,* 1950, vol. 37, 17-23
- **N. MORIS ; C. PINA ; A. M. ARIAS.** Transition states and cell fate decisions in epigenetic landscapes. *Nat. Rev. Genet.,* 2016, vol. 17, 693-703
- **A. FILIPCZYK et al.** Network plasticity of pluripotency transcription factors in embryonic stem cells. *Nat. Cell Biol.,* 2015, vol. 17, 1235-1246
- **S. HORMOZ et al.** Inferring Cell-State Transition Dynamics from Lineage Trees and Endpoint Single-Cell Measurements. *Cell Syst.,* 2016, vol. 3, 419-433.e8
- **V. A. HERZOG et al.** Thiol-linked alkylation of RNA to assess expression dynamics. *Nat. Methods.,* 2017, vol. 14, 1198-1204
- **J. A. SCHOFIELD ; E. E. DUFFY ; L. KIEFER ; M. C. SULLIVAN ; M. D. SIMON.** TimeLapse-seq: adding a temporal dimension to RNA sequencing through nucleoside recoding. *Nat. Methods.,* 2018, vol. 15, 221-225
- **J. C. BUCKINGHAM.** Br. J. Pharmacol. *Glucocorticoids: exemplars of multi-tasking.,* 2006, vol. 147, S258
- **M. D. CLEARY ; C. D. MEIERING ; E. JAN ; R. GUYMON ; J. C. BOOTHROYD.** Biosynthetic labeling of RNA with uracil phosphoribosyltransferase allows cell-specific microarray analysis of mRNA synthesis and decay. *Nat. Biotechnol.,* 2005, vol. 23, 232-237
- **L. DOLKEN et al.** High-resolution gene expression profiling for simultaneous kinetic parameter analysis of RNA synthesis and decay. *RNA.,* 2008, vol. 14, 1959-1972

- **C. MILLER et al.** Dynamic transcriptome analysis measures rates of mRNA synthesis and decay in yeast. *Mol. Syst. Biol.,* 2014, vol. 7, 458-458
- **E. E. DUFFY et al.** Tracking Distinct RNA Populations Using Efficient and Reversible Covalent Chemistry. *Mol. Cell,* 2015, vol. 59, 858-866
- **B. SCHWALB et al.** TT-seq maps the human transient transcriptome. *Science,* 2016, vol. 352, 1225-1228
- **M. RABANI et al.** Metabolic labeling of RNA uncovers principles of RNA production and degradation dynamics in mammalian cells. *Nat. Biotechnol.,* 2011, vol. 29, 436-442
- **M. R. MILLER ; K. J. ROBINSON ; M. D. CLEARY ; C. Q. DOE.** TU-tagging: cell type-specific RNA isolation from intact complex tissues. *Nat. Methods.,* 2009, vol. 6, 439-441
- **D. A. CUSANOVICH et al.** Multiplex single cell profiling of chromatin accessibility by combinatorial cellular indexing. *Science,* 2015, vol. 348, 910-914
- **J. CAO et al.** Comprehensive single-cell transcriptional profiling of a multicellular organism. *Science,* 2017, vol. 357, 661-667
- **J. CAO et al.** Joint profiling of chromatin accessibility and gene expression in thousands of single cells. *Science,* 2018, vol. 361, 1380-1385
- **V. RAMANI et al.** *Massively multiplex single-cell Hi-C,* 2016
- **R. M. MULQUEEN et al.** Highly scalable generation of DNA methylation profiles in single cells. *Nat. Biotechnol.,* 2018, vol. 36, 428-431
- **S. A. VITAK et al.** Sequencing thousands of single-cell genomes with combinatorial indexing. *Nat. Methods.,* 2017, vol. 14, 302-308
- **Y. YIN et al.** *High-throughput mapping of meiotic crossover and chromosome mis-segregation events in interspecific hybrid mice,* 2018
- **A. B. ROSENBERG et al.** Single-cell profiling of the developing mouse brain and spinal cord with split-pool barcoding. *Science,* 2018, vol. 360, 176-182
- **T. E. REDDY et al.** Genomic determination of the glucocorticoid response reveals unexpected mechanisms of gene regulation. *Genome Res.,* 2009, vol. 19, 2163-2171
- **S. JOHN et al.** Chromatin accessibility pre-determines glucocorticoid receptor binding patterns. *Nat. Genet.,* 2011, vol. 43, 264-268
- **T. E. REDDY ; J. GERTZ ; G. E. CRAWFORD ; M. J. GARABEDIAN ; R. M. MYERS.** The Hypersensitive Glucocorticoid Response Specifically Regulates Period 1 and Expression of Circadian Genes. *Mol. Cell. Biol.,* 2012, vol. 32, 3756-3767
- **C. M. VOCKLEY et al.** Direct GR Binding Sites Potentiate Clusters of TF Binding across the Human Genome. *Cell,* 2016, vol. 166, 1269-1281.e19
- **L. MCINNES ; J. HEALY ; N. SAUL ; L. GROßBERGER.** UMAP: Uniform Manifold Approximation and Projection. *Journal of Open Source Software.,* 2018, vol. 3, 861
- **A. BUTLER ; P. HOFFMAN ; P. SMIBERT ; E. PAPALEXI ; R. SATIJA.** Integrating single-cell transcriptomic data across different conditions, technologies, and species. *Nat. Biotechnol.,* 2018, vol. 36, 411-420
- The ENCODE Project Consortium, The ENCODE (ENCyclopedia Of DNA Elements) Project. *Science,* 2004, vol. 306, 636-640
- **S. AIBAR et al.** SCENIC: single-cell regulatory network inference and clustering. *Nat. Methods.,* 2017, vol. 14, 1083-1086
- **M. BORUK ; J. G. A. SAVORY ; R. J. G. HACHÉ.** AF-2-Dependent Potentiation of CCAAT Enhancer Binding Proteinβ -Mediated Transcriptional Activation by Glucocorticoid Receptor. *Mol. Endocrinol.,* 1998, vol. 12, 1749-1763
- **W. QIN et al.** Identification of functional glucocorticoid response elements in the mouse FoxO1 promoter. *Biochem. Biophys. Res. Commun.,* 2014, vol. 450, 979-983
- **C. S. SHEELA RANI ; N. ELANGO ; S.-S. WANG ; K. KOBAYASHI ; R. STRONG.** Identification of an Activator Protein-1-Like Sequence as the Glucocorticoid Response Element in the Rat Tyrosine Hydroxylase Gene. *Mol. Pharmacol.,* 2009, vol. 75, 589
- **M. FISCHER ; G. A. MÜLLER.** Cell cycle transcription control: DREAM/MuvB and RB-E2F complexes. *Crit. Rev. Biochem. Mol. Biol.,* 2017, vol. 52, 638-662
- **J. CHOU ; S. PROVOT ; Z. WERB.** GATA3 in development and cancer differentiation: cells GATA have it!. *J. Cell. Physiol.,* 2010, vol. 222, 42-49
- **J. Y. C. MADHURIMA BISWAS.** Role of Nrf1 in antioxidant response element-mediated gene expression and beyond. *Toxicol. Appl. Pharmacol.,* 2010, vol. 244, 16
- **I.-G. RYOO ; M.-K. KWAK.** Regulatory crosstalk between the oxidative stress-related transcription factor Nfe212/Nrf2 and mitochondria. *Toxicol. Appl. Pharmacol.,* 2018, vol. 359, 24-33
- **J. M. HARMON ; M. R. NORMAN ; B. J. FOWLKES ; E. B. THOMPSON.** Dexamethasone induces irreversible G1 arrest and death of a human lymphoid cell line. *J. Cell. Physiol.,* 1979, vol. 98, 267-278
- **A. K. GREENBERG et al.** Glucocorticoids inhibit lung cancer cell growth through both the extracellular signal-related kinase pathway and cell cycle regulators. *Am. J. Respir. Cell Mol. Biol.,* 2002, vol. 27, 320-328
- **J. CAO et al.** Comprehensive single-cell transcriptional profiling of a multicellular organism. *Science,* 2017, vol. 357, 661-667

- **W. MATSUSHIMA et al.** SLAM-ITseq: sequencing cell type-specific transcriptomes without cell sorting. *Development,* 2018, vol. 145
- **U. SHARMA et al.** *Small RNAs are trafficked from the epididymis to developing mammalian sperm,* 2017
- **A. MCKENNA et al.** Whole-organism lineage tracing by combinatorial and cumulative genome editing. *Science,* 2016, vol. 353, aaf7907
- **B. RAJ et al.** Simultaneous single-cell profiling of lineages and cell types in the vertebrate brain. *Nat. Biotechnol.,* 2018, vol. 36, 442-450
- **K. L. FRIEDA et al.** Synthetic recording and in situ readout of lineage information in single cells. *Nature,* 2017, vol. 541, 107-111
- **H. WICKHAM.** Elegant Graphics for Data Analysis. Springer, 2016
- **M. MUHAR et al.** SLAM-seq defines direct gene-regulatory functions of the BRD4-MYC axis. *Science,* 2018, vol. 360, 800-805
- **A. DOBIN et al.** STAR: ultrafast universal RNA-seq aligner. *Bioinformatics.,* 2013, vol. 29, 15-21
- **P. LINDENBAUM.** *JVarkit: java-based utilities for Bioinformatics,* 2015
- **FELIXKRUEGER.** FelixKrueger/TrimGalore. *GitHub, https://github.com/FelixKrueger/TrimGalore*
- **H. LI et al.** The Sequence Alignment/Map format and SAMtools. *Bioinformatics.,* 2009, vol. 25, 2078-2079
- Picard Tools. Broad Institute
- **D. C. KOBOLDT et al.** VarScan 2: somatic mutation and copy number alteration discovery in cancer by exome sequencing. *Genome Res.,* 2012, vol. 22, 568-576
- **S. L. WOLOCK ; R. LOPEZ ; A. M. KLEIN.** *Scrublet: computational identification of cell doublets in single-cell transcriptomic data,* 2018
- **X. QIU et al.** Reversed graph embedding resolves complex single-cell trajectories. *Nat. Methods.,* 2017, vol. 14, 979-982
- **J. FRIEDMAN ; T. HASTIE ; R. TIBSHIRANI.** Regularization Paths for Generalized Linear Models via Coordinate Descent. *J. Stat. Softw.,* 2010, vol. 33
- **A. RODRIGUEZ ; A. LAIO.** Clustering by fast search and find of density peaks. *Science,* 2014, vol. 344, 1492-1496
- **ARMOUR ; CHRISTOPHER D. ; JOHN C. CASTLE ; RONGHUA CHEN ; TOMAS BABAK ; PATRICK LOERCH ; STUART JACKSON ; JYOTI K. SHAH et al.** Digital Transcriptome Profiling Using Selective Hexamer Priming for cDNA Synthesis. *Nature Methods,* 2009, vol. 6 (9), 647-49
- **CAO, JUNYUE ; MALTE SPIELMANN ; XIAOJIE QIU ; XINGFAN HUANG ; DANIEL M. IBRAHIM ; ANDREW J. HILL ; FAN ZHANG et al.** The Single-Cell Transcriptional Landscape of Mammalian Organogenesis. *Nature,* 2019, vol. 566 (7745), 496-502
- **CORE, LEIGHTON J. ; ANDRÉ L. MARTINS ; CHARLES G. DANKO ; COLIN T. WATERS ; ADAM SIEPEL ; JOHN T. LIS.** Analysis of Nascent RNA Identifies a Unified Architecture of Initiation Regions at Mammalian Promoters and Enhancers. *Nature Genetics,* 2014, vol. 46 (12), 1311-20
- **GASPERINI ; MOLLY ; ANDREW J. HILL ; JOSÉ L. MCFALINE-FIGUEROA ; BETH MARTIN ; SEUNGSOO KIM ; MELISSA D. ZHANG ; DANA JACKSON et al.** A Genome-Wide Framework for Mapping Gene Regulation via Cellular Genetic Screens. *Cell,* 2019, vol. 176 (6), 1516
- **GASPERINI ; MOLLY ; ANDREW HILL ; JOSÉ L. MCFALINE-FIGUEROA ; BETH MARTIN ; COLE TRAPNELL ; NADAV AHITUV ; JAY SHENDURE.** crisprQTL Mapping as a Genome-Wide Association Framework for Cellular Genetic Screens. *bioRxiv,* 2018, https://doi.org/10.1101/314344
- **HIATT ; JOSEPH B. ; COLIN C. PRITCHARD ; STEPHEN J. SALIPANTE ; BRIAN J. O'ROAK ; JAY SHENDURE.** Single Molecule Molecular Inversion Probes for Targeted, High-Accuracy Detection of Low-Frequency Variation. *Genome Research,* 2013, vol. 23 (5), 843-54
- **MARINOV ; GEORGI K. ; BRIAN A. WILLIAMS ; KEN MCCUE ; GARY P. SCHROTH ; JASON GERTZ ; RICHARD M. MYERS ; BARBARA J. WOLD.** From Single-Cell to Cell-Pool Transcriptomes: Stochasticity in Gene Expression and RNA Splicing. *Genome Research,* 2014, vol. 24 (3), 496-510
- **SAIKIA ; MRIDUSMITA ; PHILIP BURNHAM ; SARA H. KESHAVJEE ; MICHAEL F. Z. WANG ; PABLO MORAL-LOPEZ ; MELEANA M. HINCHMAN ; CHARLES G. DANKO ; JOHN S. L. PARKER ; IWIJN DE VLAMINCK.** Simultaneous Multiplexed Amplicon Sequencing and Transcriptome Profiling in Single Cells. *bioRxiv,* 2018, https://doi.org/10.1101/328328
- **XIE, SHIQI ; JIALEI DUAN ; BOXUN LI ; PEI ZHOU ; GARY C. HON.** Multiplexed Engineering and Analysis of Combinatorial Enhancer Activity in Single Cells. *Molecular Cell,* 2017, vol. 66 (2), 285-99
- **XU, HANSEN ; BENJAMIN J. FAIR ; ZACH DWYER ; MICHAEL GILDEA ; JEFFREY A. PLEISS.** Multiplexed Primer Extension Sequencing Enables High Precision Detection of Rare Splice Isoforms. *bioRxiv,* 2018, https://doi.org/10.1101/331629.

• **ZIEGENHAIN ; CHRISTOPH ; BEATE VIETH ; SWATI PAREKH ; BJÖRN REINIUS ; AMY GUILLAUMET-ADKINS ; MARTHA SMETS ; HEIN-RICH LEONHARDT ; HOLGER HEYN ; INES HELLMANN.** Comparative Analysis of Single-Cell RNA Sequencing Methods. *Molecular Cell,* 2017, vol. 65 (4), 631-43.e4